# EUROPEAN PATENT SPECIFICATION

(11) **EP 3 083 597 B1**
(45) Date of publication and mention of the grant of the patent: **22.11.2017**
(21) Application number: 14821075.0
(22) Date of filing: 18.12.2014
(51) Int. Cl.: C07D 401/14, C07D 491/08, A61K 31/40, A61K 31/4015, C07D 405/12, C07D 417/12, C07D 207/337

(54) **FLUOROMETHYL-SUBSTITUTED PYRROLE CARBOXAMIDES AS CAV2.2 CALCIUM CHANNEL BLOCKERS**
FLUORMETHYLSUBSTITUTIERTE PYRROLCARBONSÄUREAMIDE ALS CAV2.2-CALCIUMKANAL-BLOCKIERUNGSMITTEL
CARBOXAMIDES DE PYRROLE À SUBSTITUTION FLUOROMÉTHYLE COMME AGENTS DE BLOCAGE DE CANAUX CALCIQUES CAV2.2

(30) Priority: 19.12.2013 EP 13005937
(43) Date of publication of application: 26.10.2016
(73) Proprietor: Grünenthal GmbH, 52078 Aachen (DE)
(72) Inventor: REICH, Melanie, 52072 Aachen (DE); SCHUNK, Stefan, 52080 Aachen (DE); JAKOB, Florian, 52066 Aachen (DE); DAMANN, Nils, 50354 Hürth (DE); HAURAND, Michael, 52078 Aachen (DE); HAMLYN, Richard, Sutton Cambridgeshire CB6 2NW (GB); ROGERS, Marc, Willingham Cambridgeshire CB24 5JT (GB); MACKENZIE, Kathy, Much Hadham Hertfordshire SG10 6DP (GB); SKONE, Philip, Knebworth Herts SG3 6AJ (GB)
(86) International application number: PCT/EP2014/003435
(87) International publication number: WO 2015/090603

(56) References cited:
- WO-A1-2007/141039
- WO-A1-2012/004604
- WO-A1-2014/032801
- US-A1- 2013 029 962
- TYAGARAJAN S ET AL: "A potent and selective indole N-type calcium channel (Cav2.2) blocker for the treatment of pain", BIOORGANIC & MEDICINAL CHEMISTRY LETTERS, PERGAMON, AMSTERDAM, NL, vol. 21, no. 2, 15 January 2011 (2011-01-15), pages 869-873, XP027593577, ISSN: 0960-894X, DOI: 10.1016/J.BMCL.2010.11.067 [retrieved on 2011-01-07]

## Description

### FIELD OF THE INVENTION

The invention relates to substituted pyrrole-2-yl-carboxamides bearing a fluorinated methyl moiety in 5-position as voltage gated Ca-channel (CaV) blockers, to pharmaceutical compositions containing these compounds and also to these compounds for use in the treatment and/or prophylaxis of pain and further diseases and/or disorders.

### BACKGROUND OF THE INVENTION

Ion channels are proteins that form pores in membranes of biological cells and control the flow of ions down their electrochemical gradient. They are involved in the regulation of a wide range of cellular functions in both excitable and nonexcitable cells and provide attractive therapeutic targets for the treatment of various diseases.

In the somatosensory context, CaV2.2 channels, specific cellular plasma membrane calcium channels that belong to a diverse superfamily of voltage-gated calcium channels (VGCCs), were demonstrated to play an important role in spinal nociceptive processing.

The critical role of CaV2.2 in pain processing was underlined by the clinical efficacy of the intrathecally delivered, selective CaV2.2 channel antagonist Ziconotide (SNX-111; Prialt™), a synthetic peptide derived from a w-(omega)-conotoxin peptide (Miljanich, 2004, Curr. Med. Chem., 11(23), p. 3029-40; Staats et al., 2004, JAMA, 291(1), p. 63-70). Inthrathecal administration of Ziconotide is required in order to reach the ion channel in presynaptic terminals of sensory neurons in the spinal cord. Common side effects of Ziconotide include memory impairment, dizziness, nystagmus, speech disorder, nervousness, somnolence and abnormal gait (Rauck et al., 2009, Pain Pract., 9, p. 327-37), which have been attributed to the inhibition of CaV2.2 channels in the brain by Ziconotide.

Therefore, a demand remains for the development of orally available CaV2.2 calcium channel blockers that show the desired qualities and effectively block CaV2.2 calcium channels in the nociceptive signaling pathway.

1,4-disubstituted pyrrol-2-yl carboxylic acid amides are known from WO2007/141039 A1.

### SUMMARY OF THE INVENTION

The present invention describes small molecule CaV2.2 channel blockers.

It was therefore an object of the invention to provide novel compounds, preferably having advantages over the prior-art compounds. The compounds should be suitable in particular as pharmacological active ingredients in pharmaceutical compositions, preferably in pharmaceutical compositions for the treatment and/or prophylaxis of disorders or diseases which are at least partially mediated by CaV2.2 calcium channels.

This object is achieved by the subject matter described herein.

It has surprisingly been found that the compounds of general formula (I), as given below, display outstanding affinity to CaV2.2 calcium channels and are therefore particularly suitable for the prophylaxis and/or treatment of disorders or diseases which are at least partially mediated by CaV2.2 calcium channels. A specific substitution in 5-position of the pyrrol (R⁴) render these compounds particularly suitable for the purpose of the invention.

The present invention therefore relates to a compound of general formula (I), 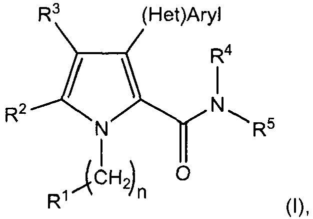 wherein
n represents 0, 1 or 2;
R¹ represents C₁₋₆-alkyl; C₂₋₆-alkenyl; C₂₋₆-alkynyl; C₃₋₆-cycloalkyl or 3 to 7 membered heterocyclyl;
R² represents CH₂F; CHF₂ or CF₃;
R³ represents H; C₁₋₆-alkyl; C₃₋₆-cycloalkyl or 3 to 7 membered heterocyclyl; OH; O-C₁₋₆-alkyl; NH₂; N(H)(C₁₋₆-alkyl); N(C₁₋₆-alkyl)₂ or SO₂(C₁₋₆-alkyl);
(Het)Aryl represents aryl or heteroaryl, each substituted by zero or one or two or three substituents of the group consisting of R⁶, R⁷ and R⁸,
wherein R⁶, R⁷ and R⁸, are each independently of one another selected from the group consisting of F; Cl; Br; I; NO₂; CN; C₁₋₆-alkyl; CF₃; CF₂H; CFH₂; CF₂Cl; CFCl₂; C(=O)-H; C(=O)-C₁₋₆-alkyl; C(=O)-OH; C(=O)-O-C₁₋₆-alkyl; C(=O)-N(H)(OH); C(=O)-NH₂; C(=O)-N(H)(C₁₋₆-alkyl); C(=O)-N(C₁₋₆-alkyl)₂; C(=N-OH)-H; C(=N-OH)-C₁₋₆-alkyl; C(=N-O-C₁₋₆-alkyl)-H; C(=N-O-C₁₋₆-alkyl)-C₁₋₆-alkyl; OH; OCF₃; OCF₂H; OCFH₂; OCF₂Cl; OCFCl₂; O-C₁₋₆-alkyl; O-C(=O)-C₁₋₆-alkyl; O-C(=O)-O-C₁₋₆-alkyl; O-(C=O)-N(H)(C₁₋₆-alkyl); O-C(=O)-N(C₁₋₆-alkyl)₂; O-S(=O)₂-C₁₋₆-alkyl; O-S(=O)₂-OH; O-S(=O)₂-O-C₁₋₆-alkyl; O-S(=O)₂-NH₂; O-S(=O)₂-N(H)(C₁₋₆-alkyl); O-S(=O)₂-N(C₁₋₆-alkyl)₂; NH₂; N(H)(C₁₋₆-alkyl); N(C₁₋₆-alkyl)₂; N(H)-C(=O)-C₁₋₆-alkyl; N(H)-C(=O)-O-C₁₋₆-alkyl; N(H)-C(=O)-NH₂; N(H)-C(=O)-N(H)(C₁₋₆-alkyl); N(H)-C(=O)-N(C₁₋₆-alkyl)₂; N(C₁₋₆-alkyl)-C(=O)-C₁₋₆-alkyl; N(C₁₋₆-alkyl)-C(=O)-O-C₁₋₆-alkyl; N(C₁₋₆-alkyl)-C(=O)-NH₂; N(C₁₋₆-alkyl)-C(=O)-N(H)(C₁₋₆-alkyl); N(C₁₋₆-alkyl)-C(=O)-N(C₁₋₆-alkyl)₂; N(H)-S(=O)₂OH; N(H)-S(=O)₂-C₁₋₆-alkyl; N(H)-S(=O)₂-O-C₁₋₆-alkyl; N(H)-S(=O)₂-NH₂; N(H)-S(=O)₂-N(H)(C₁₋₆-alkyl); N(H)-S(=O)₂N(C₁₋₆-alkyl)₂; N(C₁₋₆-alkyl)-S(=O)₂-OH; N(C₁₋₆-alkyl)-S(=O)₂-C₁₋₆-alkyl; N(C₁₋₆-alkyl)-S(=O)₂-O-C₁₋₆-alkyl; N(C₁₋₆-alkyl)-S(=O)₂-NH₂; N(C₁₋₆-alkyl)-S(=O)₂-N(H)(C₁₋₆-alkyl); N(C₁₋₆-alkyl)-S(=O)₂-N(C₁₋₆-alkyl)₂; SH; SCF₃; SCF₂H; SCFH₂; SCF₂Cl; SCFCl₂; S-C₁₋₆-alkyl; S(=O)-C₁₋₆-alkyl; S(=O)₂-C₁₋₆-alkyl; S(=O)₂-OH; S(=O)₂-O-C₁₋₆-alkyl; S(=O)₂-NH₂; S(=O)₂-N(H)(C₁₋₆-alkyl); S(=O)₂-N(C₁₋₆-alkyl)₂, C₃₋₆-cycloalkyl or 3 to 7 membered heterocyclyl;
R⁴ represents H, C₁₋₁₀-alkyl, aryl, or aryl connected via a C₁₋₈-alkylene group;
R⁵ represents H; C₁₋₁₀-alkyl, C₃₋₁₀-cycloalkyl, 3 to 10 membered heterocyclyl, aryl or heteroaryl; or C₃₋₁₀-cycloalkyl, 3 to 10 membered heterocyclyl, aryl or heteroaryl, each connected via a C₁₋₈-alkylene group; or
R⁴ and R⁵ together with the nitrogen atom connecting them form a 3 to 10 membered heterocyclyl;
wherein said C₁₋₆-alkyl, said C₁₋₁₀-alkyl, said C₂₋₆-alkenyl, said C₂₋₆-alkynyl and said C₁₋₈-alkylene in each case may be branched or unbranched and unsubstituted or mono- or poly-substituted;
and wherein said C₃₋₆-cycloalkyl, said C₃₋₁₀-cycloalkyl, said 3 to 7 membered heterocyclyl, said 3 to 10 membered heterocyclyl, said aryl and said heteroaryl in each case may be unsubstituted or mono- or polysubstituted;
optionally in the form of an individual stereoisomer or a mixture of stereoisomers,
in the form of the free compound and/or a physiologically acceptable salt and/or a physiologically acceptable solvate thereof.

### DETAILED DESCRIPTION

The term "single stereoisomer" preferably means in the sense of the present invention an individual enantiomer or diastereomer. The term "mixture of stereoisomers" means in the sense of this invention the racemate and mixtures of enantiomers and/or diastereomers in any mixing ratio.

The term "physiologically acceptable salt" preferably comprises in the sense of this invention a salt of at least one compound according to the present invention and at least one physiologically acceptable acid or base.

A physiologically acceptable salt of at least one compound according to the present invention and at least one physiologically acceptable acid preferably refers in the sense of this invention to a salt of at least one compound according to the present invention with at least one inorganic or organic acid which is physiologically acceptable - in particular when used in human beings and/or other mammals.

A physiologically acceptable salt of at least one compound according to the present invention and at least one physiologically acceptable base preferably refers in the sense of this invention to a salt of at least one compound according to the present invention as an anion with at least one preferably inorganic cation, which is physiologically acceptable - in particular when used in human beings and/or other mammals.

The term "physiologically acceptable solvate" preferably comprises in the sense of this invention an adduct of one compound according to the present invention and/or a physiologically acceptable salt of at least one compound according to the present invention with distinct molecular equivalents of one solvent or more solvents. Examples of physiologically acceptable solvents are water, alkanols, esters, ethers or ketones.

The terms "C₁₋₆-alkyl" and "C₁₋₁₀-alkyl" preferably comprise in the sense of this invention acyclic saturated aliphatic hydrocarbon residues, which can be respectively branched or unbranched and can be unsubstituted or can be mono- or polysubstituted, e.g. mono-, di- or trisubstituted, and which contain 1 to 6 carbon atoms, i.e. 1, 2, 3, 4, 5 or 6 carbon atoms, or 1 to 10, i.e. 1, 2, 3, 4, 5, 6, 7, 8, 9 or 10 carbon atoms, respectively, i.e. C₁₋₆ alkyl and C₁₋₁₀ alkyl. Preferred C₁₋₆-alkyl groups are selected from the group consisting of methyl, ethyl, n-propyl, 2-propyl, n-butyl, isobutyl, sec.-butyl, tert.-butyl, n-pentyl, isopentyl, neopentyl, and n-hexyl. Preferred C₁₋₁₀-alkyl residues are selected from the group consisting of methyl, ethyl, n-propyl, 2-propyl, n-butyl, isobutyl, sec.-butyl, tert.-butyl, n-pentyl, isopentyl, neopentyl, n-hexyl, n-heptyl, n-octyl, n-nonyl, n-decanyl and isooctyl.

In relation to the terms "C₁₋₆-alkyl" and "C₁₋₁₀-alkyl", the term "monosubstituted" or "polysubstituted" such as di- or tri-substituted refers in the sense of this invention, with respect to the corresponding groups, to the single substitution or multiple substitution, e.g. disubstitution or trisubstitution, of one or more hydrogen atoms each independently of one another by at least one substituent. The term "poly-substituted" such as di- or tri-substituted with respect to polysubstituted groups such as di- or tri-substituted groups includes the polysubstitution of these groups either on different or on the same atoms, for example trisubstituted on the same carbon atom, as in the case of CF₃ or CH₂CF₃ or at various points, as in the case of CH(OH)-CH₂CH₂-CHCl₂. The multiple substitution can be carried out using the same or using different substituents.

The term "C₃₋₆-cycloalkyl" and "C₃₋₁₀-cycloalkyl" mean for the purposes of this invention cyclic aliphatic hydrocarbons containing 3, 4, 5 or 6 carbon atoms and 3, 4, 5, 6, 7, 8, 9 or 10 carbon atoms, respectively, wherein the hydrocarbons in each case can be saturated or unsaturated (but not aromatic), unsubstituted or mono- or polysubstituted. The cycloalkyl group can be bound to the respective superordinate general structure via any desired and possible ring member of the cycloalkyl group. The cycloalkyl group can also be condensed with further saturated, (partially) unsaturated, (hetero)cyclic, aromatic or heteroaromatic ring systems, i.e. with cycloalkyl, heterocyclyl, aryl or heteroaryl residues, which in each case can in turn be unsubstituted or mono- or polysubstituted. C₃₋₁₀-cycloalkyls can furthermore be singly or multiply bridged such as, for example, in the case of adamantyl, bicycle-[2.2.1]heptyl or bicyclo[2.2.2]octyl. Preferred C₃₋₁₀-cycloalkyl groups are selected from the group consisting of cyclopropyl, cyclobutyl, cyclopentyl, cyclohexyl, cycloheptyl, cyclooctyl, cyclononyl, cyclodecyl, adamantly, cyclopentenyl, cyclohexenyl, cycloheptenyl, cyclooctenyl, 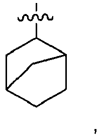 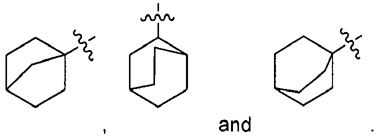 Preferred C₃₋₆-cycloalkyl groups are selected from the group consisting of cyclopropyl, cyclobutyl, cyclopentyl, cyclohexyl, cyclopentenyl and cyclohexenyl. Particularly preferred C₃₋₁₀-cycloalkyl groups and C₃₋₆-cycloalkyl groups are C₃₋₆-cycloalkyl groups such as cyclopropyl, cyclobutyl, cyclopentyl, cyclohexyl, cyclopentenyl and cyclohexenyl, in particular cyclopropyl.

The terms "3 to 7-membered heterocyclyl" and "3 to 10-membered heterocyclyl" mean for the purposes of this invention heterocycloaliphatic saturated or unsaturated (but not aromatic) residues having 3 to 7, i.e. 3, 4, 5, 6 or 7 ring members, and 3 to 10, i.e. 3, 4, 5, 6, 7, 8, 9 or 10 ring members, respectively, in which in each case at least one, if appropriate also two or three carbon atoms are replaced by a heteroatom or a heteroatom group each selected independently of one another from the group consisting of O, S, S(=O), S(=O)₂, N, NH and N(C₁₋₆-alkyl) such as N(CH₃), wherein the ring members can be unsubstituted or mono- or polysubstituted. The cycloalkyl groups can also be condensed with further saturated or (partially) unsaturated cycloalkyl or heterocyclyl, aromatic or heteroaromatic ring systems, which in each case can in turn be unsubstituted or mono- or polysubstituted. The heterocyclyl group can be bound to the superordinate general structure via any desired and possible ring member of the heterocycloaliphatic residue if not indicated otherwise.

The term "aryl" means for the purpose of this invention aromatic hydrocarbons having 6 to 14, i.e. 6, 7, 8, 9, 10, 11, 12, 13 or 14 ring members, preferably having 6 to 10, i.e. 6, 7, 8, 9 or 10 ring members, including phenyls and naphthyls. Each aryl residue can be unsubstituted or mono- or polysubstituted, wherein the aryl substituents can be the same or different and in any desired and possible position of the aryl. The aryl can be bound to the superordinate general structure via any desired and possible ring member of the aryl residue. The aryl residues can also be condensed with further saturated or (partially) unsaturated cycloalkyl or heterocyclyl, aromatic or heteroaromatic ring systems, which can in turn be unsubstituted or mono- or polysubstituted. Examples of condensed aryl residues are benzo-dioxolanyl and benzodioxanyl. Preferably, aryl is selected from the group consisting of phenyl, 1-naphthyl, 2-naphthyl, fluorenyl and anthracenyl, each of which can be respectively unsubstituted or mono- or polysubstituted. A particularly preferred aryl is phenyl, unsubstituted or mono- or poly-substituted.

The term "heteroaryl" for the purpose of this invention represents a 5-, 6-, 8-, 9- or 10-membered cyclic aromatic residue containing at least 1, if appropriate also 2, 3, 4 or 5 heteroatoms, wherein the heteroatoms are each selected independently of one another from the group S, N and O and the heteroaryl residue can be unsubstituted or mono- or polysubstituted; in the case of substitution on the heteroaryl, the substituents can be the same or different and be in any desired and possible position of the heteroaryl. The binding to the superordinate general structure can be carried out via any desired and possible ring member of the heteroaryl residue if not indicated otherwise. The heteroaryl can also be part of a bi- or polycyclic system having up to 10 ring members, wherein the ring system can be formed with further saturated or (partially) unsaturated cycloalkyl or heterocyclyl, aromatic or heteroaromatic ring systems, which can in turn be unsubstituted or mono- or polysubstituted. It is preferable for the heteroaryl residue to be selected from the group consisting of benzofuranyl, benzoimidazolyl, benzothienyl, benzothiadiazolyl, benzothiazolyl, benzotriazolyl, benzooxazolyl, benzooxadiazolyl, quinazolinyl, quinoxalinyl, carbazolyl, quinolinyl, dibenzofuranyl, dibenzothienyl, furyl (furanyl), imidazolyl, imidazothiazolyl, indazolyl, indolizinyl, indolyl, isoquinolinyl, isoxazoyl, isothiazolyl, indolyl, naphthyridinyl, oxazolyl, oxadiazolyl, phenazinyl, phenothiazinyl, phthalazinyl, pyrazolyl, pyridyl (2-pyridyl, 3-pyridyl, 4-pyridyl), pyrrolyl, pyridazinyl, pyrimidinyl, pyrazinyl, purinyl, phenazinyl, thienyl (thiophenyl), triazolyl, tetrazolyl, thiazolyl, thiadiazolyl and triazinyl.

The terms "C₁₋₈-alkylene" and "C₂₋₆-alkylene" means in the sense of this invention a bivalent acyclic saturated, aliphatic hydrocarbon residue, which can be branched or unbranched and also unsubstituted or mono- or polysubstituted, which contain 1 to 8 carbon atoms or 2 to 6 carbon atoms respectively. Preferred C₁₋₈-alkylene groups are selected from the group consisting of CH₂, CH₂CH₂, CH(CH₃), CH₂CH₂CH₂, CH(CH₃)CH₂, CH(CH₂CH₃), CH₂(CH₂)₂CH₂, CH(CH₃)CH₂CH₂, CH₂CH(CH₃)CH₂, CH(CH₃)CH(CH₃), CH(CH₂CH₃)CH₂, C(CH₃)₂CH₂, CH(CH₂CH₂CH₃) and C(CH₃)(CH₂CH₃). Preferred C₂₋₆-alkylene groups are selected from the group consisting of CH₂CH₂, CH(CH₃), CH₂CH₂CH₂, CH(CH₃)CH₂, CH(CH₂CH₃), CH₂(CH₂)₂CH₂, CH(CH₃)CH₂CH₂, CH₂CH(CH₃)CH₂, CH(CH₃)CH(CH₃), CH(CH₂CH₃)CH₂, C(CH₃)₂CH₂, CH(CH₂CH₂CH₃) and C(CH₃)(CH₂CH₃).

In relation to the terms "C₁₋₆-alkyl", "C₁₋₁₀-alkyl", "C₁₋₆-alkylene", "C₃₋₆-cycloalkyl", "C₃₋₁₀-cycloalkyl", "3 to 7-membered heterocyclyl" and "3 to 10-membered heterocyclyl", the term "mono- or polysubstituted" refers in the sense of this invention, with respect to the corresponding residues or groups, to the single substitution or multiple substitution, e.g. disubstitution, trisubstitution, tetrasubstitution, or pentasubstitution, of one or more hydrogen atoms each independently of one another by at least one substituent selected from the group consisting of F; Cl; Br; I; NO₂; CN; =O; =NH; =N(OH); =N(O-C₁₋₆-alkyl); CF₃; CF₂H; CFH₂; CF₂Cl; CFCl₂; C₁₋₆-alkyl; (C₁₋₈-alkylene)-OH; C(=O)-H; C(=O)-C₁₋₆-alkyl; C(=O)-OH; C(=O)-O-C₁₋₆-alkyl; C(=O)-N(H)(OH); C(=O)-NH₂; C(=O)-N(H)(C₁₋₆-alkyl); C(=O)-N(C₁₋₆-alkyl)₂; C(=N-OH)-H; C(=N-OH)-C₁₋₆-alkyl; C(=N-O-C₁₋₆-alkyl)-H; C(=N-O-C₁₋₆-alkyl)-C₁₋₆-alkyl; OH; OCF₃; OCF₂H; OCFH₂; OCF₂Cl; OCFCl₂; O-C₁₋₆-alkyl; O-(C₁₋₈-alkylene)-OH; O-(C₁₋₈-alkylene)-O-C₁₋₆-alkyl; O-C(=O)-C₁₋₆-alkyl; O-C(=O)-O-C₁₋₆-alkyl; O-(C=O)-N(H)(C₁₋₆-alkyl); O-C(=O)-N(C₁₋₆-alkyl)₂; O-S(=O)₂-C₁₋₆-alkyl; O-S(=O)₂-OH; O-S(=O)₂-O-C₁₋₆-alkyl; O-S(=O)₂-NH₂; O-S(=O)₂-N(H)(C₁₋₆-alkyl); O-S(=O)₂-N(C₁₋₆-alkyl)₂; NH₂; N(H)(C₁₋₆-alkyl); N(C₁₋₆-alkyl)₂; N(H)-C(=O)-C₁₋₆-alkyl; N(H)-C(=O)-O-C₁-₆-alkyl; N(H)-C(=O)-NH₂; N(H)-C(=O)-N(H)(C₁₋₆-alkyl); N(H)-C(=O)-N(C₁₋₆-alkyl)₂; N(C₁₋₆-alkyl)-C(=O)-C₁₋₆-alkyl; N(C₁₋₆-alkyl)-C(=O)-O-C₁₋₆-alkyl; N(C₁₋₆-alkyl)-C(=O)-NH₂; N(C₁₋₆-alkyl)-C(=O)-N(H)(C₁₋₆-alkyl); N(C₁₋₆-alkyl)-C(=O)-N(C₁₋₆-alkyl)₂; N(H)-S(=O)₂-OH; N(H)-S(=O)₂-C₁₋₆-alkyl; N(H)-S(=O)₂-O-C₁₋₆-alkyl; N(H)-S(=O)₂-NH₂; N(H)-S(=O)₂-N(H)(C₁₋₆-alkyl); N(H)-S(=O)₂-N(C₁₋₆-alkyl)₂; N(C₁₋₆-alkyl)-S(=O)₂-OH; N(C₁₋₆-alkyl)-S(=O)₂-C₁₋₆-alkyl; N(C₁₋₆-alkyl)-S(=O)2-O-C₁₋₆-alkyl; N(C₁₋₆-alkyl)-S(=O)₂-NH₂; N(C₁₋₆-alkyl)-S(=O)₂-N(H)(C₁₋₆-alkyl); N(C₁₋₆-alkyl)-S(=O)₂-N(C₁₋₆-alkyl)₂; SH; SCF₃; SCF₂H; SCFH₂; SCF₂Cl; SCFCl₂; S-C₁₋₆-alkyl; S(=O)-C₁₋₆-alkyl; S(=O)₂-C₁₋₆-alkyl; S(=O)₂-OH; S(=O)₂-O-C₁₋₆-alkyl; S(=O)₂-NH₂; S(=O)₂-N(H)(C₁₋₆-alkyl); S(=O)₂-N(C₁₋₆-alkyl)₂; C₃₋₆-cycloalkyl or 3 to 7 membered heterocyclyl. The term "polysubstituted" with respect to polysubstituted residues and groups includes the polysubstitution of these residues and groups either on different or on the same atoms, for example trisubstituted on the same carbon atom, as in the case of CF₃, CH₂CF₃ or 1,1-difluorocyclohexyl, or at various points, as in the case of CH(OH)-CHCl₂ or 1-chloro-3-fluorocyclohexyl. A substituent can if appropriate for its part in turn be mono- or polysubstituted. The multiple substitution can be carried out using the same or using different substituents.

Preferred substituents of "C₁₋₆-alkyl", "C₁₋₁₀-alkyl", "C₁₋₈-alkylene" and "C₂₋₆-alkylene" are selected from the group consisting of F; Cl; Br; I; NO₂; CF₃; CN; =O; =NH; C₁₋₆-alkyl; (C₁₋₈-alkylene)-OH; C(=O)-H; C(=O)-C₁₋₆-alkyl; C(=O)-OH; C(=O)-O-C₁₋₆-alkyl; C(=O)-NH₂; C(=O)-N(H)(C₁₋₆-alkyl); C(=O)-N(C₁₋₆-alkyl)₂; OH; O-C₁₋₆-alkyl; O-C(=O)-C₁₋₆-alkyl; O-(C₁₋₈-alkylene)-OH; O-(C₁₋₈-alkylene)-O-C₁₋₆-alkyl; OCF₃; NH₂; N(H)(C₁₋₆-alkyl); N(C₁₋₆-alkyl)₂; N(H)-C(=O)-C₁₋₆-alkyl; N(H)-S(=O)₂-C₁₋₆-alkyl; N(C₁₋₆-alkyl)-S(=O)₂-C₁₋₆-alkyl; N(H)-C(=O)-NH₂; N(H)-C(=O)-N(H)(C₁₋₆-alkyl); N(H)-C(=O)-N(C₁₋₆-alkyl)₂; N(H)-S(=O)₂-NH₂; N(H)-S(=O)₂-N(H)(C₁₋₆-alkyl); N(H)-S(=O)₂-N(C₁₋₆-alkyl)₂; N(C₁₋₆-alkyl)-S(=O)₂-NH₂; N(C₁₋₆-alkyl)-S(=O)₂-N(H)(C₁₋₆-alkyl); N(C₁₋₆-alkyl)-S(=O)₂-N(C₁₋₆-alkyl)₂; SH; SCF₃; S-C₁₋₆-alkyl; S(=O)₂ C₁₋₆-alkyl; S(=O)₂OH; S(=O)₂O-C₁₋₆-alkyl and S(=O)₂-NH₂; S(=O)₂-N(H)(C₁₋₆-alkyl); and S(=O)₂-N(C₁₋₆-alkyl)₂.

Particularly preferred substituents of "C₁₋₆-alkyl". "C₁₋₁₀-alkyl", "C₁₋₈-alkylene" and "C₂₋₆-alkylene" are selected from the group consisting of F; Cl; Br; I; CF₃; C(=O)-NH₂; C(=O)-N(H)(C₁₋₆-alkyl); C(=O)-N(C₁₋₆-alkyl)₂; OH; O-C₁₋₆-alkyl; O-(C₁₋₈-alkylene)-OH; O-(C₁₋₈-alkylene)-O-C₁₋₆-alkyl; NH₂; N(H)(C₁₋₆-alkyl); N(C₁₋₆-alkyl)₂; N(H)-C(=O)-C₁₋₆-alkyl; N(H)-S(=O)₂-C₁₋₆-alkyl; N(C₁₋₆-alkyl)-S(=O)₂-C₁₋₆-alkyl; N(H)-S(=O)₂-NH₂; SH; S-C₁₋₆-alkyl; S(=O)₂ C₁₋₆-alkyl and S(=O)₂-N(H)(C₁₋₆-alkyl).

Preferred substituents of "C₃₋₆-cycloalkyl", "C₃₋₁₀-cycloalkyl", "3 to 7-membered heterocyclyl" and "3 to 10-membered heterocyclyl" are selected from the group consisting of F; Cl; Br; I; NO₂; CF₃; CN; =O; C₁₋₆-alkyl; C₃₋₆-cycloalkyl or 3 to 7 membered heterocyclyl; C₃₋₆-cycloalkyl or 3 to 7 membered heterocyclyl, each bridged via a C₁₋₈-alkylene; CHO; C(=O)-C₁₋₆-alkyl; CO₂H; C(=O)O-C₁₋₆-alkyl; CONH₂; C(=O)NH-C₁₋₆-alkyl; C(=O)N(C₁₋₆-alkyl)₂; OH; O-C₁₋₆-alkyl; OCF₃; O-(C₁₋₈-alkylene)-OH; O-(C₁₋₈-alkylene)-O-C₁₋₆-alkyl; O-C(=O)-C₁₋₆-alkyl; NH₂; NH-C₁₋₆-alkyl; N(C₁₋₆-alkyl)₂; NH-C(=O)-C₁₋₆-alkyl; SH; S-C₁₋₆-alkyl; SCF₃; S(=O)₂-C₁₋₆-alkyl; S(=O)₂OH; S(=O)₂O-C₁₋₆-alkyl and S(=O)₂-NH-C₁₋₆-alkyl.

In relation to the terms "aryl" and "heteroaryl", the term "mono- or polysubstituted" refers in the sense of this invention, with respect to the corresponding residues or groups, to the single substitution or multiple substitution, e.g. disubstitution, trisubstitution, tetrasubstitution, or pentasubstitution, of one or more hydrogen atoms each independently of one another by at least one substituent selected from the group consisting of F; Cl; Br; NO₂; CN; CF₃; CF₂H; CFH₂; CF₂Cl; CFCl₂; C₁₋₆-alkyl; C₃₋₆-cycloalkyl; 3 to 7 membered heterocyclyl; aryl; heteroaryl; aryl, heteroaryl, C₃₋₆-cycloalkyl or 3 to 7 membered heterocyclyl, each connected via a C₁₋₈-alkylene; C(=O)H; C(=O)-(C₁₋₆-alkyl); C(=O)-(C₃₋₆-cycloalkyl); C(=O)-(3 to 7 membered heterocyclyl); C(=O)-(aryl); C(=O)-(heteroaryl); C(=O)OH; C(=O)-O(C₁₋₆-alkyl); C(=O)-O(C₃₋₆-cycloalkyl); C(=O)-O(3 to 7 membered heterocyclyl); C(=O)-O(aryl); C(=O)-O(heteroaryl); C(=O)-NH₂; C(=O)-N(H)(C₁₋₆-alkyl); C(=O)-N(H)(C₃₋₆-cycloalkyl); C(=O)-N(H)(3 to 7 membered heterocycloalkyl); C(=O)-N(H)(aryl); C(=O)-N(H)(heteroaryl); C(=O)-N(C₁₋₆-alkyl)₂; C(=O)-N(C₁₋₆-alkyl)(C₃₋₆-cycloalkyl); C(=O)-N(C₁₋₆-alkyl)(3 to 7 membered heterocyclyl); C(=O)-N(C₁₋₆-alkyl)(aryl); C(=O)-N(C₁₋₆-alkyl)(heteroaryl); OH; =O; O-(C₁₋₆-alkyl); O-(C₃₋₆-cycloalkyl); O-(3 to 7 membered heterocyclyl); O-(aryl); O-(heteroaryl); OCF₃; OCF₂H; OCFH₂; OCF₂Cl; OCFCl₂; O-C(=O)-(C₁₋₆-alkyl); O-C(=O)-(C₃₋₆-cycloalkyl); O-C(=O)-(3 to 7 membered heterocyclyl); O-C(=O)-(aryl); C(=O)-(heteroaryl); O-C(=O)-NH₂; O-C(=O)-N(H)(C₁₋₆-alkyl); O-C(=O)-N(H)(C₃₋₆-cycloalkyl); O-C(=O)-N(H)(3 to 7 membered heterocyclyl); O-C(=O)-N(H)(aryl); O-C(=O)-N(H)(heteroaryl); O-C(=O)-N(C₁₋₆-alkyl)₂; O-C(=O)-N(C₁₋₆-alkyl)(C₃₋₆-cycloalkyl); O-C(=O)-N(C₁₋₆-alkyl)(3 to 7 membered heterocyclyl); O-C(=O)-N(C₁₋₆-alkyl)(aryl); O-C(=O)-N(C₁₋₆-alkyl)(heteroaryl); NH₂; N(H)(C₁₋₆-alkyl); N(H)(C₃₋₆-cycloalkyl); N(H)(3 to 7 membered heterocyclyl); N(H)(aryl); N(H)(heteroaryl); N(C₁₋₆-alkyl)₂; N(C₁₋₆-alkyl)(C₃₋₆-cycloalkyl); N(C₁₋₆-alkyl)(3 to 7 membered heterocyclyl); N(C₁₋₆-alkyl) (aryl); N(C₁₋₆-alkyl)(heteroaryl); N(H)-C(=O)-(C₁₋₆-alkyl); N(H)-C(=O)-(C₃₋₆-cycloalkyl); N(H)-C(=O)-(3 to 7 membered heterocyclyl); N(H)-C(=O)-(aryl); N(H)-C(=O)-(heteroaryl); N(C₁₋₆-alkyl)-C(=O)-(C₁₋₆-alkyl); N(C₁₋₆-alkyl)-C(=O)-(C₃₋₆-cycloalkyl); N(C₁₋₆-alkyl)-C(=O)-(3 to 7 membered heterocyclyl); N(C₁₋₆-alkyl)-C(=O)-(aryl); N(C₁₋₆-alkyl)-C(=O)-(heteroaryl); N(H)-S(=O)₂-(C₁₋₆-alkyl); N(H)-S(=O)₂-(C₃₋₆-cycloalkyl); N(H)-S(=O)₂-(3 to 7 membered heterocyclyl); N(H)-S(=O)₂-(aryl); N(H)-S(=O)₂-(heteroaryl); N(C₁₋₄-alkyl)-S(=O)₂-(C₁₋₆-alkyl); N(C₁₋₆-alkyl)-S(=O)₂-(C₃₋₆-cycloalkyl); N(C₁₋₆-alkyl)-S(=O)₂-(3 to 7 membered heterocyclyl); N(C₁₋₆-alkyl)-S(=O)₂-(aryl); N(C₁₋₆-alkyl)-S(=O)₂-(heteroaryl); N(H)-C(=O)-O(C₁₋₆-alkyl); N(H)-C(=O)-O(C₃₋₆-cycloalkyl); N(H)-C(=O)-O(3 to 7 membered heterocyclyl); N(H)-C(=O)-O(aryl); N(H)-C(=O)-O(heteroaryl); N(C₁₋₆-alkyl)-C(=O)-O(C₁₋₆-alkyl); N(C₁₋₆-alkyl)-C(=O)-O(C₃₋₆-cycloalkyl); N(C₁₋₆-alkyl)-C(=O)-O(3 to 7 membered heterocyclyl); N(C₁₋₆-alkyl)-C(=O)-O(aryl); N(C₁₋₆-alkyl)-C(=O)-O(heteroaryl); N(H)-C(=O)-NH₂; N(H)-C(=O)-N(H)(C₁₋₆-alkyl); N(H)-C(=O)-N(H)(C₃₋₆-cycloalkyl); N(H)-C(=O)-N(H)(3 to 7 membered heterocyclyl); N(H)-C(=O)-N(H)(aryl); N(H)-C(=O)-N(H)(heteroaryl); N(C₁₋₆-alkyl)-C(=O)-NH₂; N(C₁₋₆-alkyl)-C(=O-N(H)(C₁₋₆-alkyl); N(C₁₋₆-alkyl)-C(=O)-N(H)(C₃₋₆-cycloalkyl); N(C₁₋₆-alkyl)-C(=O)-N(H)(3 to 7 membered heterocyclyl); N(C₁₋₆-alkyl)-C(=O)-N(H)(aryl); N(C₁₋₆-alkyl)-C(=O)-N(H)(heteroaryl); N(H)-C(=O)-N(C₁₋₆-alkyl)₂; N(H)-C(=O)-N(C₁₋₆-alkyl)(C₃₋₆-cycloalkyl); N(H)-C(=O)-N(C₁₋₆-alkyl)(3 to 7 membered heterocyclyl); N(H)-C(=O)-N(C₁₋₆-alkyl)(aryl); N(H)-C(=O)-N(C₁₋₆-alkyl) (heteroaryl); N(C₁₋₆-alkyl)-C(=O)-N(C₁₋₆-alkyl)₂; N(C₁₋₆-alkyl)-C(=O)-N(C₁₋₆-alkyl)(C₃₋₆-cycloalkyl); N(C₁₋₆-alkyl)-C(=O)-N(C₁₋₆-alkyl)(3 to 7 membered heterocyclyl); N(C₁₋₆-alkyl)-C(=O)-N(C₁₋₆-alkyl)(aryl); N(C₁₋₆-alkyl)-C(=O)-N(C₁₋₆-alkyl) heteroaryl); SH; S-(C₁₋₆-alkyl); S-(C₃₋₆-cycloalkyl); S-(3 to 7 membered heterocyclyl); S-(aryl); S-(heteroaryl); SCF₃; S(=O)₂OH; S(=O)-(C₁₋₆-alkyl); S(=O)-(C₃₋₆-cycloalkyl); S(=O)-(3 to 7 membered heterocyclyl); S(=O)-(aryl); S(=O)-(heteroaryl); S(=O)₂-(C₁₋₆-alkyl); S(=O)₂-(C₃₋₆-cycloalkyl); S(=O)₂-(3 to 7 membered heterocyclyl); S(=O)₂-(aryl); S(=O)₂-(heteroaryl); S(=O)₂-O(C₁₋₆-alkyl); S(=O)₂-O(C₃₋₆-cycloalkyl); S(=O)₂-O(3 to 7 membered heterocyclyl); S(=O)₂-O(aryl); S(=O)₂-O(heteroaryl); S(=O)₂-N(H)(C₁₋₆-alkyl); S(=O)₂-N(H)(C₃₋₆-cycloalkyl); S(=O)₂-N(H)(3 to 7 membered heterocyclyl); S(=O)₂-N(H)(aryl); S(=O)₂-N(H)(heteroaryl); S(=O)₂-N(C₁₋₆-alkyl)₂; S(=O)₂-N(C₁₋₆-alkyl)(C₃₋₆-cycloalkyl); S(=O)₂-N(C₁₋₆-alkyl)(3 to 7 membered heterocyclyl); S(=Oh-N(C₁₋₆-alkyl)-(aryl) and S(=O)₂-N(C₁₋₆-alkyl)(heteroaryl).

Preferred substituents of "aryl" and "heteroaryl" are selected from the group consisting of F; Cl; Br; NO₂; CN; CF₃; CF₂H; CFH₂; CF₂Cl; CFCl₂; C₁₋₆-alkyl; aryl; heteroaryl; C₃₋₆-cycloalkyl; 3 to 6 membered heterocyclyl; aryl, heteroaryl, C₃₋₆-cycloalkyl or 3 to 6 membered heterocycloaliphatic, each connected via a C₁₋₈-alkylene; C(=O)-H; C(=O)-C₁₋₆-alkyl; C(=O)aryl; C(=O)heteroaryl; C(=O)-OH; C(=O)-O-C₁₋₆-alkyl; C(=O)O-aryl; C(=O)O-heteroaryl; CO-NH₂; C(=O)-N(H)C₁₋₆-alkyl; C(=O)-N(C₁₋₆-alkyl)₂; C(=O)NH-aryl; C(=O)N(aryl)₂; C(=O)NH-heteroaryl; C(=O)N(heteroaryl)₂; C(=O)N(C₁₋₆-alkyl)(aryl); C(=O)N(C₁₋₆-alkyl)(heteroaryl); C(=O)N(heteroaryl)(aryl); OH; OCF₃; OCF₂H; OCFH₂; OCF₂Cl; OCFCl₂; O-C₁₋₆-alkyl; O-benzyl; O-aryl; O-heteroaryl; O-C(=O)-C₁₋₆-alkyl; O-C(=O)aryl; O-C(=O)heteroaryl; O-C(=O)-O-C₁₋₆-alkyl; O-(C=O)-N(H)C₁₋₆-alkyl; O-C(=O)-N(C₁₋₆-alkyl)₂; O-S(=O)₂-C₁₋₆-alkyl; O-S(=O)₂-OH; O-S(=O)₂-O-C₁₋₆-alkyl; O-S(=O)₂-NH₂; O-S(=O)₂-N(H)C₁₋₆-alkyl; O-S(=O)₂-N(C₁₋₆-alkyl)₂; NH₂; N(H)C₁₋₆-alkyl; N(C₁₋₆-alkyl)₂; N(H)-C(=O)-C₁₋₆-alkyl; N(H)-C(=O)-aryl; N(H)-C(=O)-heteroaryl; N(H)-C(=O)-O-C₁₋₆-alkyl; N(H)C(=O)-NH₂; N(H)-C(=O)-N(H)C₁₋₆-alkyl; N(H)-C(=O)-N(C₁₋₆-alkyl)₂; N(C₁₋₆-alkyl)-C(=O)C₁₋₆-alkyl; N(C₁₋₆-alkyl)-C(=O)-O-C₁₋₆-alkyl; N(C₁₋₆-alkyl)C(=O)-NH₂; N(C₁₋₆-alkyl)-C(=O)-N(H)C₁₋₆-alkyl; N(C₁₋₆-alkyl)-C(=O)-N(C₁₋₆-alkyl)₂; N(H)-S(=O)₂-OH; N(H)-S(=O)₂-C₁₋₆-alkyl; N(H)-S(=O)₂-O-C₁₋₆-alkyl; N(H)-S(=O)₂-NH₂; N(H)S(=O)₂-N(H)C₁₋₆-alkyl; N(H)-S(=O)₂-N(C₁₋₆-alkyl)₂; N(C₁₋₆-alkyl)-S(=O)₂-OH; N(C₁₋₆-alkyl)-S(=O)₂(C₁₋₆-alkyl); N(C₁₋₆-alkyl)-S(=O)₂-O(C₁₋₆-alkyl); N(C₁₋₆-alkyl)-S(=O)₂-NH₂; N(C₁₋₆-alkyl)-S(=O)₂-N(H)C₁₋₆-alkyl; N(C₁₋₆-alkyl)S(=O)₂-N(C₁₋₆-alkyl)₂; SH; SCF₃; SCF₂H; SCFH₂; SCF₂Cl; SCFCl₂; S-C₁₋₆-alkyl; S-benzyl; S-aryl; Sheteroaryl; S(=O)-C₁₋₆-alkyl; S(=O)₂-C₁₋₆-alkyl; S(=O)₂-aryl; S(=O)₂-heteroaryl; S(=O)₂-OH; S(=O)₂-OC₁₋₆-alkyl; S(=O)₂O-aryl; S(=O)₂O-heteroaryl; S(=O)₂-NH₂; S(=O)₂-N(H)C₁₋₆-alkyl, S(=O)₂-N(H)-aryl; S(=O)₂-N(H)-heteroaryl and S(=O)₂-N(C₁₋₆-alkyl)₂.

More preferred substituents of "aryl" and "heteroaryl" are selected from the group consisting of F; Cl; CF₃; CN; C₁₋₆-alkyl; C(=O)-OH; C(=O)-O-C₁₋₆-alkyl; CO-NH₂; C(=O)-N(H)C₁₋₆-alkyl; C(=O)-N(C₁₋₆-alkyl)₂; OH; O-C₁₋₆-alkyl; O-C(=O)-C₁₋₆-alkyl; OCF₃; OCHF₂; OCH₂F; NH₂; N(H)C₁₋₆-alkyl; N(C₁₋₆-alkyl)₂; N(H)-C(=O)-C₁₋₆-alkyl; N(C₁₋₆-alkyl)-C(=O)C₁₋₆-alkyl; N(H)-S(=O)₂-C₁₋₆-alkyl; N(C₁₋₆-alkyl)-S(=O)₂(C₁₋₆-alkyl); N(H)C(=O)NH₂; N(H)C(=O)-N(H)C₁₋₆-alkyl; N(H)-C(=O)-N(C₁₋₆-alkyl)₂; N(C₁₋₆-alkyl)-C(=O)-NH₂; N(C₁₋₆-alkyl)C(=O)-N(H)C₁₋₆-alkyl; N(C₁₋₆-alkyl)-C(=O)-N(C₁₋₆-alkyl)₂; S(=O)₂C₁₋₆-alkyl; S(=O)₂-NH₂; S(=O)₂-N(H)C₁₋₆-alkyl and S(=O)₂-N(C₁₋₆-alkyl)₂.

The compounds according to the invention are defined by substituents, for example by R¹, R² and R³ (1^{st} generation substituents) which are for their part if appropriate themselves substituted (2^{nd} generation substituents). Depending on the definition, these substituents of the substituents can for their part be resubstituted (3^{rd} generation substituents). If, for example, R¹ = a C₁₋₆-alkyl (1^{st} generation substituent), then the C₁₋₆-alkyl can for its part be substituted, for example with a NH- C₁₋₆-alkyl (2^{nd} generation substituent). This produces the functional group R¹ = (C₁₋₆-alkyl-NH-C₁₋₆-alkyl). The NH-C₁₋₆-alkyl can then for its part be resubstituted, for example with Cl (3^{rd} generation substituent). Overall, this produces the functional group R¹ = C₁₋₆-alkyl-NH-C₁₋₆-alkyl, wherein the C₁₋₆-alkyl of the NH-C₁₋₆-alkyl is substituted by Cl. However, in a preferred embodiment, the 3^{rd} generation substituents may not be resubstituted, i.e. there are then no 4^{th} generation substituents. If a residue occurs multiply within a molecule, then this residue can have respectively different meanings for various substituents: if, for example, both R¹ and R² denote a 3 to 10 membered heterocyclyl, then the 3 to 10 membered heterocyclyl can e.g. represent morpholinyl for R¹ and can represent piperazinyl for R².

Within the scope of the present invention, the symbols 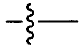 or ------ used in the formulae denotes a link of a corresponding residue to the respective superordinate general structure.

In one embodiment of the first aspect of the invention, the compound according to general formula (I) is characterized in that
R¹ represents
C₁₋₆-alkyl, C₂₋₆-alkenyl or C₂₋₆-alkynyl,
in each case branched or unbranched, and in each case unsubstituted or mono- or polysubstituted by one or more substituents selected from
F; Cl; CN; CF₃; CF₂H; CFH₂; C(=O)-OH; C(=O)-O-C₁₋₆-alkyl; C(=O)-NH₂; C(=O)-N(H)(C₁₋₆-alkyl); C(=O)-N(C₁₋₆-alkyl)₂; OH; OCF₃; OCF₂H; OCFH₂; O-C₁₋₆-alkyl; O-C(=O)-C₁₋₆-alkyl; O-S(=O)₂-C₁₋₆-alkyl; NH₂; N(H)(C₁₋₆-alkyl); N(C₁₋₆-alkyl)₂; N(H)-C(=O)-C₁₋₆-alkyl; N(H)-C(=O)-N(H)(C₁₋₆-alkyl); N(H)-C(=O)-N(C₁₋₆-alkyl)₂; N(C₁₋₆-alkyl)-C(=O)-C₁₋₆-alkyl; N(H)-S(=O)₂-C₁₋₆-alkyl; N(C₁₋₆-alkyl)-S(=O)₂-C₁₋₆-alkyl; S(=O)-C₁₋₆-alkyl, S(=O)₂-C₁₋₆-alkyl, C₃₋₆-cycloalkyl or 3 to 7 membered heterocyclyl,
or R¹ represents
C₃₋₆-cycloalkyl or 3 to 7 membered heterocyclyl, in each case unsubstituted or mono- or polysubstituted by one or more substituents selected from
F; Cl; CN; C₁₋₆-alkyl; CF₃; CF₂H; CFH₂; C(=O)-OH; C(=O)-O-C₁₋₆-alkyl; C(=O)-NH₂; C(=O)-N(H)(C₁₋₆-alkyl); C(=O)-N(C₁₋₆-alkyl)₂; OH; =O; OCF₃; OCF₂H; OCFH₂; O-C₁₋₆-alkyl; O-C(=O)-C₁₋₆-alkyl; O-S(=O)₂-C₁₋₆-alkyl; NH₂; N(H)(C₁₋₆-alkyl); N(C₁₋₆-alkylh; N(H)-C(=O)-C₁₋₆-alkyl; N(H)-C(=O)-N(H)(C₁₋₆-alkyl); N(H)-C(=O)-N(C₁₋₆-alkyl)₂; N(C₁₋₆-alkyl)-C(=O)-C₁₋₆-alkyl; N(H)-S(=O)₂-C₁₋₆-alkyl; N(C₁₋₆-alkyl)-S(=O)₂-C₁₋₆-alkyl; S(=O)-C₁₋₆-alkyl or S(=O)₂-C₁₋₆-alkyl.

Preferably,
R¹ represents
C₁₋₆-alkyl, C₂₋₆-alkenyl or C₂₋₆-alkynyl,
in each case branched or unbranched, and in each case unsubstituted or mono- or polysubstituted by one or more substituents selected from
F; Cl; CN; CF₃; C(=O)-NH₂; C(=O)-N(H)(C₁₋₆-alkyl); C(=O)-N(C₁₋₆-alkyl)₂; OH; O-C₁₋₆-alkyl; NH₂; N(H)(C₁₋₆-alkyl); N(C₁₋₆-alkyl)₂; N(H)-C(=O)-C₁₋₆-alkyl; S(=O)-C₁₋₆-alkyl; S(=O)₂-C₁₋₆-alkyl or cyclopropyl,
or
R¹ represents 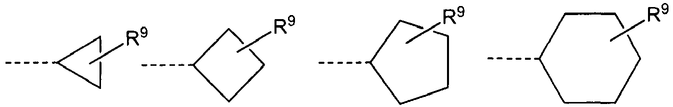 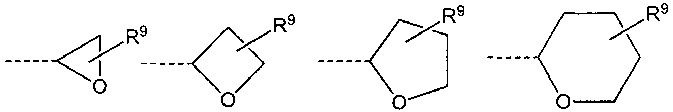 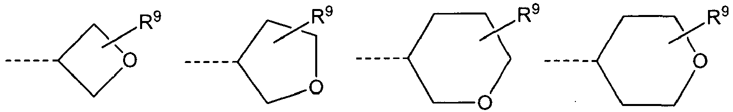 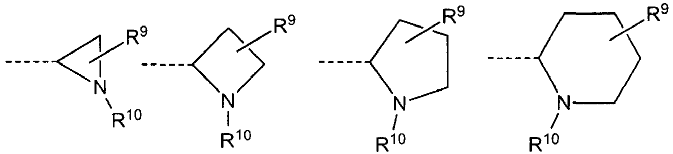 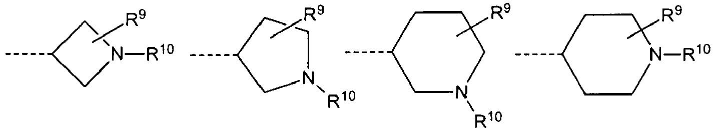 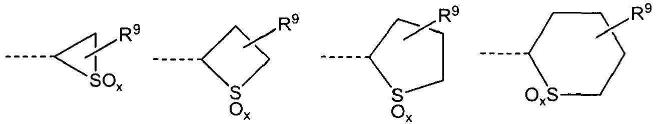 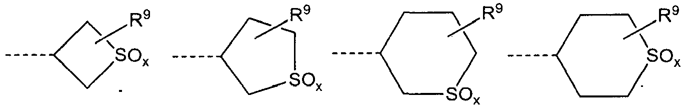 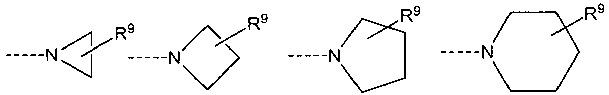
wherein x is 0, 1 or 2;
R⁹ represents 0, 1 or 2 substituents, independently selected from the group consisting of F; CN; C₁₋₆-alkyl; CF₃; OH; =O; O-C₁₋₆-alkyl; NH₂; N(H)(C₁₋₆-alkyl) or N(C₁₋₆-alkyl)₂ and
R¹⁰ is selected from H, C₁₋₆-alkyl, C(=O)-C₁₋₆-alkyl and S(=O)₂-C₁₋₆-alkyl.

Preferably,
R¹ represents
C₁₋₆-alkyl, C₂₋₆-alkenyl or C₂₋₆-alkynyl,
in each case branched or unbranched, and in each case unsubstituted or mono- or polysubstituted by one or more substituents selected from
F; Cl; CN; CF₃; C(=O)-NH₂; C(=O)-N(H)(C₁₋₆-alkyl); C(=O)-N(C₁₋₆-alkyl)₂; OH; O-C₁₋₆-alkyl; NH₂; N(H)(C₁₋₆-alkyl); N(C₁₋₆-alkyl)₂; N(H)-C(=O)-C₁₋₆-alkyl; S(=O)-C₁₋₆-alkyl; S(=O)₂-C₁₋₆-alkyl or cyclopropyl,
or R¹ represents
cyclopropyl, cyclobutyl, cyclopentyl, cyclohexyl, oxetanyl, azetidinyl, tetrahydrofuranyl, tetrahydropyranyl, pyrrolidinyl, piperidinyl and piperazinyl,
in each case unsubstituted or mono- or polysubstituted by one or more substituents selected from F; Cl; CN; C₁₋₆-alkyl; CF₃; OH; =O; O-C₁₋₆-alkyl; O-C(=O)-C₁₋₆-alkyl; O-S(=O)₂-C₁₋₆-alkyl; NH₂; N(H)(C₁₋₆-alkyl); N(C₁₋₆-alkyl)₂; N(H)-C(=O)-C₁₋₆-alkyl; N(H)-S(=O)₂-C₁₋₆-alkyl or S(=O)₂-C₁₋₆-alkyl.

More preferably,
R¹ represents CH₃, CH₂CH₃, CH(CH)₃, C(CH₃)₃, CF₃, CH₂S(=O)₂CH₃, CH₂OCH₃, CH₂OH, CH₂NH(C=O)CH₃, CH₂C(=O)-NH₂, CH₂N(CH₃)₂, C=C-cyclopropyl, or 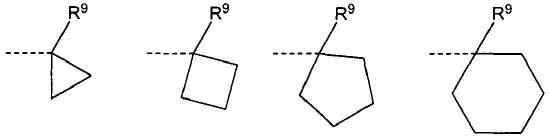 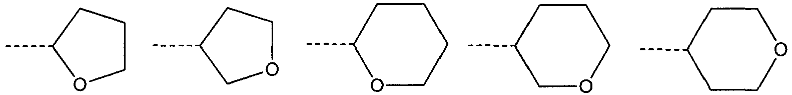 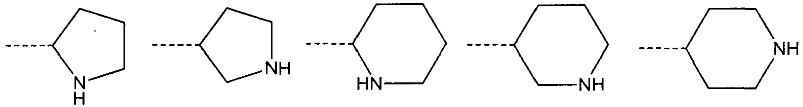 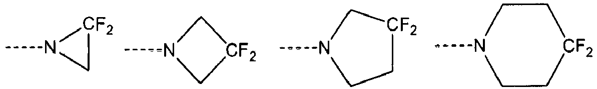 wherein R⁹ represents 0 or 1 substituent, selected from the group consisting of F; CN; CH₃; OH; OCH₃; NH₂; N(H)(CH₃) or N(CH₃)₂.

In another embodiment of the first aspect of the invention, the compound according to general formula (I) is characterized in that n represents 0 or 1.

In another embodiment of the first aspect of the invention, the compound according to general formula (I) is characterized in that R² represents CH₂F, CHF₂ or CF₃. Preferably, R² represents CHF₂ or CF₃.

A particularly preferred compound according to formula (I) is characterized in that R² represents CF₃.

Another particularly preferred compound according to formula (I) is characterized in that R² represents CHF₂.

In a further embodiment of the first aspect of the invention, the compound according to general formula (I) is characterized in that R³ represents H, C₁₋₆-alkyl, branched or unbranched, unsubstituted or mono- or poly-substituted, C₃₋₆-cycloalkyl or 3 to 7 membered heterocyclyl, in each case unsubstituted or mono- or polysubstituted; OH; O-C₁₋₆-alkyl; NH₂; N(H)-C₁₋₆-alkyl; N(-C₁₋₆-alkyl)₂ or SO₂(-C₁₋₆-alkyl), wherein in each case C₁₋₆-alkyl may be branched or unbranched and may be unsubstituted or mono- or polysubstituted.

Preferably, R³ is selected from the group consisting of H, methyl, ethyl, n-propyl, iso-propyl, n-butyl, sec-butyl, iso-butyl, tert-butyl, cyclopropyl, methoxy, ethoxy, methylsulfonyl, 2-oxetyl, 3-oxetyl, 2-tetrahydrofuranyl and 3-tetrahydrofuranyl.

More preferably, R³ is selected from the group consisting of H, methyl, ethyl, iso-propyl and cyclopropyl.

Even more preferably, R³ represents H or methyl.

In a particularly preferred embodiment of the invention, the compound according to general formula (I) is characterized in that R³ represents methyl (CH₃). In another particularly preferred embodiment of the invention, the compound according to general formula (I) is characterized in that R³ represents H.

In another embodiment of the first aspect of the invention, the compound according to general formula (I) is characterized in that (Het)Aryl is selected from the group consisting of phenyl, naphthyl, pyrrol, furanyl, thienyl, pyrazolyl, imidazolyl, isoxazolyl, isothiazolyl, oxazolyl, thiazolyl, triazolyl, oxadiazolyl, thiadiazolyl, tetrazolyl, indolyl, benzofuranyl, benzothienyl, indazolyl, benzimidazolyl, benzoxazolyl, benzothiazolyl, benzotriazolyl, pyridinyl, pyrazinyl, pyrimidinyl, pyridazinyl, triazinyl, quinolinyl, isoquinolinyl, quinoxalinyl, quinazolinyl, cinnolinyl, 1,5-naphthyridinyl, 1,6-naphthyridinyl, 1,7-naphthyridinyl, 1,8-naphthyridinyl, 2,3-naphthyridinyl, 2,6-naphthyridinyl and 2,7-naphthyridinyl, each substituted by zero or one or two or three substituents of the group consisting of R⁶, R⁷ and R⁸.

Particularly preferred compounds according to the invention are characterized in that the (Het)Aryl substituent is selected from aryl substituents. Therefore, in one preferred embodiment of the first aspect of the invention, the compound according to general formula (I) is characterized in that (Het)Aryl is selected from the group consisting of phenyl, 1-naphthyl or 2-naphthyl, each substituted by zero or one or two or three substituents of the group consisting of R⁶, R⁷ and R⁸.

Also particularly preferred compounds according to the invention are characterized in that the (Het)Aryl substituent is selected from heteroaryl substituents. Therefore, in another preferred embodiment of the first aspect of the invention, the compound according to general formula (I) is characterized in that (Het)Aryl is selected from the group consisting of pyrrol, furanyl, thienyl, pyrazolyl, imidazolyl, isoxazolyl, isothiazolyl, oxazolyl, thiazolyl, triazolyl, oxadiazolyl, thiadiazolyl, tetrazolyl, indolyl, benzofuranyl, benzothienyl, indazolyl, benzimidazolyl, benzoxazolyl, benzothiazolyl, benzotriazolyl, pyridinyl, pyrazinyl, pyrimidinyl, pyridazinyl, triazinyl, quinolinyl, isoquinolinyl, quinoxalinyl, quinazolinyl, cinnolinyl, 1,5-naphthyridinyl, 1,6-naphthyridinyl, 1,7-naphthyridinyl, 1,8-naphthyridinyl, 2,3-naphthyridinyl, 2,6-naphthyridinyl and 2,7-naphthyridinyl, each substituted by zero or one or two or three substituents of the group consisting of R⁶, R⁷ and R⁸

Preferably, (Het)Aryl is selected from the group consisting of phenyl, pyrrol, pyrazolyl, imidazolyl, isoxazolyl, oxazolyl, thiazolyl, pyridinyl, pyrazinyl, pyrimidinyl and pyridazinyl, each substituted by zero or one or two or three substituents of the group consisting of R⁶, R⁷ and R⁸.

More preferably, (Het)Aryl represents phenyl, substituted by zero or one or two or three substituents of the group consisting of R⁶, R⁷ and R⁸.

In another embodiment of the first aspect of the invention, the compound according to general formula (I) is characterized in that R⁶, R⁷ and R⁸ are idependently selected from the group consisting of F; Cl; CN; C₁₋₆-alkyl; CF₃; CF₂H; CFH₂; OH; OCF₃; OCF₂H; OCFH₂; O-C₁₋₆-alkyl; O-C(=O)-C₁₋₆-alkyl; NH₂; N(H)(C₁₋₆-alkyl); N(C₁₋₆-alkyl)₂; SCF₃; S(=O)-C₁₋₆-alkyl; S(=O)₂-C₁₋₆-alkyl; S(=O)₂-OH; S(=O)₂-O-C₁₋₆-alkyl; S(=O)₂-NH₂; S(=O)₂-N(H)(C₁₋₆-alkyl); S(=O)₂-N(C₁₋₆-alkyl)₂; C₃₋₆-cycloalkyl or O-C₃₋₆-cycloalkyl, wherein in each case said C₁₋₆-alkyl may be branched or unbranched and wherein in each case said C₃₋₆-cycloalkyl may be unsubstituted or mono- or polysubstituted.

Preferably, R⁶, R⁷ and R⁸ are each independently selected from the group consisting of F; Cl; CN; C₁₋₆-alkyl; CF₃; CF₂H; CFH₂; OH; OCF₃; OCF₂H; OCFH₂; O-C₁₋₆-alkyl; S(=O)-C₁₋₆-alkyl; S(=O)₂-C₁₋₆-alkyl; cyclopropyl and O-cyclopropyl.

More preferably, (Het)Aryl is selected from the group consisting of phenyl, pyrrol, pyrazolyl, imidazolyl, isoxazolyl, oxazolyl, thiazolyl, pyridinyl, pyrazinyl, pyrimidinyl and pyridazinyl, each substituted by zero or one or two or three substituents of the group consisting of R⁶, R⁷ and R⁸,
wherein R⁶, R⁷ and R⁸ are each independently of one another selected from the group consisting of F; Cl; CN; C₁₋₆-alkyl; CF₃; CF₂H; CFH₂; OH; OCF₃; OCF₂H; OCFH₂; O-C₁₋₆-alkyl; O-C(=O)-C₁₋₆-alkyl; NH₂; N(H)(C₁₋₆-alkyl); N(C₁₋₆-alkyl)₂; SCF₃; S(=O)-C₁₋₆-alkyl; S(=O)₂-C₁₋₆-alkyl; S(=O)₂-OH; S(=O)₂-O-C₁₋₆-alkyl; S(=O)₂-NH₂; S(=O)₂-N(H)(C₁₋₆-alkyl); S(=O)₂-N(C₁₋₆-alkyl)₂; C₃₋₆-cycloalkyl or O-C₃₋₆-cycloalkyl,
wherein in each case said C₁₋₆-alkyl may be branched or unbranched and wherein in each case said C₃₋₆-cycloalkyl may be unsubstituted or mono- or polysubstituted.

Even more preferably, (Het)Aryl is selected from the group consisting of phenyl, pyrrol, pyrazolyl, imidazolyl, isoxazolyl, oxazolyl, thiazolyl, pyridinyl, pyrazinyl, pyrimidinyl and pyridazinyl, each substituted by zero or one or two or three substituents of the group consisting of R⁶, R⁷ and R⁸, wherein R⁶, R⁷ and R⁸ are each independently of one another selected from the group consisting of F; Cl; CN; C₁₋₆-alkyl; CF₃; CF₂H; CFH₂; OH; OCF₃; OCF₂H; OCFH₂; O-C₁₋₆-alkyl; S(=O)-C₁₋₆-alkyl; S(=O)₂-C₁₋₆-alkyl; cyclopropyl and O-cyclopropyl.

Yet more preferably, (Het)Aryl is selected from the group consisting of phenyl, pyridin-2-yl, pyridin-3-yl, pyridin-4-yl, pyrazin-2-yl, pyrimidin-2-yl, pyrimidin-4-yl, pyrimidin-5-yl, pyridazin-3-yl, pyridazin-4-yl, pyrazol-3-yl, pyrazol-4-yl, pyrazol-5-yl, isoxazol-3-yl, isoxazol-4-yl, isoxazol-5-yl, oxazol-2-yl, oxazol-4-yl, oxazol-5-yl, thiazol-2-yl, thiazol-4-yl, thiazol-5-yl, each substituted by zero or one or two substituents of the group consisting of R⁶ and R⁷, wherein R⁶ and R⁷ are each independently of one another selected from the group consisting of F; Cl; CN; CF₃; CH₃; OH; OCF₃; OCH₃; S(=O)-CH₃; S(=O)₂-CH₃; cyclopropyl and O-cyclopropyl.

Even more preferably, (Het)Aryl represenrs phenyl, pyridinyl or pyrimidinyl, substituted by zero or one or two substituents of the group consisting of R⁶ and R⁷, wherein R⁶ and R⁷ are each independently of one another selected from the group consisting of F; Cl; CN; CF₃; CH₃; OH; OCF₃; OCH₃; S(=O)-CH₃; S(=O)₂-CH₃; cyclopropyl and O-cyclopropyl.

Particularly preferred, (Het)Aryl is selected from the group consisting of phenyl, 2-fluoro-phenyl, 3-fluoro-phenyl, 4-fluoro-phenyl, 2-chloro-phenyl, 3-chloro-phenyl, 4-chloro-phenyl, 2-cyano-phenyl, 3-cyano-phenyl, 4-cyano-phenyl, 2-methoxy-phenyl, 3-methoxy-phenyl, 4-methoxy-phenyl, 2-methyl-phenyl, 3-methyl-phenyl, 4-methyl-phenyl, 2-trifluoromethyl-phenyl, 3-trifluoromethyl-phenyl, 4-trifluoromethyl-phenyl, 2,3-difluoro-phenyl, 2,4-difluoro-phenyl, 2,5-difluoro-phenyl, 2,6-difluoro-phenyl, 3,4-difluoro-phenyl, 3,5-difluoro-phenyl, 2,3-dichloro-phenyl, 2,4-dichloro-phenyl, 2,5-dichloro-phenyl, 2,6-dichloro-phenyl, 3,4-dichloro-phenyl, 3,5-dichloro-phenyl, 4-chloro-2-fluoro-phenyl, 3-chloro-2-fluoro-phenyl, 5-chloro-2-fluoro-phenyl, 6-chloro-2-fluoro-phenyl, 4-chloro-3-fluoro-phenyl, 2-chloro-3-fluoro-phenyl, 5-chloro-3-fluoro-phenyl, 6-chloro-3-fluoro-phenyl, 2-chloro-4-fluoro-phenyl, 3-chloro-4-fluoro-phenyl, 6-hydroxy-pyridin-3-yl, 3-fluoro-5-(trifluoromethyl)-pyridin-2-yl, 6-cyano-4-methyl-pyridin-3-yl, 6-chloro-4-methyl-pyridin-3-yl, 6-methoxy-4-methyl-pyridin-3-yl, 2-cyano-4-methyl-pyridin-5-yl, pyridin-2-yl, 3-fluoro-pyridin-2-yl, 4-fluoro-pyridin-2-yl, 5-fluoro-pyridin-2-yl, 6-fluoro-pyridin-2-yl, 3-chloro-pyridin-2-yl, 4-chloro-pyridin-2-yl, 5-chloro-pyridin-2-yl, 6-chloro-pyridin-2-yl, 3-cyano-pyridin-2-yl, 4-cyano-pyridin-2-yl, 5-cyano-pyridin-2-yl, 6-cyano-pyridin-2-yl, 3-methoxy-pyridin-2-yl, 4-methoxy-pyridin-2-yl, 5-methoxy-pyridin-2-yl, 6-methoxy-pyridin-2-yl, 3-methyl-pyridin-2-yl, 4-methyl-pyridin-2-yl, 5-methyl-pyridin-2-yl, 6-methyl-pyridin-2-yl, 3-trifluoromethyl-pyridin-2-yl, 4-trifluoromethyl-pyridin-2-yl, 5-trifluoromethyl-pyridin-2-yl, 6-trifluoromethyl-pyridin-2-yl, pyridin-3-yl, 2-fluoro-pyridin-3-yl, 4-fluoro-pyridin-3-yl, 5-fluoro-pyridin-3-yl, 6-fluoro-pyridin-3-yl, 2-chloro-pyridin-3-yl, 4-chloro-pyridin-3-yl, 5-chloro-pyridin-3-yl, 6-chloro-pyridin-3-yl, 2-cyano-pyridin-3-yl, 4-cyano-pyridin-3-yl, 5-cyano-pyridin-3-yl, 6-cyano-pyridin-3-yl, 2-methoxy-pyridin-3-yl, 4-methoxy-pyridin-3-yl, 5-methoxy-pyridin-3-yl, 6-methoxy-pyridin-3-yl, 2-methyl-pyridin-3-yl, 4-methyl-pyridin-3-yl, 5-methyl-pyridin-3-yl, 6-methyl-pyridin-3-yl, 2-trifluoromethyl-pyridin-3-yl, 4-trifluoromethyl-pyridin-3-yl, 5-trifluoromethyl-pyridin-3-yl, 6-trifluoromethyl-pyridin-3-yl, pyridin-4-yl, 2-fluoro-pyridin-4-yl, 3-fluoro-pyridin-4-yl, 2-chloro-pyridin-4-yl, 3-chloro-pyridin-4-yl, 2-cyano-pyridin-4-yl, 3-cyano-pyridin-4-yl, 2-methoxy-pyridin-4-yl, 3-methoxy-pyridin-4-yl, 2-methyl-pyridin-4-yl, 3-methyl-pyridin-4-yl, 2-trifluoromethyl-pyridin-4-yl, 3-trifluoromethyl-pyridin-4-yl, pyrimidin-2-yl, 4-fluoro-pyrimidin-2-yl, 4-chloro-pyrimidin-2-yl, 5-fluoro-pyrimidin-2-yl, 5-chloro-pyrimidin-2-yl, 4-methoxy-pyrimidin-2-yl, 4-methyl-pyrimidin-2-yl, 5-methoxy-pyrimidin-2-yl, 5-methyl-pyrimidin-2-yl, 4-trifluoromethyl-pyrimidin-2-yl, 4-cyano-pyrimidin-2-yl, 5-trifluoromethyl-pyrimidin-2-yl, 5-cyano-pyrimidin-2-yl, pyrimidin-4-yl, 2-fluoro-pyrimidin-4-yl, 2-chloro-pyrimidin-4-yl, 5-fluoro-pyrimidin-4-yl, 5-chloro-pyrimidin-4-yl, 6-fluoro-pyrimidin-4-yl, 6-chloro-pyrimidin-4-yl, 2-trifluoromethyl-pyrimidin-4-yl, 2-cyano-pyrimidin-4-yl, 5-trifluoromethyl-pyrimidin-4-yl, 5-cyano-pyrimidin-4-yl, 6-trifluoromethyl-pyrimidin-4-yl, 6-cyano-pyrimidin-4-yl, 2-methyl-pyrimidin-4-yl, 2-methoxy-pyrimidin-4-yl, 5-methyl-pyrimidin-4-yl, 5-methoxy-pyrimidin-4-yl, 6-methyl-pyrimidin-4-yl, 6-methoxy-pyrimidin-4-yl, pyrimidin-5-yl, 2-fluoro-pyrimidin-5-yl, 2-chloro-pyrimidin-5-yl, 4-fluoro-pyrimidin-5-yl, 4-chloro-pyrimidin-5-yl, 2-methyl-pyrimidin-5-yl, 2-methoxy-pyrimidin-5-yl, 4-methyl-pyrimidin-5-yl, 4-methoxy-pyrimidin-5-yl, 2-trifluoromethyl-pyrimidin-5-yl, 2-cyano-pyrimidin-5-yl, 4-trifluoromethyl-pyrimidin-5-yl, 4-cyano-pyrimidin-5-yl, pyrazin-2-yl, 2-methoxy-pyrazin-2-yl, 5-methoxy-pyrazin-2-yl, 6-methoxy-pyrazin-2-yl, 2-methyl-pyrazin-2-yl, 5-methyl-pyrazin-2-yl, 6-methyl-pyrazin-2-yl, 2-fluoro-pyrazin-2-yl, 5-fluoro-pyrazin-2-yl, 6-fluoro-pyrazin-2-yl, 2-chloro-pyrazin-2-yl, 5-chloro-pyrazin-2-yl, 6-chloro-pyrazin-2-yl, 2-trifluoromethyl-pyrazin-2-yl, 5-trifluoromethyl-pyrazin-2-yl, 6-trifluoromethyl-pyrazin-2-yl, 2-cyano-pyrazin-2-yl, 5-cyano-pyrazin-2-yl, 6-cyano-pyrazin-2-yl, pyridazin-3-yl, 4-methoxy-pyridazin-3-yl, 5-methoxy-pyridazin-3-yl, 6-methoxy-pyridazin-3-yl, 4-methyl-pyridazin-3-yl, 5-methyl-pyridazin-3-yl, 6-methyl-pyridazin-3-yl, 4-fluoro-pyridazin-3-yl, 5-fluoro-pyridazin-3-yl, 6-fluoro-pyridazin-3-yl, 4-chloro-pyridazin-3-yl, 5-chloro-pyridazin-3-yl, 6-chloro-pyridazin-3-yl, 4-trifluoromethyl-pyridazin-3-yl, 5-trifluoromethyl-pyridazin-3-yl, 6-trifluoromethyl-pyridazin-3-yl, 4-cyano-pyridazin-3-yl, 5-cyano-pyridazin-3-yl, 6-cyano-pyridazin-3-yl, pyridazin-4-yl, 3-methoxy-pyridazin-4-yl, 5-methoxy-pyridazin-4-yl, 6-methoxy-pyridazin-4-yl, 3-methyl-pyridazin-4-yl, 5-methyl-pyridazin-4-yl, 6-methyl-pyridazin-4-yl, 3-fluoro-pyridazin-4-yl, 5-fluoro-pyridazin-4-yl, 6-fluoro-pyridazin-4-yl, 3-chloro-pyridazin-4-yl, 5-chloro-pyridazin-4-yl, 6-chloro-pyridazin-4-yl, 3-trifluoromethyl-pyridazin-4-yl, 5-trifluoromethyl-pyridazin-4-yl, 6-trifluoromethyl-pyridazin-4-yl, 3-cyano-pyridazin-4-yl, 5-cyano-pyridazin-4-yl, 6-cyano-pyridazin-4-yl, thiophen-2-yl, 3,5-dimethyl-isoxazol-4-yl, 3,5-di-(trifluoromethyl)-isoxazol-4-yl, 1H-pyrazol-4-yl, 1-methyl-1H-pyrazol-4-yl, 5-trifluoromethyl-1-methyl-1H-pyrazol-4-yl, 3-trifluoromethyl-1-methyl-1H-pyrazol-4-yl, 1,5-dimethyl-1H-pyrazol-4-yl, 1,3-dimethyl-1H-pyrazol-4-yl, 1,3,5-trimethyl-1H-pyrazol-4-yl, 1H-pyrazol-3-yl, 1-methyl-1H-pyrazol-3-yl, 5-trifluoromethyl-1-methyl-1H-pyrazol-3-yl, 4-trifluoromethyl-1-methyl-1H-pyrazol-3-yl, 1,5-dimethyl-1H-pyrazol-3-yl, 1,4-dimethyl-1H-pyrazol-3-yl and 1,4,5-trimethyl-1H-pyrazol-3-yl.

Particularly preferred, (Het)Aryl is selected from the group consisting of phenyl, 2-fluoro-phenyl, 3-fluoro-phenyl, 4-fluoro-phenyl, 2-chloro-phenyl, 3-chloro-phenyl, 4-chloro-phenyl, 2,4-difluoro-phenyl, 3,4-difluoro-phenyl, 2,4-dichloro-phenyl, 3,4-dichloro-phenyl, 4-chloro-2-fluoro-phenyl, 4-chloro-3-fluoro-phenyl, 2-chloro-4-fluoro-phenyl, 3-chloro-4-fluoro-phenyl, pyridin-2-yl, 4-fluoro-pyridin-2-yl, 4-chloro-pyridin-2-yl, 4-cyano-pyridin-2-yl, 4-methoxy-pyridin-2-yl, 4-methyl-pyridin-2-yl, 4-trifluoromethyl-pyridin-2-yl, pyrimidin-2-yl, 5-fluoro-pyrimidin-2-yl, 5-chloro-pyrimidin-2-yl, 5-methoxy-pyrimidin-2-yl, 5-methyl-pyrimidin-2-yl, 5-trifluoromethyl-pyrimidin-2-yl and 5-cyano-pyrimidin-2-yl.

In another embodiment of the first aspect of the invention, the compound according to general formula (I) is characterized in that is a compound according to general formula (Ib), 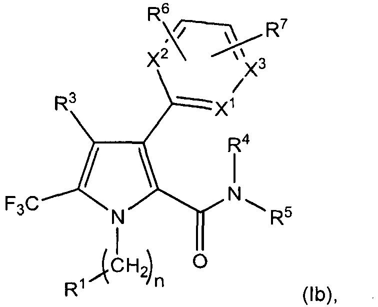 wherein
R³ represents H or CH₃ or cyclopropyl;
X¹ is N or CH; X² is N or CH; X³ is N or CH; and
R⁶ and R⁷ are independently absent or are each independently of one another selected from the group consisting of F; Cl; CN; C₁₋₆-alkyl; CF₃; CF₂H; CFH₂; OH; OCF₃; OCF₂H; OCFH₂ or O-C₁₋₆-alkyl.

In yet another embodiment of the first aspect of the invention, the compound according to general formula (I) is characterized in that is a compound according to general formula (Ia), 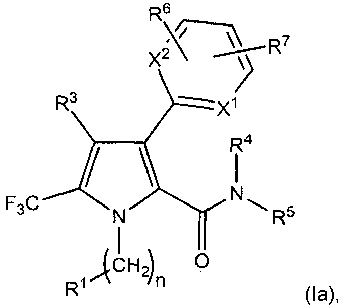 wherein
R³ represents H or CH₃ or cyclopropyl;
X¹ is N or CH; X² is N or CH; and
R⁶ and R⁷ are independently absent or are each independently of one another selected from the group consisting of F; Cl; CN; C₁₋₆-alkyl; CF₃; CF₂H; CFH₂; OH; OCF₃; OCF₂H; OCFH₂ or O-C₁₋₆-alkyl.

In another embodiment of the first aspect of the invention, the compound according to general formula (I) is characterized in that
R⁴ represents H or
C₁₋₆-alkyl, branched or unbranched and unsubstituted or substituted with 1, 2, 3, 4 or 5 substituents independently selected from the group consisting of OH, =O, O-C₁₋₆-alkyl, S(=O)-C₁₋₆-alkyl, S(=O)₂-C₁₋₆-alkyl, N(H)-S(=O)-C₁₋₆-alkyl, N(C₁₋₆-alkyl)-S(=O)-C₁₋₆-alkyl, N(H)-S(=O)₂-C₁₋₆-alkyl, N(C₁₋₆-alkyl)-S(=O)₂-C₁₋₆-alkyl, C(=O)-NH₂, C(=O)-N(H)(C₁₋₆-alkyl), C(=O)-N(C₁₋₆-alkyl)₂, C(=O)-O-C₁₋₆-alkyl; N(H)-C(=O)-C₁₋₆-alkyl, and N(C₁₋₆-alkyl)-C(=O)-C₁₋₆-alkyl; or
C₃₋₆-cycloalkyl, unsubstituted or substituted with 1, 2, 3, 4 or 5 substituents independently selected from the group consisting of F, Cl, CF₃, =O, OCF₃; C₁₋₈-alkylen-OH, C₁₋₆-alkyl, OH, O-C₁₋₆-alkyl, S(=O)-C₁₋₆-alkyl, S(=O)₂-C₁₋₆-alkyl, N(H)-S(=O)-C₁₋₆-alkyl, N(C₁₋₆-alkyl)-S(=O)-C₁₋₆-alkyl, N(H)-S(=O)₂-C₁₋₆-alkyl, N(C₁₋₆-alkyl)-S(=O)₂-C₁₋₆-alkyl, C(=O)-NH₂, C(=O)-N(H)(C₁₋₆-alkyl), C(=O)-N(C₁₋₆-alkyl)₂, C(=O)-O-C₁₋₆-alkyl; N(H)-C(=O)-C₁₋₆-alkyl, and N(C₁₋₆-alkyl)-C(=O)-C₁₋₆-alkyl; wherein said C₃₋₆-cycloalkyl residue is optionally connected via a C₁₋₈-alkylene group, which in turn may be branched or unbranched and unsubstituted or substituted with 1, 2, 3, 4 or 5 substituents independently selected from the group consisting of F, Cl, CF₃, =O, OCF₃, OH, O-C₁₋₆-alkyl and C₁₋₈-alkylen-OH; or 3 to 7 membered heterocyclyl, which is unsubstituted or substituted with 1, 2, 3, 4 or 5 substituents independently selected from the group consisting of F, Cl, CF₃, =O, OCF₃; C₁₋₈-alkylen-OH, C₁₋₆-alkyl, OH, O-C₁₋₆-alkyl, S(=O)-C₁₋₆-alkyl, S(=O)₂-C₁₋₆-alkyl, N(H)-S(=O)-C₁₋₆-alkyl, N(C₁₋₆-alkyl)-S(=O)-C₁₋₆-alkyl, N(H)-S(=O)₂-C₁₋₆-alkyl, N(C₁₋₆-alkyl)-S(=O)₂-C₁₋₆-alkyl, C(=O)-NH₂, C(=O)-N(H)(C₁₋₆-alkyl), C(=O)-N(C₁₋₆-alkyl)₂, C(=O)-O-C₁₋₆-alkyl; N(H)-C(=O)-C₁₋₆-alkyl, and N(C₁₋₆-alkyl)-C(=O)-C₁₋₆-alkyl, wherein said 3 to 7membered heterocyclyl is optionally connected via a C₁₋₈-alkylene group, which in turn may be branched or unbranched and unsubstituted or substituted with 1, 2, 3, 4 or 5 substituents independently selected from the group consisting of F, Cl, CF₃, =O, OCF₃, OH, O-C₁₋₆-alkyl and C₁₋₈-alkylen-OH.

More preferably,
R⁴ represents represents H or
C₁₋₆-alkyl, branched or unbranched and unsubstituted or substituted with 1, 2, 3, 4 or 5 substituents independently selected from the group consisting of OH, =O, O-C₁₋₆-alkyl, S(=O)-C₁₋₆-alkyl, S(=O)₂-C₁₋₆-alkyl, N(H)-S(=O)-C₁₋₆-alkyl, N(C₁₋₆-alkyl)-S(=O)-C₁₋₆-alkyl, N(H)-S(=O)₂-C₁₋₆-alkyl, N(C₁₋₆-alkyl)-S(=O)₂-C₁₋₆-alkyl, C(=O)-NH₂, C(=O)-N(H)(C₁₋₆-alkyl), C(=O)-N(C₁₋₆-alkyl)₂, C(=O)-O-C₁₋₆-alkyl; N(H)-C(=O)-C₁₋₆-alkyl, and N(C₁₋₆-alkyl)-C(=O)-C₁₋₆-alkyl.

Still more preferably, R⁴ represents represents H or C₁₋₆-alkyl. Particularly preferred, R⁴ represents represents H, methyl, ethyl, iso-propyl or n-propyl.

In a preferred embodiment of the first aspect of the present invention, R⁴ denotes methyl. In another preferred embodiment of the first aspect of the present invention, R⁴ represents H.

In another embodiment of the first aspect of the invention, the compound according to general formula (I) is characterized in that
R⁵ represents
H or
C₁₋₆-alkyl, branched or unbranched and unsubstituted or substituted with 1, 2, 3, 4 or 5 substituents independently selected from the group consisting of F, Cl, CF₃, CN, OH, =O, OCF₃, O-C₁₋₆-alkyl, O-(C=O)C₁₋₆-alkyl, S(=O)-C₁₋₆-alkyl, S(=O)₂-C₁₋₆-alkyl, S(=O)₂-NH₂, S(=O)₂-N(H)C₁₋₆-alkyl, S(=O)₂-N(C₁₋₆-alkyl)₂, NH₂, NH(C₁₋₆-alkyl), N(C₁₋₆-alkyl)₂, N(H)-S(=O)-C₁₋₆-alkyl, N(C₁₋₆-alkyl)-S(=O)-C₁₋₆-alkyl, N(H)-S(=O)₂-C₁₋₆-alkyl, N(C₁₋₆-alkyl)-S(=O)₂-C₁₋₆-alkyl, N(H)-C(=O)-NH₂, N(H)-C(=O)-N(H)(C₁₋₆-alkyl), N(H)-C(=O)-N(C₁₋₆-alkyl)₂, N(H)-C(=O)-O-C₁₋₆-alkyl; O-C(=O)-NH₂, O-C(=O)-N(H)(C₁₋₆-alkyl), O-C(=O)-N(C₁₋₆-alkyl)₂, C(=O)-NH₂, C(=O)-N(H)(C₁₋₆-alkyl), C(=O)-N(C₁₋₆-alkyl)₂, C(=O)-O-C₁₋₆-alkyl; N(H)-C(=O)-C₁₋₆-alkyl, and N(C₁₋₆-alkyl)-C(=O)-C₁₋₆-alkyl; or
C₃₋₆-cycloalkyl, unsubstituted or substituted with 1, 2, 3, 4 or 5 substituents independently selected from the group consisting of F, Cl, CN, CF₃, =O, OCF₃, C₁₋₈-alkylen-OH, C₁₋₆-alkyl, OH, O-C₁₋₆-alkyl, O-(C=O)C₁₋₆-alkyl, S(=O)-C₁₋₆-alkyl, S(=O)₂-C₁₋₆-alkyl, S(=O)₂-NH₂, S(=O)₂-N(H)C₁₋₆-alkyl, S(=O)₂-N(C₁₋₆-alkyl)₂, NH₂, NH(C₁₋₆-alkyl), N(C₁₋₆-alkyl)₂, N(H)-S(=O)-C₁₋₆-alkyl, N(C₁₋₆-alkyl)-S(=O)-C₁₋₆-alkyl, N(H)-S(=O)₂-C₁₋₆-alkyl, N(C₁₋₆-alkyl)-S(=O)₂-C₁₋₆-alkyl, N(H)-C(=O)-O-C₁₋₆-alkyl; O-C(=O)-NH₂, O-C(=O)-N(H)(C₁₋₆-alkyl), O-C(=O)-N(C₁₋₆-alkyl)₂, N(H)-C(=O)-NH₂, N(H)-C(=O)-N(H)(C₁₋₆-alkyl), N(H)-C(=O)-N(C₁₋₆-alkyl)₂, C(=O)-NH₂, C(=O)-N(H)(C₁₋₆-alkyl), C(=O)-N(C₁₋₆-alkyl)₂, C(=O)-O-C₁₋₆-alkyl; N(H)-C(=O)-C₁₋₆-alkyl, and N(C₁₋₆-alkyl)-C(=O)-C₁₋₆-alkyl; wherein said C₃₋₆-cycloalkyl is optionally connected via a C₁₋₈-alkylene group, which in turn may be branched or unbranched and unsubstituted or substituted with 1, 2, 3, 4 or 5 substituents independently selected from the group consisting of F, Cl, CF₃, =O, OCF₃, OH, O-C₁₋₆-alkyl and C₁₋₈-alkylen-OH; or
3 to 7 membered heterocyclyl, which is unsubstituted or substituted with 1, 2, 3, 4 or 5 substituents independently from one another selected from the group consisting of F, Cl, CN, CF₃, =O, OCF₃, C₁₋₆-alkylen-OH, C₁₋₆-alkyl, OH, O-C₁₋₆-alkyl, O-(C=O)C₁₋₆-alkyl, S(=O)-C₁₋₆-alkyl, S(=O)₂-C₁₋₆-alkyl, S(=O)₂-NH₂, S(=O)₂-N(H)C₁₋₆-alkyl, S(=O)₂-N(C₁₋₆-alkyl)₂, NH₂, NH(C₁₋₆-alkyl), N(C₁₋₆-alkyl)₂, N(H)-S(=O)-C₁₋₆-alkyl, N(C₁₋₆-alkyl)-S(=O)-C₁₋₆-alkyl, N(H)-S(=O)₂-C₁₋₆-alkyl, N(C₁₋₆-alkyl)-S(=O)₂-C₁₋₆-alkyl, N(H)-C(=O)-O-C₁₋₆-alkyl; O-C(=O)-NH₂, O-C(=O)-N(H)(C₁₋₆-alkyl), O-C(=O)-N(C₁₋₆-alkyl)₂, N(H)-C(=O)-NH₂, N(H)-C(=O)-N(H)(C₁₋₆-alkyl), N(H)-C(=O)-N(C₁₋₆-alkyl)₂, (C=O)C₁₋₆-alkyl, C(=O)-NH₂, C(=O)-N(H)(C₁₋₆-alkyl), C(=O)-N(C₁₋₆-alkyl)₂, C(=O)-O-C₁₋₆-alkyl; N(H)-C(=O)-C₁₋₆-alkyl, and N(C₁₋₆-alkyl)-C(=O)-C₁₋₆-alkyl; wherein said 3 to 7 membered heterocyclyl is optionally connected via a C₁₋₈-alkylene group, which in turn may be branched or unbranched and unsubstituted or substituted with 1, 2, 3, 4 or 5 substituents independently selected from the group consisting of F, Cl, CF₃, =O, OCF₃, OH, O-C₁₋₆-alkyl and C₁₋₈-alkylen-OH; or
aryl or heteroaryl, which is unsubstituted or substituted with 1, 2, 3, 4 or 5 substituents selected from the group consisting of F, Cl, CN, CF₃, OCF₃, C₁₋₈-alkylen-OH, C₁₋₆-alkyl, OH, O-C₁₋₆-alkyl, O-(C=O)C₁₋₆-alkyl, S(=O)-C₁₋₆-alkyl, S(=O)₂-C₁₋₆-alkyl, S(=O)₂-NH₂, S(=O)₂-N(H)C₁₋₆-alkyl, S(=O)₂-N(C₁₋₆-alkyl)₂, NH₂, NH(C₁₋₆-alkyl), N(C₁₋₆-alkyl)₂, N(H)-S(=O)-C₁₋₆-alkyl, N(C₁₋₆-alkyl)-S(=O)-C₁₋₆-alkyl, N(H)-S(=O)₂-C₁₋₆-alkyl, N(C₁₋₆-alkyl)-S(=O)₂-C₁₋₆-alkyl, N(H)-C(=O)-O-C₁₋₆-alkyl; O-C(=O)-NH₂, O-C(=O)-N(H)(C₁₋₆-alkyl), O-C(=O)-N(C₁₋₆-alkyl)₂, N(H)-C(=O)-NH₂, N(H)-C(=O)-N(H)(C₁₋₆-alkyl), N(H)-C(=O)-N(C₁₋₆-alkyl)₂, C(=O)-NH₂, C(=O)-N(H)(C₁₋₆-alkyl), C(=O)-N(C₁₋₆-alkyl)₂, C(=O)-O-C₁₋₆-alkyl; N(H)-C(=O)-C₁₋₆-alkyl, and N(C₁₋₆-alkyl)-C(=O)-C₁₋₆-alkyl, wherein said aryl or heteroaryl is optionally connected via a C₁₋₈-alkylene group, which in turn may be branched or unbranched and unsubstituted or substituted with 1, 2, 3, 4 or 5 substituents independently from one another selected from the group consisting of F, Cl, CF₃, =O, OCF₃, OH, O-C₁₋₆-alkyl and C₁₋₈-alkylen-OH.

In another preferred embodiment of the first aspect of the invention, the compound according to general formula (I) is characterized in that R⁵ represents
C₃₋₆-cycloalkyl, which is unsubstituted or substituted with 1, 2, 3, 4 or 5 substituents independently from one another selected from the group consisting of F, Cl, CF₃, =O, OCF₃, OH, O-C₁₋₆-alkyl, C₁₋₈-alkylen-OH and C₁₋₆-alkyl; or
3 to 7 membered heterocyclyl, which contains 1 or 2 heteroatoms or heteroatom groups independently from one another selected from the group consisting of O, S, S(=O), S(=O)₂, NH and N-C₁₋₆-alkyl, and which is unsubstituted or substituted with 1, 2, 3, 4 or 5 substituents independently from one another selected from the group consisting of F, Cl, CF₃, OCF₃, CN, =O, C₁₋₆-alkyl, C₁₋₈-alkylen-OH and O-C₁₋₆-alkyl; or
aryl or heteroaryl, which contains at least one nitrogen atom, and wherein said aryl or heteroaryl is unsubstituted or substituted with 1, 2 or 3 substituents independently from one another selected from the group consisting of F, Cl, CN, CF₃, OCF₃, C₁₋₈-alkylen-OH, C₁₋₆-alkyl, OH, O-C₁₋₆-alkyl, S(=O)₂-C₁₋₆-alkyl, S(=O)₂-NH₂. NH₂, NH(C₁₋₆-alkyl), N(C₁₋₆-alkyl)₂, O-C(=O)-NH₂, C(=O)-NH₂, C(=O)-N(H)(C₁₋₆-alkyl), C(=O)-N(C₁₋₆-alkyl)₂, C(=O)-O-C₁₋₆-alkyl; or
a part structure of general formula SF-III 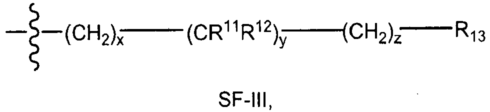 wherein
x represents 0, 1 or 2; y represents 0, 1 or 2; z represents 0, 1 or 2;
   on the condition that the sum of x, y and z is 1, 2, 3, 4, 5 or 6;
R¹¹ and R¹² are independently from one another selected from H or C₁₋₆-alkyl; or
R¹¹ and R¹² together with the carbon atom connecting them form a C₃₋₆-cycloalkyl or a 3 to 7 membered heterocyclyl, which contains 1 or 2 heteroatoms or heteroatom groups independently from one another selected from the group consisting of O, S, S(=O), S(=O)₂, NH and N-C₁₋₆-alkyl, wherein said C₃₋₆-cycloalkyl or 3 to 7 membered heterocyclyl may be unsubstituted or substituted with 1, 2, 3, 4 or 5 substituents independently from one another selected from the group consisting of F, Cl, CF₃, OCF₃, CN, C₁₋₆-alkyl and O-C₁₋₆-alkyl;
R¹³ is selected from the group consisting of
H, F, Cl, CN, OH, O-C₁₋₆-alkyl, O-(C=O)C₁₋₆-alkyl, S(=O)-C₁₋₆-alkyl, S(=O)₂-C₁₋₆-alkyl, S(=O)₂-NH₂, S(=O)₂-N(H)C₁₋₆-alkyl, S(=O)₂-N(C₁₋₆-alkyl)₂, NH₂, NH(C₁₋₆-alkyl), N(C₁₋₆-alkyl)₂, N(H)-C(=O)-C₁₋₆-alkyl, N(C₁₋₆-alkyl)-C(=O)-C₁₋₆-alkyl, N(H)-S(=O)-C₁₋₆-alkyl, N(C₁₋₆-alkyl)-S(=O)-C₁₋₆-alkyl, N(H)-S(=O)₂-C₁₋₆-alkyl, N(C₁₋₆-alkyl)-S(=O)₂-C₁₋₆-alkyl, C(=O)-NH₂, C(=O)-N(H)(C₁₋₆-alkyl), C(=O)-N(C₁₋₆-alkyl)₂, C(=O)-O-C₁₋₆-alkyl, N(H)-C(=O)-NH₂, N(H)-C(=O)-N(H)(C₁₋₆-alkyl), N(H)-C(=O)-N(C₁₋₆-alkyl)₂, N(H)-C(=O)-O-C₁₋₆-alkyl; O-C(=O)-NH₂, O-C(=O)-N(H)(C₁₋₆-alkyl), O-C(=O)-N(C₁₋₆-alkyl)₂;
or represents
C₃₋₆-cycloalkyl, which is unsubstituted or substituted with 1, 2, 3, 4, or 5 substituents independently from one another selected from the group consisting of F, Cl, CF₃, =O, OCF₃, OH, O-C₁₋₆-alkyl, C₁₋₆-alkylen-OH and C₁₋₆-alkyl; or
3-7-membered heterocyclyl, which contains 1 or 2 heteroatoms or heteroatom groups independently from one another selected from the group consisting of O, S, S(=O), S(=O)₂, NH and N-C₁₋₆-alkyl, and which is unsubstituted or substituted with 1, 2, 3, 4 or 5 substituents independently from one another selected from the group consisting of F, Cl, CF₃, OCF₃, CN, C₁₋₆-alkyl and O-C₁₋₆-alkyl; or
aryl or heteroaryl, which contains at least one nitrogen atom and wherein said aryl or heteroaryl is unsubstituted or substituted with 1, 2 or 3 substituents independently from one another selected from the group consisting of F, Cl, CN, CF₃, OCF₃, C₁₋₆-alkylen-OH, C₁₋₆-alkyl, OH, O-C₁₋₆-alkyl, S(=O)₂-C₁₋₆-alkyl, S(=O)₂-NH₂. NH₂, NH(C₁₋₆-alkyl), N(C₁₋₆-alkyl)₂, O-C(=O)-NH₂, C(=O)-NH₂, C(=O)-N(H)(C₁₋₆-alkyl), C(=O)-N(C₁₋₆-alkyl)₂, C(=O)-O-C₁₋₆-alkyl.

Preferred heteroaryl residues, which contain at least one nitrogen atom, are selected from pyridine, pyrimidine, pyrazine, pyridazine, triazine, quinoline, isoquinoline, phthalazine, naphtheridine, quinoxaline, quinazoline, indole, isoindole, pyrrole, imidazole, pyrazole, 1,2,3-triazole, 1,2,4-triazole, oxazole, isoxazole, thiazole, isothiazole, oxadiazole and thiadiazole. Preferred aryl residue is phenyl.

More preferably, R⁵ represents a part structure of general formula SF-III,
wherein R¹³ is selected from the group consisting of
H, F, Cl, CN, CF₃, OCF₃, O-C₁₋₆-alkylen-OH, C₁₋₈-alkylen-OH, C₁₋₆-alkyl, OH, O-C₁₋₆-alkyl, S(=O)-C₁₋₆-alkyl, S(=O)₂-C₁₋₆-alkyl, S(=O)₂-NH₂. S(=O)₂-N(H)C₁₋₆-alkyl, S(=O)₂-N(C₁₋₆-alkyl)₂, NH₂, NH(C₁₋₆-alkyl), N(C₁₋₆-alkyl)₂, N(H)-S(=O)-C₁₋₆-alkyl, N(C₁₋₆-alkyl)-S(=O)-C₁₋₆-alkyl, N(H)-S(=O)₂-C₁₋₆-alkyl, N(C₁₋₆-alkyl)-S(=O)₂-C₁₋₆-alkyl, N(H)-C(=O)-NH₂, N(H)-C(=O)-N(H)(C₁₋₆-alkyl), N(H)-C(=O)-N(C₁₋₆-alkyl)₂, C(=O)-NH₂, C(=O)-N(H)(C₁₋₆-alkyl), C(=O)-N(C₁₋₆-alkyl)₂, C(=O)-O-C₁₋₆-alkyl; N(H)-C(=O)-C₁₋₆-alkyl and N(C₁₋₆-alkyl)-C(=O)-C₁₋₆-alkyl.

Still more preferably, R⁵ represents a part structure of general formula SF-III,
wherein R¹³ is selected from the group consisting of H, OH, F, Cl, CN, S(=O)₂-C₁₋₆-alkyl, NH₂, N(H)-C(=O)-C₁₋₆-alkyl, N(H)-S(=O)₂-C₁₋₆-alkyl, O-C₁₋₆-alkyl, C(=O)-NH₂, C(=O)-N(H)(C₁₋₆-alkyl) and C(=O)-O-C₁₋₆-alkyl.

In a preferred embodiment of the invention, the general formula SF-III is selected from formulae SF-IIIa to SF-IIIo 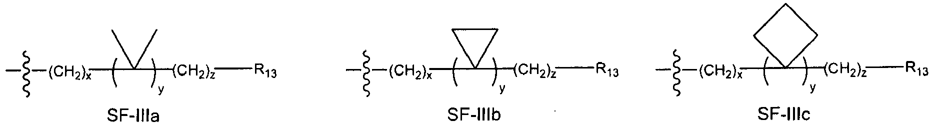 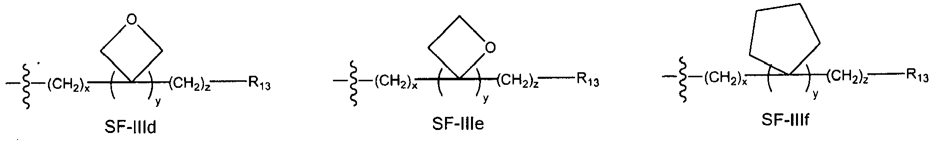 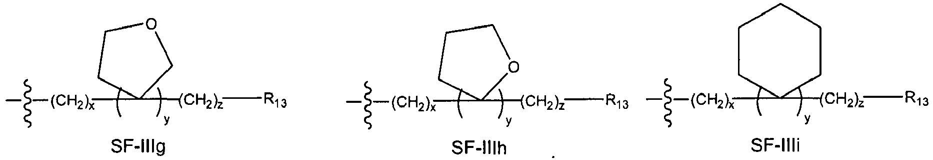 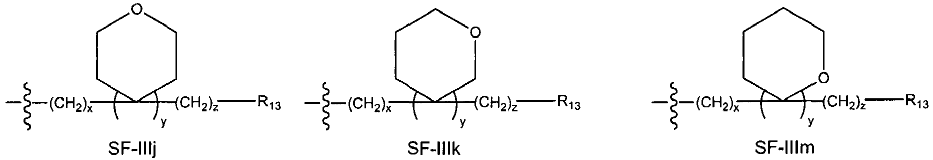 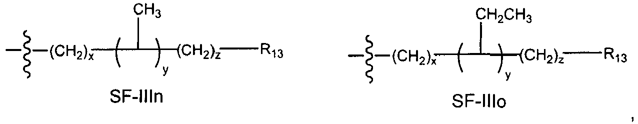 wherein
x represents 0, 1 or 2; y represents 0 or 1; z represents 0, 1 or 2; on the condition that the sum of x, y and z is 1, 2, 3, 4, 5 or 6.

Preferred are compounds according formula (I), that are characterized that R⁵ is represented by any part structure the general formulae SF-IIIa to SF-IIIo, wherein
x represents 1, y respresents 0 and z represents 0.

Also preferred are compounds according formula (I), that are characterized that R⁵ is represented by any part structure the general formulae SF-IIIa to SF-IIIo, wherein x represents 1, y respresents 1 and z represents 0.

Also preferred are compounds according formula (I), that are characterized that R⁵ is represented by any part structure the general formulae SF-IIIa to SF-IIIo, wherein x represents 0, y respresents 1 and z represents 0.

Also preferred are compounds according formula (I), that are characterized that R⁵ is represented by any part structure the general formulae SF-IIIa to SF-IIIo, wherein x represents 0, y respresents 1 and z represents 1.

In a particularity preferred embodiment of the first aspect of the invention, the compound according to general formula (I) is characterized in that R⁵ is selected from the group consisting of
methyl, ethyl, 2-propyl (iso-propyl), 1-propyl (n-propyl), 1-butyl, 2-butyl, 2-methyl-propyl, 1,1-dimethyl-ethyl (tert-butyl), 1-pentyl, 2-pentyl, 3-pentyl, 2-methyl-butyl, 2,2-dimethyl-propyl (neo-pentyl), 1-hexyl, 2-hexyl, 3-hexyl, 3,3-dimethyl-butyl, cyclopropyl, cyclopropylmethyl, 2-cyclopropyl-ethyl, 1-cyclopropyl-ethyl as well as 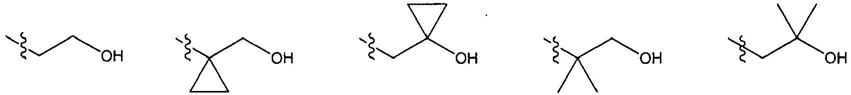 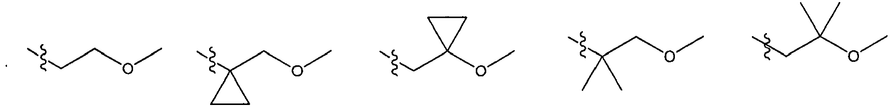 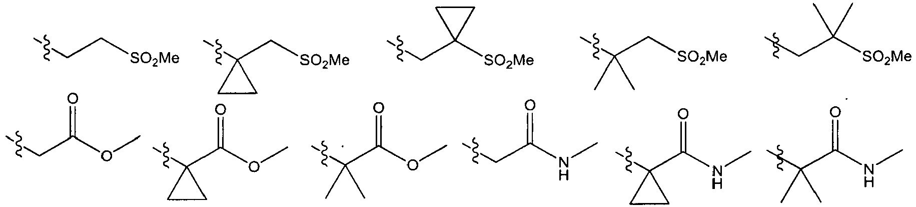 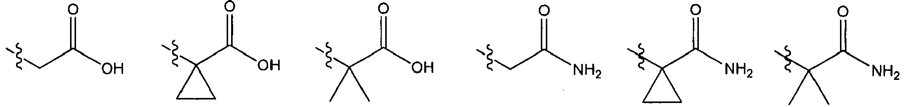 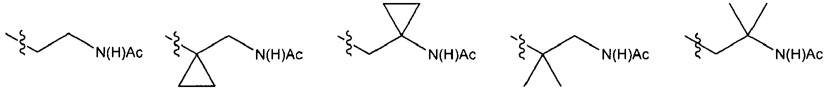 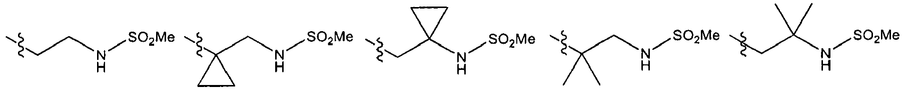 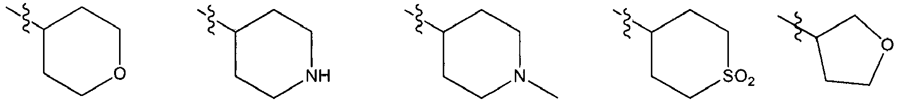 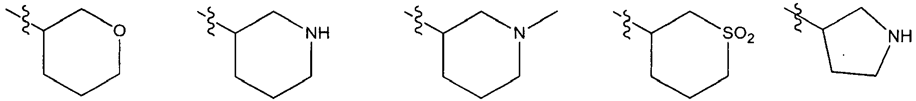 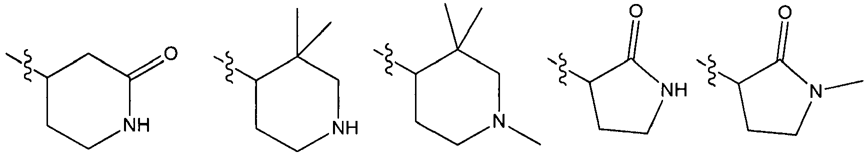 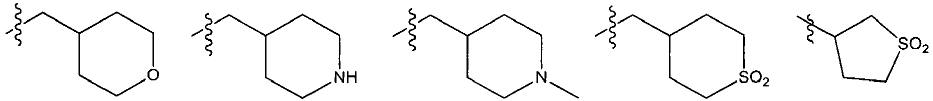 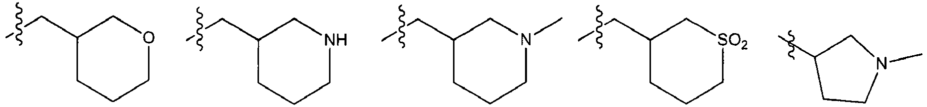 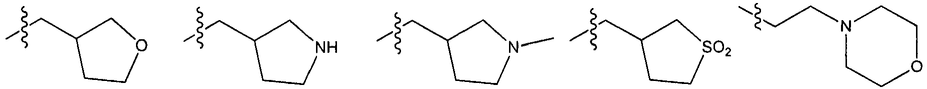 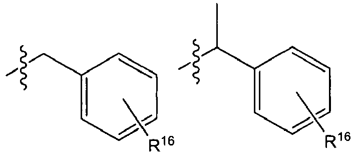 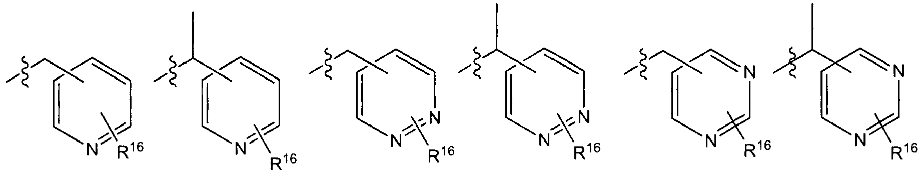 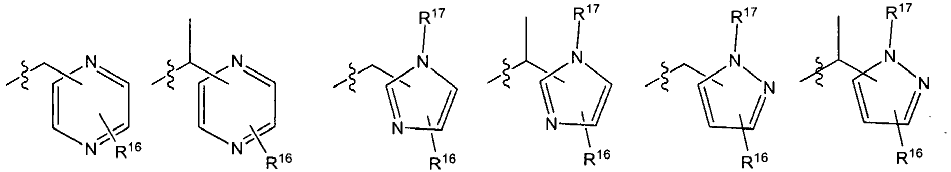 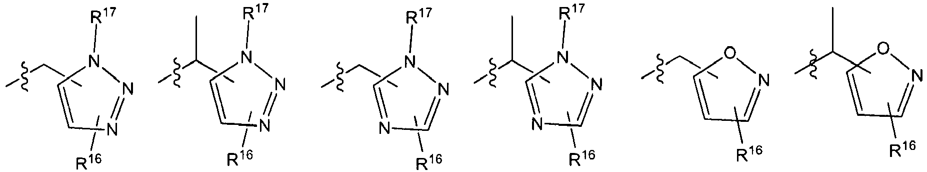 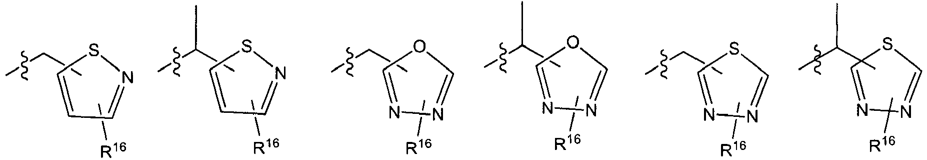 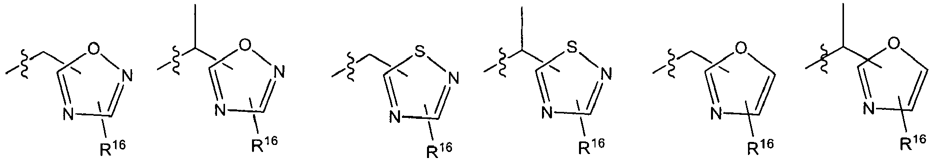 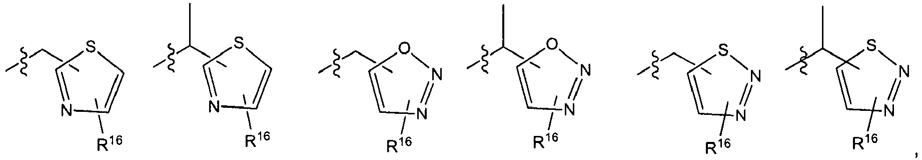 wherein
R¹⁶ represents 1, 2 or 3 substituents, selected from C₁₋₆-alkyl, CF₃, F, Cl, CN, OH, OCF₃, O-C₁₋₆-alkyl, NH₂, N(H)C₁₋₆-alkyl, N(C₁₋₆-alkyl)₂, 1-pyrrolidinyl, 1-piperidinyl, 4-methyl-piperidin-1-yl or 1-morpholinyl,
and R¹⁷ represents H or C₁₋₆-alkyl.

In a particularly preferred embodiment, R⁴ represents H or C₁₋₆-alkyl or benzyl and
R⁵ represents
C₃₋₆-cycloalkyl, which is unsubstituted or substituted with 1, 2, 3, 4 or 5 substituents independently from one another selected from the group consisting of F, Cl, CF₃, =O, OCF₃, OH, O-C₁₋₆-alkyl, C₁₋₈-alkylen-OH and C₁₋₆-alkyl; or
3 to 7 membered heterocyclyl, which contains 1 or 2 heteroatoms or heteroatom groups independently from one another selected from the group consisting of O, S, S(=O), S(=O)₂, NH and N-C₁₋₆-alkyl, and which is unsubstituted or substituted with 1, 2, 3, 4 or 5 substituents independently from one another selected from the group consisting of F, Cl, CF₃, OCF₃, CN, C₁₋₆-alkyl, C₁₋₈-alkylen-OH and O-C₁₋₆-alkyl; or
aryl or heteroaryl, which contains at least one nitrogen atom, and wherein said aryl or heteroaryl is unsubstituted or substituted with 1, 2 or 3 substituents independently from one another selected from the group consisting of F, Cl, CN, CF₃, OCF₃, C₁₋₈-alkylen-OH, C₁₋₆-alkyl, OH, O-C₁₋₆-alkyl, S(=O)₂-C₁₋₆-alkyl, S(=O)₂-NH₂. NH₂, NH(C₁₋₆-alkyl), N(C₁₋₆-alkyl)₂, O-C(=O)-NH₂, C(=O)-NH₂, C(=O)-N(H)(C₁₋₆-alkyl), C(=O)-N(C₁₋₆-alkyl)₂, C(=O)-O-C₁₋₆-alkyl; or
a part structure of general formula SF-III 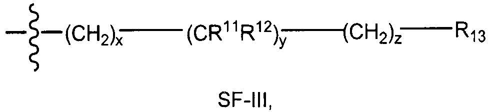 wherein
x represents 0, 1 or 2; y represents 0, 1 or 2; z represents 0, 1 or 2; on the condition that the sum of x, y and z is 1, 2, 3, 4, 5 or 6;
R¹¹ and R¹² are independently from one another selected from H or C₁₋₆-alkyl; or
R¹¹ and R¹² together with the carbon atom connecting them form a C₃₋₆-cycloalkyl or a 3 to 7 membered heterocyclyl, which contains 1 or 2 heteroatoms or heteroatom groups independently from one another selected from the group consisting of O, S, S(=O), S(=O)₂, NH and N-C₁₋₆-alkyl, wherein said C₃₋₆-cycloalkyl or 3 to 7 membered heterocyclyl may be unsubstituted or substituted with 1, 2, 3, 4 or 5 substituents independently from one another selected from the group consisting of F, Cl, CF₃, OCF₃, CN, =O, C₁₋₆-alkyl and O-C₁₋₆-alkyl;
R¹³ is selected from the group consisting of
H, F, Cl, CN, CF₃, OCF₃, C₁₋₈-alkylen-OH, C₁₋₆-alkyl, OH, O-C₁₋₆-alkyl, S(=O)-C₁₋₆-alkyl, S(=O)₂-C₁₋₆-alkyl, S(=O)₂-NH₂, S(=O)₂-N(H)C₁₋₆-alkyl, S(=O)₂-N(C₁₋₆-alkyl)₂, NH₂, NH(C₁₋₆-alkyl), N(C₁₋₆-alkyl)₂, N(H)-S(=O)-C₁₋₆-alkyl, N(C₁₋₆-alkyl)-S(=O)-C₁₋₆-alkyl, N(H)-S(=O)₂-C₁₋₆-alkyl, N(C₁₋₆-alkyl)-S(=O)₂-C₁₋₆-alkyl, N(H)-C(=O)-NH₂, N(H)-C(=O)-N(H)(C₁₋₆-alkyl), N(H)-C(=O)-N(C₁₋₆-alkyl)₂, C(=O)-NH₂, C(=O)-N(H)(C₁₋₆-alkyl), C(=O)-N(C₁₋₆-alkyl)₂, C(=O)-O-C₁₋₆-alkyl; N(H)-C(=O)-C₁₋₆-alkyl and N(C₁₋₆-alkyl)-C(=O)-C₁₋₆-alkyl,
or represents
C₃₋₆-cycloalkyl, which is unsubstituted or substituted with 1, 2, 3, 4, or 5 substituents independently from one another selected from the group consisting of F, Cl, CF₃, =O, OCF₃, OH, O-C₁₋₆-alkyl, C₁₋₈-alkylen-OH and C₁₋₆-alkyl; or
3 to 7 membered heterocyclyl, which contains 1 or 2 heteroatoms or heteroatom groups independently from one another selected from the group consisting of O, S, S(=O), S(=O)₂, NH and N-C₁₋₆-alkyl, and which is unsubstituted or substituted with 1, 2, 3, 4 or 5 substituents independently from one another selected from the group consisting of F, Cl, CF₃, OCF₃, CN, C₁₋₆-alkyl and O-C₁₋₆-alkyl; or
aryl or heteroaryl, which contains at least one nitrogen atom, and wherein said aryl or heteroaryl is unsubstituted or substituted with 1, 2 or 3 substituents independently from one another selected from the group consisting of F, Cl, CN, CF₃, OCF₃, C₁₋₈-alkylen-OH, C₁₋₆₋alkyl, OH, O-C₁₋₆-alkyl, S(=O)₂-C₁₋₆-alkyl, S(=O)₂-NH₂, NH₂, NH(C₁₋₆-alkyl), N(C₁₋₆-alkyl)₂, O-C(=O)-NH₂, C(=O)-NH₂, C(=O)-N(H)(C₁₋₆-alkyl), C(=O)-N(C₁₋₆-alkyl)₂, C(=O)-O-C₁₋₆-alkyl.

In another embodiment of the first aspect of the invention, the compound according to general formula (I) is characterized in that R⁴ and R⁵ together with the nitrogen atom connecting them form a 3 to 7 membered heterocyclyl, unsubstituted or substituted with 1, 2, 3, 4 or 5 substituents selected from the group consisting of F, Cl, CN, CF₃, =O, OH, C₁₋₆-alkyl, O-C₁₋₆-alkyl, C₁₋₆-alkylen-OH, OCF₃, SO₂(C₁₋₆-alkyl), SO₂NH₂, SO₂N(H)C₁₋₆-alkyl, SO₂N(C₁₋₆-alkyl)₂, C₁₋₆-alkylen-SO₂(C₁₋₆-alkyl), NH₂, NH(C₁₋₆-alkyl), N(C₁₋₆-alkyl)₂, (C=O)C₁₋₆-alkyl, C₃₋₆-cycloalkyl or 3 to 7 membered heterocyclyl, aryl, heteroaryl, O-aryl and O-heteroaryl, in each case unsubstituted or mono- or polysubstituted.

Preferably, R⁴ and R⁵ together with the nitrogen atom connecting them form a heterocyclyl selected from the group consisting of 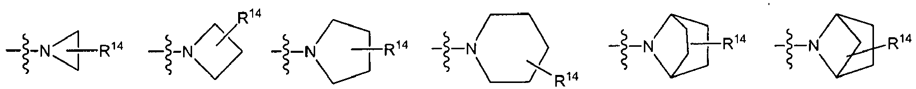 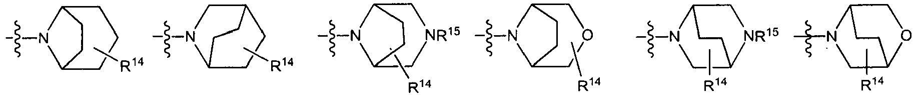 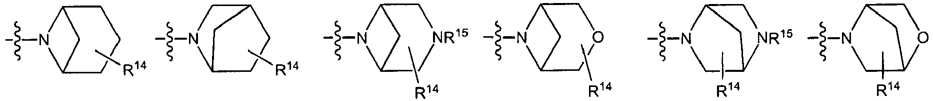 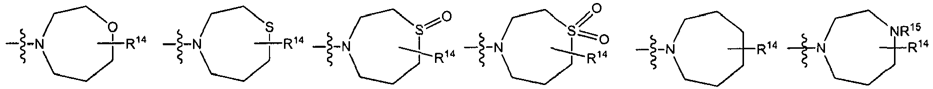 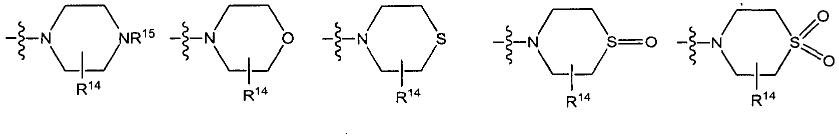 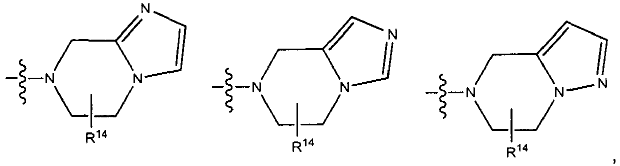 wherein
R¹⁴ denotes 0, 1, 2, 3 or 4 substituents which are in each case independently of each other selected from the group consisting of F, Cl, CF₃, =O, OCF₃, OH, O-C₁₋₆-alkyl, C₁₋₈-alkylen-OH, SO₂(C₁₋₆-alkyl), C₁₋₈-alkylen-SO₂(C₁₋₆-alkyl), C₁₋₆-alkyl, aryl, heteroaryl, O-aryl and O-heteroaryl,
wherein said aryl or said heteroaryl is unsubstituted or substituted with 1, 2, 3, 4 or 5 substituents independently from one another selected from the group consisting of F, Cl, CN, CF₃, OCF₃, C₁₋₆-alkylen-OH, C₁₋₆-alkyl, OH, O-C₁₋₆-alkyl, NH₂, NH(C₁₋₆-alkyl), N(C₁₋₆-alkyl)₂, N(H)-S(=O)₂-C₁₋₆-alkyl, C(=O)-NH₂ or C(=O)-N(H)(C₁₋₆-alkyl), C(=O)-N(C₁₋₆-alkyl)₂; or
R¹⁴ denotes at least two substituents, wherein two substituents R¹⁴ stand together for a C₁₋₈-alkylen-group, substituted or unsubstituted, wherein optionally one or more C-atoms of the C₁₋₈-alkylen-group is replaced by a heteroatom or heteroatom group, selected of O, N-R¹⁵, S, S(O) and S(O)₂, and wherein these two substituents R¹⁴ are positioned at different carbon atoms of the heterocyclyl residue, so the C₁₋₈-alkylen-group represents a bridge to form a bicyclic heterocyclyl residue; or
R¹⁴ denotes at least two substituents, wherein two substituents R¹⁴ stand together for a C₂₋₆-alkylen-group, substituted or unsubstituted, wherein optionally one or more C-atoms of the C₂₋₆-alkylen-group is replaced by a heteroatom or heteroatom group, selected from O, N-R¹⁵, S, S(O) and S(O)₂, and wherein these two substituents R¹⁴ are positioned at the same carbon atom of the heterocyclyl residue, so the C₂₋₆-alkylen-group forms a spiro-heterocyclyl residue; and
R¹⁵ represents H, C₁₋₆-alkyl, (C=O)C₁₋₆-alkyl, (C=O)NH₂, (C=O)NH(C₁₋₆-alkyl) or (C=O)N(C₁₋₆-alkyl)₂.

More preferably,
R⁴ and R⁵ together with the nitrogen atom connecting them form a heterocycloaliphatic residue selected from the group consisting of 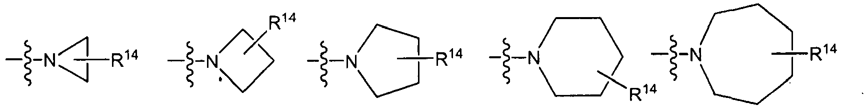 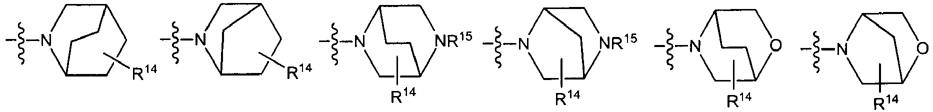 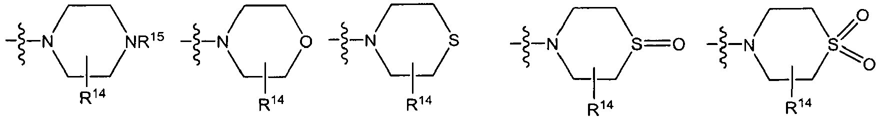 wherein
R¹⁴ denotes 0, 1, 2, 3 or 4 substituents which are in each case independently of each other selected from the group consisting of F, Cl, CF₃, =O, OCF₃, OH, O-C₁₋₆-alkyl, C₁₋₆-alkylen-OH, C₁₋₆-alkylen-SO₂(C₁₋₆-alkyl), SO₂(C₁₋₆-alkyl), C₁₋₆-alkyl, aryl, heteroaryl, O-aryl and O-heteroaryl, wherein said aryl or said heteroaryl is unsubstituted or substituted with 1, 2 or 3 substituents independently from one another selected from the group consisting of F, Cl, CN, CF₃, OCF₃, C₁₋₆-alkylen-OH, C₁₋₆₋alkyl, OH or O-C₁₋₆-alkyl;
   or
R¹⁴ denotes at least two substituents, wherein two substituents R¹⁴ stand together for a C₁₋₆-alkylen-group, substituted or unsubstituted, wherein optionally one or more C-atoms of the C₁₋₆-alkylen-group is replaced by a heteroatom or heteroatom group, selected of O, N-R¹⁵, S, S(O) and S(O)₂, and
wherein these two substituents R¹⁴ are positioned at different carbon atoms of the heterocyclyl, so the C₁₋₆-alkylen-group represents a bridge to form a bicyclic heterocyclyl;
   or
R¹⁴ denotes at least two substituents, wherein two substituents R¹⁴ stand together for a C₂₋₆-alkylen-group, substituted or unsubstituted, wherein optionally one or more C-atoms of the C₂₋₆-alkylen-group is replaced by a heteroatom or heteroatom group, selected of O, N-R¹⁵, S, S(O) and S(O)₂, and
wherein these two substituents R¹⁴ are positioned at the same carbon atom of the heterocyclyl, so the C₂₋₆-alkylen-group forms a spiro-heterocyclyl; and
R¹⁵ represents H, C₁₋₆-alkyl, (C=O)C₁₋₆-alkyl, (C=O)NH₂, (C=O)NH(C₁₋₆-alkyl) or (C=O)N(C₁₋₆-alkyl)₂.

Most preferred,
R⁴ and R⁵ together with the nitrogen atom connecting them form a heterocycloaliphatic residue selected from the group consisting of

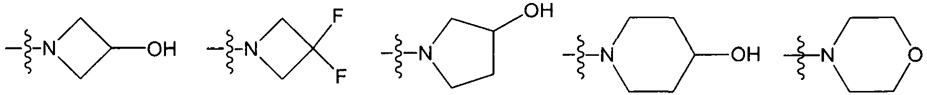

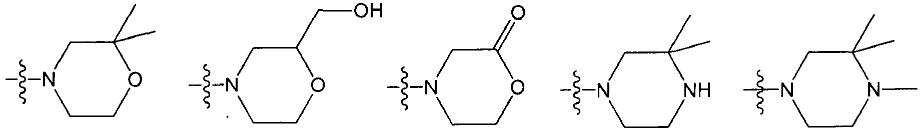

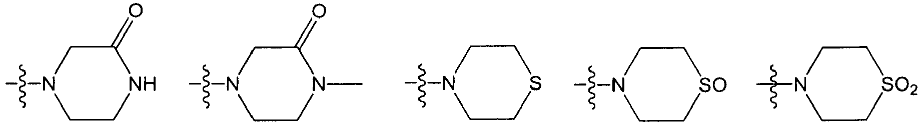

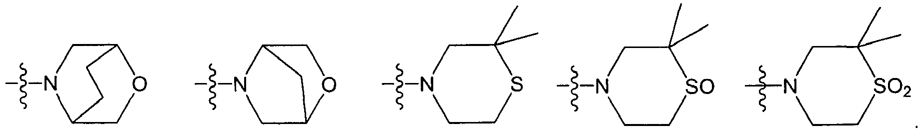

In another embodiment of the first aspect of the invention, the compound according to general formula (I) is characterized in that the compound of general formula (I) is a compound according to general formula (Ia) or (Ib),
wherein
n is 0 or 1,
R¹ represents
C₁₋₆-alkyl, C₂₋₆-alkenyl or C₂₋₆-alkynyl,
in each case branched or unbranched, and in each case unsubstituted or mono- or polysubstituted by one or more substituents selected from
F; Cl; CN; CF₃; C(=O)-NH₂; C(=O)-N(H)(C₁₋₆-alkyl); C(=O)-N(C₁₋₆-alkyl)₂; OH; O-C₁₋₆-alkyl; NH₂; N(H)(C₁₋₆-alkyl); N(C₁₋₆-alkyl)₂; N(H)-C(=O)-C₁₋₆-alkyl; S(=O)-C₁₋₆-alkyl; S(=O)₂-C₁₋₆-alkyl or cyclopropyl,
or R⁷ represents
cyclopropyl, cyclobutyl, cyclopentyl, cyclohexyl, oxetanyl, azetidinyl, tetrahydrofuranyl, tetrahydropyranyl, pyrrolidinyl, piperidinyl and piperazinyl,
in each case unsubstituted or mono- or polysubstituted by one or more substituents selected from F; Cl; CN; C₁₋₆-alkyl; CF₃; OH; =O; O-C₁₋₆-alkyl; O-C(=O)-C₁₋₆-alkyl; O-S(=O)₂-C₁₋₆-alkyl; NH₂; N(H)(C₁₋₆-alkyl); N(C₁₋₆-alkyl)₂; N(H)-C(=O)-C₁₋₆-alkyl; N(H)-S(=O)₂-C₁₋₆-alkyl or S(=O)₂-C₁₋₆-alkyl;
R³ represents H or CH₃ or cyclopropyl;
X¹ is N or CH; X² is N or CH; X³ is N or CH
R⁶ and R⁷ are independently absent or are each independently of one another selected from the group consisting of F; Cl; CN; C₁₋₆-alkyl; CF₃; CF₂H; CFH₂; OH; OCF₃; OCF₂H; OCFH₂ or O-C₁₋₆-alkyl;
R⁴ represents H or C₁₋₆-alkyl or benzyl; and
R⁵ represents
C₃₋₆-cycloalkyl, which is unsubstituted or substituted with 1, 2, 3, 4 or 5 substituents independently from one another selected from the group consisting of F, Cl, CF₃, =O, OCF₃, OH, O-C₁₋₆-alkyl, C₁₋₆-alkylen-OH, C₁₋₆-alkyl, C(=O)-NH₂, C(=O)-N(H)(C₁₋₆-alkyl) and C(=O)-N(C₁₋₆-alkyl)₂; or
5- or 6-membered heterocyclyl, which contains 1 or 2 heteroatoms or heteroatom groups independently from one another selected from the group consisting of O, S, S(=O), S(=O)₂, NH and N-C₁₋₆-alkyl, and which is unsubstituted or substituted with 1, 2, 3, 4 or 5 substituents independently from one another selected from the group consisting of F, Cl, CF₃, OCF₃, CN, =O, C₁₋₆-alkyl, C₁₋₆-alkylen-OH and O-C₁₋₆-alkyl;
or a part structure of general formula SF-III 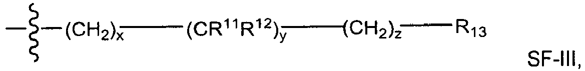 wherein
x represents 1 and y and z each represent 0 or x and y each represent 1 and z represents 0 or x and z each represent 1 and y represents 0 or x, y and z each represent 1;
R¹¹ and R¹² are independently from one another selected from H or CH₃;
R¹³ is selected from the group consisting of
   H, F, Cl, CN, OH, O-C₁₋₆-alkyl, O-(C=O)C₁₋₆-alkyl, S(=O)-C₁₋₆-alkyl, S(=O)₂-C₁₋₆-alkyl, NH₂, NH(C₁₋₆-alkyl), N(C₁₋₆-alkyl)₂, N(H)-C(=O)-C₁₋₆-alkyl, N(H)-S(=O)₂-C₁₋₆-alkyl, C(=O)-NH₂, C(=O)-N(H)(C₁₋₆-alkyl), C(=O)-N(C₁₋₆-alkyl)₂, N(H)-C(=O)-NH₂, N(H)-C(=O)-N(H)(C₁₋₆-alkyl), N(H)-C(=O)-N(C₁₋₆-alkyl)₂,
or represents
   C₃₋₆-cycloalkyl or
   3-7-membered heterocyclyl, which contains 1 or 2 heteroatoms or heteroatom groups independently from one another selected from the group consisting of O, S, S(=O), S(=O)₂, NH and N-C₁₋₆-alkyl, and which is unsubstituted or substituted with 1, 2, 3, 4 or 5 substituents independently from one another selected from the group consisting of F, Cl, CF₃, OCF₃, CN, C₁₋₆-alkyl and O-C₁₋₆-alkyl; or
   phenyl or heteroaryl, selected from pyrrolyl, pyrazolyl, imidazolyl, oxazolyl, isoxazolyl, thiazolyl, isothiazolyl, pyridinyl, pyrazinyl, pyrimidinyl and pyridazinyl,
   in each unsubstituted or substituted with 1, 2 or 3 substituents independently from one another selected from the group consisting of F, Cl, CN, CF₃, OCF₃, C₁₋₆-alkylen-OH, C₁₋₆-alkyl, OH, O-C₁₋₆-alkyl, S(=O)₂-C₁₋₆-alkyl, S(=O)₂-NH₂, NH₂, NH(C₁₋₆-alkyl), N(C₁₋₆-alkyl)₂, O-C(=O)-NH₂, C(=O)-NH₂, C(=O)-N(H)(C₁₋₆-alkyl), C(=O)-N(C₁₋₆-alkyl)₂, C(=O)-O-C₁₋₆-alkyl;
   or
R⁴ and R⁵ together with the nitrogen atom connecting them form a heterocyclyl, selected from the group consisting of 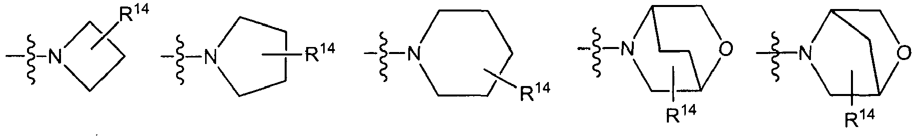 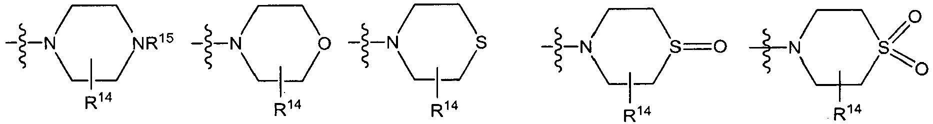 wherein
   R¹⁴ denotes 0, 1 or 2 substituents which are in each case independently of each other selected from the group consisting of F, Cl, CF₃, =O, OCF₃, OH, O-C₁₋₆-alkyl, C₁₋₆-alkylen-OH, C₁₋₆-alkylen-SO₂(C₁₋₆-alkyl), SO₂(C₁₋₆-alkyl), C₁₋₆-alkyl, aryl, heteroaryl, O-aryl and O-heteroaryl, wherein said aryl or said heteroaryl is unsubstituted or substituted with 1, 2 or 3 substituents independently from one another selected from the group consisting of F, Cl, CN, CF₃, OCF₃, C₁₋₆-alkylen-OH, C₁₋₆₋alkyl, OH or O-C₁₋₆-alkyl; and
   R¹⁵ represents H, C₁₋₆-alkyl, (C=O)C₁₋₆-alkyl, (C=O)NH₂, (C=O)NH(C₁₋₆-alkyl) or (C=O)N(C₁₋₆-alkyl)₂.

In another preferred embodiment of the first aspect of the invention, the compound according to general formula (I) is characterized in that
the compound of general formula (I) is a compound according to general formula (Ia),
wherein
n represents 0 or 1;
R¹ represents CH₃, CH₂CH₃, CH(CH)₃, C(CH₃)₃, CF₃, CH₂S(=O)₂CH₃, CH₂OCH₃, CH₂OH, CH₂NH(C=O)CH₃, CH₂C(=O)-NH₂, CH₂N(CH₃)₂, C≡C-cyclopropyl, or 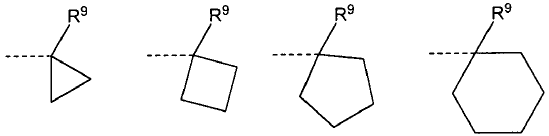 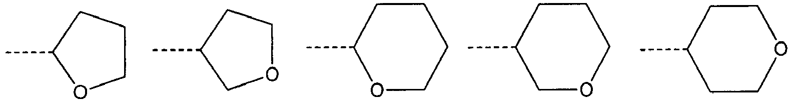 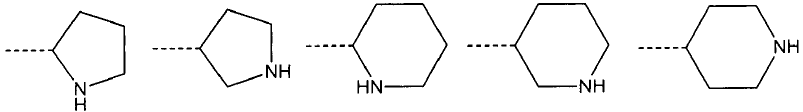 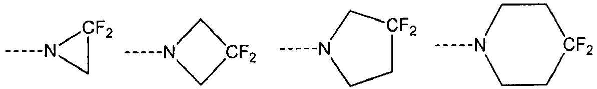 wherein R⁹ represents 0 or 1 substituent, selected from the group consisting of F; CN; CH₃; OH; OCH₃; NH₂; N(H)(CH₃) or N(CH₃)₂;
R⁴ represents H or methyl; and
R⁵ represents
ethyl, 2-propyl (iso-propyl), 1-propyl (n-propyl), 1-butyl, 2-butyl, 2-methyl-propyl, 1,1-dimethyl-ethyl (tert-butyl), 1-pentyl, 2-pentyl, 3-pentyl, 2-methyl-butyl, 2,2-dimethyl-propyl (neo-pentyl), 1-hexyl, 2-hexyl, 3-hexyl, 3,3-dimethyl-butyl, cyclopropyl, cyclopropylmethyl, 2-cyclopropyl-ethyl, 1-cyclopropyl-ethyl as well as 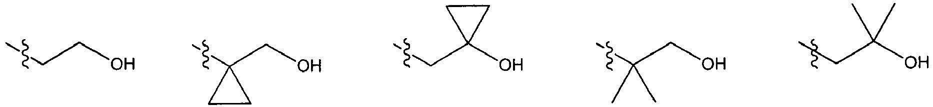 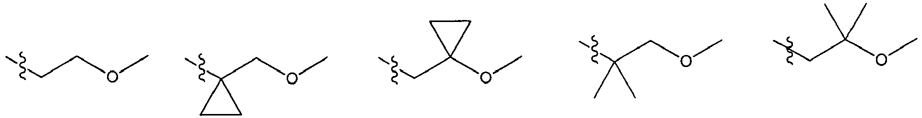 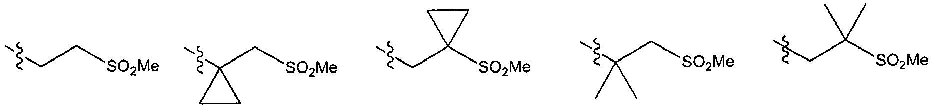 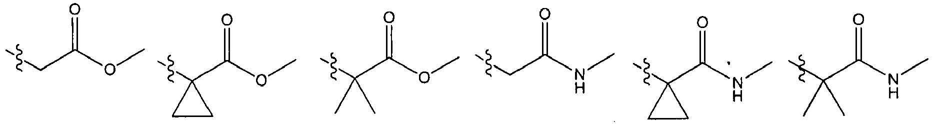 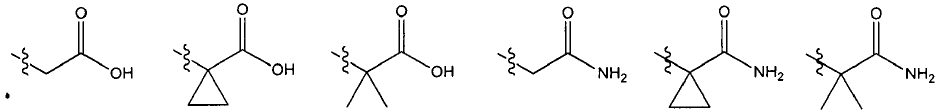 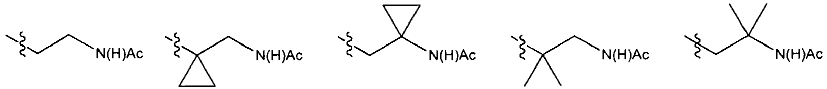 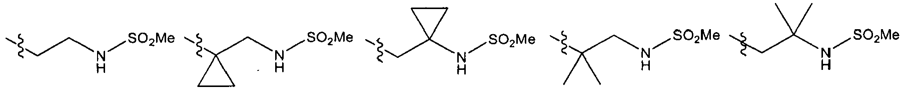 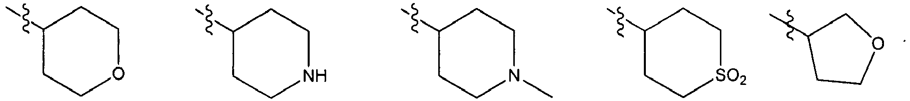 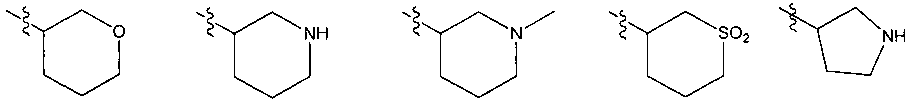 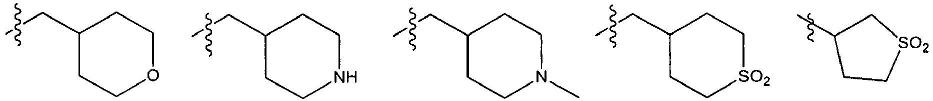 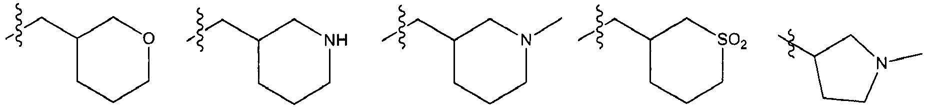 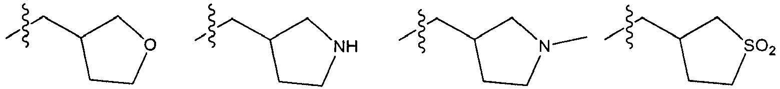 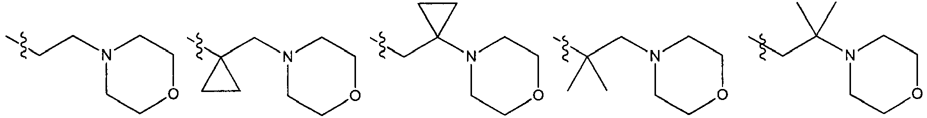 or
R⁴ and R⁵ together with the nitrogen atom connecting them form a heterocyclyl, selected from the group consisting of

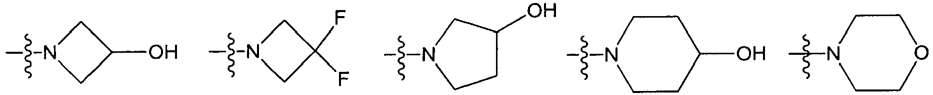

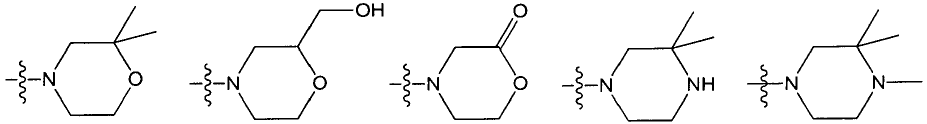

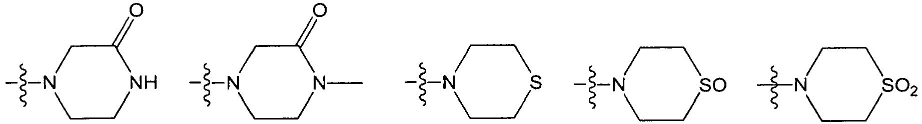

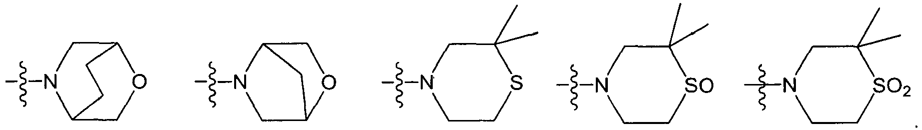

Particularly preferred compounds according to the invention are selected from the group consisting of

| | |
|---|---|
| **001** | N-Benzyl-3-(4-chlorophenyl)-1,4-dimethyl-N-tetrahydro-pyran-4-yl-5-(trifluoromethyl)-1H-pyrrole-2-carboxylic acid amide |
| **002** | 3-(4-Chlorophenyl)-N-(2,2-dimethyl-propyl)-N,1,4-trimethyl-5-(trifluoromethyl)-1H-pyrrole-2-carboxylic acid amide |
| **003** | N-Benzyl-3-(4-chlorophenyl)-N-(2,2-dimethyl-propyl)-1,4-dimethyl-5-(trifluoromethyl)-1H-pyrrole-2-carboxylic acid amide |
| **004** | [3-(4-Chlorophenyl)-1,4-dimethyl-5-(trifluoromethyl)-1H-pyrrol-2-yl]-morpholin-4-yl-methanone |
| **005** | 3-(4-Chlorophenyl)-N-(2,2-dimethyl-propyl)-1-ethyl-N,4-dimethyl-5-(trifluoromethyl)-1H-pyrrole-2-carboxylic acid amide |
| **006** | [3-(4-Chlorophenyl)-1-isopropyl-4-methyl-5-(trifluoromethyl)-1H-pyrrol-2-yl]-(2,2-dimethyl-morpholin-4-yl)-methanone |
| **007** | N-(1-Carbamoyl-cyclopropyl)-3-(4-chlorophenyl)-1-cyclopropyl-N,4-dimethyl-5-(trifluoromethyl)-1H-pyrrole-2-carboxylic acid amide |
| **008** | 3-(4-Chlorophenyl)-1-cyclopropyl-N-(1,1-dioxo-thian-4-yl)-N,4-dimethyl-5-(trifluoromethyl)-1H-pyrrole-2-carboxylic acid amide |
| **009** | N-(2-Carbamoyl-2-methyl-propyl)-3-(4-chlorophenyl)-1-cyclopropyl-N,4-dimethyl-5-(trifluoromethyl)-1H-pyrrole-2-carboxylic acid amide |
| **010** | 3-(4-Chlorophenyl)-1-cyclopropyl-N-[(4-methoxyphenyl)-methyl]-N,4-dimethyl-5-(trifluoromethyl)-1H-pyrrole-2-carboxylic acid amide |
| **011** | 3-(4-Chlorophenyl)-1-cyclopropyl-N-[(4-methoxyphenyl)-methyl]-N-methyl-5-(trifluoromethyl)-1H-pyrrole-2-carboxylic acid amide |
| **012** | 3-(4-Chlorophenyl)-N,1-dicyclopropyl-N,4-dimethyl-5-(trifluoromethyl)-1H-pyrrole-2-carboxylic acid amide |
| **013** | 3-(4-Chlorophenyl)-N,1-dicyclopropyl-N-methyl-5-(trifluoromethyl)-1H-pyrrole-2-carboxylic acid amide |
| **014** | 3-(4-Chlorophenyl)-1-cyclopropyl-N,4-dimethyl-N-tetrahydro-pyran-4-yl-5-(trifluoromethyl)-1H-pyrrole-2-carboxylic acid amide |
| **015** | 3-(4-Chlorophenyl)-1-cyclopropyl-N-methyl-N-tetrahydro-pyran-4-yl-5-(trifluoromethyl)-1H-pyrrole-2-carboxylic acid amide |
| **016** | 3-(4-Chlorophenyl)-1-cyclopropyl-N-(2,2-dimethyl-3-morpholin-4-yl-propyl)-N,4-dimethyl-5-(trifluoromethyl)-1H-pyrrole-2-carboxylic acid amide |
| **017** | 3-(4-Chlorophenyl)-1-cyclopropyl-N-(2,2-dimethyl-3-morpholin-4-yl-propyl)-N-methyl-5-(trifluoromethyl)-1H-pyrrole-2-carboxylic acid amide |
| **018** | 3-(4-Chlorophenyl)-1-cyclopropyl-N-(2-hydroxy-2-methyl-propyl)-N,4-dimethyl-5-(trifluoromethyl)-1H-pyrrole-2-carboxylic acid amide |
| **019** | 3-(4-Chlorophenyl)-1-cyclopropyl-N-(2-hydroxy-2-methyl-propyl)-N-methyl-5-(trifluoromethyl)-1H-pyrrole-2-carboxylic acid amide |
| **020** | 3-(4-Chloro-2-fluoro-phenyl)-1-cyclopropyl-N,4-dimethyl-N-[(5-methyl-isoxazol-3-yl)-methyl]-5-(trifluoromethyl)-1H-pyrrole-2-carboxylic acid amide |
| **021** | 3-(4-Chloro-2-fluoro-phenyl)-1-cyclopropyl-N,4-dimethyl-N-(pyrimidin-4-yl-methyl)-5-(trifluoromethyl)-1H-pyrrole-2-carboxylic acid amide |
| **022** | 3-(4-Chlorophenyl)-1-cyclopropyl-N,4-dimethyl-N-(tetrahydro-furan-3-yl-methyl)-5-(trifluoromethyl)-1H-pyrrole-2-carboxylic acid amide |
| **023** | 3-(4-Chlorophenyl)-1-cyclopropyl-N-methyl-N-(tetrahydro-furan-3-yl-methyl)-5-(trifluoromethyl)-1H-pyrrole-2-carboxylic acid amide |
| **024** | 3-(4-Chloro-2-fluoro-phenyl)-1-cyclopropyl-N,4-dimethyl-N-[(5-methyl-pyrazin-2-yl)-methyl]-5-(trifluoromethyl)-1H-pyrrole-2-carboxylic acid amide |
| **025** | [3-(4-Chlorophenyl)-1-cyclopropyl-4-methyl-5-(trifluoromethyl)-1H-pyrrol-2-yl]-(3-hydroxy-azetidin-1-yl)-methanone |
| **026** | [3-(4-Chlorophenyl)-1-cyclopropyl-5-(trifluoromethyl)-1H-pyrrol-2-yl]-(3-hydroxy-azetidin-1-yl)-methanone |
| **027** | [3-(4-Chlorophenyl)-1-cyclopropyl-4-methyl-5-(trifluoromethyl)-1H-pyrrol-2-yl]-[4-(3-methyl-[1,2,4]oxadiazol-5-yl)-piperidin-1-yl]-methanone |
| **028** | [3-(4-Chloro-2-fluoro-phenyl)-1-cyclopropyl-4-methyl-5-(trifluoromethyl)-1H-pyrrol-2-yl]-[4-(3-methyl-[1,2,4]oxadiazol-5-yl)-piperidin-1-yl]-methanone |
| **029** | [3-(4-Chlorophenyl)-1-cyclopropyl-5-(trifluoromethyl)-1H-pyrrol-2-yl]-[4-(3-methyl-[1,2,4]oxadiazol-5-yl)-piperidin-1-yl]-methanone |
| **030** | [3-(4-Chlorophenyl)-1-cyclopropyl-4-methyl-5-(trifluoromethyl)-1H-pyrrol-2-yl]-[4-[5-(trifluoromethyl)-pyridin-2-yl]oxy-piperidin-1-yl]-methanone |
| **031** | [3-(4-Chlorophenyl)-1-cyclopropyl-5-(trifluoromethyl)-1H-pyrrol-2-yl]-[4-[5-(trifluoromethyl)-pyridin-2-yl]oxy-piperidin-1-yl]-methanone |
| **032** | [3-(4-Chlorophenyl)-1-cyclopropyl-4-methyl-5-(trifluoromethyl)-1H-pyrrol-2-yl]-(2,2-dimethyl-morpholin-4-yl)-methanone |
| **033** | [3-(4-Chloro-2-fluoro-phenyl)-1-cyclopropyl-4-methyl-5-(trifluoromethyl)-1H-pyrrol-2-yl]-(2,2-dimethyl-morpholin-4-yl)-methanone |
| **034** | [3-(4-Chlorophenyl)-1-cyclopropyl-5-(trifluoromethyl)-1H-pyrrol-2-yl]-(2,2-dimethyl-morpholin-4-yl)-methanone |
| **035** | 3-(4-Chlorophenyl)-1-cyclopropyl-N-(2,2-dimethyl-propyl)-N-methyl-5-(trifluoromethyl)-1H-pyrrole-2-carboxylic acid amide |
| **036** | 3-(4-Chlorophenyl)-1-cyclopropyl-N-(3-hydroxy-2,2-dimethyl-propyl)-N-methyl-5-(trifluoromethyl)-1H-pyrrole-2-carboxylic acid amide |
| **037** | 3-(4-Chlorophenyl)-1-cyclopropyl-N-methyl-N-(2-methylsulfonyl-ethyl)-5-(trifluoromethyl)-1H-pyrrole-2-carboxylic acid amide |
| **038** | [3-(4-Chlorophenyl)-1-cyclopropyl-5-(trifluoromethyl)-1H-pyrrol-2-yl]-morpholin-4-yl-methanone |
| **039** | 3-(4-Chlorophenyl)-1-cyclopropyl-N-(2,2-dimethyl-propyl)-N,4-dimethyl-5-(trifluoromethyl)-1H-pyrrole-2-carboxylic acid amide |
| **040** | 3-(4-Chlorophenyl)-1-cyclopropyl-N-(3-hydroxy-2,2-dimethyl-propyl)-N,4-dimethyl-5-(trifluoromethyl)-1H-pyrrole-2-carboxylic acid amide |
| **041** | 3-(4-Chlorophenyl)-1-cyclopropyl-N,4-dimethyl-N-(2-methylsulfonyl-ethyl)-5-(trifluoromethyl)-1H-pyrrole-2-carboxylic acid amide |
| **042** | [3-(4-Chlorophenyl)-1-cyclobutyl-4-methyl-5-(trifluoromethyl)-1H-pyrrol-2-yl]-(2,2-dimethyl-morpholin-4-yl)-methanone |
| **043** | [3-(4-Chlorophenyl)-1-cyclopropyl-4-methyl-5-(trifluoromethyl)-1H-pyrrol-2-yl]-morpholin-4-yl-methanone |
| **044** | [3-(4-Chlorophenyl)-1-cyclopropyl-4-methyl-5-(trifluoromethyl)-1H-pyrrol-2-yl]-(2,2-dimethyl-1,1-dioxo-[1,4]thiazinan-4-yl)-methanone |
| **045** | (2,2-Dimethyl-morpholin-4-yl)-[3-(4-fluorophenyl)-1-(2-methyl-propyl)-5-(trifluoromethyl)-1H-pyrrol-2-yl]-methanone |
| **046** | 3-(4-Fluorophenyl)-N-methyl-1-(2-methyl-propyl)-N-tetrahydro-pyran-4-yl-5-(trifluoromethyl)-1H-pyrrole-2-carboxylic acid amide |
| **047** | 3-(4-Fluorophenyl)-N-methyl-N-[(5-methyl-isoxazol-3-yl)-methyl]-1-(2-methyl-propyl)-5-(trifluoromethyl)-1H-pyrrole-2-carboxylic acid amide |
| **048** | 3-(4-Chlorophenyl)-N,4-dimethyl-1-(2-methyl-propyl)-N-(pyrimidin-4-yl-methyl)-5-(trifluoromethyl)-1H-pyrrole-2-carboxylic acid amide |
| **049** | 3-(4-Fluorophenyl)-N-methyl-1-(2-methyl-propyl)-N-[(5-methyl-pyrazin-2-yl)-methyl]-5-(trifluoromethyl)-1H-pyrrole-2-carboxylic acid amide |
| **050** | 3-(4-Fluorophenyl)-N-methyl-1-(2-methyl-propyl)-N-(2-methylsulfonyl-ethyl)-5-(trifluoromethyl)-1H-pyrrole-2-carboxylic acid amide |
| **051** | N-(1-Carbamoyl-cyclopropyl)-3-(4-chlorophenyl)-N,4-dimethyl-1-(2-methyl-propyl)-5-(trifluoromethyl)-1H-pyrrole-2-carboxylic acid amide |
| **052** | N-(2-Carbamoyl-2-methyl-propyl)-3-(4-chlorophenyl)-N,4-dimethyl-1-(2-methyl-propyl)-5-(trifluoromethyl)-1H-pyrrole-2-carboxylic acid amide |
| **053** | N-(2,2-Dimethyl-propyl)-3-(4-fluorophenyl)-N-methyl-1-(2-methyl-propyl)-5-(trifluoromethyl)-1H-pyrrole-2-carboxylic acid amide |
| **054** | N-(2,2-Dimethyl-3-morpholin-4-yl-propyl)-3-(4-fluorophenyl)-N-methyl-1-(2-methyl-propyl)-5-(trifluoromethyl)-1H-pyrrole-2-carboxylic acid amide |
| **055** | 3-(4-Chlorophenyl)-N-(3-hydroxy-2,2-dimethyl-propyl)-N,4-dimethyl-1-(2-methyl-propyl)-5-(trifluoromethyl)-1H-pyrrole-2-carboxylic acid amide |
| **056** | 3-(4-Fluorophenyl)-N-(3-hydroxy-2,2-dimethyl-propyl)-N-methyl-1-(2-methyl-propyl)-5-(trifluoromethyl)-1H-pyrrole-2-carboxylic acid amide |
| **057** | 3-(4-Chlorophenyl)-N,4-dimethyl-N-[(5-methyl-isoxazol-3-yl)-methyl]-1-(2-methyl-propyl)-5-(trifluoromethyl)-1H-pyrrole-2-carboxylic acid amide |
| **058** | 3-(4-Fluorophenyl)-N-methyl-1-(2-methyl-propyl)-N-(tetrahydro-furan-3-yl-methyl)-5-(trifluoromethyl)-1H-pyrrole-2-carboxylic acid amide |
| **059** | 3-(4-Chlorophenyl)-N,4-dimethyl-1-(2-methyl-propyl)-N-[(5-methyl-pyrazin-2-yl)-methyl]-5-(trifluoromethyl)-1H-pyrrole-2-carboxylic acid amide |
| **060** | N-[(2-Dimethylamino-pyrimidin-5-yl)-methyl]-3-(4-fluorophenyl)-N-methyl-1-(2-methyl-propyl)-5-(trifluoromethyl)-1H-pyrrole-2-carboxylic acid amide |
| **061** | 3-(4-Chlorophenyl)-N,4-dimethyl-1-(2-methyl-propyl)-N-(2-methylsulfonyl-ethyl)-5-(trifluoromethyl)-1H-pyrrole-2-carboxylic acid amide |
| **062** | [3-(4-Chlorophenyl)-4-methyl-1-(2-methyl-propyl)-5-(trifluoromethyl)-1H-pyrrol-2-yl]-[4-(3-methyl-[1,2,4]oxadiazol-5-yl)-piperidin-1-yl]-methanone |
| **063** | 4-[3-(4-Fluorophenyl)-1-(2-methyl-propyl)-5-(trifluoromethyl)-1H-pyrrole-2-carbonyl]-piperazin-2-one |
| **064** | [3-(4-Chlorophenyl)-4-methyl-1-(2-methyl-propyl)-5-(trifluoromethyl)-1H-pyrrol-2-yl]-(2,2-dimethyl-morpholin-4-yl)-methanone |
| **065** | [3-(4-Fluorophenyl)-1-(2-methyl-propyl)-5-(trifluoromethyl)-1H-pyrrol-2-yl]-morpholin-4-yl-methanone |
| **066** | N-Cyclopropyl-3-(4-fluorophenyl)-1-(2-methyl-propyl)-5-(trifluoromethyl)-1H-pyrrole-2-carboxylic acid amide |
| **067** | 3-(4-Chlorophenyl)-N-(2-cyano-2-methyl-propyl)-N,4-dimethyl-1-(2-methyl-propyl)-5-(trifluoromethyl)-1H-pyrrole-2-carboxylic acid amide |
| **068** | 3-(4-Chlorophenyl)-N,4-dimethyl-1-[(1-methyl-cyclopropyl)-methyl]-N-(2-methylsulfonyl-ethyl)-5-(trifluoromethyl)-1H-pyrrole-2-carboxylic acid amide |
| **069** | N-[(2-Dimethylamino-pyrimidin-5-yl)-methyl]-3-(4-fluorophenyl)-N-methyl-1-(tetrahydro-furan-2-yl-methyl)-5-(trifluoromethyl)-1H-pyrrole-2-carboxylic acid amide |
| **070** | 3-(4-Fluorophenyl)-N-methyl-1-(tetrahydro-furan-2-yl-methyl)-N-tetrahydro-pyran-4-yl-5-(trifluoromethyl)-1H-pyrrole-2-carboxylic acid amide |
| **071** | N-(1,1-Dioxo-thiolan-3-yl)-3-(4-fluorophenyl)-N-methyl-1-(tetrahydro-furan-2-yl-methyl)-5-(trifluoromethyl)-1H-pyrrole-2-carboxylic acid amide |
| **072** | N-(2-Carbamoyl-2-methyl-propyl)-3-(4-fluorophenyl)-N-methyl-1-(tetrahydro-fu ran-2-yl-methyl)-5-(trifluoromethyl)-1H-pyrrole-2-carboxylic acid amide |
| **073** | N-(2,2-Dimethyl-propyl)-3-(4-fluorophenyl)-N-methyl-1-(tetrahydro-furan-2-yl-methyl)-5-(trifluoromethyl)-1H-pyrrole-2-carboxylic acid amide |
| **074** | 3-(4-Fluorophenyl)-N-(3-hydroxy-2,2-dimethyl-propyl)-N-methyl-1-(tetrahydro-furan-2-yl-methyl)-5-(trifluoromethyl)-1H-pyrrole-2-carboxylic acid amide |
| **075** | 3-(4-Fluorophenyl)-N-methyl-N-[(5-methyl-isoxazol-3-yl)-methyl]-1-(tetrahydro-furan-2-yl-methyl)-5-(trifluoromethyl)-1H-pyrrole-2-carboxylic acid amide |
| **076** | 3-(4-Fluorophenyl)-N-methyl-N,1-bis(tetrahydro-furan-2-yl-methyl)-5-(trifluoromethyl)-1H-pyrrole-2-carboxylic acid amide |
| **077** | 3-(4-Fluorophenyl)-N-methyl-N-[(5-methyl-pyrazin-2-yl)-methyl]-1-(tetrahydro-furan-2-yl-methyl)-5-(trifluoromethyl)-1H-pyrrole-2-carboxylic acid amide |
| **078** | 3-(4-Fluorophenyl)-N-methyl-N-(2-methylsulfonyl-ethyl)-1-(tetrahydro-furan-2-yl-methyl)-5-(trifluoromethyl)-1H-pyrrole-2-carboxylic acid amide |
| **079** | 4-[3-(4-Fluorophenyl)-1-(tetrahydro-furan-2-yl-methyl)-5-(trifluoromethyl)-1H-pyrrole-2-carbonyl]-piperazin-2-one |
| **080** | (2,2-Dimethyl-morpholin-4-yl)-[3-(4-fluorophenyl)-1-(tetrahydro-furan-2-yl-methyl)-5-(trifluoromethyl)-1H-pyrrol-2-yl]-methanone |
| **081** | [3-(4-Fluorophenyl)-1-(tetrahydro-furan-2-yl-methyl)-5-(trifluoromethyl)-1H-pyrrol-2-yl]-morpholin-4-yl-methanone |
| **082** | N-Cyclopropyl-3-(4-fluorophenyl)-1-(tetrahydro-furan-2-yl-methyl)-5-(trifluoromethyl)-1H-pyrrole-2-carboxylic acid amide |
| **083** | 3-(4-Chlorophenyl)-N-(2,2-dimethyl-propyl)-N-methyl-1-(tetrahydro-furan-2-yl-methyl)-5-(trifluoromethyl)-1H-pyrrole-2-carboxylic acid amide |
| **084** | [3-(4-Chlorophenyl)-1-(cyclopropyl-methyl)-4-methyl-5-(trifluoromethyl)-1H-pyrrol-2-yl]-(2,2-dimethyl-morpholin-4-yl)-methanone |
| **085** | [3-(4-Chlorophenyl)-1-(cyclobutyl-methyl)-4-methyl-5-(trifluoromethyl)-1H-pyrrol-2-yl]-(2,2-dimethyl-morpholin-4-yl)-methanone |
| **086** | [3-(4-Chlorophenyl)-1-(3-cyclopropyl-prop-2-ynyl)-4-methyl-5-(trifluoromethyl)-1H-pyrrol-2-yl]-morpholin-4-yl-methanone |
| **087** | [3-(4-Chlorophenyl)-4-methyl-1-[(1-methyl-cyclopropyl)-methyl]-5-(trifluoromethyl)-1H-pyrrol-2-yl]-morpholin-4-yl-methanone |
| **088** | 1-[(1-Amino-cyclopropyl)-methyl]-3-(4-chlorophenyl)-N-(2,2-dimethyl-propyl)-N-methyl-5-(trifluoromethyl)-1H-pyrrole-2-carboxylic acid amide |
| **089** | [3-(4-Chlorophenyl)-1-[(1-hydroxy-cyclopropyl)-methyl]-4-methyl-5-(trifluoromethyl)-1H-pyrrol-2-yl]-morpholin-4-yl-methanone |
| **090** | 3-(4-Chlorophenyl)-N-(2,2-dimethyl-propyl)-1-[(1-hydroxy-cyclopentyl)-methyl]-N-methyl-5-(trifluoromethyl)-1H-pyrrole-2-carboxylic acid amide |
| **091** | 3-(4-Chlorophenyl)-N-(2,2-dimethyl-propyl)-N-methyl-1-(pyrrolidin-2-yl-methyl)-5-(trifluoromethyl)-1H-pyrrole-2-carboxylic acid amide |
| **092** | 3-(4-Chlorophenyl)-N-(2,2-dimethyl-propyl)-N-methyl-1-(tetrahydro-pyran-4-yl-methyl)-5-(trifluoromethyl)-1H-pyrrole-2-carboxylic acid amide |
| **093** | 1-[(1-Amino-cyclopropyl)-methyl]-3-(4-chlorophenyl)-N,4-dimethyl-N-(2-methylsulfonyl-ethyl)-5-(trifluoromethyl)-1H-pyrrole-2-carboxylic acid amide |
| **094** | 3-(4-Chlorophenyl)-1-[(1-hydroxy-cyclopentyl)-methyl]-N,4-dimethyl-N-(2-methylsulfonyl-ethyl)-5-(trifluoromethyl)-1H-pyrrole-2-carboxylic acid amide |
| **095** | [1-[(1-Amino-cyclopropyl)-methyl]-3-(4-chlorophenyl)-4-methyl-5-(trifluoromethyl)-1H-pyrrol-2-yl]-morpholin-4-yl-methanone |
| **096** | [3-(4-Chlorophenyl)-1-[(1-hydroxy-cyclopentyl)-methyl]-4-methyl-5-(trifluoromethyl)-1H-pyrrol-2-yl]-morpholin-4-yl-methanone |
| **097** | [3-(4-Chlorophenyl)-4-methyl-1-(tetrahydro-pyran-4-yl-methyl)-5-(trifluoromethyl)-1H-pyrrol-2-yl]-morpholin-4-yl-methanone |
| **098** | [3-(4-Chlorophenyl)-1-[2-(3,3-difluoro-azetidin-1-yl)-ethyl]-4-methyl-5-(trifluoromethyl)-1H-pyrrol-2-yl]-morpholin-4-yl-methanone |
| **099** | [3-(4-Chlorophenyl)-4-methyl-1-(2-methylsulfonyl-ethyl)-5-(trifluoromethyl)-1H-pyrrol-2-yl]-morpholin-4-yl-methanone |
| **100** | 3-(4-Chlorophenyl)-1-[(1-cyano-cyclopropyl)-methyl]-N-(2,2-dimethyl-propyl)-N-methyl-5-(trifluoromethyl)-1H-pyrrole-2-carboxylic acid amide |
| **101** | 3-(4-Chlorophenyl)-N-(2,2-dimethyl-propyl)-1-[(1-hydroxy-cyclobutyl)-methyl]-N-methyl-5-(trifluoromethyl)-1H-pyrrole-2-carboxylic acid amide |
| **102** | [3-(4-Chlorophenyl)-1,4-dimethyl-5-(trifluoromethyl)-1H-pyrrol-2-yl]-(2,2-dimethyl-morpholin-4-yl)-methanone |
| **103** | [3-(4-Chlorophenyl)-1-ethyl-4-methyl-5-(trifluoromethyl)-1H-pyrrol-2-yl]-(2,2-dimethyl-morpholin-4-yl)-methanone |
| **104** | [3-(4-Chlorophenyl)-4-methyl-1-(2-methyl-propyl)-5-(trifluoromethyl)-1H-pyrrol-2-yl]-(2-oxa-5-azabicyclo[2.2.1]heptan-5-yl)-methanone |
| **105** | N-(2-Carbamoyl-2-methyl-propyl)-3-(4-chlorophenyl)-1-isopropyl-N,4-dimethyl-5-(trifluoromethyl)-1H-pyrrole-2-carboxylic acid amide |
| **106** | 3-(4-Chlorophenyl)-N-(2-cyano-2-methyl-propyl)-1-isopropyl-N,4-dimethyl-5-(trifluoromethyl)-1H-pyrrole-2-carboxylic acid amide |
| **107** | 3-(4-Chlorophenyl)-1-isopropyl-N,4-dimethyl-N-[(5-methyl-isoxazol-3-yl)-methyl]-5-(trifluoromethyl)-1H-pyrrole-2-carboxylic acid amide |
| **108** | 3-(4-Chlorophenyl)-N,1-diisopropyl-N,4-dimethyl-5-(trifluoromethyl)-1H-pyrrole-2-carboxylic acid amide |
| **109** | 3-(4-Chlorophenyl)-N-isopropyl-N,4-dimethyl-1-(2-methyl-propyl)-5-(trifluoromethyl)-1H-pyrrole-2-carboxylic acid amide |
| **110** | 3-(4-Chlorophenyl)-1-isopropyl-N,4-dimethyl-N-(2-methylsulfonyl-ethyl)-5-(trifluoromethyl)-1H-pyrrole-2-carboxylic acid amide |
| **111** | 3-(4-Chlorophenyl)-1-isopropyl-N,4-dimethyl-N-[(2-methyl-2H-pyrazol-3-yl)-methyl]-5-(trifluoromethyl)-1H-pyrrole-2-carboxylic acid amide |
| **112** | [3-(5-Chloro-pyridin-2-yl)-1-isopropyl-4-methyl-5-(trifluoromethyl)-1H-pyrrol-2-yl]-(2,2-dimethyl-morpholin-4-yl)-methanone |
| **113** | 3-(5-Chloro-pyridin-2-yl)-1-isopropyl-N,4-dimethyl-N-[(5-methyl-isoxazol-3-yl)-methyl]-5-(trifluoromethyl)-1H-pyrrole-2-carboxylic acid amide |
| **114** | 3-(5-Chloro-pyridin-2-yl)-1-isopropyl-N,4-dimethyl-N-[(2-methyl-2H-pyrazol-3-yl)-methyl]-5-(trifluoromethyl)-1H-pyrrole-2-carboxylic acid amide |
| **115** | 3-(4-Chlorophenyl)-1-isopropyl-N,4-dimethyl-N-(2-oxo-pyrrolidin-3-yl)-5-(trifluoromethyl)-1H-pyrrole-2-carboxylic acid amide |
| **116** | N-(2-Carbamoyl-2-methyl-propyl)-3-(5-chloro-pyridin-2-yl)-1-isopropyl-N,4-dimethyl-5-(trifluoromethyl)-1H-pyrrole-2-carboxylic acid amide |
| **117** | 3-(5-Chloro-pyridin-2-yl)-N-(2-cyano-2-methyl-propyl)-1-isopropyl-N,4-dimethyl-5-(trifluoromethyl)-1H-pyrrole-2-carboxylic acid amide |
| **118** | 3-(5-Chloro-pyridin-2-yl)-1-isopropyl-N,4-dimethyl-N-(2-methylsulfonyl-ethyl)-5-(trifluoromethyl)-1H-pyrrole-2-carboxylic acid amide |
| **119** | 3-(5-Chloro-pyridin-2-yl)-1-isopropyl-N,4-dimethyl-N-(2-oxo-pyrrolidin-3-yl)-5-(trifluoromethyl)-1H-pyrrole-2-carboxylic acid amide |
| **120** | 3-(5-Chloro-pyridin-2-yl)-N,1-diisopropyl-N,4-dimethyl-5-(trifluoromethyl)-1H-pyrrole-2-carboxylic acid amide |
| **121** | [3-(5-Chloro-pyrimidin-2-yl)-1-isopropyl-4-methyl-5-(trifluoromethyl)-1H-pyrrol-2-yl]-(2,2-dimethyl-morpholin-4-yl)-methanone |
| **122** | 3-(5-Chloro-pyrimidin-2-yl)-1-isopropyl-N,4-dimethyl-N-[(2-methyl-2H-pyrazol-3-yl)-methyl]-5-(trifluoromethyl)-1H-pyrrole-2-carboxylic acid amide |
| **123** | 3-(5-Chloro-pyrimidin-2-yl)-1-isopropyl-N,4-dimethyl-N-[(5-methyl-isoxazol-3-yl)-methyl]-5-(trifluoromethyl)-1H-pyrrole-2-carboxylic acid amide |
| **124** | 3-(5-Chloro-pyrimidin-2-yl)-1-isopropyl-N,4-dimethyl-N-(2-oxo-pyrrolidin-3-yl)-5-(trifluoromethyl)-1H-pyrrole-2-carboxylic acid amide |
| **125** | N-(2-Carbamoyl-2-methyl-propyl)-3-(5-chloro-pyrimidin-2-yl)-1-isopropyl-N,4-dimethyl-5-(trifluoromethyl)-1H-pyrrole-2-carboxylic acid amide |
| **126** | 3-(5-Chloro-pyrimidin-2-yl)-N-(2-cyano-2-methyl-propyl)-1-isopropyl-N,4-dimethyl-5-(trifluoromethyl)-1H-pyrrole-2-carboxylic acid amide |
| **127** | 3-(5-Chloro-pyrimidin-2-yl)-1-isopropyl-N,4-dimethyl-N-(2-methylsulfonyl-ethyl)-5-(trifluoromethyl)-1H-pyrrole-2-carboxylic acid amide |
| **128** | 3-(5-Chloro-pyrimidin-2-yl)-N,1-diisopropyl-N,4-dimethyl-5-(trifluoromethyl)-1H-pyrrole-2-carboxylic acid amide |
| **129** | N-tert-Butyl-4-[3-(5-chloro-pyridin-2-yl)-1-isopropyl-4-methyl-5-(trifluoromethyl)-1H-pyrrole-2-carbonyl]-piperazine-1-carboxylic acid amide |
| **130** | [3-(5-Chloro-pyridin-2-yl)-1-isopropyl-4-methyl-5-(trifluoromethyl)-1H-pyrrol-2-yl]-(2,2-dimethyl-1-oxo-[1,4]thiazinan-4-yl)-methanone |
| **131** | N-tert-Butyl-4-[3-(4-chlorophenyl)-1-isopropyl-4-methyl-5-(trifluoromethyl)-1H-pyrrole-2-carbonyl]-piperazine-1-carboxylic acid amide |
| **132** | 3-(4-Chlorophenyl)-N-[1-(hydroxymethyl)-3-methyl-butyl]-1-isopropyl-N,4-dimethyl-5-(trifluoromethyl)-1H-pyrrole-2-carboxylic acid amide |
| **133** | N-[1-(tert-Butyl-carbamoyl)-ethyl]-3-(4-chlorophenyl)-1-isopropyl-N,4-dimethyl-5-(trifluoromethyl)-1H-pyrrole-2-carboxylic acid amide |
| **134** | 3-(5-Chloro-pyridin-2-yl)-N-(3,3-dimethyl-piperidin-4-yl)-1-isopropyl-N,4-dimethyl-5-(trifluoromethyl)-1H-pyrrole-2-carboxylic acid amide |
| **135** | 3-(4-Chlorophenyl)-N-(3,3-dimethyl-piperidin-4-yl)-1-isopropyl-N,4-dimethyl-5-(trifluoromethyl)-1H-pyrrole-2-carboxylic acid amide |

optionally in the form of a single stereoisomer or a mixture of stereoisomers, in the form of the free compound and/or a physiologically acceptable salt or solvate thereof.

Furthermore, preference may be given to compounds according to the invention that cause at least a 50% inhibition, which is present at a concentration of 3 µM, in a fluorescent assay for CaV2.2 channels with HEK293 cells in which human CaV2.2 channels were stably expressed at a concentration of less 3 µM, preferably less than 1000 nM, particularly preferably less than 300 nM, most particularly preferably less than 100 nM, even more preferably less than 75 nM, additionally preferably less than 50 nM, most preferably less than 10 nM.

In the process, the Ca²⁺ influx is quantified in the FLIPR assay with the aid of a Ca²⁺-sensitive dye (type Fluo-4, Molecular Probes Europe BV, Leiden, the Netherlands) in a fluorescent imaging plate reader (FLIPR 3, Molecular Devices, Sunnyvale, USA), as described hereinafter.

The compounds according to the invention and corresponding stereoisomers and also the respective corresponding acids, bases, salts and solvates are suitable for the treatment and/or prophylaxis of one or more disorders and/or diseases selected from the group consisting of pain, preferably pain selected from the group consisting of acute pain, chronic pain, visceral pain, headache pain, inflammatory pain and mixed pain; stroke (the neuronal damage resulting from head trauma); mood disorders; epilepsy; schizophrenia, and neurodegenerative disorders.

The present invention further relates to a compound according to the present invention for CaV2.2 calcium channel regulation, preferably for use in CaV2.2 calcium channel blockage.

The present invention therefore further relates to a compound according to the present invention for the prophylaxis and/or treatment of disorders and/or diseases which are mediated, at least in part, at least in part, by CaV2.2 channels.

The term "disorders and/or diseases which are mediated, at least in part, by CaV2.2 channels", is intended to include each of or all of the disease states.

The substances according to the invention hence act, for example, on CaV2.2 channels relevant in connection with various diseases, so that they are suitable as a pharmacologically active compound in pharamceutical compositions.

The compounds according to the first aspect of the present invention and the corresponding stereoisomers and the respective salts and solvates are toxicologically safe and are therefore suitable as pharmacologically active ingredients in pharmaceutical compositions.

In another sepcet of the present invention, the invention therefore also provides pharmaceutical compositions, containing at least one compound according to the invention and optionally one or more suitable, pharmaceutically compatible auxiliaries and/or, if appropriate, one or more further pharmacologically active compounds.

The pharmaceutical composition according to the invention is suitable for administration to adults and children, including toddlers and babies.

The pharmaceutical composition according to the invention may be found as a liquid, semisolid or solid pharmaceutical form, for example in the form of injection solutions, drops, juices, syrups, sprays, suspensions, tablets, patches, capsules, plasters, suppositories, ointments, creams, lotions, gels, emulsions, aerosols or in multiparticulate form, for example in the form of pellets or granules, if appropriate pressed into tablets, decanted in capsules or suspended in a liquid, and also be administered as much.

In addition to at least one compound according to the invention, if appropriate in the form of one of its pure stereoisomers, in particular enantiomers or diastereomers, its racemate or in the form of mixtures of the stereoisomers, in particular the enantiomers or diastereomers, in any desired mixing ratio, or if appropriate in the form of a corresponding salt or respectively in the form of a corresponding solvate, the pharmaceutical composition according to the invention conventionally contains further physiologically compatible pharmaceutical auxiliaries which can for example be selected from the group consisting of excipients, fillers, solvents, diluents, surface-active substances, dyes, preservatives, blasting agents, slip additives, lubricants, aromas and binders.

The selection of the physiologically compatible auxiliaries and also the amounts thereof to be used depend on whether the pharmaceutical composition is to be applied orally, subcutaneously, parenterally, intravenously, intraperitoneally, intradermally, intramuscularly, intranasally, buccally, rectally or locally, for example to infections of the skin, the mucous membranes and of the eyes. Preparations in the form of tablets, dragees, capsules, granules, pellets, drops, juices and syrups are preferably suitable for oral application; solutions, suspensions, easily reconstitutable dry preparations and also sprays are preferably suitable for parenteral, topical and inhalative application. The compounds according to the invention used in the pharmaceutical composition according to the invention in a repository in dissolved form or in a plaster, agents promoting skin penetration being added if appropriate, are suitable percutaneous application preparations. Orally or percutaneously applicable preparation forms can release the respective compound according to the invention also in a delayed manner.

The pharmaceutical compositions according to the invention are prepared with the aid of conventional means, devices, methods and process known in the art, such as are described for example in "Remington's Pharmaceutical Sciences", A.R. Gennaro (Editor), 17th edition, Mack Publishing Company, Easton, Pa, 1985, in particular in Part 8, Chapters 76 to 93. The corresponding description is introduced herewith by way of reference and forms part of the disclosure. The amount to be administered to the patient of the respective compounds according to the invention of the above-indicated general formula I may vary and is for example dependent on the patient's weight or age and also on the type of application, the indication and the severity of the disorder. Conventionally 0.001 to 100 mg/kg, preferably 0.05 to 75 mg/kg, particularly preferably 0.05 to 50 mg of at least one such compound according to the invention are applied per kg of the patient's body weight.

CaV2.2 channels are believed to be involved in a variety of diseases or disorders in mammals such as humans. These include pain (e.g.; acute pain, chronic pain, visceral pain, headache pain, inflammatory pain, mixed pain), stroke (the neuronal damage resulting from head trauma), epilepsy, mood disorders, schizophrenia, neurodegenerative disorders.

Another embodiment of the present invention is at least one compound according the present invention for the treatment and/or prophylaxis of one or more disorders selected from the group consisting of pain, preferably pain selected from the group consisting of acute pain, chronic pain, visceral pain, headache pain, inflammatory pain and mixed pain; stroke (the neuronal damage resulting from head trauma); mood disorders; epilepsy; schizophrenia, and neurodegenerative disorders.

Another embodiment of the present invention is at least one compound according to the present invention for the treatment and/or prophylaxis of pain, in particular acute pain and/or chronic pain and/or visceral pain and/or headache pain and/or inflammatory pain and/or mixed pain.

Acute pain according to the invention might include nociceptive pain and post-operative or surgical pain. Chronic pain according to the invention might include peripheral neuropathic pain such as post-herpetic neuralgia, traumatic nerve injury, nerve compression or entrapment, small fibre neuropathy, diabetic neuropathy, neuropathic cancer pain, failed back surgery Syndrome, trigeminal neuralgia, phantom limb pain; neuroma pain, complex regional pain syndrome, chronic arthritic pain and related neuralgias, and pain associated with cancer, chemotherapy, HIV and HIV treatment-induced neuropathy; central neuropathic pain such as multiple sclerosis related pain, Parkinson disease related pain, post-stroke pain, post-traumatic spinal cord injury pain, and pain in dementia; musculoskeletal pain such as osteoarthritic pain and fibromyalgia syndrome. In treating osteoarthritic pain, joint mobility will also improve as the underlying chronic pain is reduced. Thus, at least one compound for treatment of osteoarthritic pain inherently will also improve joint mobility in patients suffering from osteoarthritis. Visceral pain according to the invention might include interstitial cystitis, irritable bowel syndrome, Crohn's disease and chronic pelvic pain syndrome. Inflammatory pain according to the invention might include rheumatoid arthritis and endometriosis. Headachepain according to the invention might include migraine, cluster headache, tension headache syndrome, facial pain and headache caused by other diseases. Mixed pain according to the invention might include lower back pain, neck and shoulder pain, burning mouth syndrome and complex regional pain syndrome.

In another embodiment of the invention, at least one compound according to the present invention is particularily suitable for the treatment and/or prophylaxis of mood disorders.

Mood disorders according to the invention might include anxiety disorder, social anxiety disorder, panic disorder, specific phobias, for example, specific animal phobias, social phobias, obsessive-compulsive disorder, agoraphobia, post-traumatic stress syndrome, addiction (including dependence, withdrawal and/or relapse of medication, including opioids, but also drugs such as cocaine, opioids, alcohol and nicotine), generalised anxiety disorders, single episodic or recurrent major depressive disorders and dysthymic disorders, or bipolar disorders, for example, bipolar I disorder, bipolar II disorder and cyclothymic disorder.

In another embodiment of the invention, at least one compound according to the present invention is particularity suitable for the treatment and/or prophylaxis of epilepsy.

Epilepsy according to the invention might include partial seizures such as temporal lobe epilepsy, absence seizures generalized seizures, and tonic/clonic seizures.

In yet another embodiment of the invention, at least one compound according to the present invention is particularity suitable for the treatment and/or prophylaxis of neurodegenerative disorders.

Neurodegenerative disorders according to the invention might include Parkinson's disease, Alzheimer's disease, multiple sclerosis, neuropathies, Huntington's disease, presbycusis and amyotrophic lateral sclerosis (ALS).

Particularly preferably, at least one compound according to the present invention is suitable for the treatment and/or prophylaxis of one or more disorders and/or diseases selected from the group consisting of pain, preferably of pain selected from the group consisting of acute pain, chronic pain, visceral pain, headache pain, inflammatory pain and mixed pain; migraine; depression; neurodegenerative diseases, preferably selected from the group consisting of multiple sclerosis, Alzheimer's disease, Parkinson's disease and Huntington's disease; cognitive dysfunctions, preferably cognitive deficiency states, particularly preferably memory disorders; medication dependency; misuse of medication; withdrawal symptoms in medication dependency; development of tolerance to medication, preferably development of tolerance to natural or synthetic opioids; drug dependency; misuse of drugs; withdrawal symptoms in drug dependency; alcohol dependency; misuse of alcohol and withdrawal symptoms in alcohol dependency.

Most particularly preferably, at least one compound according to the present invention according to the invention is suitable for the treatment and/or prophylaxis of pain, preferably of pain selected from the group consisting of acute pain, chronic pain, visceral pain, headache pain, inflammatory pain and mixed pain.

The present invention further relates to a compound according to the present invention and one or more additional pharmaceutically active agents for use in the prophylaxis and/or treatment of disorders and/or diseases which are mediated, at least in part, at least in part, by CaV2.2 channels.

In particular, the present invention therefore further relates to a compound according to the present invention and one or more additional pharmaceutically active agents for the prophylaxis and/or treatment of disorders and/or diseases selected from the group consisting of pain, preferably pain selected from the group consisting of acute pain, chronic pain, visceral pain, headache pain, inflammatory pain and mixed pain; stroke (the neuronal damage resulting from head trauma); mood disorders; epilepsy; schizophrenia, and neurodegenerative disorders.

Most particularly preferred is a compound according to the present invention one or more additional pharmaceutically active agents for the prophylaxis and/or treatment of pain, preferably of pain selected from the group consisting of acute pain, chronic pain, visceral pain, headache pain, inflammatory pain and mixed pain.

Additional pharmaceutically active agents in the treatment of pain may include, for example, i) opiate agonists or antagonists, ii) calcium channel antagonists, iii) 5HT receptor agonists or antagonists, iv) sodium channel antagonists, v) NMDA receptor agonists or antagonists, vi) COX-2 selective inhibitors, vii) NKI antagonists, viii) non-steroidal anti-inflammatory drugs ("NSAID"), ix) selective serotonin reuptake inhibitors ("SSRI") and/or selective serotonin and norepinephrine reuptake inhibitors ("SSNRI"), x) tricyclic antidepressant drugs, xi) norepinephrine modulators, xii) lithium, xiii) valproate, xiv) neurontin (gabapentin), xv) pregabalin.

Additional pharmaceutically active agents in the treatment of depression or anxiety can include other anti-depressant or anti-anxiety agents, such as norepinephrine reuptake inhibitors, selective serotonin reuptake inhibitors (SSRIs), monoamine oxidase inhibitors (MAOIs), reversible inhibitors of monoamine oxidase (RIMAs), serotonin and noradrenaline reuptake inhibitors (SNRIs), adrenoreceptor antagonists, atypical anti-depressants, benzodiazepines, 5-HT1 A agonists or antagonists, especially 5-HT1A partial agonists, neurokinin 1 receptor antagonists, corticotropin releasing factor (CRF) antagonists, and pharmaceutically acceptable salts thereof.

Another embodiment of the present invention therefore relates to use of at least one compound according to the present invention for the preparation of a pharmaceutical composition for the treatment and/or prophylaxis of one or more disorders or diseases, particularly selected from the group consisting of pain, preferably pain selected from the group consisting of acute pain, chronic pain, visceral pain, headache pain, inflammatory pain and mixed pain; stroke; mood disorders; epilepsy; schizophrenia, and neurodegenerative disorders.

Another aspect of the present invention is a method of treatment and/or prophylaxis of disorders and/or diseases in a mammal, preferably of disorders and/or diseases selected from the group consisting of pain, preferably pain selected from the group consisting of acute pain, chronic pain, visceral pain, headache pain, inflammatory pain and mixed pain; stroke; mood disorders; epilepsy; schizophrenia, and neurodegenerative disorders, which comprises administering an effective amount of at least one compound according to the present invention to the mammal.

Another embodiment of the present invention is a method for CaV2.2 calcium channel regulation, preferably for use in CaV2.2 calcium channel blockage, and, further, a method of treatment and/or prophylaxis of disorders and/or diseases, which are mediated, at least in part, by CaV2.2 channels, in a mammal, preferably of disorders and/or diseases selected from the group consisting of pain, preferably pain selected from the group consisting of acute pain, chronic pain, visceral pain, headache pain, inflammatory pain and mixed pain; stroke; mood disorders; epilepsy; schizophrenia, and neurodegenerative disorders, which comprises administering an effective amount of at least one compound according to the present invention to the mammal.

All preferred embodiments of the first aspect of the invention are preferred *vice versa* for the other aspects and embodiments.

The effectiveness against pain can be shown, for example, in the *Bennett* or *Chung* model (Bennett, G.J. and Xie, Y.K., A peripheral mononeuropathy in rat that produces disorders of pain sensation like those seen in man, Pain 1988, 33(1), 87-107; Kim, S.H. and Chung, J.M., An experimental model for peripheral neuropathy produced by segmental spinal nerve ligation in the rat, Pain 1992, 50(3), 355-363), by tail flick experiments (e.g. according to D'Amour und Smith (J. Pharm. Exp. Ther. 72, 74 79 (1941)) or by the formalin test (e.g. according to D. Dubuisson et al., Pain 1977, 4, 161-174).

### EXAMPLES

The compounds according to the invention can be prepared in the manner described below. The following examples further illustrate the invention but are not to be construed as limiting its scope.

All starting materials which are not explicitly described were either commercially available (the details of suppliers such as for example Acros, Avocado, Aldrich, Apollo, Bachem, Fluka, FluoroChem, Lancaster, Manchester Organics, MatrixScientific, Maybridge, Merck, Rovathin, Sigma, TCI, Oakwood, etc. can be found in the Symyx® Available Chemicals Database of MDL, San Ramon, US or the SciFinder® Database of the ACS, Washington DC, US, respectively, for example) or the synthesis thereof has already been described precisely in the specialist literature (experimental guidelines can be found in the Reaxys® Database of Elsevier, Amsterdam, NL or the SciFinder® Database of the ACS, Washington DC, US, repspectively, for example) or can be prepared using the conventional methods known to the person skilled in the art.

The stationary phase used for the column chromatography was silica gel 60 (0.04 - 0.063 mm) from E. Merck, Darmstadt. The reactions were, if necessary, carried out under an inert amosphere (mostly nitrogen).

The yields of the compounds prepared are not optimized. The mixing ratios of solvents are usually stated in the volume / volume ratio. The reactions were, if necessary, carried out under an inert amosphere (mostly N₂). The number of equivalents of reagents and the amounts of solvents employed as well as the reaction temperatures and times can vary slightly between different reactions carried out by the same (general) method. The work-up and purification methods were adapted according to the characteristic properties of each compound and can vary slightly for analogous/ general methods.

All the intermediate products and exemplary compounds were analytically characterized by means of ¹H-NMR spectroscopy. In addition, mass spectrometry tests (MS, m/z for [M+H]⁺) were carried out for all the exemplary compounds and selected intermediate products.

The indication "equivalents" ("eq." or "eq" or "equiv.") means molar equivalents, "RT" or "rt" means RT (23 ± 7 °C), "M" are indications of concentration in mol/l, "aq." means aqueous, "sat." means saturated, "sol." means solution, "conc." means concentrated. The mixing ratios of solvents are usually stated in the volume / volume ratio.

The following abbreviations are used in the descriptions of the experiments:
Boc = tert-butyloxycarbonyl; BOP-Cl = bis(2-oxo-3-oxazolidinyl)phosphinic chloride; CC = column chromatography; d = day(s); DBU = 1,8-Diazabicyclo[5.4.0]undec-7-ene; DIAD = Diisopropyl azodicarboxylate; DCE = 1,1-dichloroethane; DCM = dichloromethane; DIAD = diisopropyl azodicarboxylate; DIPEA = N,N-diisopropylethylamine; DME = 1,2-dimethoxyethane; DMF = N,N-dimethylformamid; DMSO = dimethylsulfoxide; EDCI = N-(3-dimethylaminopropyl)-N'-ethylcarbodiimide hydrochloride; Et₂O = diethyl ether; EtOAc = ethylacetate; EtOH = ethanol; h = hour(s); HATU = O-(7-azabenzotriazol-1-yl)N,N,N',N'-tetramethyluronium hexafluorophosphate; HOAt = 1-hydroxy-7-azabenzotriazole; HOBt = 1-hydroxybenzotriazole hydrate; MeCN = acetonitrile; Mel = iodomethane; MeOH = methanol; NBS = N-bromo-succinimide; OAc = acetate; min = minute(s); n-BuLi = n-butyllithium; Pd₂(dba)₃ = tris(dibenzylideneacetone)dipalladium(0); Pd(dppf)Cl₂ = [1,1'-bis(diphenylphosphino)ferrocene]dichloropalladium(II); PPh₃ = triphenylphosphine; PyBOP= (benzotriazol-1-yl-oxytripyrrolidinophosphonium-hexafluorophosphat); RT = room temperature; TBD = 1,5,7-triazabicyclo[4.4.0]dec-5-ene; tert = tertiary; THF = tetrahydrofuran; TosOH = p-Toluolsulfonic acid; XPhos = 2-di-tert-butylphosphino-2',4',6'-triisopropylbiphenyl.

### 1.Synthesis of example compounds

### 1.1 Synthesis of carboxylic acid building blocks (ACl)

### 1.1.1 3-(4-Chlorophenyl)-1-cyclopropyl-4-methyl-5-(trifluoromethyl)-1H-pyrrole-2-carboxylic acid (ACl-1)

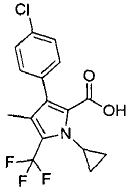

The synthesis of ACl-1 was carried out in analogy to ACl-3.

### 1.1.2 3-(4-Chlorophenyl)-1-cyclopropyl-5-(trifluoromethyl)-1H-pyrrole-2-carboxylic acid (ACl-2)

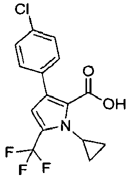

The first 3 steps of the synthesis of ACl-2 were carried out in analogy to ACl-4; last 3 steps in analogy to ACl-3.

### 1.1.3 3-(4-Chloro-2-fluorophenyl)-1-cyclopropyl-4-methyl-5-(trifluoromethyl)-1H-pyrrole-2-carboxylic acid (ACl-3)

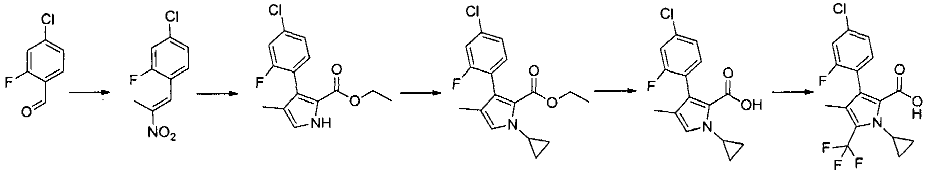

### Step 1: (E)-4-Chloro-2-fluoro-1-(2-nitroprop-1-en-1-yl)benzene

4-Chloro-2-fluorobenzaldehyde (25 g, 157.6 mmol), nitroethane (12.43 mL, 173.35 mmol), trimethyl orthoformate (38 mL, 346.62 mmol), methylamine·HCl (8.3 g, 122.9 mmol) & potassium acetate (10.8 g, 98.14 mmol) in MeOH (125 mL) was heated at reflux for 18 h. The reaction mixture was cooled to RT and diluted with H₂O (200 mL) and extracted with Et₂O (3 x 100 mL). The combined organic layer was successively washed with H₂O (200 mL), brine (200 mL), dried (Na₂SO₄), filtered and concentrated. The residue upon trituration with MeOH (30m L) gave yellow solid. It was filtered off and washed with chilled MeOH (25 mL) to afford 15 g (44 %) of the desired product as a yellow solid.

### Step 2: Ethyl 3-(4-chloro-2-fluorophenyl)-4-methyl-1H-pyrrole-2-carboxylate

(E)-4-Chloro-2-fluoro-1-(2-nitroprop-1-en-1-yl)benzene (15 g, 215 mmol) in THF (100 mL) was treated with ethyl isocyanoacetate (9.6 mL, 88.35 mmol) and DBU (6.97 mL, 88.31 mmol) at 0°C. The reaction mixture was stirred for 2 h at RT. The reaction mixture was partitioned between EtOAc (100 mL) and H₂O (200 mL). The organic layer was successively washed with H₂O (2 x 200 mL), brine solution (200 mL), dried (Na₂SO₄) and concentrated in vacuo. The residue upon purification by column chromatography(silica gel; 100-200 mesh); the pure product eluted with 5% EtOAc in pet-ether to give the desired product (10.5 g, 53 %) as an off white solid.

### Step 3: Ethyl 3-(4-chloro-2-fluorophenyl)-1-cyclopropyl-4-methyl-1H-pyrrole-2-carboxylate

A stirred solution of ethyl 3-(4-chloro-2-fluorophenyl)-4-methyl-1H-pyrrole-2-carboxylate (5 g, 17.79 mmol), cyclopropylboronic acid (3.06 g, 35.58 mmol) and Na₂CO₃ (3.77 g, 35.58 mmol) in 1,2-Dichloro ethane (100 mL) was added to a stirred suspension of Copper(II)acetate (8.72 g, 48.05 mmol), 2,2'-Bipyridyl (2.77 g, 17.79 mmol) in 1,2-Dichloro ethane (100 mL) at RT. The reaction mixture was then heated at 80°C for 48 h. The reaction mixture was allowed to cool to RT and filtered. The filtrate was diluted with H₂O (100 mL) and separated the organic layer. The aqueous layer was extracted with DCM (2x100 mL). The combined organic layer was washed with brine (100 mL), dried (Na₂SO₄) and concentrated to give the crude compound, which was purified by CC (silica gel; 60-120mesh); the product was eluted by 10-12 %EtOAc -pet ether to yield the desired product (3.70 g, 65.02 %) as a brown solid.

### Step 4: 3-(4-Chloro-2-fluorophenyl)-1-cyclopropyl-4-methyl-1H-pyrrole-2-carboxylic acid

8M NaOH (20 mL) was added to a solution of ethyl 3-(4-chloro-2-fluorophenyl)-1-cyclopropyl-4-methyl-1H-pyrrole-2-carboxylate (4 g, 12.5 mmol) in EtOH (50 mL) at RT and refluxed at 100°C for 18 h. The reaction mixture was concentrated to give the residue which was acidified (pH∼2) with 6N HCl at 0°C. The aqueous layer was extracted with EtOAc (2x 100mL). The combined organic layer was washed with brine (200 mL), dried (Na₂SO₄), filtered and concentrated to afford the desired product (3.1 g, 84.9 %) as an off white solid.

### Step 5: 3-(4-Chloro-2-fluorophenyl)-1-cyclopropyl-4-methyl-5-(trifluoromethyl)-1H-pyrrole-2-carboxylic acid (ACl-3)

CF₃I gas (10 g) was bubbled through the solution of 3-(4-chloro-2-fluorophenyl)-1-cyclopropyl-4-methyl-1H-pyrrole-2-carboxylic acid (4.5 g, 15.35 mmol) and FeSO₄.7H₂O (2.56g, 9.21 mmol) in DMSO (45 mL) at RT for 5 min. 30% aqueous H₂O₂ (10 mL, 92.1 mmol) was added at 0°C and whole then stirred at RT for 16 h. The mixture was diluted with H₂O (100 mL) and extracted with Et₂O (3 x 50 mL).The combined organic layer was washed with H₂O (100 mL), brine (100 mL), dried (Na₂SO₄) and concentrated to get the crude, which was purified by CC (silica gel; 60-120mesh); the product eluted with 13-15% EtOAc in petroleum ether to give the desired product (1.97 g, 35.6 %) as an white solid.

### 1.1.4 3-(4-Fluorophenyl)-1-isobutyl-5-(trifluoromethyl)-1H-pyrrole-2-carboxylic acid (ACl-4)

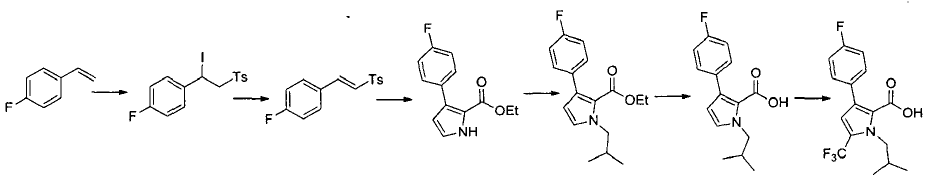

### Step 1: 1-Fluoro-4-(1-iodo-2-tosylethyl)benzene

4-Fluorostyrene (10.5 g, 85.96 mmol) & ammonium ceric nitrate (94.25 g, 171.93 mmol) were added successively to a suspension of sodium p-toluenesulfinate (22.95 g, 128.93 mmol) and Nal (19.3 g, 128.95 mmol) in MeCN (400 mL) under Ar atmosphere at RT and stirred for 12 h. The reaction mixture was concentrated in vacuo and the residue was partitioned between H₂O (300 mL) and DCM (250 mL). The layers were separated and the aqueous phase was extracted with DCM (2 x 250 mL). The combined organic layer was washed with aqueous Na₂S₂O₃ (500 mL), brine (500 mL), dried over anhydrous Na₂SO₄, filtered and concentrated to give the crude product (21 g, 60%).

### Step 2: (E)-1-Fluoro-4-(2-tosylvinyl)benzene

Triethylamine (14.46 mL, 103.94 mmol) was added to a solution of 1-fluoro-4-(1-iodo-2-tosylethyl)-benzene (21 g, 51.9 mmol) in MeCN (210 mL) and stirred for 1 h at RT. The reaction mixture was concentrated under reduced pressure to obtain solid which was dissolved in DCM (200 mL).The organic layer was washed successively with 1*M* KHSO₄ (500 mL), H₂O (500 mL), brine solution (500 mL); dried over anhydrous Na₂SO₄, filtered and concentrated to give crude. The crude was purified by CC [silica gel (100-200mesh; EtOAc: petroleum ether= 1:9%)] to give 8.9 g of the desired product (62%) as a white solid.

### Step 3: Ethyl 3-(4-fluorophenl)-1H-pyrrole-2-carboxylate

NaH (60% in mineral oil; 1.54 g, 38.68 mmol) was added portionwise to a solution of (E)-1-fluoro-4-(2-tosylvinyl)benzene (8.9 g, 32.23 mmol) and ethyl isocyanoacetate (3.85 mL, 35.46 mmol) in THF (200 mL) at 0°C and the reaction mixture then stirred at RT for 2 h. EtOH (20 mL) was added and the solvents were evaporated to give a residue. The residue was partitioned between EtOAc (200 mL) and brine (200 mL) and the layers were separated. The organic layer was washed with H₂O (200 mL), dried (Na₂SO₄), filtered and concentrated to give the crude. The crude was purified by CC (neutral alumina; EtOAc: petroleum ether = 2:8) to give 5 g of the desired product (66 %).

### Step 4: Ethyl 3-(4-fluorophenyl)-1-isobutyl-1H-pyrrole-2-carboxylate

A solution of ethyl 3-(4-fluorophenyl)-1H-pyrrole-2-carboxylate (7 g, 30.03 mmol) in DMF (40 mL) was added to a mixture of 60 % NaH (1.8 g, 45.04 mmol) in DMF (30 mL) at 0°C under N₂ atmosphere and stirred for 15 min. Isobutyl bromide (3.6 mL, 33.01 mmol) was added and the reaction mixture was allowed to warm to RT and then stirred at 80°C for 16 h. The reaction mixture was cooled to RT and quenched with brine (20 mL) at 0°C. Concentration in vacuo gave a residue. The residue was diluted with H₂O (200 mL) and extracted with EtOAc (2 x 100 mL). The combined organic layers were dried (Na₂SO₄), filtered and evaporated in vacuo to give the crude product. The crude product was purified by CC (silica gel (60-120mesh; EtOAc: petroleum ether = 2:8) to give 7 g of the desired product (80%) as a yellow liquid.

### Step 5: 3-(4-Fluorophenyl)-1-isobutyl-1H-pyrrole-2-carboxylic acid

8M NaOH (70 mL) was added to a solution of ethyl 3-(4-fluorophenyl)-1-isobutyl-1H-pyrrole-2-carboxylate (7 g, 24.20 mmol) in EtOH (30 mL) at 0°C. The resulting reaction mixture was stirred at reflux for 16 h. The reaction mixture was cooled to 0°C, acidified (pH∼2) with 6*N* HCl and the aqueous layer was extracted with EtOAc (2 x 100 mL). The combined organic layer was washed with brine (200 mL), dried (Na₂SO₄), filtered and evaporated in vacuo to give crude. The crude product was washed with petroleum ether (2 x 20 mL) and dried under vacuum to give the desired product (4 g, 63%) as a colorless solid.

### Step 6: 3-(4-Fluorophenyl)-1-isobutyl-5-(trifluoromethyl)-1H-pyrrole-2-carboxylic acid (ACl-4)

CF₃I gas (10 g) was bubbled through a solution of 3-(4-fluorophenyl)-1-isobutyl-1H-pyrrole-2-carboxylic acid (4 g, 15.29 mmol) in DMSO (40 mL) and FeSO₄·7H₂O (2.55 g, 9.177 mmol) at RT for 5 min 30% aqueous H₂O₂ (10.4 mL, 91.77 mmol) was added at 0°C and the mixture then stirred at RT for 16 h. The mixture was diluted with H₂O (100 mL) and extracted with EtOAc (3 x 50 mL). The combined organic layer was washed successively with H₂O (150 mL), brine (150 mL), dried (Na₂SO₄) and concentrated in vacuo to give the crude product, which was purified by CC (silica gel; 100-200mesh; 5-10% EtOAc in petroleum ether) to give a solid. Triturating with pentane gave the desired product (1.6 g, 41%) as colorless solid.

### 1.1.5 3-(4-Chlorophenyl)-1-isobutyl-4-methyl-5-(trifluoromethyl)-1H-pyrrole-2-carboxylic acid (ACl-5)

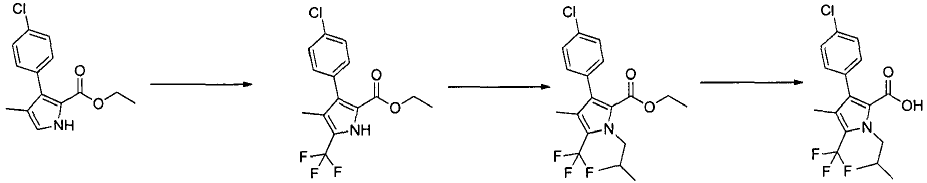

### Step 1: Ethyl 3-(4-chlorophenyl)-4-methyl-5-(trifluoromethyl)-1H-pyrrole-2-carboxylate

A solution of ethyl 3-(4-chlorophenyl)-4-methyl-1H-pyrrole-2-carboxylate [step 2, PY-1] (4.42 g, 16.78 mmol) in dry DMF (75 mL) was cooled in an ice bath under N₂. Finely grounded FeSO₄.7H₂O (2.80 g, 10.07 mmol) was added, followed by trifluoromethanesulfonyl chloride (3.58 mL, 33.6 mmol). Subsequently, 35% aqueous H₂O₂ (4.41 mL, 50.3 mmol) was added dropwise over 5 min. The mixture was stirred at 0°C for 15 min. More FeSO₄.7H₂O (2.80 g, 10.07 mmol) was added, followed by trifluoromethanesulfonyl chloride (1.8 mL, 16.78 mmol) and the dropwise addition of 35% aqueous H₂O₂ (2.0 mL, 22.84 mmol). The mixture was stirred at 0°C for 15 min. The reaction mixture was poured out in ice water (200 mL) and stirred for 30 min. The solid was filtered off and washed with H₂O (2x 20 mL). The product was dissolved in EtOAc (50 mL) and washed with brine (2x 20 mL) before drying on Na₂SO₄ and concentration *in vacuo.* The product was purified using gravity CC (silica, heptane/EtOAc, 98:2 → 9:1) to give the desired product (3.2 g, 57%) as a white solid.

### Step 2: Ethyl 3-(4-chlorophenyl)-1-isobutyl-4-methyl-5-(trifluoromethyl)-1H-pyrrole-2-carboxylate

To a solution of Ethyl 3-(4-chlorophenyl)-4-methyl-5-(trifluoromethyl)-1H-pyrrole-2-carboxylate (3.2 g, 9.65 mmol) in dry DMF (25 mL) under N₂, was added Cs₂CO₃ (6.29 g, 19.29 mmol), followed by 1-iodo-2-methylpropane (1.23 mL, 10.61 mmol). The mixture was stirred at 50°C for 20 h. The reaction mixture was diluted with *i*-Pr₂O (25 mL) and the solid was filtered off. The filtrate was diluted with *i-*Pr₂O (100 mL) and washed with saturated aqueous NaHCO₃ (2x 50 mL) and brine (2x 50 mL) before drying on Na₂SO₄ and concentration *in vacuo.* The product was purified using flash chromatography (silica, gradient heptane/EtOAc, 98:2 → 8:2). Also, a product-containing mix fraction was obtained which was purified further using flash chromatography (silica, gradient heptane/EtOAc, 100:0 → 95:5). Both batches of product were combined and purified further using flash chromatography (silica, heptane/EtOAc, 100:0 → 98:2) to give the desired product (1.83 g, 49%) as a yellow oil.

### Step 3: 3-(4-Chlorophenyl)-1-isobutyl-4-methyl-5-(trifluoromethyl)-1H-pyrrole-2-carboxylic acid (ACl-5)

A solution of NaOH (1.89 g, 47.2 mmol) in H₂O (10 mL) was added to a solution of ethyl 3-(4-chlorophenyl)-1-isobutyl-4-methyl-5-(trifluoromethyl)-1H-pyrrole-2-carboxylate (1.83 g, 4.72 mmol) in a mixture of THF (10 mL) and EtOH (10 mL). The clear yellow solution was heated to 80°C using a reflux condenser for 1 h. The reflux condenser was removed and the heating was continued for 3 h. The reflux condenser was replaced and the heating was continued for 20 h at oil bath temperature 70°C. The reaction mixture was concentrated *in vacuo* to give a yellow solid. The product was diluted with H₂O (20 mL) and cooled in an ice bath before the addition of aqueous 1M KHSO₄ (48 mL) to give a white solid. Filtration was not successful. The product was dissolved in EtOAc (150 mL) and combined with the aqueous filtrate. The aqueous layer was extracted with EtOAc (2x 50 mL) and the combined organic layers were washed with aqueous 1M KHSO₄(2x 50 mL) and brine (2x 50 mL) before drying on Na₂SO₄ and concentration *in vacuo.* The product was purified using gravity CC (silica, heptane/EtOAc, 1:1) to give the desired product (1.407 g, 83%).

### 1.1.6 3-(4-Fluorophenyl)-1-((tetrahydrofuran-2-yl)methyl)-5-(trifluoromethyl)-1H-pyrrole-2-carboxylic acid (ACl-6)

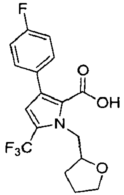

The synthesis of ACl-6 was carried out in analogy to ACl-4.

### 1.1.7 3-(4-Chlorophenyl)-1,4-dimethyl-5-(trifluoromethyl)-1H-pyrrole-2-carboxylic acid (ACl-7)

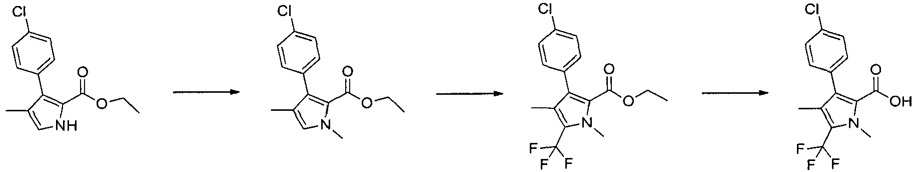

### Step 1: Ethyl 3-(4-chlorophenyl)-1,4-dimethyl-1H-pyrrole-2-carboxylate

To a suspension of ethyl 3-(4-chlorophenyl)-4-methyl-1H-pyrrole-2-carboxylate [step 2, PY-1] (4 g, 15.17 mmol) and K₂CO₃ (4.19 g, 30.3 mmol) in MeCN (50 mL) was added CH I (2.83 mL, 45.5 mmol). The reaction mixture was stirred at 80°C and cooled to RT after 7 h. K₂CO₃ (4.19 g, 30.3 mmol) and Mel (2.83 mL, 45.5 mmol) were added and stirring was continued overnight. The reaction was heated for 7 h at 60°C, cooled to RT and diluted with brine. The aqueous layer was extracted with DCM (250 mL), the combined solvents dried and evaporated to result 4 g yellow oil, which was a mixture of starting material and product (2:1). This mixture was dissolved in dry THF (30 mL) under N₂ atmosphere and 60% NaH in mineral oil (0.780 g, 19.5 mmol) was added portionwise. After 20 min MeI (2.83 mL, 45.5 mmol) was added and the reaction was stirred for 4 h at RT. The reaction mixture was poured into H₂O (300 mL) and extracted with EtOAc (350 mL). The solvents were dried and evaporated, which gave the desired product (3.7 g, 88%) as a yellow oil.

### Step 2: Ethyl 3-(4-chlorophenyl)-1,4-dimethyl-5-(trifluoromethyl)-1H-pyrrole-2-carboxylate

Ethyl 3-(4-chlorophenyl)-1,4-dimethyl-1H-pyrrole-2-carboxylate (2.68 g, 9.65 mmol) was dissolved in DMSO (50 mL) and FeSO₄·7 H₂O (1.610 g, 5.79 mmol) was added. The dark brown solution was stirred for 5 min, then CF₃I (gas) was bubbled through for 2 min. The mixture was stirred for 5 min at RT. Subsequently, the mixture was cooled in an ice bath until the mixture solidifies. Aqueous 30% H₂O₂ (1.971 mL, 19.30 mmol) was added in a slow stream *via* a syringe and the mixture was stirred in an ice bath for 10 min. The reaction was quenched by careful dropwise addition of brine (25 mL) and the mixture was stirred for 5 min at RT. The mixture was diluted with brine (100 mL) and the product was extracted with EtOAc (2x 150mL). The combined organic layers were washed with brine (2x 100 mL) and dried on Na₂SO₄ before concentration *in vacuo* to give 3.13 g of a brown oil. The product was purified using flash CC (silica, gradient heptane/EtOAc 95:5 to 7:3) to give the desired product (1.58 g, 47%) as a yellow oil.

### Step 3: 3-(4-Chlorophenyl)-1,4-dimethyl-5-(trifluoromethyl)-1H-pyrrole-2-carboxylic acid (ACl-7)

A solution of NaOH (1.712 g, 42.8 mmol) in H₂O (7.5 mL) was added to a solution of Ethyl 3-(4-chlorophenyl)-1,4-dimethyl-5-(trifluoromethyl)-1H-pyrrole-2-carboxylate (1.48 g, 4.28 mmol) in a mixture of MeOH (7.5 mL) and THF (7.5 mL). The yellow solution was stirred at reflux for 1 h. The reaction mixture was cooled to RT, the volatiles were removed *in vacuo* and the residue was cooled in an ice bath. The mixture was acidified using aqueous 1M KHSO₄ solution (50 mL) and brine (25 mL) was added. The product was extracted with EtOAc (2x 50 mL). The combined organic layers were washed with brine (25 mL) and dried over Na₂SO₄ before concentration *in vacuo.* The product was recrystallised from *i*-Pr₂O to give the desired product (703 mg, 51%) as colorless flakes.

### 1.1.8 3-(4-chlorophenyl)-1-ethyl-4-methyl-5-(trifluoromethyl)-1H-pyrrole-2-carboxylic acid (ACl-8)

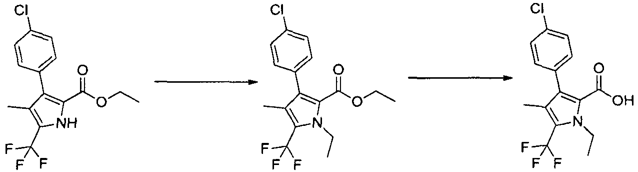

### Step 1: ethyl 3-(4-chlorophenyl)-1-ethyl-4-methyl-5-(trifluoromethyl)-1H-pyrrole-2-carboxylate

To a solution of ethyl 3-(4-chlorophenyl)-4-methyl-5-(trifluoromethyl)-1H-pyrrole-2-carboxylate (0.310 g, 0.94 mmol) in MeCN (10 mL) were added K₂CO₃ (0.260 g, 1.88 mmol) and ethyl iodide (0.440 g, 2.82 mmol, 0.23 mL) The reaction mixture was refluxed overnight under N₂ atmosphere. The reaction mixture was concentrated under reduced pressure. The residue was diluted with H₂O and extracted with EtOAc. The combined organic layer was washed with brine, dried over anhydrous Na₂SO₄ and evaporated under reduced pressure. The obtained crude product was purified by flash CC (silica, gradient 10% hexane/EtOAc 90:10) to afford desired product (0.325 g, 96 %) as brown sticky material.

### Step 2: 3-(4-chlorophenyl)-1-ethyl-4-methyl-5-(trifluoromethyl)-1H-pyrrole-2-carboxylic acid (ACl-8)

To a solution of ethyl 3-(4-chlorophenyl)-1-ethyl-4-methyl-5-(trifluoromethyl)-1H-pyrrole-2-carboxylate (0.325 g, 0.9 mmol) in a mixture of solvents THF : MeOH : H₂O (3 : 2 : 1) (8 mL) was added LiOH·H₂O (0.378 g, 9 mmol).The reaction mixture was refluxed for 4 h. The reaction mixture was concentrated under reduced pressure. The residue was diluted with H₂O, acidified (pH 2) with aqueous 6N HCl solution and extracted with EtOAc. The combined organic layers were washed with brine, dried over anhydrous Na₂SO₄ and evaporated under reduced pressure to afford desired compound (0.325 g, 96 %) as off-white solid.

### 1.1.9 3-(4-Chlorophenyl)-1-isopropyl-4-methyl-5-(trifluoromethyl)-1H-pyrrole-2-carboxylic acid (ACl-9)

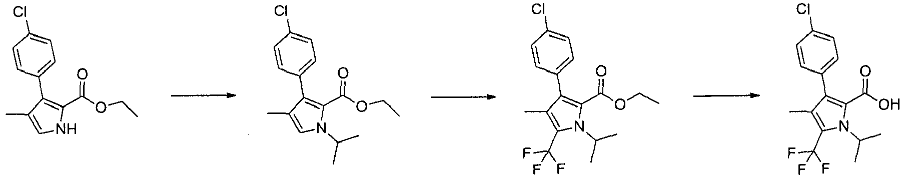

### Step 1: Ethyl 3-(4-chlorophenyl)-1-isopropyl-4-methyl-1H-pyrrole-2-carboxylate

To a solution of ethyl 3-(4-chlorophenyl)-4-methyl-1H-pyrrole-2-carboxylate [step 2, PY-1 (3.0 g, 11.38 mmol) in dry DMF (30 mL) was added Cs₂CO₃ (18.53 g, 56.9 mmol) and 2-iodopropane (3.41 mL, 34.1 mmol). The mixture was stirred in a sealed vessel at RT for 20 h. More Cs₂CO₃ (7.41 g, 22.75 mmol) was added, followed by 2-iodopropane (1.14 mL, 11.38 mmol). Stirring was continued at RT for 72 h. The mixture was cautiously acidified with aqueous 1M KHSO₄ and the product was extracted with EtOAc/*i*-Pr₂O (1/1, v/v, 300 mL). The organic layer was washed with aqueous 1M KHSO₄ (2x50 mL) and brine (2x50 mL) before drying on Na₂SO₄ and concentration *in vacuo* to give the desired product (3.34 g, 96%) as an orange oil which solidified on standing.

### Step 2: Ethyl 3-(4-chlorophenyl)-1-isopropyl-4-methyl-5-(trifluoromethyl)-1H-pyrrole-2-carboxylate

To a cooled solution of ethyl 3-(4-chlorophenyl)-1-isopropyl-4-methyl-1H-pyrrole-2-carboxylate (3.3 g, 10.79 mmol) in DMF (100 mL) was added trifluoromethanesulfonyl chloride (2.46 mL, 21.58 mmol) and finely grounded FeSO₄·7H₂O (1.80 g, 6.47 mmol). After 5 min, 30% aqueous H₂O₂ (3.63 mL, 32.4 mmol) was added dropwise *via* a syringe over 30 min, keeping the temperature below 10°C. The mixture was stirred at 3°C for 1.5 h. The reaction mixture was poured onto ice cold H₂O (400 mL) and the product was extracted with *i*-Pr₂O (2x250 mL). The combined organic layers were washed with saturated aqueous NaHCO₃ (2x50 mL) and brine (2x50 mL) before drying on Na₂SO₄ and concentration *in vacuo.* The crude product was purified using flash cc (silica, gradient heptane/EtOAc, 100:0 → 95:5) to give the desired product (1190 mg, 29%) as a white solid. Impure fractions were purified further using flash chromatography (silica, gradient heptane/EtOAc, 99:1 → 97:3) and purified again using flash cc (silica, gradient heptane/EtOAc, 100:0 → 98:2) to give another batch of the desired product (822 mg, 20%) as an off-white solid. Total yield: 2.01 g (50%).

### Step 3: 3-(4-Chlorophenyl)-1-isopropyl-4-methyl-5-(trifluoromethyl)-1H-pyrrole-2-carboxylic acid (ACl-9)

To a solution of ethyl 3-(4-chlorophenyl)-1-isopropyl-4-methyl-5-(trifluoromethyl)-1H-pyrrole-2-carboxylate (823 mg, 2.202 mmol) in DMSO (4 mL) was added NaOH (352 mg, 8.81 mmol) and the mixture was stirred at RT for 3 h. More DMSO (4 mL) was added to aid stirring and also more NaOH (176 mg, 4.4 mmol) was added. Stirring was continued for 1 h, the reaction mixture was cooled in an ice bath and acidified using aqueous 1M KHSO₄ (14.3 mL). The suspension was stirred vigorously to break up sticky particles. A solid had formed which was filtered off and washed with H₂O (2x10 mL) and EtOAc (2x25 mL) and discarded. Also a lump of gum was formed on the stirring bar, which was dissolved in EtOAc/H₂O (1/1, v/v, 25 mL). All layers were combined and separated. The organic layer was washed with aqueous 1M KHSO₄ (2x20 mL) and brine (2x20 mL) before drying on Na₂SO₄ and concentration *in vacuo* to give the desired product (757 mg, 99%) as an off-white solid.

### 1.1.10 3-(5-Chloropyridin-2-yl)-1-isopropyl-4-methyl-5-(trifluoromethyl)-1H-pyrrole-2-carboxylic acid (ACl-10)

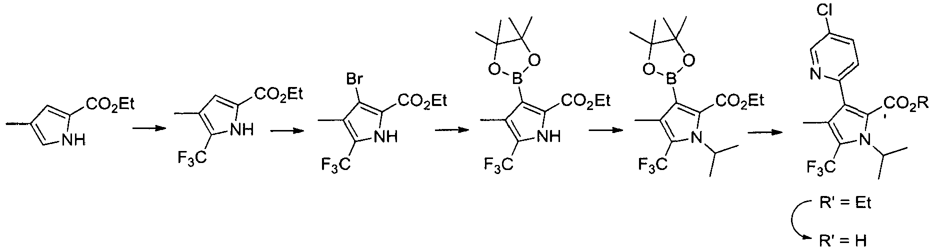

### Step 1: Ethyl 4-methyl-5-(trifluoromethyl)-1H-pyrrole-2-carboxylate

To a solution of ethyl 4-methyl-1H-pyrrole-2-carboxylate (9.05 g, 59.1 mmol) and trifluoromethanesulfonyl chloride (12.5 mL, 118 mmol) in dry DMF (275 mL) was added FeSO₄.7H₂O (9.86 g, 35.4 mmol). The reaction was cooled with an icebath and 30% aqueous H₂O₂ (12.1 mL, 118 mmol) was dropwise added. The reaction mixture was stirred at RT for 1.5 h. The reaction mixture was cooled with an icebath and subsequently trifluoromethanesulfonyl chloride (12.5 mL, 118 mmol), FeSO₄·7H₂O (9.86 g, 35.4 mmol) and dropwise 30% aqueous H₂O₂ (12.1 mL, 118 mmol) were added. The reaction mixture was stirred at RT for 1h and the reaction mixture was poured into icewater while stirring vigourously. A white solid was filtered off and washed with ice cold H₂O (2x 50 mL), dissolved in EtOAc, washed with brine, dried (Na₂SO₄), filtered and concentrated resulting in the desired product (10.51 g, 74%).

### Step 2: Ethyl 3-bromo-4-methyl-5-(trifluoromethyl)-1H-pyrrole-2-carboxylate

To a solution of ethyl 4-methyl-5-(trifluoromethyl)-1H-pyrrole-2-carboxylate (10.5 g, 43.7 mmol) in MeCN (125 mL) was added K₂CO₃ (6.56 g, 47.5 mmol). The reaction mixture was cooled with an icebath and NBS (8.45 g, 47.5 mmol) was portionwise added. The reaction mixture was stirred for 10 min and allowed to warm to RT. To the reaction mixture was added H₂O (250 mL) and the reaction mixture was stirred for 45 min and the precipitate was filtered off, washed with H₂O (2x 250 mL) and dried on filter for 1h. The residue was dissolved in Et₂O, dried (Na₂SO₄) and concentrated to give the desired product (12.22 g, 80%).

### Step 3: Ethyl 4-methyl-3-(4,4,5,5-tetramethyl-1,3,2-dioxaborolan-2-yl)-5-(trifluoromethyl)-1H-pyrrole-2-carboxylate

XPhos (1.10 g, 2.30 mmol) was added to a solution of ethyl 3-bromo-4-methyl-5-(trifluoromethyl)-1H-pyrrole-2-carboxylate (6.00 g, 18.6 mmol) in dry dioxane (60 mL) and argon was bubbled through the reaction mixture for 15 min. To the reaction mixture was added 4,4,5,5-tetramethyl-1,3,2-dioxaborolane (8.70 mL, 60.0 mmol), Et₃N (8.36 mL, 60 mmol) and Pd₂(dba)₃ (0.549 g, 0.600 mmol) and the reaction mixture was stirred at 100 °C for 1h (pre-heated oil bath). The reaction mixture was allowed to cool to to RT The reaction mixture was diluted with EtOAc, washed with saturated aqueous NH₄Cl and brine, dried (Na₂SO₄) and concentrated. CC (silica, heptane/EtOAc, 6:1) of the residue yielded the desired product (6.07 g, 93%).

### Step 4: Ethyl 1-isopropyl-4-methyl-3-(4,4,5,5-tetramethyl-1,3,2-dioxaborolan-2-yl)-5-(trifluoromethyl)-1H-pyrrole-2-carboxylate

Under an argon atmosphere and cooled with an ice-bath, DIAD (2.12 mL, 10.9 mmol) was added dropwise to a solution of PPh₃ (3.58 g, 13.7 mmol) and ethyl 4-methyl-3-(4,4,5,5-tetramethyl-1,3,2-dioxaborolan-2-yl)-5-(trifluoromethyl)-1H-pyrrole-2-carboxylate (3.16 g, 9.10 mmol) in dry THF (30 mL). The reaction mixture was stirred vigourously for 30 min and *i*-PrOH (0.842 mL, 10.9 mmol) was dropwise added. The ice-bath was removed and the reaction mixture was stirred for 1h. Extra PPh₃ (3.58 g, 13.7 mmol) was added followed by the dropwise addition of DIAD (2.12 mL, 10.9 mmol) and the reaction mixture was stirred for 15 min. To the reaction mixture was dropwise added *i*-PrOH (0.842 mL, 10.9 mmol) and stirring was continued for 2 h and the reaction mixture was concentrated. To the residue was added *i*-Pr₂O and the resulting precipitate was filtered off and the filtrate was concentrated. The crude product was purified by cc (1^{st}: silica, heptane/EtOAc, 9:1; 2^{nd}: silica, heptane/EtOAc, 9:1) to give the desired product (2.28 g, 64%).

### Step 5: Ethyl 3-(5-chloropyridin-2-yl)-1-isopropyl-4-methyl-5-(trifluoromethyl)-1H-pyrrole-2-carboxylate

To a solution of ethyl 1-isopropyl-4-methyl-3-(4,4,5,5-tetramethyl-1,3,2-dioxaborolan-2-yl)-5-(trifluoromethyl)-1H-pyrrole-2-carboxylate (2.05 g, 5.27 mmol) and 2-bromo-5-chloropyridine (1.520 g, 7.90 mmol) in DME (40 mL) was added Cs₂CO₃ (5.15 g, 15.8 mmol) and H₂O (10 mL). The mixture was degassed by bubbling Argon for 30 min. Pd(dppf)Cl₂ (385 mg, 0.527 mmol) was added and the mixture was placed in a pre-heated oil-bath at 105 °C. The mixture was heated to reflux for 3 h. More 2-bromo-5-chloropyridine (0.052 g, 0.270 mmol) and Pd(dppf)Cl₂ (0.1 g, 0.137 mmol) were added and heating was continued for 30 min. The mixture was cooled to RT and diluted with CH₂Cl₂ (50 mL). The solution was filtered over Celite and the residue washed with CH₂Cl₂. The combined filtrate was concentrated *in vacuo* and the residue was partitioned between EtOAc (100 mL) and saturated aqueous NaHCO₃ (100 mL). The organic layer was washed with saturated aqueous NaHCO₃ (2x50 mL) and brine (2x50 mL) before drying on Na₂SO₄ and concentration *in vacuo.* The crude product was purified using flash cc (silica, gradient heptane/EtOAc, 1:0 → 9:1) to give the desired product (1.48 g, 75%) as a white solid.

### Step 6: 3-(5-Chloropyridin-2-yl)-1-isopropyl-4-methyl-5-(trifluoromethyl)-1H-pyrrole-2-carboxylic acid (ACl-10)

To a solution of ethyl 3-(5-chloropyridin-2-yl)-1-isopropyl-4-methyl-5-(trifluoromethyl)-1H-pyrrole-2-carboxylate (1.48 g, 3.95 mmol) in DMSO (25 mL) was added NaOH (0.948 g, 23.69 mmol) and the resulting white suspension was stirred at RT for 1.5 h. The mixture was stirred at 40 °C for 1.5 h. The reaction mixture was diluted with H₂O (25 mL) and aqueous 1M KHSO₄ (24 mL) was added. The solid was filtered off, the residue washed with H₂O (2x25 mL) and dried on air under a N₂-flow to give the desired product (1.48 g, '108%') as a white solid.

### 1.1.11 3-(5-Chloropyrimidin-2-yl)-1-isopropyl-methyl-5-(trifluoromethyl)-1H-pyrrole-2-carboxylic acid (ACl-11)

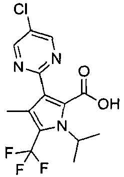

The synthesis of ACl-11 was carried out in analogy to ACl-10.

### 1.2 Synthesis of pyrrole building blocks (PY)

### 1.2.1 (3-(4-Chlorophenyl)-4-methyl-1H-pyrrol-2-yl)(morpholino)methanone (PY-1)

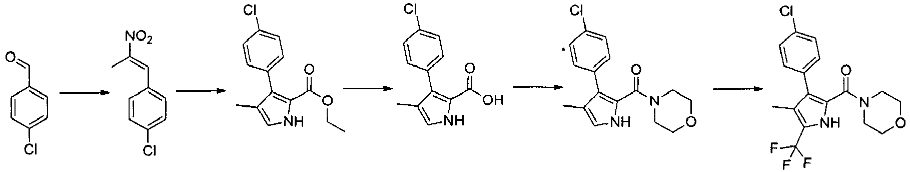

### Step 1: (E)-1-Chloro-4-(2-nitroprop-1-en-1-yl)benzene

A solution of 4-chlorobenzaldehyde (58.5 g, 416 mmol), nitroethane (90 mL, 1249 mmol) and piperidine (8.22 mL, 83 mmol) in toluene (400 mL) was stirred at reflux (Dean-Stark) for 4 h. The mixture was left standing at RT overnight. The solvent was removed under reduced pressure and the residue recrystallised from absolute EtOH to furnish 51.81 g (63%) of the desired product.

### Step 2: Ethyl 3-(4-chlorophenyl)-4-methyl-1H-pyrrole-2-carboxylate

To a suspension of (E)-1-chloro-4-(2-nitroprop-1-en-1-yl)benzene (11.6 g, 58.7 mmol) and ethyl 2-isocyanoacetate (7.0 g, 62 mmol) in dry THF (30 mL) and *i*-PrOH (30 mL) was added 2.6 mmol TBD/g polystyrene (24.8 g, 64.4 mmol). The reaction mixture was stirred at RT overnight. The suspension was filtered, the residue washed with *i*-PrOH/THF (1/1, v/v, 40 mL), followed by EtOH (ca. 20 mL). The combined filtrate was evaporated under reduced pressure, to furnish 16.95 g ('109%') of the desired product.

### Step 3: 3-(4-Chlorophenyl)-4-methyl-1H-pyrrole-2-carboxylic acid

To a solution of ethyl 3-(4-chlorophenyl)-4-methyl-1H-pyrrole-2-carboxylate (16.95 g, max. 58.7 mmol) in absolute EtOH (30 mL) and THF (30 mL) was added H₂O (30 mL) and NaOH (51.4 g, 1.285 mol) and the reaction mixture was stirred at reflux for 90 min and left standing at RT overnight. The reaction mixture was concentrated *in vacuo.* Upon cooling on an ice bath, ice (250 mL) was added followed by the dropwise addition of aqueous 6 M HCl (250 mL). After stirring at 0°C for 2 h, the suspension was filtered and the residue washed with H₂O (2x 50 mL). The product was dried on a filter overnight to obtain 12.30 g (89% over two steps) of the desired product.

### Step 4: (3-(4-Chlorophenyl)-4-methyl-1H-pyrrol-2-yl)(morpholino)methanone

A suspension of 3-(4-chlorophenyl)-4-methyl-1H-pyrrole-2-carboxylic acid (7.23 g, 30.7 mmol) and morpholine (6.68 mL, 77 mmol) in DCM (100 mL) was cooled to 0°C. EDCI (7.65 g, 39.9 mmol) was added followed by HOAt (3.76 g, 27.6 mmol). The reaction mixture was stirred at RT overnight. DCM (100 mL) was added and the mixture was extracted with aqueous 1M KHSO₄ (3x 200 mL), saturated aqueous NaHCO₃ (200 mL), brine (200 mL), dried (Na₂SO₄) and evaporated under reduced pressure, to give 7.91 g (85%) of the desired product.

### Step 5: (3-(4-Chlorophenyl)-4-methyl-5-(trifluoromethyl)-1H-pyrrol-2-yl)(morpholino)methanone (PY-1)

Trifluoromethanesulfonyl chloride (0.522 mL, 4.92 mmol) was added to a solution of (3-(4-chlorophenyl)-4-methyl-1H-pyrrol-2-yl)(morpholino)methanone (1.00 g, 3.28 mmol) in dry DMF (30 mL). At 0°C, FeSO₄·7H₂O (0.365 g, 1.312 mmol) was added followed by the dropwise addition of 30% aqueous H₂O₂ (0.670 mL, 6.56 mmol). After 1 h, the reaction mixture was added to ice/H₂O (100 mL) and stirred vigorously for 15 min. Filtration was attempted, but not successful. The mixture was extracted with EtOAc (2x100 mL). The combined organic layers were extracted with brine (2x 100 mL), dried (Na₂SO₄) and left standing overnight. The suspension was filtered and the filtrate evaporated under reduced pressure. The product was combined with the batch of crude product described below and purified. Trifluoromethanesulfonyl chloride (4.60 mL, 43.4 mmol) was added to a solution of(3-(4-chlorophenyl)-4-methyl-1H-pyrrol-2-yl)(morpholino)methanone (6.62 g, 21.72 mmol) in dry DMF (200 mL). At 0°C, FeSO₄·7H₂O (3.62 g, 13.03 mmol) was added followed by the dropwise addition of 30% aqueous H₂O₂ (6.66 mL, 65.2 mmol). After 30 min, the reaction mixture was added to ice/H₂O (500 mL) and stirred vigorously for 15 min. Filtration was attempted, but not successful. The mixture was extracted with EtOAc (3x200 mL). The combined organic layer was extracted with brine (2x100 mL), dried (Na₂SO₄), evaporated under reduced pressure and co-evaporated with toluene (2x). The resulting batch of crude product was combined with the crude batch described above and purified by CC (silica, heptane/EtOAc, 4:1 → 3:1 → 2:1 → 1:1), to afford 6.03 g (65%) of the desired product.

### 1.2.2 3-(4-Chlorophenyl)-N-methyl-N-neopentyl-5-(trifluoromethyl)-1H-pyrrole-2-carboxamide (PY-2)

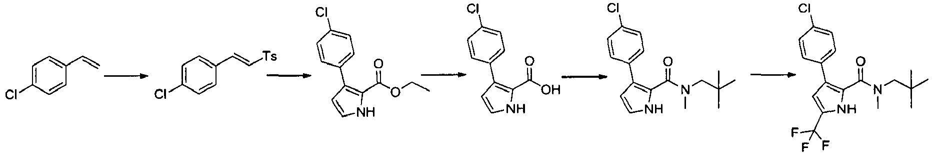

### Step 1: (E)-1-Chloro-4-(2-tosylvinyl)benzene

To a suspension of sodium 4-methylbenzenesulfinate (68.7 g, 386 mmol) in DMSO (350 mL) was added AcOH (350 mL). Subsequently, KI (61.1 g, 368 mmol), 2,2'-bypyridine (5.48 g, 35.1 mmol), CuI (6.68 g, 35.1 mmol) and 4-chlorostyrene (40.7 mL, 317 mmol) were added. The reaction mixture was directly warmed to 100°C with a preheated oil bath and stirred at this temperature overnight. The reaction mixture was allowed to cool to RT and then poured into ice water (1.4 L). The formed suspension was stirred for 0.5 h and the precipitate was filtered, washed with H₂O (3x 700 mL) and a small amount of *i*-Pr₂O (2x). The precipitate was dissolved in hot toluene (1 L), filtered and the remaining impurities were washed with hot toluene (2x). The combined filtrate was left standing to crystallize. The crystals were washed with toluene (2x) and dried on filter to give the desired product (37.07 g). The mother liquor was concentrated and crystallized (*i*-PrOH) to give extra product (21.54 g). The crystals were combined (58.61 g, 63%).

### Step 2: Ethyl 3-(4-chlorophenyl)-1H-pyrrole-2-carboxylate

During a period of 30 min, a solution of ethyl 2-isocyanoacetate (25.0 g, 221 mmol) and (E)-1-chloro-4-(2-tosylvinyl)benzene (58.6 g, 200 mmol) in dry THF (200 mL) and dry DMF (200 mL) was added to a suspension of 60% NaH in mineral oil (9.61 g, 240 mmol) in dry THF (400 mL) while cooling with a water bath. The reaction mixture was stirred for 2 h, then quenched with saturated aqueous NH₄Cl (400 mL) and concentrated to a smaller volume. The residue was extracted with toluene (1 L). The organic layer was washed with saturated aqueous NaHCO₃ (400 mL) and brine (2x 400 mL), dried (Na₂SO₄) and concentrated. The residue was dissolved in hot Et₂O, filtered and the filtrate was concentrated. The residue was subjected to CC (silica, toluene/acetone, 99:1) giving two fractions.

The first fraction was pure product (18.2 g) and the second fraction was purified further by crystallization (MeCN/H₂O) to give the desired product (1.93 g). The mother liquor was concentrated and crystallization (MeCN/H₂O/EtOH) of the residue gave also product (1.37 g). The combined yield of the desired product was 21.5 g (43%).

### Step 3: 3-(4-Chlorophenyl)-1H-pyrrole-2-carboxylic acid

To a suspension of ethyl 3-(4-chlorophenyl)-1H-pyrrole-2-carboxylate (20.1 g, 80.0 mmol) in EtOH (80 mL) and H₂O (80 mL) was added LiOH·H₂O (16.9 g, 402 mmol) and the reaction mixture was stirred at 80°C overnight. Extra LiOH·H₂O (6.76 g, 161 mmol) was added and the reaction mixture was stirred at 100°C for 3 h. The reaction mixture was concentrated to a smaller volume and extra H₂O was added. The reaction mixture was stirred at 100°C for 2 h and at RT over the weekend. The reaction mixture was acidified with aqueous 1M KHSO₄ while cooling with an ice bath. The formed precipitate was filtered off, washed with H₂O (2x) and dried on filter overnight. The residue was suspended in EtOH (200 mL) and H₂O (200 mL) and LiOH·H₂O (33.8 g, 805 mmol) was added. The reaction mixture was stirred at reflux overnight, allowed to cool to RT and concentrated. The reaction mixture was acidified with aqueous 1M KHSO₄ while cooling with an icebath. The formed precipitate was filtered, washed with H₂O (2x) and dried on filter overnight. The product was dissolved in EtOAc, dried (Na₂SO₄) and concentrated to give impure product (14.8 g, 52% pure, 43%).

### Step 4: 3-(4-Chlorophenyl)-N-methyl-N-neopentyl-1H-pyrrole-2-carboxamide

To 3-(4-chlorophenyl)-1H-pyrrole-2-carboxylic acid (14.8 g, 52% pure, 34.7 mmol) in dry DME (200 mL) was added DIPEA (25 mL, 143 mmol), N,2,2-trimethylpropan-1-amine hydrochloride **AMN-1** (7.17 g, 52.1 mmol) and BOP-Cl (10.6 g, 41.7 mmol). The reaction mixture was stirred at reflux temperature for 1h. The reaction mixture was diluted with EtOAc (1 L), washed with aqueous 1M KHSO₄ and saturated aqueous NaHCO₃, dried (Na₂SO₄) and concentrated. The residue was stirred in *i*-Pr₂O for 10 min. and the solids were filtered off. Crystallisation (EtOAc) of the residue and its subsequent mother liquor gave the desired product (7.45 g, 70%).

### Step 5: 3-(4-Chlorophenyl)-N-methyl-N-neopentyl-5-(trifluoromethyl)-1H-pyrrole-2-carboxamide

K₂HPO₄ (1.71 g, 9.84 mmol) was added to a solution of 3-(4-chlorophenyl)-N-methyl-N-neopentyl-1H-pyrrole-2-carboxamide (1.00 g, 3.28 mmol) in dry DMF (30 mL) and argon was bubbled through the reaction mixture for 15 min. Dichlorotris(1,10-phenanthroline)ruthenium(II) hydrate (0.118 g, 0.164 mmol) and trifluoromethanesulfonyl chloride (0.522 mL, 4.92 mmol) were added and the suspension was irradiated by a light bulb (E27-23W, 4000K, 165 mA) overnight. Extra trifluoromethanesulfonyl chloride (0.522 mL, 4.92 mmol) was added to the reaction mixture and the suspension was irradiated by a light bulb for another 4 h. The reaction mixture was poured into ice cold H₂O, stirred for 10 min and the precipitate was filtered off. The precipitate was washed with H₂O (2x) and dried on filter. The residue was first purified by CC (silica, heptane/EtOAc, 3:1) and then by crystallisation (*i*-Pr₂O/ heptane) to give the desired product (400 mg, 33%). The mother liquor was concentrated and crystallisation (i-Pr₂O/heptane) of the residue gave extra product (69 mg, 6%). Total yield: 469 mg (39%).

### 1.2.3 (3-(4-Chlorophenyl)-4-methyl-5-(trifluoromethyl)-1H-pyrrol-2-yl)(2,2-dimethylmorpholino)-methanone (PY-3)

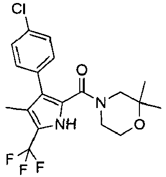

The synthesis of PY-3 was carried out in analogy to PY-1.

### 1.2.4 3-(4-Chlorophenyl)-N,4-dimethyl-N-(2-(methylsulfonyl)ethyl)-5-(trifluoromethyl)-1H-pyrrole-2-carboxamide (PY-4)

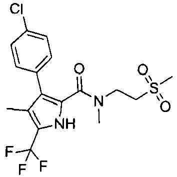

The synthesis of PY-4 was carried out in analogy to PY-1.

### 1.3 Synthesis of alcohol building blocks (AOH)

### 1.3.1 2-(3,3-Difluoroazetidin-1-yl)ethanol (AOH-1)

A suspension of 3,3-difluoroazetidine hydrochloride (100 mg, 0.772 mmol) and K₂CO₃ (320 mg, 2.316 mmol) in MeCN (3 mL) was prepared and 2-bromoethanol (0.071 mL, 1.00 mmol) was added. The reaction mixture was stirred at 80°C overnight with exclusion of light. The suspension was decanted and the supernatant was filtered over Celite. The residue was combined with EtOAc (20 mL) and stirred well, the resulting supernatant was filtered over Celite. The combination of filtrates was concentrated *in vacuo* to result in 58 mg of crude product as a turbid oil.

### 1.3.2 3-Cyclopropylprop-2-yn-1-ol (AOH-2)

2.5 M *n*-BuLi in hexanes (13.31 mL, 33.3 mmol) was added to cyclopropyl acetylene (2.56 mL, 30.3 mmol) in dry THF (50 mL) at 0°C. The reaction mixture was stirred at 0°C for 1 h, then cooled to -78°C. Paraformaldehyde (1.14 g, 37.8 mmol) was added at -78°C. The reaction mixture was then stirred at -78°C and warmed to RT overnight. The reaction mixture was then concentrated *in vacuo,* EtOAc (100 mL) was added and the organic layer was washed with brine (50 mL). The aqueous layer was extracted with EtOAc. The combined organic layer was concentrated *in vacuo.* The product was purified by flash chromatography (silica, gradient heptane/EtOAc, 1:0 → 65:35) to give a slightly yellow coloured oil of the desired product (1.52 g, 52%).

### 1.3.3 tert-Butyl (1-(hydroxymethyl)cyclopropyl)carbamate (AOH-3)

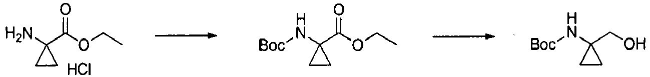

### Step 1: Ethyl 1-((tert-butoxycarbonyl)amino)cyclopropanecarboxylate

To a solution of ethyl 1-aminocyclopropanecarboxylate hydrochloride (3.22 g, 19.4 mmol) in DCM (35 mL) was added triethylamine (2.71 mL, 19.4 mmol) upon which a suspension was obtained. A solution of Boc₂O (4.24 g, 19.4 mmol) in DCM (5 mL) was added dropwise over ca. 2 min. The reaction mixture was stirred at RT overnight. Aqueous 1 M KHSO₄ (100 mL) and DCM (50 mL) were added. The organic layer was separated, dried (Na₂SO₄), evaporated under reduced pressure and co-evaporated with THF (2x), to afford 4.31 g (97%) of the desired product.

### Step 2: tert-Butyl (1-(hydroxymethyl)cyclopropyl)carbamate (AOH-3)

Ethyl 1-((tert-butoxycarbonyl)amino)cyclopropanecarboxylate (4.31 g, 18.80 mmol) was dissolved in dry THF (20 mL), 2 M LiBH₄ in THF (10.34 mL, 20.68 mmol) was added dropwise and the reaction mixture was stirred at RT overnight. More 2 M LiBH₄ in THF (5 mL, 10 mmol) was added and stirring at RT was continued for 6 h. Na₂SO₄ (15 g) was added followed by H₂O (10 mL). The suspension was stirred at RT over the weekend. The suspension was filtered over a pad of Na₂SO₄ and the residue washed with DCM. The combined filtrate was evaporated under reduced pressure. The product was purified by CC (silica, heptane/EtOAc, 1:1), to furnish 2.78 g (79%) of the desired product.

### 1.3.4 (1-((Tetrahydro-2H-pyran-2-yl)oxy)cyclopropyl)MeOH (AOH-4)

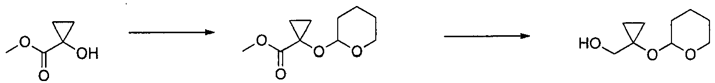

### Step 1: Methyl 1-((tetrahydro-2H-pyran-2-yl)oxy)cyclopropanecarboxylate

Methyl 1-hydroxycyclopropanecarboxylate (1.12 g, 9.65 mmol) was dissolved in DCM (15 mL) and 3,4-dihydro-2H-pyran (0.9 mL, 10.13 mmol) was added, followed by pyridinium p-toluenesulfonate (0.242 g, 0.965 mmol). The colorless solution was stirred at RT in a sealed vessel for 16 h. The reaction mixture was concentrated *in vacuo.* The white suspension was partitioned between brine (20 mL) and Et₂O (20 mL) and the layers were separated. The organic layer was washed with brine (2x 5 mL) and dried on Na₂SO₄ before concentration *in vacuo.* The product was purified using CC (silica, gradient heptane/EtOAc, 1:0 → 8:2) to give the desired product (1.16 g, 60%) as a colorless oil.

### Step 2: (1-((Tetrahydro-2H-pyran-2-yl)oxy)cyclopropyl)MeOH (AOH-4)

Under N₂ atmosphere, methyl 1-((tetrahydro-2H-pyran-2-yl)oxy)cyclopropanecarboxylate (520 mg, 2.60 mmol) was dissolved in dry Et₂O (15 mL) and 4 M LiAlH₄ in Et₂O (0.69 mL, 2.73 mmol) was added slowly *via* a syringe. The resulting solution was stirred heated to reflux for 2 h. More 4 M LiAlH₄ in Et₂O (0.65 mL, 2.60 mmol) was added and the heating was continued for 2 h. The heating was ceased and the stirring was continued for 16 h. The reaction was quenched by careful addition of ice-H₂O (30 mL). The product was extracted with EtOAc (2x25 mL) and the organic layer was washed with saturated aqueous NaHCO₃ (3x 20 mL) and brine (2x20 mL) before drying on Na₂SO₄ and concentration *in vacuo* to give a light yellow oil. The product was dissolved in heptane and purified using flash CC (silica, gradient heptane/EtOAc, 95:5 → 1:1) to give the desired product (245 mg, 55%) as a colorless oil.

### 1.4 Synthesis of amine building blocks (AMN)

### 1.4.1 N,2,2-Trimethylpropan-1-amine hydrochloride (AMN-1)

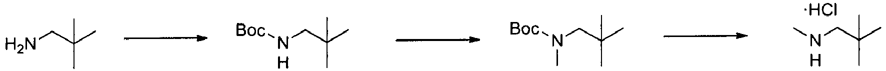

### Step 1: tert-Butyl neopentylcarbamate

To a solution of 2,2-dimethylpropan-1-amine (17.90 g, 205 mmol) in DCM (150 mL) was dropwise added a solution of Boc₂O (44.8 g, 205 mmol) in DCM (50 mL) at 0°C. After complete addition, stirring was continued at RT overnight. The solvent was removed under reduced pressure and the residue co-evaporated with DCM (3x). EtOAc (250 mL) was added and the mixture was washed with H₂O (2x 250 mL). The organic layer was dried (Na₂SO₄) and evaporated under reduced pressure, to give 36.98 g (96%) of the desired product.

### Step 2: tert-Butyl methyl(neopentyl)carbamate

A solution of tert-butyl neopentylcarbamate (37.0 g, 198 mmol) in dry DMF (100 mL) was added to a suspension of 60% NaH in mineral oil (15.8 g, 395 mmol) in dry DMF (200 mL) under a nitrogen atmosphere in 10 min and the reaction mixture was stirred for 1 h. To the reaction mixture was added Mel (30.9 mL, 494 mmol) in 10 min while cooling with an icebath and the reaction mixture was stirred at RT overnight. The reaction mixture was quenched with ice/H₂O (600 mL) and extracted with Et₂O (1 L). The organic layer was washed with brine, dried (Na₂SO₄) and concentrated to result in the desired product (45.6 g, '115%').

### Step 3: N,2,2-Trimethylpropan-1-amine hydrochloride (AMN-1)

To a solution of tert-butyl methyl(neopentyl)carbamate (45.6 g, max. 198 mmol) in dry 1,4-dioxane (200 mL) was dropwise added 4M HCl in dioxane (200 mL, 800 mmol) and the reaction mixture was stirred overnight. The reaction mixture was concentrated and stirred in Et₂O for 1 d. The product was filtered under a nitrogen stream, washed with a small amount of Et₂O (2x) and dried on filter for 10 min. yielding the desired product (26.0 g, 95% over two steps).

### 1.4.2 2,2-Dimethyl-3-(methylamino)propanamide hydrochloride (AMN-2)

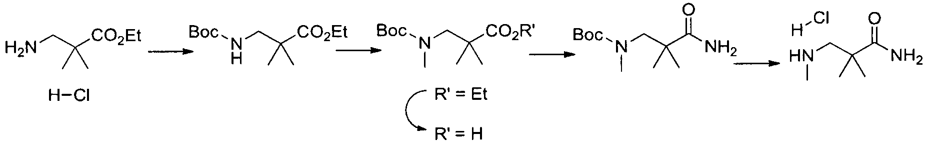

### Step 1: Ethyl 3-((tert-butoxycarbonyl)amino)-2,2-dimethylpropanoate

At 0°C, ethyl 3-amino-2,2-dimethylpropanoate hydrochloride (4.5 g, 24.77 mmol) was dissolved in a mixture of DCM (150 mL) and pentane (150 mL). Et₃N (4.14 mL, 29.7 mmol) was added, followed by Boc₂O (5.41 g, 24.77 mmol) and the white suspension was stirred at RT for 20 h. The reaction mixture was concentrated *in vacuo* and the residue was partitioned between H₂O (100 mL) and *i*-Pr₂O (100 mL). The aqueous layer was extracted with *i*-Pr₂O (100 mL) and the combined organic layers were washed with H₂O (2x50 mL) and brine (2x50 mL) before drying on Na₂SO₄ and concentration *in vacuo* to give the desired product (6.05 g, 100%) as a yellow oil.

### Step 2: Ethyl 3-((tert-butoxycarbonyl)(methyl)amino)-2,2-dimethylpropanoate

To a cooled (0°C) solution of ethyl 3-((tert-butoxycarbonyl)amino)-2,2-dimethylpropanoate (6.05 g, 24.66 mmol) in dry DMF (40 mL) was added 60% NaH in mineral oil (1.085 g, 27.1 mmol). The mixture was stirred for 5 min at 0°C, then warmed up to RT over 15 min. The mixture was cooled to 0°C again and Mel (3.1 mL, 49.6 mmol) was added *via* a syringe over 2 min. The mixture was stirred at RT for 3 h. The reaction mixture was poured out in aqueous 1M KHSO₄ (100 mL) with vigorous stirring and the product was extracted with *i*-Pr₂O/EtOAc (1:1, v/v, 250 mL). The organic layer was washed with aqueous 1M KHSO₄ (2x50 mL), saturated aqueous NaHCO₃ (2x50 mL) and aqueous 1M Na₂S₂O₃ (2x50 mL) before washing with brine (2x50 mL) and drying on Na₂SO₄. The solvent was removed *in vacuo* to give the desired product (5.72 g, 89%) as a colorless oil.

### Step 3: 3-((tert-Butoxycarbonyl)(methyl)amino)-2,2-dimethylpropanoic acid

A suspension of LiOH·H₂O (9 g, 216 mmol) in H₂O (25 mL) was added to a solution of ethyl 3-((tert-butoxycarbonyl)(methyl)amino)-2,2-dimethylpropanoate (5.6 g, 21.59 mmol) in a mixture of THF (25 mL) and EtOH (25 mL). The mixture was stirred at 60°C for 3 h. The volatiles were removed *in vacuo* and the aqueous mixture was cooled in an ice bath. Aqueous 1M KHSO₄ (250 mL) was added. The product was extracted with EtOAc (3x75 mL) and the combined organic layers were washed with aqueous 1M KHSO₄ (2x50 mL) and brine (2x50 mL) before drying on Na₂SO₄ and concentration *in vacuo* to give the desired product (4.9 g, 98%) as a yellow oil which crystallised on standing.

### Step 4: tert-Butyl (3-amino-2,2-dimethyl-3-oxopropyl)(methyl)carbamate

To a solution of 3-((tert-butoxycarbonyl)(methyl)amino)-2,2-dimethylpropanoic acid (4.22 g, 18.25 mmol) in EtOAc (200 mL) was added CDI (3.55 g, 21.89 mmol). The mixture was stirred at RT for 1.5 h. The reaction mixture was cooled in an ice bath and aqueous 25% NH₄OH (34 mL, 219 mmol) was added and the resulting suspension was stirred at RT for 2 h. The reaction mixture was concentrated *in vacuo* to approximately 200 mL. The residue was washed with saturated aqueous NaHCO₃ (3x50 mL), aqueous 1M KHSO₄ (2x50 mL) and brine (2x50 mL) before drying on Na₂SO₄ and concentration *in vacuo* to give a white solid. The product was crystallised from hot EtOH and dried on air to give the desired product (2.8 g, 66%) as colorless crystals.

### Step 5: 2,2-Dimethyl-3-(methylamino)propanamide hydrochloride (AMN-2)

To a solution of tert-butyl (3-amino-2,2-dimethyl-3-oxopropyl)(methyl)carbamate (1.8 g, 7.82 mmol) in DCM (40 mL) was added 4 M HCl in dioxane (39 mL, 156 mmol). The mixture was stirred at RT for 3 h. The reaction mixture was concentrated *in vacuo* to give the desired product (1.27 g, 98%) as a white solid.

### 1.4.3 2,2-Dimethyl-3-(methylamino)propanenitrile hydrochloride (AMN-3)

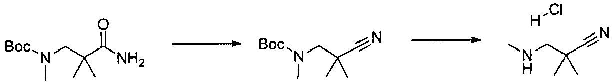

### Step 1: tert-Butyl (2-cyano-2-methylpropyl)(methyl)carbamate

A solution of tert-butyl (3-amino-2,2-dimethyl-3-oxopropyl)(methyl)carbamate [see step 4 AMN-2] (1 g, 4.34 mmol) in pyridine (10 mL) was cooled in an ice bath. POCl₃ (405 µL, 4.34 mmol) was added *via* a syringe and the resulting white suspension was stirred at 0°C for 30 min. The reaction mixture was poured out in aqueous 5M HCl (26 mL) and the product was extracted with EtOAc (1x50 mL, 1x25 mL). The combined organic layers were washed with aqueous 1M HCl (2x20 mL), saturated aqueous NaHCO₃ (2x20 mL) and brine (2x20 mL) before drying on Na₂SO₄ and concentration *in vacuo* to give the desired product (689 mg, 74%) as a colorless oil.

### Step 2: 2,2-Dimethyl-3-(methylamino)propanenitrile hydrochloride (AMN-3)

To a solution of tert-butyl (2-cyano-2-methylpropyl)(methyl)carbamate (0.689 g, 3.25 mmol) in DCM (16 mL) was added 4M HCl in dioxane (16.2 mL, 64.9 mmol) and the solution was stirred at RT for 1 h. The reaction mixture was concentrated *in vacuo* to give a white solid. The product was crystallised from hot EtOH to give the desired product (287 mg, 59%) as colorless crystals.

### 1.5 Pyrrole derivatives:

### General method for synthesis of pyrrole derivatives:

### General Procedure 1 (GP-1):

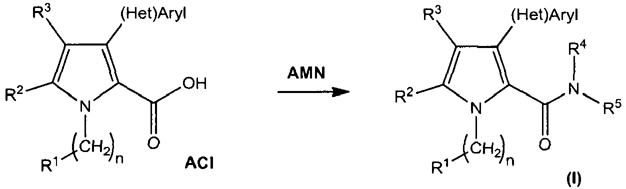

To a cooled (0°C) stirred solution of carboxylic acid **ACl** (1 eq.) and DIPEA (2 - 4 eq.) in DCM was added EDCI (1.2 eq.), followed by HOBt (0.2 eq.) and stirring was continued for 15 min at RT before cooling again to 0 °C. Amine **AMN** [or the corresponding hydrochloride salt] (1 eq.) was added and the solution was allowed to warm to RT and stir for 12-72 h. The reaction mixture was washed with saturated aqueous NaHCO₃ and concentrated *in vacuo.* The crude product was purified by flash CC.

### General Procedure 2 (GP-2):

To a cooled (0°C) stirred solution of carboxylic acid **ACI** (1 eq.) and DIPEA (2 - 4 eq.) in DCM or THF was added HATU (1.0 eq.), and stirring was continued for 15 min at RT before cooling again to 0°C. Amine **AMN** [or the corresponding hydrochloride salt] (1 eq.) was then added and the solution was allowed to warm to RT and stir for 12-72 h. The reaction mixture was diluted with DCM, washed with saturated aqueous NaHCO₃ and concentrated *in vacuo.* The crude product was purified by flash CC.

### General Procedure 3 (GP-3):

Carboxylic acid **ACl** (1 eq.) and amine **AMN** (1 - 1.5 eq.) were dissolved in DME or DMF, BOP-Cl (1 - 2.5 eq.) and DIPEA (3 - 5 eq.) were added. The reaction mixture was stirred at 60°C for 2-4 h and then cooled to RT. The reaction mixture was poured in aqueous 1M KHSO₄ and the product was extracted using EtOAc or DCM (3x). The combined organic layers were washed with aqueous 1M KHSO₄ (2x), aqueous saturated NaHCO₃ (3x) and brine (2x) before drying on Na₂SO₄ and concentration *in vacuo.* The product was purified using flash CC (silica).

### General Procedure 4 (GP-4):

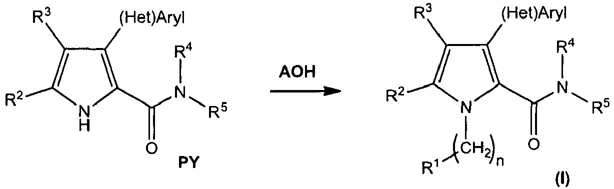

Pyrrole **PY** (1 eq), PPh₃ (1.15 eq) and alcohol **AOH** (1.25 eq) were dissolved in dry THF at RT. To the solution was added DIAD (1.1 eq) and the reaction mixture was stirred for 1 h to 5 d at RT. In some cases the order of addition was changed and the alcohol was added last, after DIAD. Also, in some cases it was necessary to add extra reagent (up to 3x PPh₃, DIAD, alcohol). The reaction mixture was diluted with EtOAc and saturated aqueous NaHCO₃ was added and the layers were separated. The aqueous layer was extracted with EtOAc. The combined organic layer was dried and the solvent was evaporated. The crude product was purified by flash CC (silica) and in some cases preparative LCMS or reversed phase chromatography.

### General Procedure 5 (GP-5):

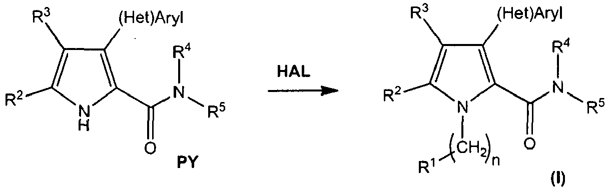

To a solution of pyrrole **PY**(1 eq) in dry DMF or MeCN, Cs₂CO₃ (2-3 eq) and bromide **HAL** (1-2 eq, optionally on silica) were added and the reaction mixture was stirred at 50°C for 18 h. The reaction mixture was either (A) filtered through silica and washed with EtOAc (2x10 mL) or (B) poured out in H₂O, extracted with EtOAc, followed by washing the organic layer with brine (2x) and dried over Na₂SO₄. The solvent was removed under reduced pressure and the residue purified using flash chromatography (silica).

### General Procedure 6 (GP-6):

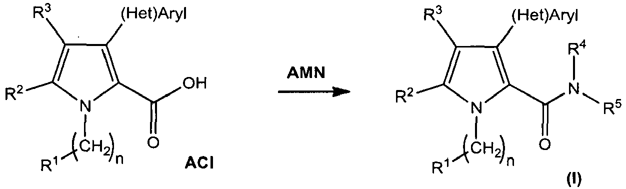

To a solution of carboxylic acid **ACl** (1 eq.) and DIPEA (3 - 4 eq.) in dry THF was added PyBOP (1.1 eq.) and amine **AMN** [or the corresponding hydrochloride salt] (1.2 eq.). The reaction mixture was stirred for 1 h and diluted with EtOAc and saturated aqueous NaHCO₃. The organic layer was separated, washed with brine, dried (Na₂SO₄) and concentrated. The crude product was purfied by flash cc and in some cases recrystallised. In some cases additional PyBOP and amine **AMN** was dosed.

**Table Synthesis of pyrrole derivatives (according to formula (I)):**

| **Example No.** | **Name** | **Synthesis according to (yield)** |
|---|---|---|
| **001** | N-Benzyl-3-(4-chlorophenyl)-1,4-dimethyl-N-tetrahydro-pyran-4-yl-5-(trifluoromethyl)-1H-pyrrole-2-carboxylic acid amide | GP-3 from ACI-7 & AMN-1 (26%) |
| **002** | 3-(4-Chlorophenyl)-N-(2,2-dimethyl-propyl)-N,1,4-trimethyl-5-(trifluoromethyl)-1H-pyrrole-2-carboxylic acid amide | GP-3 from ACI-7 & AMN-1 (77%) |
| **003** | N-Benzyl-3-(4-chlorophenyl)-N-(2,2-dimethyl-propyl)-1,4-dimethyl-5-(trifluoromethyl)-1H-pyrrole-2-carboxylic acid amide | GP-3 from ACI-7 (68%) |
| **004** | [3-(4-Chlorophenyl)-1,4-dimethyl-5-(trifluoromethyl)-1H-pyrrol-2-yl]-morpholin-4-yl-methanone | see below |
| **005** | 3-(4-Chlorophenyl)-N-(2,2-dimethyl-propyl)-1-ethyl-N,4-dimethyl-5-(trifluoromethyl)-1H-pyrrole-2-carboxylic acid amide | GP-2 from ACI-8 & AMN-1 (41%) |
| **006** | [3-(4-Chlorophenyl)-1-isopropyl-4-methyl-5-(trifluoromethyl)-1H-pyrrol-2-yl]-(2,2-dimethyl-morpholin-4-yl)-methanone | GP-4 from PY-3 (34%) |
| **007** | N-(1-Carbamoyl-cyclopropyl)-3-(4-chlorophenyl)-1-cyclopropyl-N,4-dimethyl-5-(trifluoromethyl)-1H-pyrrole-2-carboxylic acid amide | see below |
| **008** | 3-(4-Chlorophenyl)-1-cyclopropyl-N-(1,1-dioxo-thian-4-yl)-N,4-dimethyl-5-(trifluoromethyl)-1H-pyrrole-2-carboxylic acid amide | GP-3 from ACl-1 (68%) |
| **009** | N-(2-Carbamoyl-2-methyl-propyl)-3-(4-chlorophenyl)-1-cyclopropyl-N,4-dimethyl-5-(trifluoromethyl)-1H-pyrrole-2-carboxylic acid amide | in analogy to SC-007 |
| **010** | 3-(4-Chlorophenyl)-1-cyclopropyl-N-[(4-methoxyphenyl)-methyl]-N,4-dimethyl-5-(trifluoromethyl)-1H-pyrrole-2-carboxylic acid amide | GP-1 from ACl-1 (50%) |
| **011** | 3-(4-Chlorophenyl)-1-cyclopropyl-N-[(4-methoxyphenyl)-methyl]-N-methyl-5-(trifluoromethyl)-1H-pyrrole-2-carboxylic acid amide | GP-1 from ACl-2 (50%) |
| **012** | 3-(4-Chlorophenyl)-N,1-dicyclopropyl-N,4-dimethyl-5-(trifluoromethyl)-1H-pyrrole-2-carboxylic acid amide | GP-1 from ACl-1 (26%) |
| **013** | 3-(4-Chlorophenyl)-N,1-dicyclopropyl-N-methyl-5-(trifluoromethyl)-1H-pyrrole-2-carboxylic acid amide | GP-1 from ACl-2 (26%) |
| **014** | 3-(4-Chlorophenyl)-1-cyclopropyl-N,4-dimethyl-N-tetrahydropyran-4-yl-5-(trifluoromethyl)-1H-pyrrole-2-carboxylic acid amide | GP-1 from ACl-1 (62%) |
| **015** | 3-(4-Ch lorophenyl)-1-cyclopropyl-N-methyl-N-tetrahydro-pyran-4-yl-5-(trifluoromethyl)-1H-pyrrole-2-carboxylic acid amide | GP-1 from ACI-2 (77%) |
| **016** | 3-(4-Chlorophenyl)-1-cyclopropyl-N-(2,2-dimethyl-3-morpholin-4-yl-propyl)-N,4-dimethyl-5-(trifluoromethyl)-1H-pyrrole-2-carboxylic acid amide | GP-1 from ACI-1 (48%) |
| **017** | 3-(4-Chlorophenyl)-1-cyclopropyl-N-(2,2-dimethyl-3-morpholin-4-yl-propyl)-N-methyl-5-(trifluoromethyl)-1H-pyrrole-2-carboxylic acid amide | GP-1 from ACI-2 (38%) |
| **018** | 3-(4-Chlorophenyl)-1-cyclopropyl-N-(2-hydroxy-2-methylpropyl)-N,4-dimethyl-5-(trifluoromethyl)-1H-pyrrole-2-carboxylic acid amide | GP-1 from ACI-1 (72%) |
| **019** | 3-(4-Chlorophenyl)-1-cyclopropyl-N-(2-hydroxy-2-methylpropyl)-N-methyl-5-(trifluoromethyl)-1H-pyrrole-2-carboxylic acid amide | GP-1 from ACI-2 (75%) |
| **020** | 3-(4-Chloro-2-fluoro-phenyl)-1-cyclopropyl-N,4-dimethyl-N-[(5-methyl-isoxazol-3-yl)-methyl]-5-(trifluoromethyl)-1H-pyrrole-2-carboxylic acid amide | GP-1 from ACI-3 (69%) |
| **021** | 3-(4-Chloro-2-fluoro-phenyl)-1-cyclopropyl-N,4-dimethyl-N-(pyrimidin-4-yl-methyl)-5-(trifluoromethyl)-1H-pyrrole-2-carboxylic acid amide | GP-1 from ACI-3 (62%) |
| **022** | 3-(4-Chlorophenyl)-1-cyclopropyl-N,4-dimethyl-N-(tetrahydrofuran-3-yl-methyl)-5-(trifluoromethyl)-1H-pyrrole-2-carboxylic acid amide | GP-1 from ACI-1 (68%) |
| **023** | 3-(4-Chlorophenyl)-1-cyclopropyl-N-methyl-N-(tetrahydro-furan-3-yl-methyl)-5-(trifluoromethyl)-1H-pyrrole-2-carboxylic acid amide | GP-1 from ACI-2 (29%) |
| **024** | 3-(4-Chloro-2-fluoro-phenyl)-1-cyclopropyl-N,4-dimethyl-N-[(5-methyl-pyrazin-2-yl)-methyl]-5-(trifluoromethyl)-1H-pyrrole-2-carboxylic acid amide | GP-1 from ACI-3 (60%) |
| **025** | [3-(4-Chlorophenyl)-1-cyclopropyl-4-methyl-5-(trifluoromethyl)-1H-pyrrol-2-yl]-(3-hydroxy-azetidin-1-yl)-methanone | GP-1 from ACI-1 (60%) |
| **026** | [3-(4-Chlorophenyl)-1-cyclopropyl-5-(trifluoromethyl)-1H-pyrrol-2-yl]-(3-hydroxy-azetidin-1-yl)-methanone | GP-1 from ACI-2 (80%) |
| **027** | [3-(4-Chlorophenyl)-1-cyclopropyl-4-methyl-5-(trifluoromethyl)-1H-pyrrol-2-yl]-[4-(3-methyl-[1,2,4]oxadiazol-5-yl)-piperidin-1-yl]-methanone | GP-1 from ACI-1 (84%) |
| **028** | [3-(4-Chloro-2-fluoro-phenyl)-1-cyclopropyl-4-methyl-5-(trifluoromethyl)-1H-pyrrol-2-yl]-[4-(3-methyl-[1,2,4]oxadiazol-5-yl)-piperidin-1-yl]-methanone | GP-1 from ACI-3 (57%) |
| **029** | [3-(4-Chlorophenyl)-1-cyclopropyl-5-(trifluoromethyl)-1H-pyrrol-2-yl]-[4-(3-methyl-[1,2,4]oxadiazol-5-yl)-piperidin-1-yl]-methanone | GP-1 from ACI-2 (83%) |
| **030** | [3-(4-Chlorophenyl)-1-cyclopropyl-4-methyl-5-(trifluoromethyl)-1H-pyrrol-2-yl]-[4-[5-(trifluoromethyl)-pyridin-2-yl]oxy-piperidin-1-yl]-methanone | GP-1 from ACI-1 (60%) |
| **031** | [3-(4-Chlorophenyl)-1-cyclopropyl-5-(trifluoromethyl)-1H-pyrrol-2-yl]-[4-[5-(trifluoromethyl)-pyridin-2-yl]oxy-piperidin-1-yl]-methanone | GP-1 from ACl-2 (59%) |
| **032** | [3-(4-Chlorophenyl)-1-cyclopropyl-4-methyl-5-(trifluoromethyl)-1H-pyrrol-2-yl]-(2,2-dimethyl-morpholin-4-yl)-methanone | GP-1 from ACl-1 (62%) |
| **033** | [3-(4-Chloro-2-fluoro-phenyl)-1-cyclopropyl-4-methyl-5-(trifluoromethyl)-1H-pyrrol-2-yl]-(2,2-dimethyl-morpholin-4-yl)-methanone | GP-1 from ACl-3 (47%) |
| **034** | [3-(4-Chlorophenyl)-1-cyclopropyl-5-(trifluoromethyl)-1H-pyrrol-2-yl]-(2,2-dimethyl-morpholin-4-yl)-methanone | GP-1 from ACl-2 (54%) |
| **035** | 3-(4-Chlorophenyl)-1-cyclopropyl-N-(2,2-dimethyl-propyl)-N-methyl-5-(trifluoromethyl)-1H-pyrrole-2-carboxylic acid amide | GP-3 from ACl-2 & AMN-1 (76%) |
| **036** | 3-(4-Chlorophenyl)-1-cyclopropyl-N-(3-hydroxy-2,2-dimethylpropyl)-N-methyl-5-(trifluoromethyl)-1H-pyrrole-2-carboxylic acid amide | GP-3 from ACl-2 (72%) |
| **037** | 3-(4-Chlorophenyl)-1-cyclopropyl-N-methyl-N-(2-methylsulfonylethyl)-5-(trifluoromethyl)-1H-pyrrole-2-carboxylic acid amide | GP-3 from ACl-2 (79%) |
| **038** | [3-(4-Chlorophenyl)-1-cyclopropyl-5-(trifluoromethyl)-1H-pyrrol-2-yl]-morpholin-4-yl-methanone | In analogy to step-3 ACI-3 (5x amount of reagents was dosed sequentially) |
| **039** | 3-(4-Chlorophenyl)-1-cyclopropyl-N-(2,2-dimethyl-propyl)-N,4-dimethyl-5-(trifluoromethyl)-1H-pyrrole-2-carboxylic acid amide | GP-3 from ACI-1 & AMN-1 (77%) |
| **040** | 3-(4-Chlorophenyl)-1-cyclopropyl-N-(3-hydroxy-2,2-dimethylpropyl)-N,4-dimethyl-5-(trifluoromethyl)-1H-pyrrole-2-carboxylic acid amide | GP-3 from ACI-1 (62%) |
| **041** | 3-(4-Chlorophenyl)-1-cyclopropyl-N,4-dimethyl-N-(2-methylsulfonyl-ethyl)-5-(trifluoromethyl)-1H-pyrrole-2-carboxylic acid amide | GP-3 from ACI-1 (76%) |
| **042** | [3-(4-Chlorophenyl)-1-cyclobutyl-4-methyl-5-(trifluoromethyl)-1H-pyrrol-2-yl]-(2,2-dimethyl-morpholin-4-yl)-methanone | GP-4 from PY-3 (14%) |
| **043** | [3-(4-Chlorophenyl)-1-cyclopropyl-4-methyl-5-(trifluoromethyl)-1H-pyrrol-2-yl]-morpholin-4-yl-methanone | see below |
| **044** | [3-(4-Chlorophenyl)-1-cyclopropyl-4-methyl-5-(trifluoromethyl)-1H-pyrrol-2-yl]-(2,2-dimethyl-1,1-dioxo-[1,4]thiazinan-4-yl)-methanone | see below |
| **045** | (2,2-Dimethyl-morpholin-4-yl)-[3-(4-fluorophenyl)-1-(2-methylpropyl)-5-(trifluoromethyl)-1H-pyrrol-2-yl]-methanone | GP-1 from ACI-4 (66%) |
| **046** | 3-(4-Fluorophenyl)-N-methyl-1-(2-methyl-propyl)-N-tetrahydro-pyran-4-yl-5-(trifluoromethyl)-1H-pyrrole-2-carboxylic acid amide | GP-1 from ACI-4 (81%) |
| **047** | 3-(4-Fluorophenyl)-N-methyl-N-[(5-methyl-isoxazol-3-yl)-methyl]-1-(2-methyl-propyl)-5-(trifluoromethyl)-1H-pyrrole-2-carboxylic acid amide . | GP-1 from ACI-4 (66%) |
| **048** | 3-(4-Chlorophenyl)-N,4-dimethyl-1-(2-methyl-propyl)-N-(pyrimidin-4-yl-methyl)-5-(trifluoromethyl)-1H-pyrrole-2-carboxylic acid amide | GP-1 from ACI-5 (70%) |
| **049** | 3-(4-Fluorophenyl)-N-methyl-1-(2-methyl-propyl)-N-[(5-methyl-pyrazin-2-yl)-methyl]-5-(trifluoromethyl)-1H-pyrrole-2-carboxylic acid amide | GP-1 from ACI-4 (59%) |
| **050** | 3-(4-Fluorophenyl)-N-methyl-1-(2-methyl-propyl)-N-(2-methylsulfonyl-ethyl)-5-(trifluoromethyl)-1H-pyrrole-2-carboxylic acid amide | GP-1 from ACI-4 (21%) |
| **051** | N-(1-Carbamoyl-cyclopropyl)-3-(4-chlorophenyl)-N,4-dimethyl-1-(2-methyl-propyl)-5-(trifluoromethyl)-1H-pyrrole-2-carboxylic acid amide | see below |
| **052** | N-(2-Carbamoyl-2-methyl-propyl)-3-(4-chlorophenyl)-N,4-dimethyl-1-(2-methyl-propyl)-5-(trifluoromethyl)-1H-pyrrole-2-carboxylic acid amide | GP-1 from ACl-5 (61%) |
| **053** | N-(2,2-Dimethyl-propyl)-3-(4-fluorophenyl)-N-methyl-1-(2-methyl-propyl)-5-(trifluoromethyl)-1H-pyrrole-2-carboxylic acid amide | GP-1 from ACl-4 (49%) |
| **054** | N-(2,2-Dimethyl-3-morpholin-4-yl-propyl)-3-(4-fluorophenyl)-N-methyl-1-(2-methyl-propyl)-5-(trifluoromethyl)-1H-pyrrole-2-carboxylic acid amide | GP-1 from ACl-4 (44%) |
| **055** | 3-(4-Chlorophenyl)-N-(3-hydroxy-2,2-dimethyl-propyl)-N,4-dimethyl-1-(2-methyl-propyl)-5-(trifluoromethyl)-1H-pyrrole-2-carboxylic acid amide | GP-3 from ACl-5 (57%) |
| **056** | 3-(4-Fluorophenyl)-N-(3-hydroxy-2,2-dimethyl-propyl)-N-methyl-1-(2-methyl-propyl)-5-(trifluoromethyl)-1H-pyrrole-2-carboxylic acid amide | GP-1 from ACl-4 (56%) |
| **057** | 3-(4-Chlorophenyl)-N,4-dimethyl-N-[(5-methyl-isoxazol-3-yl)-methyl]-1-(2-methyl-propyl)-5-(trifluoromethyl)-1H-pyrrole-2-carboxylic acid amide | GP-1 from ACl-5 (85%) |
| **058** | 3-(4-Fluorophenyl)-N-methyl-1-(2-methyl-propyl)-N-(tetrahydrofuran-3-yl-methyl)-5-(trifluoromethyl)-1H-pyrrole-2-carboxylic acid amide | GP-1 from ACl-4 (77%) |
| **059** | 3-(4-Chlorophenyl)-N,4-dimethyl-1-(2-methyl-propyl)-N-[(5-methyl-pyrazin-2-yl)-methyl]-5-(trifluoromethyl)-1H-pyrrole-2-carboxylic acid amide | GP-1 from ACl-5 (75%) |
| **060** | N-[(2-Dimethylamino-pyrimidin-5-yl)-methyl]-3-(4-fluorophenyl)-N-methyl-1-(2-methyl-propyl)-5-(trifluoromethyl)-1H-pyrrole-2-carboxylic acid amide | GP-1 from ACl-4 (83%) |
| **061** | 3-(4-Chlorophenyl)-N,4-dimethyl-1-(2-methyl-propyl)-N-(2-methylsulfonyl-ethyl)-5-(trifluoromethyl)-1H-pyrrole-2-carboxylic acid amide | GP-3 from ACl-5 (55%) |
| **062** | [3-(4-Chlorophenyl)-4-methyl-1-(2-methyl-propyl)-5-(trifluoromethyl)-1H-pyrrol-2-yl]-[4-(3-methyl-[1,2,4]oxadiazol-5-yl)-piperidin-1-yl]-methanone | GP-1 from ACl-5 (92%) |
| **063** | 4-[3-(4-Fluorophenyl)-1-(2-methyl-propyl)-5-(trifluoromethyl)-1H-pyrrole-2-carbonyl]-piperazin-2-one | GP-1 from ACl-4 (30%) |
| **064** | [3-(4-Chlorophenyl)-4-methyl-1-(2-methyl-propyl)-5-(trifluoromethyl)-1H-pyrrol-2-yl]-(2,2-dimethyl-morpholin-4-yl)-methanone | GP-3 from ACl-5 (51%) |
| **065** | [3-(4-Fluorophenyl)-1-(2-methyl-propyl)-5-(trifluoromethyl)-1H-pyrrol-2-yl]-morpholin-4-yl-methanone | GP-1 from ACl-4 (36%) |
| **066** | N-Cyclopropyl-3-(4-fluorophenyl)-1-(2-methyl-propyl)-5-(trifluoromethyl)-1H-pyrrole-2-carboxylic acid amide | GP-1 from ACl-4 (78%) |
| **067** | 3-(4-Chlorophenyl)-N-(2-cyano-2-methyl-propyl)-N,4-dimethyl-1-(2-methyl-propyl)-5-(trifluoromethyl)-1H-pyrrole-2-carboxylic acid amide | GP-3 from ACl-5 (32%) |
| **068** | 3-(4-Chlorophenyl)-N,4-dimethyl-1-[(1-methyl-cyclopropyl)-methyl]-N-(2-methylsulfonyl-ethyl)-5-(trifluoromethyl)-1H-pyrrole-2-carboxylic acid amide | See below |
| **069** | N-[(2-Dimethylamino-pyrimidin-5-yl)-methyl]-3-(4-fluorophenyl)-N-methyl-1-(tetrahydro-furan-2-yl-methyl)-5-(trifluoromethyl)-1H-pyrrole-2-carboxylic acid amide | GP-1 from ACl-6 (88%) |
| **070** | 3-(4-Fluorophenyl)-N-methyl-1-(tetrahyd ro-fu ran-2-yl-methyl)-N-tetrahydro-pyran-4-yl-5-(trifluoromethyl)-1H-pyrrole-2-carboxylic acid amide | GP-1 from ACl-6 (49%) |
| **071** | N-(1,1-Dioxo-thiolan-3-yl)-3-(4-fluorophenyl)-N-methyl-1-(tetrahydro-furan-2-yl-methyl)-5-(trifluoromethyl)-1H-pyrrole-2-carboxylic acid amide | GP-2 from ACl-6 (37%) |
| **072** | N-(2-Carbamoyl-2-methyl-propyl)-3-(4-fluorophenyl)-N-methyl-1-(tetrahydro-furan-2-yl-methyl)-5-(trifluoromethyl)-1H-pyrrole-2-carboxylic acid amide | GP-1 from ACl-6 (61%) |
| **073** | N-(2,2-Dimethyl-propyl)-3-(4-fluorophenyl)-N-methyl-1-(tetrahydro-furan-2-yl-methyl)-5-(trifluoromethyl)-1H-pyrrole-2-carboxylic acid amide | GP-1 from ACl-6 (61%) |
| **074** | 3-(4-Fluorophenyl)-N-(3-hydroxy-2,2-dimethyl-propyl)-N-methyl-1-(tetrahydro-furan-2-yl-methyl)-5-(trifluoromethyl)-1H-pyrrole-2-carboxylic acid amide | GP-1 from ACl-6 (39%) |
| **075** | 3-(4-Fluorophenyl)-N-methyl-N-[(5-methyl-isoxazol-3-yl)-methyl]-1-(tetrahydro-furan-2-yl-methyl)-5-(trifluoromethyl)-1H-pyrrole-2-carboxylic acid amide | GP-1 from ACl-6 (65%) |
| **076** | 3-(4-Fluorophenyl)-N-methyl-N,1-bis(tetrahydro-furan-2-yl-methyl)-5-(trifluoromethyl)-1H-pyrrole-2-carboxylic acid amide | GP-1 from ACl-6 (79%) |
| **077** | 3-(4-Fluorophenyl)-N-methyl-N-[(5-methyl-pyrazin-2-yl)-methyl]-1-(tetrahydro-furan-2-yl-methyl)-5-(trifluoromethyl)-1H-pyrrole-2-carboxylic acid amide | GP-1 from ACl-6 (59%) |
| **078** | 3-(4-Fluorophenyl)-N-methyl-N-(2-methylsulfonyl-ethyl)-1-(tetrahydro-furan-2-yl-methyl)-5-(trifluoromethyl)-1H-pyrrole-2-carboxylic acid amide | GP-1 from ACI-6 (33%) |
| **079** | 4-[3-(4-Fluorophenyl)-1-(tetrahydro-furan-2-yl-methyl)-5-(trifluoromethyl)-1H-pyrrole-2-carbonyl]-piperazin-2-one | GP-1 from ACI-6 (56%) |
| **080** | (2,2-Dimethyl-morpholin-4-yl)-[3-(4-fluorophenyl)-1-(tetrahydrofuran-2-yl-methyl)-5-(trifluoromethyl)-1H-pyrrol-2-yl]-methanone | GP-1 from ACI-6 (69%) |
| **081** | [3-(4-Fluorophenyl)-1-(tetrahydro-furan-2-yl-methyl)-5-(trifluoromethyl)-1H-pyrrol-2-yl]-morpholin-4-yl-methanone | GP-1 from ACI-6 (73%) |
| **082** | N-Cyclopropyl-3-(4-fluorophenyl)-1-(tetrahydro-furan-2-yl-methyl)-5-(trifluoromethyl)-1H-pyrrole-2-carboxylic acid amide | GP-1 from ACI-6 (66%) |
| **083** | 3-(4-Chlorophenyl)-N-(2,2-dimethyl-propyl)-N-methyl-1-(tetrahydro-furan-2-yl-methyl)-5-(trifluoromethyl)-1H-pyrrole-2-carboxylic acid amide | GP-4 from PY-2 (88%) |
| **084** | [3-(4-Chlorophenyl)-1-(cyclopropyl-methyl)-4-methyl-5-(trifluoromethyl)-1H-pyrrol-2-yl]-(2,2-dimethyl-morpholin-4-yl)-methanone | GP-4 from PY-3 (76%) |
| **085** | [3-(4-Chlorophenyl)-1-(cyclobutyl-methyl)-4-methyl-5-(trifluoromethyl)-1H-pyrrol-2-yl]-(2,2-dimethyl-morpholin-4-yl)-methanone | GP-5 from PY-3 (76%) |
| **086** | [3-(4-Chlorophenyl)-1-(3-cyclopropyl-prop-2-ynyl)-4-methyl-5-(trifluoromethyl)-1H-pyrrol-2-yl]-morpholin-4-yl-methanone | GP-4 from PY-1 & AOH-2 (42%) |
| **087** | [3-(4-Chlorophenyl)-4-methyl-1-[(1-methyl-cyclopropyl)-methyl]-5-(trifluoromethyl)-1H-pyrrol-2-yl]-morpholin-4-yl-methanone | GP-4 from PY-1 (57%) |
| **088** | 1-[(1-Amino-cyclopropyl)-methyl]-3-(4-chlorophenyl)-N-(2,2-dimethyl-propyl)-N-methyl-5-(trifluoromethyl)-1H-pyrrole-2-carboxylic acid amide | in analogy to SC-095 from PY-2 |
| **089** | [3-(4-Chlorophenyl)-1-[(1-hydroxy-cyclopropyl)-methyl]-4-methyl-5-(trifluoromethyl)-1H-pyrrol-2-yl]-morpholin-4-yl-methanone | see below |
| **090** | 3-(4-Chlorophenyl)-N-(2,2-dimethyl-propyl)-1-[(1-hydroxycyclopentyl)-methyl]-N-methyl-5-(trifluoromethyl)-1H-pyrrole-2-carboxylic acid amide | see below |
| **091** | 3-(4-Chlorophenyl)-N-(2,2-dimethyl-propyl)-N-methyl-1-(pyrrolidin-2-yl-methyl)-5-(trifluoromethyl)-1H-pyrrole-2-carboxylic acid amide | see below |
| **092** | 3-(4-Chlorophenyl)-N-(2,2-dimethyl-propyl)-N-methyl-1-(tetrahydro-pyran-4-yl-methyl)-5-(trifluoromethyl)-1H-pyrrole-2-carboxylic acid amide | GP-4 from PY-2 (44%) |
| **093** | 1-[(1-Amino-cyclopropyl)-methyl]-3-(4-chlorophenyl)-N,4-dimethyl-N-(2-methylsulfonyl-ethyl)-5-(trifluoromethyl)-1H-pyrrole-2-carboxylic acid amide | in analogy to SC-095 from PY-4 |
| **094** | 3-(4-Chlorophenyl)-1-[(1-hydroxy-cyclopentyl)-methyl]-N,4-dimethyl-N-(2-methylsulfonyl-ethyl)-5-(trifluoromethyl)-1H-pyrrole-2-carboxylic acid amide | in analogy to SC-090 from PY-4 (step 4) |
| **095** | [1-[(1-Amino-cyclopropyl)-methyl]-3-(4-chlorophenyl)-4-methyl-5-(trifluoromethyl)-1H-pyrrol-2-yl]-morpholin-4-yl-methanone | see below |
| **096** | [3-(4-Chlorophenyl)-1-[(1-hydroxy-cyclopentyl)-methyl]-4-methyl-5-(trifluoromethyl)-1H-pyrrol-2-yl]-morpholin-4-yl-methanone | see below |
| **097** | [3-(4-Ch lorophenyl)-4-methyl-1-(tetrahyd ro-pyran-4-yl-methyl)-5-(trifluoromethyl)-1H-pyrrol-2-yl]-morpholin-4-yl-methanone | GP-4 from PY-1 (66%) |
| **098** | [3-(4-Chlorophenyl)-1-[2-(3,3-difluoro-azetidin-1-yl)-ethyl]-4-methyl-5-(trifluoromethyl)-1H-pyrrol-2-yl]-morpholin-4-yl-methanone | GP-4 from PY-1 & AOH-1 (15%) |
| **099** | [3-(4-Chlorophenyl)-4-methyl-1-(2-methylsulfonyl-ethyl)-5-(trifluoromethyl)-1H-pyrrol-2-yl]-morpholin-4-yl-methanone | see below |
| **100** | 3-(4-Chlorophenyl)-1-[(1-cyano-cyclopropyl)-methyl]-N-(2,2-dimethyl-propyl)-N-methyl-5-(trifluoromethyl)-1H-pyrrole-2-carboxylic acid amide | GP-5 from PY-2 (42%) |
| **101** | 3-(4-Chlorophenyl)-N-(2,2-dimethyl-propyl)-1-[(1-hydroxy-cyclobutyl)-methyl]-N-methyl-5-(trifluoromethyl)-1H-pyrrole-2-carboxylic acid amide | see below |
| **102** | [3-(4-Chlorophenyl)-1,4-dimethyl-5-(trifluoromethyl)-1H-pyrrol-2-yl]-(2,2-dimethyl-morpholin-4-yl)-methanone | GP-4 from PY-3 (80%) |
| **103** | [3-(4-Chlorophenyl)-1-ethyl-4-methyl-5-(trifluoromethyl)-1H-pyrrol-2-yl]-(2,2-dimethyl-morpholin-4-yl)-methanone | GP-4 from PY-3 (86%) |
| **104** | [3-(4-Ch lorophenyl)-4-methyl-1-(2-methyl-propyl)-5-(trifluoromethyl)-1H-pyrrol-2-yl]-(2-oxa-5-azabicyclo[2.2.1]heptan-5-yl)-methanone | GP-3 from ACI-5 (43%) |
| **105** | N-(2-Carbamoyl-2-methyl-propyl)-3-(4-chlorophenyl)-1-isopropyl-N,4-dimethyl-5-(trifluoromethyl)-1H-pyrrole-2-carboxylic acid amide | GP-3 from ACI-9 & AMN-2 (62%) |
| **106** | 3-(4-Chlorophenyl)-N-(2-cyano-2-methyl-propyl)-1-isopropyl-N,4-dimethyl-5-(trifluoromethyl)-1H-pyrrole-2-carboxylic acid amide | GP-3 from ACI-9 & AMN-3 (53%) |
| **107** | 3-(4-Chlorophenyl)-1-isopropyl-N,4-dimethyl-N-[(5-methyl-isoxazol-3-yl)-methyl]-5-(trifluoromethyl)-1H-pyrrole-2-carboxylic acid amide | GP-3 from ACI-9 (64%) |
| **108** | 3-(4-Chlorophenyl)-N,1-diisopropyl-N,4-dimethyl-5-(trifluoromethyl)-1H-pyrrole-2-carboxylic acid amide | GP-3 from ACI-9 (57%) |
| **109** | 3-(4-Chlorophenyl)-N-isopropyl-N,4-dimethyl-1-(2-methylpropyl)-5-(trifluoromethyl)-1H-pyrrole-2-carboxylic acid amide | GP-3 from ACI-5 (42%) |
| **110** | 3-(4-Chlorophenyl)-1-isopropyl-N,4-dimethyl-N-(2-methylsulfonyl-ethyl)-5-(trifluoromethyl)-1H-pyrrole-2-carboxylic acid amide | GP-3 from ACl-9 (70%) |
| **111** | 3-(4-Chlorophenyl)-1-isopropyl-N,4-dimethyl-N-[(2-methyl-2H-pyrazol-3-yl)-methyl]-5-(trifluoromethyl)-1H-pyrrole-2-carboxylic acid amide | GP-3 from ACl-9 (60%) |
| **112** | [3-(5-Chloro-pyridin-2-yl)-1-isopropyl-4-methyl-5-(trifluoromethyl)-1H-pyrrol-2-yl]-(2,2-dimethyl-morpholin-4-yl)-methanone | GP-3 from ACl-10 (44%, over 2 steps) |
| **113** | 3-(5-Chloro-pyridin-2-yl)-1-isopropyl-N,4-dimethyl-N-[(5-methyl-isoxazol-3-yl)-methyl]-5-(trifluoromethyl)-1H-pyrrole-2-carboxylic acid amide | GP-3 from ACl-10 (66%, over 2 steps) |
| **114** | 3-(5-Chloro-pyridin-2-yl)-1-isopropyl-N,4-dimethyl-N-[(2-methyl-2H-pyrazol-3-yl)-methyl]-5-(trifluoromethyl)-1H-pyrrole-2-carboxylic acid amide | GP-3 from ACl-10 (54%, over 2 steps) |
| **115** | 3-(4-Chlorophenyl)-1-isopropyl-N,4-dimethyl-N-(2-oxo-pyrrolidin-3-yl)-5-(trifluoromethyl)-1H-pyrrole-2-carboxylic acid amide | GP-3 from ACI-9 (58%) |
| **116** | N-(2-Carbamoyl-2-methyl-propyl)-3-(5-chloro-pyridin-2-yl)-1-isopropyl-N,4-dimethyl-5-(trifluoromethyl)-1H-pyrrole-2-carboxylic acid amide | GP-3 from ACl-10 & AMN-2 (48%, over 2 steps) |
| **117** | 3-(5-Chloro-pyridin-2-yl)-N-(2-cyano-2-methyl-propyl)-1-isopropyl-N,4-dimethyl-5-(trifluoromethyl)-1H-pyrrole-2-carboxylic acid amide | see below |
| **118** | 3-(5-Chloro-pyridin-2-yl)-1-isopropyl-N,4-dimethyl-N-(2-methylsulfonyl-ethyl)-5-(trifluoromethyl)-1H-pyrrole-2-carboxylic acid amide | GP-3 from ACl-10 (82%) |
| **119** | 3-(5-Chloro-pyridin-2-yl)-1-isopropyl-N,4-dimethyl-N-(2-oxopyrrolidin-3-yl)-5-(trifluoromethyl)-1H-pyrrole-2-carboxylic acid amide | GP-3 from ACl-10 (75%) |
| **120** | 3-(5-Chloro-pyridin-2-yl)-N,1-diisopropyl-N,4-dimethyl-5-(trifluoromethyl)-1H-pyrrole-2-carboxylic acid amide | GP-3 from ACl-10 (72%) |
| **121** | [3-(5-Chloro-pyrimidin-2-yl)-1-isopropyl-4-methyl-5-(trifluoromethyl)-1H-pyrrol-2-yl]-(2,2-dimethyl-morpholin-4-yl)-methanone | GP-3 from ACl-11 (58%) |
| **122** | 3-(5-Chloro-pyrimidin-2-yl)-1-isopropyl-N,4-dimethyl-N-[(2-methyl-2H-pyrazol-3-yl)-methyl]-5-(trifluoromethyl)-1H-pyrrole-2-carboxylic acid amide | GP-3 from ACl-11 (27%) |
| **123** | 3-(5-Chloro-pyrimidin-2-yl)-1-isopropyl-N,4-dimethyl-N-[(5-methyl-isoxazol-3-yl)-methyl]-5-(trifluoromethyl)-1H-pyrrole-2-carboxylic acid amide | GP-3 from ACl-11 (41%) |
| **124** | 3-(5-Chloro-pyrimidin-2-yl)-1-isopropyl-N,4-dimethyl-N-(2-oxopyrrolidin-3-yl)-5-(trifluoromethyl)-1H-pyrrole-2-carboxylic acid amide | GP-6 from ACl-11 (30%) |
| **125** | N-(2-Carbamoyl-2-methyl-propyl)-3-(5-chloro-pyrimidin-2-yl)-1-isopropyl-N,4-dimethyl-5-(trifluoromethyl)-1H-pyrrole-2-carboxylic acid amide | GP-6 from ACl-11 & AMN-2 (25%) |
| **126** | 3-(5-Chloro-pyrimidin-2-yl)-N-(2-cyano-2-methyl-propyl)-1-isopropyl-N,4-dimethyl-5-(trifluoromethyl)-1H-pyrrole-2-carboxylic acid amide | GP-6 from ACl-11 & AMN-3 (39%) |
| **127** | 3-(5-Chloro-pyrimidin-2-yl)-1-isopropyl-N,4-dimethyl-N-(2-methylsulfonyl-ethyl)-5-(trifluoromethyl)-1H-pyrrole-2-carboxylic acid amide | GP-6 from ACl-11 (69%) |
| **128** | 3-(5-Chloro-pyrimidin-2-yl)-N,1-diisopropyl-N,4-dimethyl-5-(trifluoromethyl)-1H-pyrrole-2-carboxylic acid amide | GP-6 from ACl-11 (62%) |
| **129** | N-tert-Butyl-4-[3-(5-chloro-pyrid in-2-yl)-1-isopropyl-4-methyl-5-(trifluoromethyl)-1H-pyrrole-2-carbonyl]-piperazine-1-carboxylic acid amide | GP-3 from ACl-10 (72% over 2 steps) |
| **130** | [3-(5-Chloro-pyridin-2-yl)-1-isopropyl-4-methyl-5-(trifluoromethyl)-1H-pyrrol-2-yl]-(2,2-dimethyl-1-oxo-[1,4]thiazinan-4-yl)-methanone | GP-3 from ACl-10 (18% over 2 steps) |
| **131** | N-tert-Butyl-4-[3-(4-chlorophenyl)-1-isopropyl-4-methyl-5-(trifluoromethyl)-1H-pyrrole-2-carbonyl]-piperazine-1-carboxylic acid amide | GP-3 from ACl-9 (80%) |
| **132** | 3-(4-Chlorophenyl)-N-[1-(hydroxymethyl)-3-methyl-butyl]-1-isopropyl-N,4-dimethyl-5-(trifluoromethyl)-1H-pyrrole-2-carboxylic acid amide | GP-3 from ACl-9 (54%) |
| **133** | N-[1-(tert-Butyl-carbamoyl)-ethyl]-3-(4-chlorophenyl)-1-isopropyl-N,4-dimethyl-5-(trifluoromethyl)-1H-pyrrole-2-carboxylic acid amide | GP-3 from ACl-9 (43%) |
| **134** | 3-(5-Chloro-pyridin-2-yl)-N-(3,3-dimethyl-piperidin-4-yl)-1-isopropyl-N,4-dimethyl-5-(trifluoromethyl)-1H-pyrrole-2-carboxylic acid amide | see below |
| **135** | 3-(4-Chlorophenyl)-N-(3,3-dimethyl-piperidin-4-yl)-1-isopropyl-N,4-dimethyl-5-(trifluoromethyl)-1H-pyrrole-2-carboxylic acid amide | see below |

### [3-(4-Chlorophenyl)-1,4-dimethyl-5-(trifluoromethyl)-1H-pyrrol-2-yl]-morpholin-4-yl-methanone (example 004)

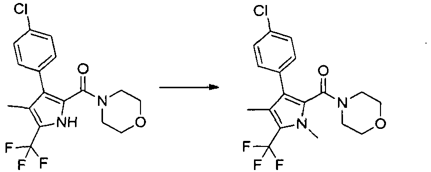

The reaction was carried out in 2 batches. To (3-(4-chlorophenyl)-4-methyl-1H-pyrrol-2-yl)-(morpholino)methanone (PY-1) (80 mg, 0.215 mmol) was added MeCN (2 mL) followed by K₂CO₃ (59 mg, 0.43 mmol) and Mel (40 µL, 0.64 mmol). The reaction mixture was stirred at 80°C in a closed reaction vessel for 5 h and then cooled to RT. Brine (10 mL) and DCM (10 mL) were added. The organic layer was dried (Na₂SO₄) and evaporated under reduced pressure. For the 2^{nd} batch to (3-(4-chlorophenyl)-4-methyl-1H-pyrrol-2-yl)(morpholino)methanone (PY-1) (20 mg, 0.054 mmol) was added MeCN (0.5 mL) followed by K₂CO₃ (15 mg, 0.11 mmol) and Mel (10 µL, 0.16 mmol). The reaction mixture was stirred at 80°C in a closed reaction vessel for 5 h and left standing at RT for 5 d. More Mel (5 µL, 0.08 mmol) was added and stirring at 80°C was continued for 4 h. The mixture was left standing at RT overnight. Brine (5 mL) and DCM (5 mL) were added. The aqueous layer was extracted with DCM (5 mL). Organic layers were combined, dried (Na₂SO₄) and evaporated under reduced pressure. Both batches of crude product were combined and purified by flash chromate-graphy (silica, gradient heptane/CH₂Cl₂, 1:1 → 0:1) to afford 87 mg (84%) of the desired product.

### N-(1-Carbamoyl-cyclopropyl)-3-(4-chlorophenyl)-1-cyclopropyl-N,4-dimethyl-5-(trifluoromethyl)-1H-pyrrole-2-carboxylic acid amide (example 007)

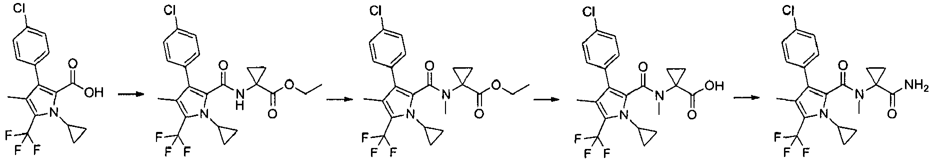

The first 3 steps were carried out in analogy to 051 starting from carboxylic acid ACI-1.

### Step 4: N-(1-Carbamoyl-cyclopropyl)-3-(4-chlorophenyl)-1-cyclopropyl-N,4-dimethyl-5-(trifluoromethyl)-1H-pyrrole-2-carboxylic acid amide (example 007)

NH₄Cl (273 mg, 5.10 mmol), EDCI (215 mg, 1.123 mmol) and HOAt (13.89 mg, 0.102 mmol) were added to a solution of 1-(3-(4-chlorophenyl)-1-cyclopropyl-N,4-dimethyl-5-(trifluoromethyl)-1H-pyrrole-2-carboxamido)cyclopropanecarboxylic acid (450 mg, max. 0.80 mmol) and DIPEA (0.891 mL, 5.10 mmol) in dry dioxane (10 mL). The solution was stirred for 1 h after which DIPEA (0.891 mL, 5.10 mmol), NH₄Cl (273 mg, 5.10 mmol) and EDCI (215 mg, 1.123 mmol) were added and the solution was stirred for 2 h. The mixture was poured out in saturated aqueous NaHCO₃ (100 mL) and extracted with DCM (2x 100 mL). The organic layer was washed with H₂O (100 mL) and brine (100 mL). The organic layer was dried over Na₂SO₄ and the solvents were removed under reduced pressure giving a yellow oil. The residue was purified using flash chromatography (silica, heptane/EtOAc, 7:3→0:1). Fractions containing product were evaporated and co-evaporated with heptane to afford 280 mg (80%, 2 steps) of the desired product as a white solid.

### [3-(4-Chlorophenyl)-1-cyclopropyl-4-methyl-5-(trifluoromethyl)-1H-pyrrol-2-yl]-morpholin-4-yl-methanone (example 043)

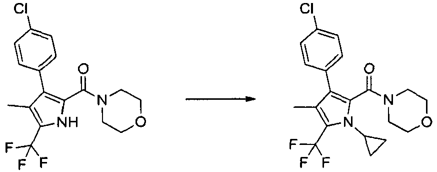

Alkylation was performed in two batches as described below.

Batch 1: To a suspension of (3-(4-chlorophenyl)-4-methyl-5-(trifluoromethyl)-1H-pyrrol-2-yl)(morpholino)methanone (PY-1) (80 mg, 0.215 mmol), cyclopropylboronic acid (36.9 mg, 0.429 mmol) and Na₂CO₃ (45.5 mg, 0.429 mmol) in DCE (1 mL) was added Cu(OAc)₂ (39.0 mg, 0.215 mmol) followed by 2,2'-bipyridyl (33.5 mg, 0.215 mmol). The reaction mixture was stirred at 80 °C in a closed reaction vessel. After 4 h, more cyclopropylboronic acid (36.9 mg, 0.429 mmol), 2,2'-bipyridyl (33.5 mg, 0.215 mmol), Na₂CO₃ (45.5 mg, 0.429 mmol) and Cu(OAc)₂ (39.0 mg, 0.215 mmol) were added and stirring at 80°C was continued for 2 h. The mixture was left standing at RT overnight. More cyclopropylboronic acid (18.43 mg, 0.215 mmol), Na₂CO₃ (22.75 mg, 0.215 mmol), Cu(OAc)₂ (19.49 mg, 0.107 mmol) and 2,2'-bipyridine (16.76 mg, 0.107 mmol) were added and stirring at 80 C was continued. After 2 h, more cyclopropylboronic acid (18.43 mg, 0.215 mmol), Na₂CO₃ (22.75 mg, 0.215 mmol), Cu(OAc)₂ (19.49 mg, 0.107 mmol) and 2,2'-bipyridine (16.76 mg, 0.107 mmol) were added and stirring at 80°C was continued for 2 h. The reaction mixture was left at RT overnight. More cyclopropylboronic acid (18.43 mg, 0.215 mmol), Na₂CO₃ (22.75 mg, 0.215 mmol), Cu(OAc)₂ (19.49 mg, 0.107 mmol), 2,2'-bipyridine (16.76 mg, 0.107 mmol) and DCE (1 mL) were added and stirring at 80°C was continued. After 2 h, more cyclopropylboronic acid (18.43 mg, 0.215 mmol), Na₂CO₃ (22.75 mg, 0.215 mmol), Cu(OAc)₂ (19.49 mg, 0.107 mmol) and 2,2'-bipyridine (16.76 mg, 0.107 mmol) were added and stirring at 80°C was continued for 2 h. The reaction mixture was stored at RT overnight. Brine (10 mL) and DCM (10 mL) were added. The aqueous layer was extracted with DCM (2 x 10 mL). Organic layers were combined, dried (Na₂SO₄) and evaporated under reduced pressure. The crude product was subjected to flash chromatography (silica, gradient heptane/EtOAc, 1:0 → 1:1).

Batch 2: To a suspension of (3-(4-chlorophenyl)-4-methyl-5-(trifluoromethyl)-1H-pyrrol-2-yl)-(morpholino)methanone (PY-1) (80 mg, 0.215 mmol), cyclopropylboronic acid (18.43 mg, 0.215 mmol) and Na₂CO₃ (22.75 mg, 0.215 mmol) in DCE (1 mL) was added Cu(OAc)₂ (19.49 mg, 0.107 mmol) followed by 2,2'-bipyridine (16.76 mg, 0.107 mmol). The reaction mixture was stirred at 80°C in a closed reaction vessel. After 2 h and 4 h, the same amounts of reagents were added again. After stirring for 1 h after the last addition of reagents, heating was stopped and the reaction mixture was left standing at RT overnight. More cyclopropylboronic acid (18.43 mg, 0.215 mmol), Na₂CO₃ (22.75 mg, 0.215 mmol), Cu(OAc)₂ (19.49 mg, 0.107 mmol) and 2,2'-bipyridine (16.76 mg, 0.107 mmol) were added and stirring was continued at 80°C for 4 h. The mixture was left standing at RT overnight. Saturated aqueous NH₄Cl (25 mL) and H₂O (25 mL) were added followed by DCM (25 mL). The aqueous layer was extracted with DCM (25 mL). Organic layers were combined, dried (Na₂SO₄) and evaporated under reduced pressure. The crude product was subjected to flash chromatography (silica, gradient heptane/EtOAc, 1:0 → 1:1). The batches of product from both reactions were combined and this combined batch was purified further by flash chromatography (silica, gradient heptane/EtOAc, 4:1 → 1:1), to give 95 mg (54%) of the desired product.

### [3-(4-Chlorophenyl)-1-cyclopropyl-4-methyl-5-(trifluoromethyl)-1H-pyrrol-2-yl]-(2,2-dimethyl-1,1-dioxo-[1,4]thiazinan-4-yl)-methanone (example 044)

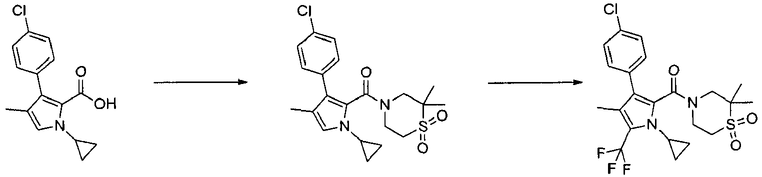

### Step 1-(3-(4-Chlorophenyl)-1-cyclopropyl-4-methyl-1H-pyrrol-2-yl)(2,2-dimethyl-1,1-dioxidothiomorpholino)methanone

To a solution of 3-(4-chlorophenyl)-1-cyclopropyl-4-methyl-1H-pyrrole-2-carboxylic acid [synthesized in analogy to ACI-3, step 4] (400 mg, 1.451 mmol) in DME (10 mL), 2,2-dimethylthiomorpholine 1,1-dioxide (355 mg, 2.176 mmol), DIPEA (0.811 mL, 4.64 mmol) and BOP-Cl (443 mg, 1.741 mmol) were added and the solution was stirred at reflux for 2 h. Subsequently, the reaction mixture was allowed to cool to RT. The reaction mixture was poured out in aqueous 1M KHSO₄ (100 mL) and extracted with DCM (2x 100 mL). The organic layer was washed with saturated aqueous NaHCO₃ (100 mL) and subsequently, with brine (100 mL). The organics were dried over Na₂SO₄ and the solvents were removed under reduced pressure. The residue was purified using flash chromatography (silica, gradient heptane/EtOAc, 9:1→1:1) to give 539 mg (88%) of the desired product as a white solid.

### Step 2: (3-(4-Chlorophenyl)-1-cyclopropyl-4-methyl-5-(trifluoromethyl)-1H-pyrrol-2-yl]-(2,2-dimethyl-1,1-dioxo-[1,4]thiazinan-4-yl)-methanone (example 044)

To a cooled (0°C) solution of (3-(4-chlorophenyl)-1-cyclopropyl-4-methyl-1H-pyrrol-2-yl)(2,2-dimethyl-1,1-dioxidothiomorpholino)methanone (519 mg, 1.233 mmol) in DMF (10 mL) under N₂ atmosphere, FeSO₄·7H₂O (686 mg, 2.466 mmol) and trifluoromethanesulfonyl chloride (0.261 mL, 2.466 mmol) were added. Subsequently, 35% aqueous H₂O₂ (0.324 mL, 3.70 mmol) was added dropwise and the solution was stirred for 30 min at 0°C. Trifluoromethanesulfonyl chloride (0.261 mL, 2.466 mmol) and 35% aqueous H₂O₂ (0.324 mL, 3.70 mmol) were added and the solution was stirred for 30 min at 0°C. The reaction mixture was added dropwise to ice-cold H₂O (150 mL) while stirring vigorously. The precipitate was filtered off and washed with ice-cold H₂O (2x 50 mL). The residue was dissolved in DCM (100 mL) and dried over Na₂SO₄. The solvent was removed under reduced pressure. The residue was purified using flash chromatography (silica, gradient heptane/EtOAc, 9:1→3:2). The product was co-evaporated with pentane (3x 50 mL) under reduced pressure to give 350 mg (58%) the desired product as a white solid.

### N-(1-Carbamoyl-cyclopropyl)-3-(4-chlorophenyl)-N,4-dimethyl-1-(2-methyl-propyl)-5-(trifluoromethyl)-1H-pyrrole-2-carboxylic acid amide (example 051)

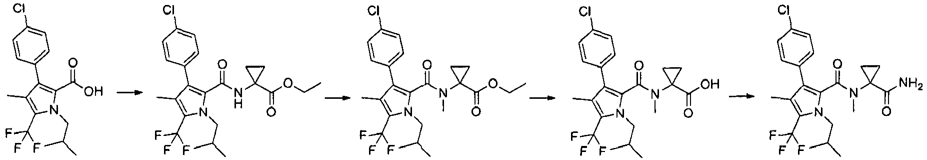

### Step 1: Ethyl 1-(3-(4-chlorophenyl)-1-isobutyl-4-methyl-5-(trifluoromethyl)-1H-pyrrole-2-carboxamido)cyclopropanecarboxylate

To a solution of 3-(4-chlorophenyl)-1-isobutyl-4-methyl-5-(trifluoromethyl)-1H-pyrrole-2-carboxylic acid (ACI-5) (300 mg, 0.834 mmol) in dry DME (5 mL) was added ethyl 1-aminocyclopropanecarboxylate hydrochloride (166 mg, 1.001 mmol) and DIPEA (612 µL, 3.50 mmol) to give a turbid yellow mixture. BOP-Cl (446 mg, 1.751 mmol) was added and the mixture was stirred at 60°C for 1 h, then at RT for 16 h. The reaction mixture was diluted with EtOAc (10 mL) and filtered off. The residue was washed with EtOAc (2x 5 mL) and the combined filtrate was concentrated *in vacuo.* The product was dissolved in EtOAc (50 mL) and washed with saturated aqueous NaHCO₃ (2x 20 mL), aqueous 1M KHSO₄ (2x10 mL) and brine (2x10 mL) before drying on Na₂SO₄ and concentration *in vacuo.* The product was absorbed on hydromatrix and purified using flash chromatography (silica, gradient heptane/EtOAc, 98:2 → 8:2) to give the desired product (158 mg, 40%) as a white solid. Also another batch (29 mg, 7%) was obtained analogously. Total yield: 187 mg (47%).

### Step 2: Ethyl 1-(3-(4-chlorophenyl)-1-isobutyl-N,4-dimethyl-5-(trifluoromethyl)-1H-pyrrole-2-carboxamido)cyclopropanecarboxylate

To a solution of ethyl 1-(3-(4-chlorophenyl)-1-isobutyl-4-methyl-5-(trifluoromethyl)-1H-pyrrole-2-carboxamido)cyclopropanecarboxylate (158 mg, 0.336 mmol) in dry DMF (2 mL) was added 60% NaH in mineral oil (14.76 mg, 0.369 mmol) at 0°C under N₂. The mixture was stirred for 10 min at 0°C, then Mel (42 µL, 0.671 mmol) was added. The mixture was stirred at RT for 1 h. Aqueous 1M KHSO₄ (25 mL) was added to the reaction mixture and the product was extracted with EtOAc (50 mL). The organic layer was washed with aqueous 1M KHSO₄ (2x 20 mL), saturated aqueous NaHCO₃ (2x2 0 mL), aqueous 1M Na₂S₂O₃ (2x 20 mL) and brine (2x 20 mL) before drying on Na₂SO₄ and concentration *in vacuo* to give the desired product (137 mg, 84%) as a colorless oil.

### Step 3: 1-(3-(4-Chlorophenyl)-1-isobutyl-N,4-dimethyl-5-(trifluoromethyl)-1H-pyrrole-2-carboxamido)-cyclopropanecarboxylic acid

To a solution of ethyl 1-(3-(4-chlorophenyl)-1-isobutyl-N,4-dimethyl-5-(trifluoromethyl)-1H-pyrrole-2-carboxamido)cyclopropanecarboxylate (137 mg, 0.283 mmol) in a mixture of THF (0.5 mL) and EtOH (0.5 mL) was added a suspension of LiOH·H₂O (119 mg, 2.83 mmol) in H₂O (0.5 mL). The mixture was stirred at 60°C in a sealed vessel for 3 h. The reaction mixture was concentrated *in vacuo* and the residue was partitioned between aqueous 1M KHSO₄ (10 mL) and EtOAc (10 mL). The aqueous layer was extracted with EtOAc (10 mL) and the combined organic layers were washed with aqueous 1M KHSO₄ (2x 10 mL) and brine (2x 10 mL) before drying on Na₂SO₄ and concentration *in vacuo* to give the desired product (90 mg, 69%) as a white solid.

### Step 4: N-(1-Carbamoyl-cyclopropyl)-3-(4-chlorophenyl)-N,4-dimethyl-1-(2-methyl-propyl)-5-(trifluoromethyl)-1H-pyrrole-2-carboxylic acid amide (example 051)

To a suspension of 1-(3-(4-Chlorophenyl)-1-isobutyl-N,4-dimethyl-5-(trifluoromethyl)-1H-pyrrole-2-carboxamido)cyclopropanecarboxylic acid (83 mg, 0.182 mmol) in dry DME (2 mL) was added 0.4 M NH₃ in THF (1.13 mL, 0.454 mmol), giving a clear light yellow solution. DIPEA (96 µL, 0.545 mmol) was added, followed by BOP-Cl (92 mg, 0.363 mmol) and the resulting white suspension was stirred at RT for 2 h. The reaction mixture was poured out in saturated aqueous NaHCO₃ (20 mL) and the product was extracted with EtOAc (2x 20 mL). The combined organic layers were washed with saturated aqueous NaHCO₃ (2x 10 mL), aqueous 1M KHSO₄ (2x 10 mL) and brine (2x 10 mL) before drying on Na₂SO₄ and concentration *in vacuo.* The product was triturated with MeOH and filtered off.

The solid was washed with MeOH and dried *in vacuo* at 50 °C to give the desired product (48 mg, 58%) as a white solid.

### 3-(4-Chlorophenyl)-N,4-dimethyl-1-[(1-methyl-cyclopropyl)-methyl]-N-(2-methylsulfonyl-ethyl)-5-(trifluoromethyl)-1H-pyrrole-2-carboxylic acid amide (example 068)

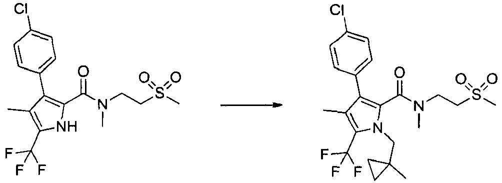

To 3-(4-Chlorophenyl)-N,4-dimethyl-N-(2-methylsulfonylethyl)-5-(trifluoromethyl)-1H-pyrrole-2-carboxamide (PY-4, 100 mg, 0.24 mmol) and PPh₃ (1.2 equiv., 0.075 g, 0.063 mL, 0.28 mmol) in THF (2 mL), 1-methylcyclopropanmethanol (1.3 equiv., 0.027 g, 0.03 mL, 0.31 mmol) was added and the reaction mixture stirred for 5 min at RT. Then diisopropyl azidoformate (1.1 equiv., 0.054 g, 0.052 mL, 0.26 mmol) was added and the reaction mixture stirred at RT for 18 h. Further PPh₃ (1.2 equiv., 0.075 g, 0.063 mL, 0.28 mmol), 1-methylcyclopropanmethanol (1.3 equiv., 0.027 g, 0.031 mL, 0.31 mmol) and diisopropyl azidoformate (1.1 equiv., 0.054 g, 0.052 mL, 0.26 mmol) was added and the reaction mixture stirred at RT. Further PPh₃ (1.2 equiv., 0.075 g, 0.063 mL, 0.28 mmol), 1-methylcyclopropanmethanol (1.3 equiv., 0.027 g, 0.031 mL, 0.31 mmol) and diisopropyl azidoformate (1.1 equiv., 0.054 g, 0.052 mL, 0.26 mmol) was added and reaction mixture stirred at RT for 2 h. The reaction mixture was diluted with 100ml EtOAc and washed with 100 ml H₂O. The aqueous phase was extracted 2 x 100ml EtOAc. The organic phases were combined and reduced under vacuum to residue, which was purified via preparative HPLC using 5-95% MeCN / H₂O (0.1% Formic Acid) over 15 min collecting at 254nm. Pure fractions combined and reduced under vacuum and further purificied via 25g SNAP silica column using 0-50% EtOAc / petroleum ether over 30 min, then 50% for 5 min collecting at 254nm. The pure fractions were combined and reduced under vacuum to yield 3-(4-chlorophenyl)-N,4-dimethyl-1-[(1-methyl-cyclopropyl)-methyl]-N-(2-methylsulfonyl-ethyl)-5-(trifluoromethyl)-1H-pyrrole-2-carboxylic acid amide **(068)** (47.6 mg, 0.048 g, 0.10 mmol) as a white solid.

### [3-(4-Chlorophenyl)-1-[(1-hydroxy-cyclopropyl)-methyl]-4-methyl-5-(trifluoromethyl)-1H-pyrrol-2-yl]-morpholin-4-yl-methanone (example 089)

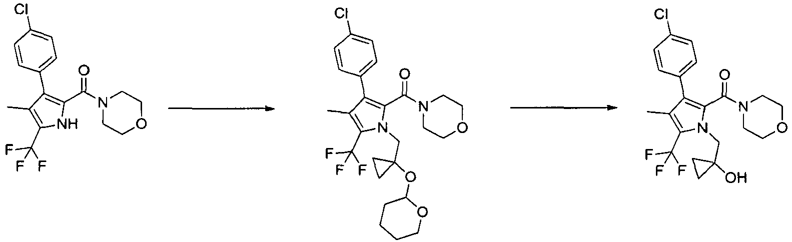

### Step 1: (3-(4-Chlorophenyl)-4-methyl-1-((1-((tetrahydro-2H-pyran-2-yl)oxy)cyclopropyl)methyl)-5-(trifluoromethyl)-1H-pyrrol-2-yl)(morpholino)methanone

DIAD (156 µL, 0.805 mmol) was added dropwise to a cooled solution of PPh₃ (216 mg, 0.825 mmol) in dry THF (5 mL). After 5 min, a suspension is formed. A solution of (3-(4-Chlorophenyl)-4-methyl-1H-pyrrol-2-yl)(morpholino)methanone (PY-1) (150 mg, 0.402 mmol) and (1-((tetrahydro-2H-pyran-2-yl)oxy)cyclopropyl)methanol (AOH-4) (104 mg, 0.604 mmol) in dry THF (2 mL) was added dropwise, giving a clear light yellow solution after addition. The mixture was stirred at RT for 16 h. The reaction mixture was concentrated *in vacuo* and the residue was partitioned between saturated aqueous NaHCO₃ (20 mL) and EtOAc (20 mL). The aqueous layer was extracted with EtOAc (20 mL). The combined organic layers were washed with saturated aqueous NaHCO₃ (2x20 mL) and brine (2x20 mL) before drying on Na₂SO₄ and concentration *in vacuo* to give a yellow oil. The product was purified using flash CC (silica, gradient heptane/EtOAc, 9:1 → 1:1) to give the desired product (174 mg, 64%) as a colorless oil.

### Step 2: [3-(4-Chlorophenyl)-1-[(1-hydroxy-cyclopropyl)-methyl]-4-methyl-5-(trifluoromethyl)-1H-pyrrol-2-yl]-morpholin-4-yl-methanone (example 089)

To a solution of (3-(4-Chlorophenyl)-4-methyl-1-((1-((tetrahydro-2H-pyran-2-yl)oxy)cyclopropyl)-methyl)-5-(trifluoromethyl)-1H-pyrrol-2-yl)(morpholino)methanone (164 mg, 0.243 mmol) in MeOH (15 mL) was added TosOH·H₂O (35 mg, 0.182 mmol) and the mixture was stirred in a sealed vessel at RT for 2 h. The reaction mixture was concentrated *in vacuo* and the residue was partitioned between aqueous 1M KHSO₄ (15 mL) and EtOAc (25 mL). The organic layer was washed with aqueous 1M KHSO₄ (2x15 mL), saturated aqueous NaHCO₃ (2x15 mL) and brine (2x15 mL) before drying on Na₂SO₄ and concentration *in vacuo* to give a colorless oil. The product was purified using flash CC(silica, gradient heptane/EtOAc, 95:5 → 2:3) to give 26 mg of a colorless oil. Also impure fractions were obtained, which were combined and purified further using flash CC (silica, gradient heptane/EtOAc, 95:5 → 2:3) to give 25 mg of a colorless oil. The batches of product were combined and dried to give the desired product (52 mg, 48%) as a colorless oil/foam.

### 3-(4-Chlorophenyl)-N-(2,2-dimethyl-propyl)-1-[(1-hydroxy-cyclopentyl)-methyl]-N-methyl-5-(trifluoromethyl)-1H-pyrrole-2-carboxylic acid amide (example 090)

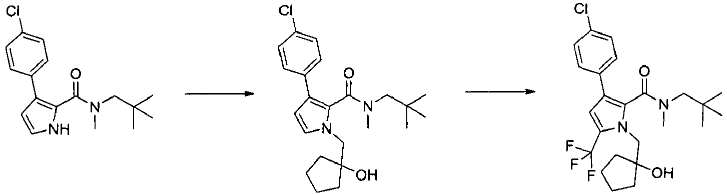

### Step 1: 3-(4-Chlorophenyl)-1-((1-hydroxycyclopentyl)methyl)-N-methyl-N-neopentyl-1H-pyrrole-2-carboxamide

3-(4-Chlorophenyl)-N-methyl-N-neopentyl-1H-pyrrole-2-carboxamide (step 4, PY-2) (400 mg, 1.31 mmol) was dissolved in dry MeCN (20 mL) and this solution was combined with Cs₂CO₃ (855 mg, 2.62 mmol) and 1-oxaspiro[2.4]heptane (283 mg, 2.89 mmol). The reaction mixture was stirred vigorously at 60°C overnight. Extra 1-oxaspiro[2.4]heptane (139 mg, 1.31 mmol) was added. The reaction mixture was stirred vigorously at 60°C overnight. The resulting reaction mixture was combined with silica (1 g). Filtration and washing of the residue with EtOAc (3x5 mL) was followed by concentration *in vacuo.* The residue was dissolved in DCM and subsequently brought on silica. The product was purified by flash chromatography (silica, gradient heptane/EtOAc, 1:0 → 65:35) to afford 360 mg (68%) of the desired product as an orange oil.

### Step 2: 3-(4-Chlorophenyl)-N-(2,2-dimethyl-propyl)-1-[(1-hydroxy-cyclopentyl)-methyl]-N-methyl-5-(trifluoromethyl)-1H-pyrrole-2-carboxylic acid amide (example 090)

3-(4-Chlorophenyl)-1-((1-hydroxycyclopentyl)methyl)-N-methyl-N-neopentyl-1H-pyrrole-2-carboxamide (238 mg, 0.591 mmol) was dissolved in DMSO (6 mL), this solution was combined with crushed FeSO₄·7H₂O (49.3 mg, 0.177 mmol). Trifluoromethyl iodide (gas) was bubbled through the solution for 3 min. Dropwise addition of aqueous 18% H₂O₂ (0.201 mL, 1.18 mmol) was followed by stirring at RT for 20 min. Extra aqueous 18% H₂O₂ (0.100 mL, 0.591 mmol) was added, followed by stirring at RT for 20 min. Careful addition of diluted brine (40 mL, 1:1 H₂O:brine) caused gas evolution. The mixture was stirred for 10 min before EtOAc (30 mL) was added. Stirring well resulted in a clear two phase system. The layers were separated and the orange aqueous layer was extracted with EtOAc (10 mL). The combination of organic layers was washed with H₂O (10 mL), brine and dried on Na₂SO₄, followed by concentration *in vacuo.* The residue was dissolved in DCM and subsequently brought on silica. The product was purified by flash chromatography (silica, gradient heptane/EtOAc, 1:0 → 7:3) to afford 87 mg (31 %) of the desired product as a fluffy white solid.

### 3-(4-Chlorophenyl)-N-(2,2-dimethyl-propyl)-N-methyl-1-(pyrrolidin-2-yl-methyl)-5-(trifluoromethyl)-1H-pyrrole-2-carboxylic acid amide (example 091)

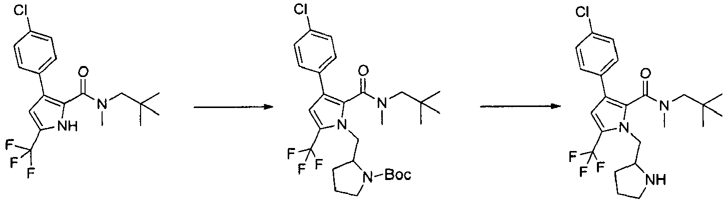

### Step 1: tert-Butyl 2-((3-(4-chlorophenyl)-2-(methyl(neopentyl)carbamoyl)-5-(trifluoromethyl)-1H-pyrrol-1-yl)methyl)gyrrolidine-1-carboxylate

A solution of 3-(4-chlorophenyl)-N-methyl-N-neopentyl-5-(trifluoromethyl)-1H-pyrrole-2-carboxamide (PY-2) (300 mg, 0.69 mmol) and PPh₃ (290 mg, 1.11 mmol) in dry THF (0.40 mL) was prepared and cooled with a water bath. DIAD (175 µL, 0.90 mmol) was added dropwise to result in a solid reaction mixture. The water bath was removed, extra dry THF (0.1 mL) was added. 1-Boc-(2-hydroxymethyl)-pyrrolidine (181 mg, 0.90 mmol) was added in three equal portions, with 15 min between every addition. The solid reaction mixture became a well stirrable suspension and was stirred overnight at RT. PPh₃ (218 mg, 0.83 mmol) and dry THF (0.40 mL) were added. DIAD (175 µL, 0.90 mmol) was added dropwise to result in a solid reaction mixture, which was sonicated for 20 min to result in a stirrable slurry. 1-Boc-(2-hydroxymethyl)pyrrolidine (181 mg, 0.90 mmol) was added in three equal portions, with 15 min between every addition. The reaction mixture became a well stirrable suspension and was stirred overnight at RT. The suspension was combined with EtOAc (1 mL), some warming resulted in a clear solution. Heptane (8 mL) was added in portions of 2 mL, to result in a precipitate.

Filtration provided a residue and a filtrate. The residue was discarded, the filtrate was concentrated *in vacuo,* dissolved in DCM (2 mL) and used for flash chromatography (silica, gradient heptane/EtOAc, 100:0 to 80:20). The impure product was dissolved in DCM (1 mL) and used for flash chromatography (silica, heptane/EtOAc, 93:7) to result in 124 mg (32%) of the desired product as a white foam.

### Step 2: 3-(4-Chlorophenyl)-N-(2,2-dimethyl-propyl)-N-methyl-1-(pyrrolidin-2-yl-methyl)-5-(trifluoromethyl)-1H-pyrrole-2-carboxylic acid amide (example 091)

tert-Butyl 2-((3-(4-chlorophenyl)-2-(methyl(neopentyl)carbamoyl)-5-(trifluoromethyl)-1H-pyrrol-1-yl)methyl)pyrrolidine-1-carboxylate (124 mg, 0.223 mmol) was dissolved in dry DCM (3 mL), 4M HCl in dioxane (3 mL, 12 mmol) was added dropwise. The reaction mixture was stirred at RT for 5 h. The reaction mixture was combined with aqueous 2M NaOH (10 mL), followed by some ice (5 mL) and DCM (20 mL). The phases were separated, the aqueous layer was extracted with DCM (10 mL). The combination of organic phases was washed with brine and dried (Na₂SO₄), followed by concentration *in vacuo.* The residue was dissolved in MeCN (10 mL), followed by concentration *in vacuo.* The residue was dissolved in MeCN (2 mL). The slightly turbid solution was filtered off, mixed with H₂O (1 mL) and used for freeze drying to result in 92 mg (90%) of the desired product as a fluffy white powder.

### [1-[(1-Amino-cyclopropyl)-methyl]-3-(4-chlorophenyl)-4-methyl-5-(trifluoromethyl)-1H-pyrrol-2-yl]-morpholin-4-yl-methanone (example 095)

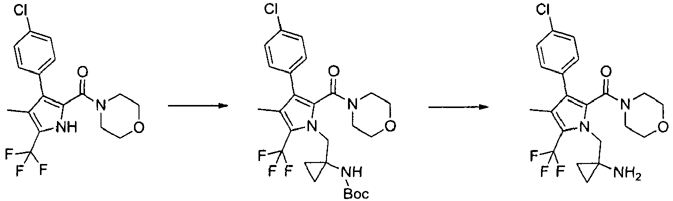

### Step 1: tert-Butyl (1-((3-(4-chlorophenyl)-4-methyl-2-(morpholine-4-carbonyl)-5-(trifluoromethyl)-1H-pyrrol-1-yl)methyl)cyclopropyl)carbamate

To (3-(4-chlorophenyl)-4-methyl-1H-pyrrol-2-yl)(morpholino)methanone (PY-1) (400 mg, 1.073 mmol), tert-Butyl (1-(hydroxymethyl)cyclopropyl)carbamate (AOH-3) (301 mg, 1.610 mmol) and PPh₃ (563 mg, 2.146 mmol) was added dry THF (0.33 mL) and the mixture was sonicated for 3 min. More dry THF (0.17 mL) was added and sonication was continued for 2 min upon which a very viscous solution was obtained. DIAD (0.417 mL, 2.146 mmol) was added dropwise and the reaction mixture was sonicated at RT for 1 h. More alcohol AOH-3 (301 mg, 1.610 mmol) and PPh₃ (563 mg, 2.146 mmol) were added and the mixture was sonicated for 5 min DIAD (0.417 mL, 2.146 mmol) was added dropwise and the mixture was sonicated for 1 h. More alcohol AOH-3 (301 mg, 1.610 mmol) and PPh₃ (563 mg, 2.146 mmol) were added and the mixture was sonicated for 5 min. DIAD (0.417 mL, 2.146 mmol) was added and sonication was continued for 1 h. Heptane (5 mL) and a stir bar were added and the mixture was stirred at RT overnight. The mixture was sonicated for 6 h and left standing over the weekend. The suspension was filtered and the residue washed with Et₂O. The combined filtrate was evaporated under reduced pressure. The product was purified by flash chromatography (silica, gradient heptane/EtOAc, 4:1 → 1:1), followed by reversed phase chromatography (C18, H₂O/MeCN/TFA). Product containing fractions were combined and concentrated to a smaller volume. Saturated aqueous NaHCO₃ (25 mL) and DCM (25 mL) were added. The organic layer was dried (Na₂SO₄) and evaporated under reduced pressure, to afford 210 mg (36%) of the desired product.

### Step 2: [1-[(1-Amino-cyclopropyl)-methyl]-3-(4-chlorophenyl)-4-methyl-5-(trifluoromethyl)-1H-pyrrol-2-yl]-morpholin-4-yl-methanone (example 095)

To a solution of tert-butyl (1-((3-(4-chlorophenyl)-4-methyl-2-(morpholine-4-carbonyl)-5-(trifluoromethyl)-1H-pyrrol-1-yl)methyl)cyclopropyl)carbamate (210 mg, 0.387 mmol) in DCM (5 mL) was added 4 M HCl in dioxane (5 mL, 20 mmol) and the reaction mixture was stirred at RT overnight. Ice (25 mL) and aqueous 1M NaOH (25 mL) were added and the mixture was extracted with DCM (3x 25 mL). Organic layers were combined, dried (Na₂SO₄) and evaporated under reduced pressure. The product was purified by flash chromatography (silica, gradient DCM/(7M NH₃ in MeOH), 1:0 → 98:2). The product was co-evaporated with MeOH (3x), transferred to a vial as a solution in MeOH. The solvent was removed with a flow of N₂ and the product dried *in vacuo,* to obtain 123 mg (72%) of the desired produc.

### [3-(4-Chlorophenyl)-1-[(1-hydroxy-cyclopentyl)-methyl]-4-methyl-5-(trifluoromethyl)-1H-pyrrol-2-yl]-morpholin-4-yl-methanone (example 096)

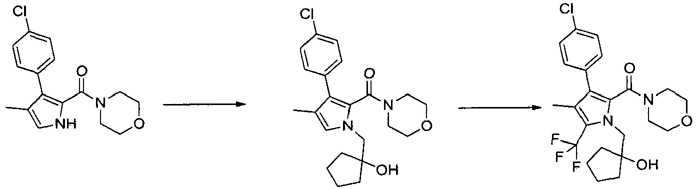

### Step 1: (3-(4-Chlorophenyl)-1-((1-hydroxycyclopentyl)methyl)-4-methyl-1H-pyrrol-2-yl)(morpholino)methanone

To a solution of (3-(4-chlorophenyl)-4-methyl-1H-pyrrol-2-yl)(morpholino)methanone [step 4, PY-1] (500 mg, 1.64 mmol) and 1-oxaspiro[2.4]heptane (177 mg, 1.81 mmol) in dry MeCN (4 mL) was added Cs₂CO₃ (641 mg, 1.97 mmol) and the reaction mixture was stirred at 60 °C overnight. The reaction mixture was allowed to cool to RT, then poured into ice water, acidified with aqueous 1M KHSO₄ (10 mL) and extracted with EtOAc. The organic layer was washed with brine, dried (Na₂SO₄) and concentrated. The residue was first purified by CC(silica, heptane/EtOAc, 1:1), followed by reversed phase chromatography (C18, H₂O/MeCN + 0.1% (v/v) TFA) to give two fractions of product. The first fraction contains nearly pure product (46 mg, purity: 94%, 7%) and the second fraction contains pure product (120 mg, 18%).

### Step 2: [3-(4-Chlorophenyl)-1-[(1-hydroxy-cyclopentyl)-methyl]-4-methyl-5-(trifluoromethyl)-1H-pyrrol-2-yl]-morpholin-4-yl-methanone (example 096)

Argon was bubbled through a suspension of (3-(4-chlorophenyl)-1-((1-hydroxycyclopentyl)methyl)-4-methyl-1H-pyrrol-2-yl)(morpholino)methanone (109 mg, 0.271 mmol) and K₂HPO₄ (141 mg, 0.812 mmol) in anhydrous MeCN (3 mL) for 30 min while stirring vigorously, after which dichlorotris(1,10-phenanthroline)ruthenium(II) hydrate (10 mg, 0.014 mmol) and trifluoromethanesulfonyl chloride (0.043 mL, 0.406 mmol) were added. The reaction mixture was irradiated with a fluorescent light bulb (E27-23W, 4000K, 165 mA) overnight and then poured into icecold H₂O. The formed precipitate was filtered off, washed with H₂O (3x) and a minimum amount of heptane. The precipitate was dried on filter for 1h and dissolved in EtOAc. The organic layer was dried (Na₂SO₄) and concentrated. Crystallisation (MeOH/H₂O) of the residue gave the desired product (69 mg, 54%).

### [3-(4-Chlorophenyl)-4-methyl-1-(2-methylsulfonyl-ethyl)-5-(trifluoromethyl)-1H-pyrrol-2-yl]-morpholin-4-yl-methanone (example 099)

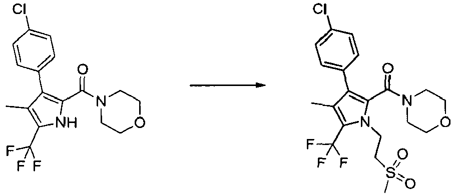

(3-(4-Chlorophenyl)-4-methyl-1H-pyrrol-2-yl)(morpholino)methanone (PY-1) (100 mg, 0.268 mmol) was combined with methyl vinyl sulfone (0.235 mL, 2.68 mmol). Dry DMF (0.25 mL) was added, followed by Cs₂CO₃ (8.74 mg, 0.027 mmol). The reaction mixture was stirred at RT for 3 d and combined with saturated aqueous NaHCO₃ (15 mL), some ice and EtOAc (20 mL). The layers were separated, the organic layer was washed with diluted aqueous NaHCO₃ (10 mL) and dried with brine and Na₂SO₄. Concentration *in vacuo* was followed by purification by flash chromatography (silica, gradient heptane/EtOAc, 1:0 → 1:4). The resulting oil was dissolved in DMF (30 mL), the solution was concentrated *in vacuo.* This was repeated two times. The residue was dissolved in EtOAc (25 mL), washed with diluted aqueous NaHCO₃ (3x20 mL) and dried with brine and Na₂SO₄. Concentration *in vacuo* was followed by lyophilisation to provide 98 mg (76%) of the desired product as a white fluffy solid.

### 3-(4-Chlorophenyl)-N-(2,2-dimethyl-propyl)-1-[(1-hydroxy-cyclobutyl)-methyl]-N-methyl-5-(trifluoromethyl)-1H-pyrrole-2-carboxylic acid amide (example 101)

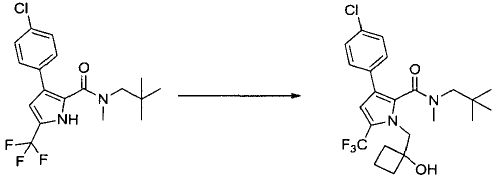

A 50 mL round bottomed flask equipped with a reflux cooler was used. 3-(4-Chlorophenyl)-N-methyl-N-neopentyl-5-(trifluoromethyl)-1H-pyrrole-2-carboxamide (PY-2) (195 mg, 0.523 mmol) was dissolved in dry MeCN (15 mL), this solution was combined with Cs₂CO₃ (511 mg, 1.569 mmol) and 1-oxaspiro-[2.3]hexane (88 mg, 1.046 mmol). The reaction mixture was stirred vigorously at 60°C overnight. Extra 1-oxaspiro[2.3]hexane (88 mg, 1.046 mmol) was added, the reflux cooler was replaced by a stopper. The reaction mixture was stirred vigorously at 60°C overnight. Extra 1-oxaspiro[2.3]hexane (88 mg, 1.046 mmol) was added, the reaction mixture was stirred vigorously at 60°C overnight. The resulting reaction mixture was combined with silica (0.5 g). Filtration and washing of the residue with EtOAc (3x 5 mL) was followed by concentration *in vacuo.* The residue was dissolved in DCM and subsequently brought on silica. The product was purified by flash chromatography (silica, gradient heptane/EtOAc, 1:0 → 3:1) to afford 66 mg (27%) of the desired product as a fluffy white solid.

### 3-(5-Chloro-pyridin-2-yl)-N-(2-cyano-2-methyl-propyl)-1-isopropyl-N,4-dimethyl-5-(trifluoromethyl)-1H-pyrrole-2-carboxylic acid amide (example 117)

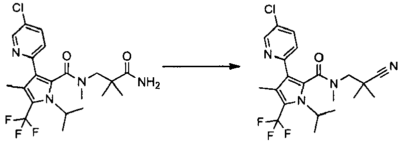

To a cooled solution of N-(2-carbamoyl-2-methyl-propyl)-3-(5-chloro-pyridin-2-yl)-1-isopropyl-N,4-dimethyl-5-(trifluoromethyl)-1H-pyrrole-2-carboxylic acid amide (example 116) (180 mg, 0.294 mmol) in pyridine (4 mL) was added trifluoroacetic anhydride (106 µL, 0.765 mmol) dropwise over 2 min under N₂. The mixture was stirred at 0 °C for 1.5 h. Aqueous 1M HCl (20 mL) was added, followed by CH₂Cl₂ (50 mL) and aqueous 5M HCl (15 mL). The layers were separated and the aqueous layer was extracted with CH₂Cl₂ (20 mL). The combined organic layers were washed with aqueous 1M KHSO₄ (2x20 mL), saturated aqueous NaHCO₃ (2x20 mL) and brine (2x20 mL) before drying on Na₂SO₄ and concentration *in vacuo.* The crude product was purified using flash cc (silica, gradient heptane/EtOAc, 1:0 → 7:3) to give the desired product (92 mg, 71%) as a colorless oil which solidified on standing.

### 3-(5-Chloro-pyridin-2-yl)-N-(3,3-dimethyl-piperidin-4-yl)-1-isopropyl-N,4-dimethyl-5-(trifluoromethyl)-1H-pyrrole-2-carboxylic acid amide (example 134)

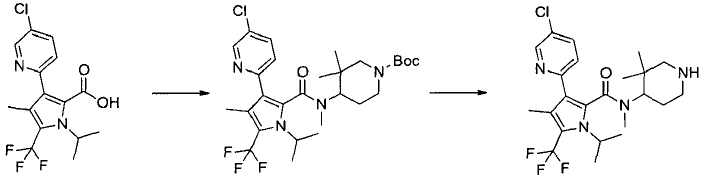

### Step 1: tert-Butyl 4-(3-(5-chloropyridin-2-yl)-1-isopropyl-N,4-dimethyl-5-(trifluoromethyl)-1H-pyrrole-2-carboxamido)-3,3-dimethylpiperidine-1-carboxylate

To a suspension of 3-(5-chloropyridin-2-yl)-1-isopropyl-4-methyl-5-(trifluoromethyl)-1H-pyrrole-2-carboxylic acid (ACI-10) (122 mg, max. 0.33 mmol), DIPEA (0.184 mL, 1.056 mmol) and BOP-Cl (179 mg, 0.704 mmol) in DME (0.5 mL) was added *tert*-butyl 3,3-dimethyl-4-(methylamino)piperidine-1-carboxylate (171 mg, 0.704 mmol). The reaction mixture was stirred at 60 °C for 2 h and left standing at room temperature overnight. Stirring was continued at 60 °C for 5 h and the mixture was left standing at room temperature overnight. More DIPEA (0.184 mL, 1.056 mmol), BOP-Cl (90 mg, 0.352 mmol) and DME (0.5 mL) were added followed by tert-butyl 3,3-dimethyl-4-(methylamino)piperidine-1-carboxylate (85 mg, 0.352 mmol). Stirring at 60 °C was continued for 6 h and the mixture was left standing at room temperature overnight. Stirring was continued for 3 h at 80 °C. EtOAc (50 mL) and H₂O (50 mL) were added. The organic layer was extracted with saturated aqueous NaHCO₃ (50 mL), dried (Na₂SO₄) and evaporated under reduced pressure. The product was purified by flash cc (silica, gradient heptane/EtOAc, 1:0 → 1:1), to afford 84 mg (45% over two steps) of the desired product.

### Step 2: 3-(5-Chloro-pyridin-2-yl)-N-(3,3-dimethyl-piperidin-4-yl)-1-isopropyl-N,4-dimethyl-5-(trifluoromethyl)-1H-pyrrole-2-carboxylic acid amide

tert-butyl 4-(3-(5-chloropyridin-2-yl)-1-isopropyl-N,4-dimethyl-5-(trifluoromethyl)-1H-pyrrole-2-carboxamido)-3,3-dimethylpiperidine-1-carboxylate (83 mg, 0.145 mmol) was dissolved in 4 M HCl in dioxane (8 mL, 32 mmol). The reaction mixture was stirred at RT for 4.5 h. CH₂Cl₂ (50 mL) was added followed by the careful addition of saturated aqueous NaHCO₃ (50 mL). The aqueous layer was extracted with CH₂Cl₂ (50 mL). The organic layers were combined, dried (Na₂SO₄) and evaporated under reduced pressure. The product was purified by flash cc (silica, gradient CH₂Cl₂/(7M NH₃ in MeOH), 1:0 → 97:3) to give 40 mg (58%) of the desired product.

### 3-(4-Chlorophenyl)-N-(3,3-dimethyl-piperidin-4-yl)-1-isopropyl-N,4-dimethyl-5-(trifluoromethyl)-1H-pyrrole-2-carboxylic acid amide (example 135)

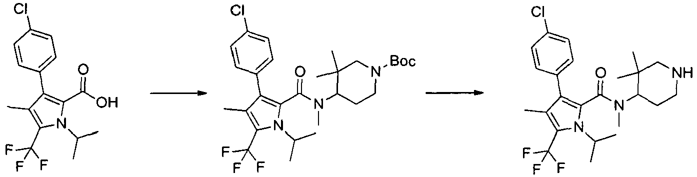

### Step 1: tert-Butyl 4-(3-(4-chlorophenyl)-1-isopropyl-N,4-dimethyl-5-(trifluoromethyl)-1H-pyrrole-2-carboxamido)-3,3-dimethylpiperidine-1-carboxylate

To a solution of 3-(4-chlorophenyl)-1-isopropyl-4-methyl-5-(trifluoromethyl)-1H-pyrrole-2-carboxylic acid (ACl-9) (130 mg, 0.376 mmol), DIPEA (0.394 mL, 2.256 mmol) and BOP-Cl (191 mg, 0.752 mmol) in dry DME (2 mL), *tert*-butyl 3,3-dimethyl-4-(methylamino)piperidine-1-carboxylate (91 mg, 0.376 mmol) was added and the reaction mixture was stirred for 18 h at 70 °C. The reaction mixture was partitioned between saturated aqueous NaHCO₃ (50 mL) and EtOAc (50 mL). The aqueous phase was extracted with EtOAc (50 mL). The combined organics were washed with brine and dried over Na₂SO₄. The solvent was removed under reduced pressure and the residue was purified using flash cc (silica, gradient heptane/EtOAc, 1:0 → 1:1) to give the desired product.

### Step 2: 3-(4-Chlorophenyl)-N-(3,3-dimethyl-piperidin-4-yl)-1-isopropyl-N,4-dimethyl-5-(trifluoromethyl)-1H-pyrrole-2-carboxylic acid amide

tert-Butyl 4-(3-(4-chlorophenyl)-1-isopropyl-N,4-dimethyl-5-(trifluoromethyl)-1H-pyrrole-2-carboxamido)-3,3-dimethylpiperidine-1-carboxylate was dissolved in 4M HCl in dioxane (10 mL, 40 mmol) and stirred for 18 h at RT. The solvent was removed under reduced pressure and the residue was partitioned between CH₂Cl₂ (10 mL) and saturated aqueous Na₂CO₃ (10 mL). The organics were separated using a phase separator and the solvent was removed under reduced pressure to afford the product as a yellow oil which was purified using reversed phase cc (C18, H₂O/MeCN/HCO₂H). Fractions containing the product were combined and partitioned between saturated aqueous Na₂CO₃ (50 mL) and EtOAc (50 mL). The aqueous phase was extracted with EtOAc (50 mL). The combined organics were washed with brine (50 mL) and dried over Na₂SO₄. The solvent was removed under reduced pressure and was subsequently lyophilised using MeCN/H₂O (3/1, v/v, 2 mL) to afford 46 mg (26% over two steps) of the desired product as an off-white solid.

### ANALYTICS

Material and Methods for LC/MS Analytics: **Hardware:** Coupled Agilent 1290 Infinity UHPLC-TOF System; *LC-Module:* MTP-Handler: Agilent, Model BenchCel 2R; Themostatic Control Autoinjector: Agilent, Modell G4226A; Column oven: Agilent, Model G1316C; DAD: Agilent, Model G4212A; Binary Pump: Agilent, Model G4220A; *Time Of Flight Mass Spectrometer:* Agilent 6224; Ion source: Dual ESI; **Column:** Supplier: Waters; Type: Acquity UPLC HSS T3 1.8µm (Part No. 186003538); Dimensions: 2.1 x 50 mm; **Eluents:** Eluent A: H₂O from Millipore Ultrapure water System: Milli-Q Integral 3 + 0.1 % Formic acid; Eluent B: MeCN, Merck KGaA: LiChrosolv Hypergrade for LC-MS (1.00029.9010) + 0.1 % Formic acid; Formic acid: Merck KGaA: Suprapure 98-100% (1.11670.1000); **LC-Method:** Flow: 2.5 mL/min; Runtime: 1.2 min; Gradient: Start 2% B, 1 min 100% B, 1.09 min 100% B, 1.11 min 2% B, 1.2 min 2% B Stop; Columntemperature: 80°C; UV: 190-400 nm; **MS-Method:** Ion Polarity: Positive; Gas Temperature: 325 °C; Gas Flow: 10 mL/min

| **No.** | **Ex. R¹** | **n** | **R²** | **R³** | **(Het)aryl** | **N(R⁴)(R⁵)** | **Target Mass** | **Target Mass Found** | **UV254-purity** |
|---|---|---|---|---|---|---|---|---|---|
| **001** | CH₃ | 0 | CF₃ | CH₃ | 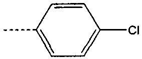 | 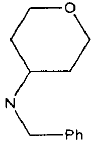 | 490.95 | Yes | 99 |
| **002** | CH₃ | 0 | CF₃ | CH₃ | 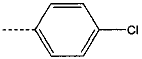 | 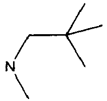 | 400.87 | Yes | 100 |
| **003** | CH₃ | 0 | CF₃ | CH₃ | 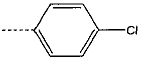 | 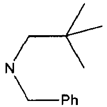 | 476.96 | Yes | 99 |
| **004** | CH₃ | 0 | CF₃ | CH₃ | 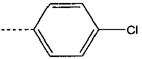 | 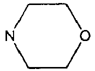 | 386.80 | Yes | 100 |
| **005** | CH₃ | 1 | CF₃ | CH₃ | 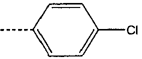 | 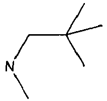 | 414.89 | Yes | 97 |
| **006** | CH(CH₃)₂ | 0 | CF₃ | CH₃ | 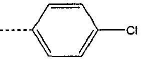 | 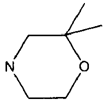 | 442.90 | Yes | 100 |
| **007** | 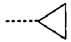 | 0 | CF₃ | CH₃ | 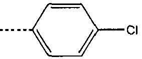 | 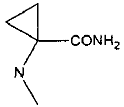 | 439.86 | Yes | 100 |
| **008** | 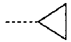 | 0 | CF₃ | CH₃ | 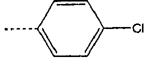 | 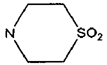 | 488.95 | Yes | 100 |
| **009** | 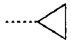 | 0 | CF₃ | CH₃ | 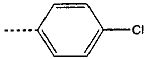 | 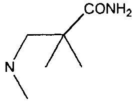 | 455.90 | Yes | 100 |
| **010** | 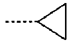 | 0 | CF₃ | CH₃ | 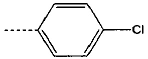 | 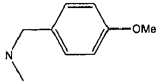 | 476.92 | Yes | 100 |
| **011** | 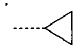 | 0 | CF₃ | H | 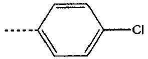 | 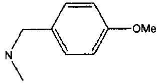 | 462.89 | Yes | 100 |
| **012** | 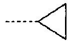 | 0 | CF₃ | CH₃ | 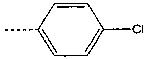 | 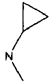 | 396.83 | Yes | 100 |
| **013** | 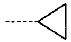 | 0 | CF₃ | H | 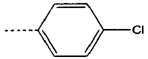 | 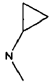 | 382.81 | Yes | 98 |
| **014** | 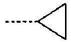 | 0 | CF₃ | CH₃ | 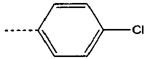 | 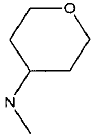 | 440.89 | Yes | 100 |
| **015** | 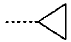 | 0 | CF₃ | H | 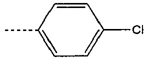 | 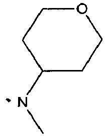 | 426.86 | Yes | 100 |
| **016** | 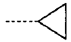 | 0 | CF₃ | CH₃ | 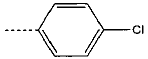 | 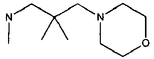 | 512.01 | Yes | 92 |
| **017** | 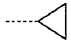 | 0 | CF₃ | H | 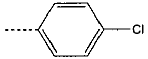 | 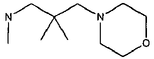 | 497.98 | Yes | 92 |
| **018** | 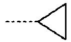 | 0 | CF₃ | CH₃ | 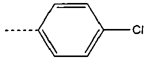 | 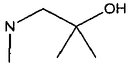 | 428.88 | Yes | 100 |
| **019** | 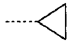 | 0 | CF₃ | H | 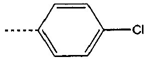 | 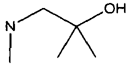 | 414.85 | Yes | 99 |
| **020** | 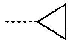 | 0 | CF₃ | CH₃ | 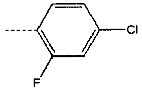 | 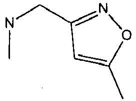 | 469.86 | Yes | 96 |
| **021** | 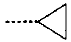 | 0 | CF₃ | CH₃ | 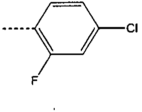 | 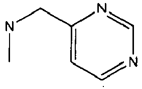 | 466.86 | Yes | 98 |
| **022** | 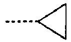 | 0 | CF₃ | CH₃ | 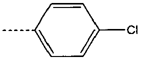 | 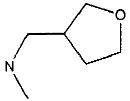 | 440.89 | Yes | 100 |
| **023** | 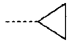 | 0 | CF₃ | H | 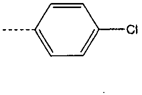 | 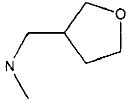 | 426.86 | Yes | 100 |
| **024** | 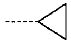 | 0 | CF₃ | CH₃ | 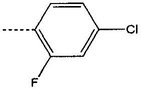 | 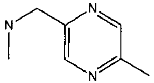 | 480.89 | Yes | 98 |
| **025** | 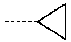 | 0 | CF₃ | CH₃ | 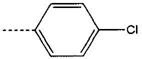 | 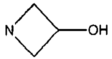 | 398.81 | Yes | 100 |
| **026** | 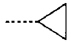 | 0 | CF₃ | H | 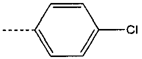 | 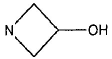 | 384.78 | Yes | 99 |
| **027** | 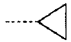 | 0 | CF₃ | CH₃ | 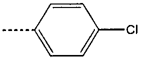 | 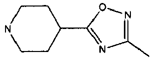 | 492.92 | Yes | 97 |
| **028** | 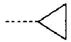 | 0 | CF₃ | CH₃ | 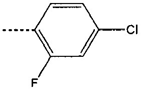 | 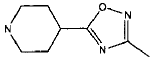 | 510.91 | Yes | 98 |
| **029** | 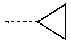 | 0 | CF₃ | H | 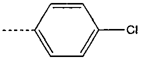 | 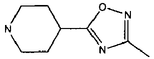 | 478.90 | Yes | 98 |
| **030** | 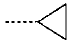 | 0 | CF₃ | CH₃ | 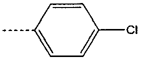 | 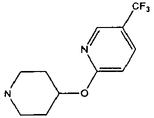 | 571.94 | Yes | 100 |
| **031** | 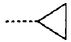 | 0 | CF₃ | H | 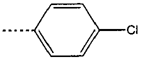 | 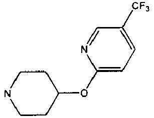 | 557.92 | Yes | 100 |
| **032** | 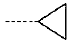 | 0 | CF₃ | CH₃ | 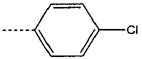 | 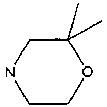 | 440.89 | Yes | 100 |
| **033** | 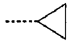 | 0 | CF₃ | CH₃ | 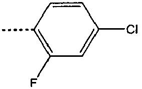 | 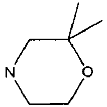 | 458.88 | Yes | 98 |
| **034** | 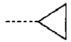 | 0 | CF₃ | H | 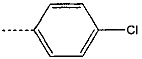 | 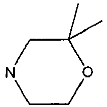 | 426.86 | Yes | 100 |
| **035** | 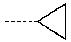 | 0 | CF₃ | H | 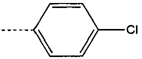 | 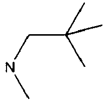 | 412.88 | Yes | 100 |
| **036** | 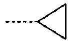 | 0 | CF₃ | H | 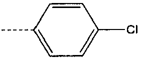 | 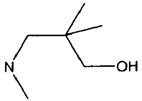 | 428.88 | Yes | 100 |
| **037** | 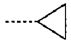 | 0 | CF₃ | H | 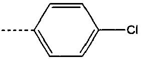 | 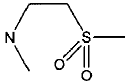 | 448.89 | Yes | 100 |
| **038** | 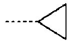 | 0 | CF₃ | H | 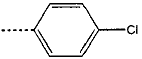 | 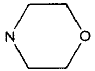 | 398.81 | Yes | 100 |
| **039** | 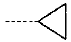 | 0 | CF₃ | CH₃ | 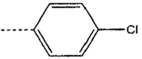 | 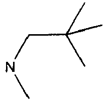 | 426.90 | Yes | 100 |
| **040** | 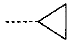 | 0 | CF₃ | CH₃ | 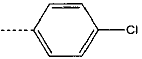 | 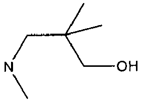 | 442.90 | Yes | 100 |
| **041** | 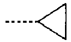 | 0 | CF₃ | CH₃ | 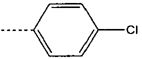 | 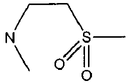 | 462.91 | Yes | 100 |
| **042** | 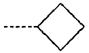 | 0 | CF₃ | CH₃ | 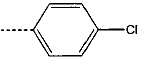 | 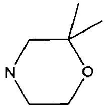 | 454.91 | Yes | 100 |
| **043** | 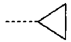 | 0 | CF₃ | CH₃ | 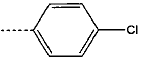 | 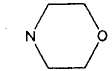 | 412.83 | Yes | 100 |
| **044** | 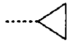 | 0 | CF₃ | CH₃ | 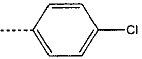 | 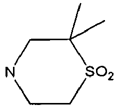 | 488.95 | Yes | 100 |
| **045** | CH(CH₃)₂ | 1 | CF₃ | H | 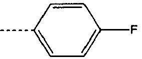 | 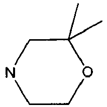 | 426.45 | Yes | 100 |
| **046** | CH(CH₃)₂ | 1 | CF₃ | H | 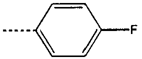 | 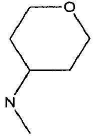 | 426.45 | Yes | 100 |
| **047** | CH(CH₃)₂ | 1 | CF₃ | H | 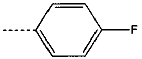 | 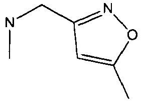 | 437.43 | Yes | 100 |
| **048** | CH(CH₃)₂ | 1 | CF₃ | CH₃ | 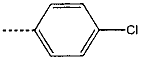 | 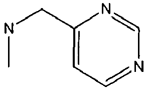 | 464.91 | Yes | 99 |
| **049** | CH(CH₃)₂ | 1 | CF₃ | H | 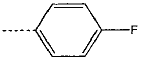 | 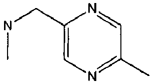 | 448.46 | Yes | 100 |
| **050** | CH(CH₃)₂ | 1 | CF₃ | H | 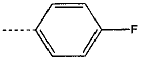 | 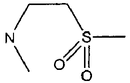 | 448.48 | Yes | 100 |
| **051** | CH(CH₃)₂ | 1 | CF₃ | CH₃ | 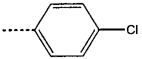 | 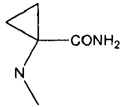 | 455.90 | Yes | 100 |
| **052** | CH(CH₃)₂ | 1 | CF₃ | CH₃ | 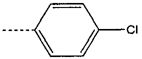 | 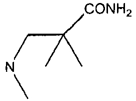 | 471.94 | Yes | 97 |
| **053** | CH(CH₃)₂ | 1 | CF₃ | H | 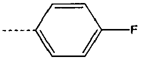 | 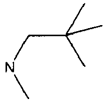 | 412.46 | Yes | 100 |
| **054** | CH(CH₃)₂ | 1 | CF₃ | H | 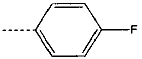 | 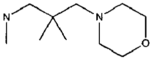 | 497.57 | Yes | 98 |
| **055** | CH(CH₃)₂ | 1 | CF₃ | CH₃ | 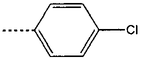 | 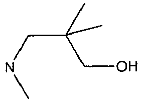 | 458.95 | Yes | 93 |
| **056** | CH(CH₃)₂ | 1 | CF₃ | H | 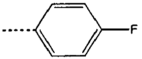 | 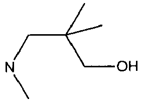 | 428.46 | Yes | 100 |
| **057** | CH(CH₃)₂ | 1 | CF₃ | CH₃ | 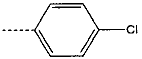 | 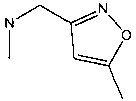 | 467.91 | Yes | 98 |
| **058** | CH(CH₃)₂ | 1 | CF₃ | H | 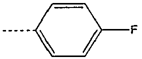 | 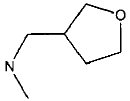 | 426.45 | Yes | 100 |
| **059** | CH(CH₃)₂ | 1 | CF₃ | CH₃ | 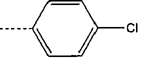 | 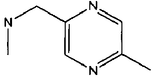 | 478.94 | Yes | 97 |
| **060** | CH(CH₃)₂ | 1 | CF₃ | H | 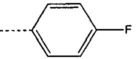 | 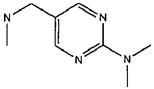 | 477.50 | Yes | 100 |
| **061** | CH(CH₃)₂ | 1 | CF₃ | CH₃ | 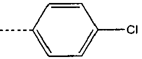 | 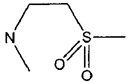 | 478.96 | Yes | 94 |
| **062** | CH(CH₃)₂ | 1 | CF₃ | CH₃ | 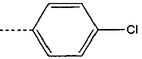 | 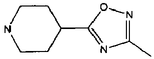 | 508.96 | Yes | 98 |
| **063** | CH(CH₃)₂ | 1 | CF₃ | H | 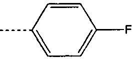 | 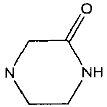 | 411.39 | Yes | 100 |
| **064** | CH(CH₃)₂ | 1 | CF₃ | CH₃ | 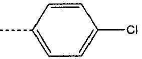 | 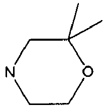 | 456.93 | Yes | 100 |
| **065** | CH(CH₃)₂ | 1 | CF₃ | H | 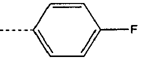 | 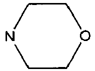 | 398.40 | Yes | 100 |
| **066** | CH(CH₃)₂ | 1 | CF₃ | H | 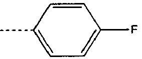 | 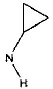 | 368.37 | Yes | 100 |
| **067** | CH(CH₃)₂ | 1 | CF₃ | CH₃ | 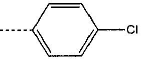 | 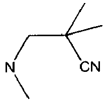 | 453.93 | Yes | 98 |
| **068** | 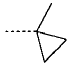 | 1 | CF₃ | CH₃ | 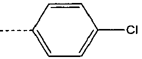 | 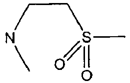 | 490.97 | Yes | 100 |
| **069** | 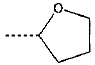 | 1 | CF₃ | H | 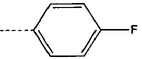 | 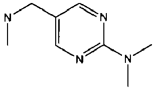 | 505.51 | Yes | 98 |
| **070** | 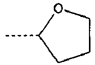 | 1 | CF₃ | H | 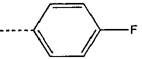 | 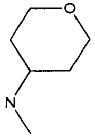 | 454.46 | Yes | 98 |
| **071** | 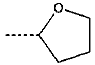 | 1 | CF₃ | H | 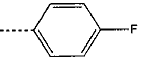 | 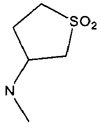 | 488.50 | Yes | 97 |
| **072** | 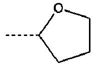 | 1 | CF₃ | H | 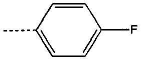 | 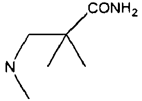 | 469.47 | Yes | 95 |
| **073** | 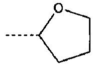 | 1 | CF₃ | H | 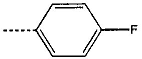 | 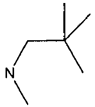 | 440.47 | Yes | 100 |
| **074** | 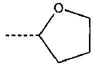 | 1 | CF₃ | H | 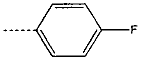 | 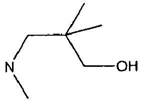 | 456.47 | Yes | 100 |
| **075** | 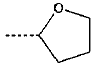 | 1 | CF₃ | H | 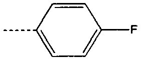 | 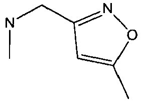 | 465.44 | Yes | 98 |
| **076** | 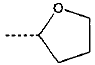 | 1 | CF₃ | H | 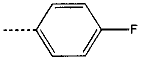 | 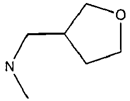 | 454.46 | Yes | 97 |
| **077** | 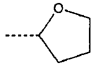 | 1 | CF₃ | H | 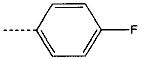 | 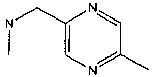 | 476.47 | Yes | 96 |
| **078** | 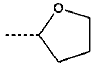 | 1 | CF₃ | H | 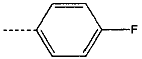 | 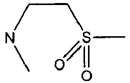 | 476.49 | Yes | 99 |
| **079** | 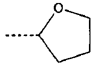 | 1 | CF₃ | H | 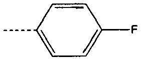 | 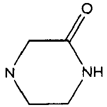 | 439.40 | Yes | 96 |
| **080** | 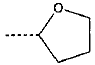 | 1 | CF₃ | H | 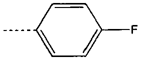 | 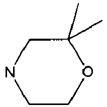 | 454.46 | Yes | 98 |
| **081** | 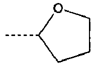 | 1 | CF₃ | H | 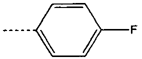 | 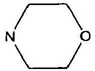 | 426.41 | Yes | 97 |
| **082** | 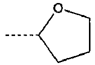 | 1 | CF₃ | H | 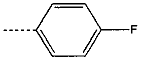 | 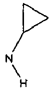 | 396.38 | Yes | 97 |
| **083** | 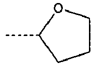 | 1 | CF₃ | H | 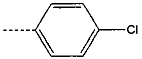 | 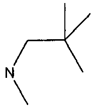 | 456.93 | Yes | 98 |
| **084** | 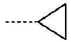 | 1 | CF₃ | CH₃ | 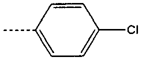 | 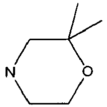 | 454.91 | Yes | 100 |
| **085** | 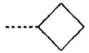 | 1 | CF₃ | CH₃ | 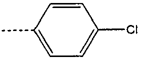 | 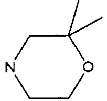 | 468.94 | Yes | 100 |
| **086** | 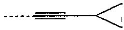 | 1 | CF₃ | CH₃ | 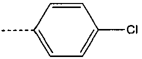 | 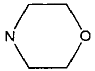 | 450.88 | Yes | 100 |
| **087** | 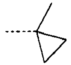 | 1 | CF₃ | CH₃ | 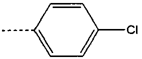 | 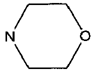 | 440.89 | Yes | 100 |
| **088** | 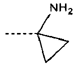 | 1 | CF₃ | H | 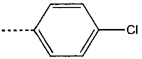 | 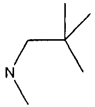 | 441.92 | Yes | 98 |
| **089** | 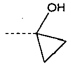 | 1 | CF₃ | CH₃ | 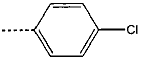 | 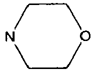 | 442.86 | Yes | 100 |
| **090** | 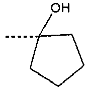 | 1 | CF₃ | H | 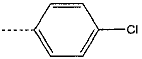 | 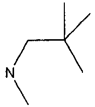 | 470.96 | Yes | 100 |
| **091** | 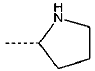 | 1 | CF₃ | H | 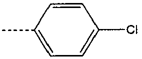 | 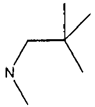 | 455.94 | Yes | 99 |
| **092** | 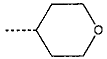 | 1 | CF₃ | H | 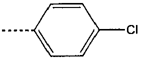 | 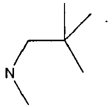 | 470.20 | Yes | 100 |
| **093** | 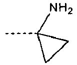 | 1 | CF₃ | CH₃ | 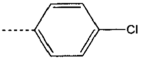 | 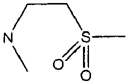 | 491.95 | Yes | 100 |
| **094** | 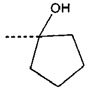 | 1 | CF₃ | CH₃ | 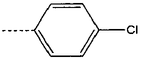 | 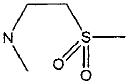 | 520.99 | Yes | 100 |
| **095** | 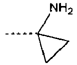 | 1 | CF₃ | CH₃ | 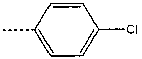 | 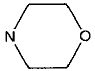 | 441.87 | Yes | 99 |
| **096** | 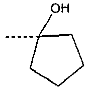 | 1 | CF₃ | CH₃ | 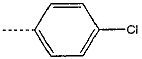 | 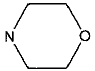 | 470.91 | Yes | 100 |
| **097** | 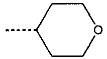 | 1 | CF₃ | CH₃ | 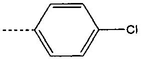 | 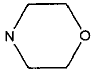 | 470.91 | Yes | 99 |
| **098** | 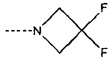 | 2 | CF₃ | CH₃ | 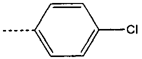 | 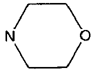 | 491.88 | Yes | 100 |
| **099** | 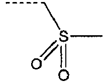 | 1 | CF₃ | CH₃ | 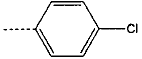 | 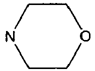 | 478.91 | Yes | 98 |
| **100** | 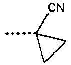 | 1 | CF₃ | H | 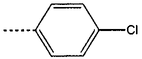 | 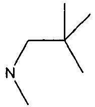 | 451.91 | Yes | 100 |
| **101** | 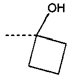 | 1 | CF₃ | H | 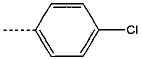 | 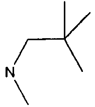 | 456.93 | Yes | 100 |
| **102** | CH₃ | 0 | CF₃ | CH₃ | 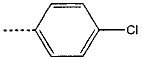 | 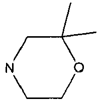 | 414.85 | Yes | 95 |
| **103** | CH₃ | 1 | CF₃ | CH₃ | 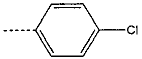 | 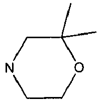 | 428.88 | Yes | 96 |
| **104** | CH(CH₃)₂ | 1 | CF₃ | CH₃ | 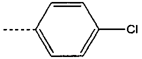 | 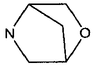 | 440.89 | Yes | 96 |
| **105** | CH(CH₃)₂ | 0 | CF₃ | H | 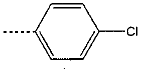 | 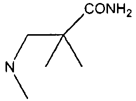 | 457.92 | Yes | 95 |
| **106** | CH(CH₃)₂ | 0 | CF₃ | CH₃ | 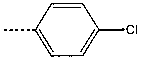 | 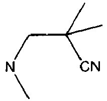 | 439.90 | Yes | 95 |
| **107** | CH(CH₃)₂ | 0 | CF₃ | CH₃ | 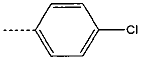 | 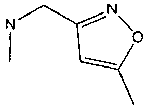 | 453.89 | Yes | 96 |
| **108** | CH(CH₃)₂ | 0 | CF₃ | CH₃ | 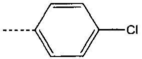 | 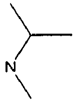 | 400.87 | Yes | 96 |
| **109** | CH(CH₃)₂ | 1 | CF₃ | CH₃ | 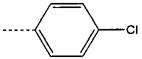 | 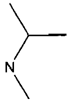 | 414.89 | Yes | 94 |
| **110** | CH(CH₃)₂ | 0 | CF₃ | CH₃ | 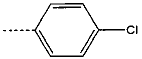 | 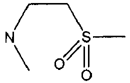 | 464.93 | Yes | 96 |
| **111** | CH(CH₃)₂ | 0 | CF₃ | CH₃ | 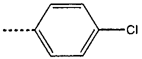 | 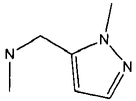 | 452.90 | Yes | 96 |
| **112** | CH(CH₃)₂ | 0 | CF₃ | CH₃ | 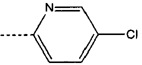 | 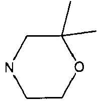 | 443.89 | Yes | 96 |
| **113** | CH(CH₃)₂ | 0 | CF₃ | CH₃ | 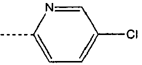 | 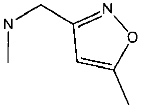 | 454.87 | Yes | 96 |
| **114** | CH(CH₃)₂ | 0 | CF₃ | CH₃ | 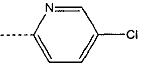 | 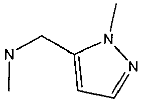 | 453.89 | Yes | 96 |
| **115** | CH(CH₃)₂ | 0 | CF₃ | CH₃ | 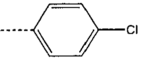 | 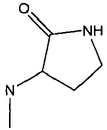 | 441.87 | Yes | 95 |
| **116** | CH(CH₃)₂ | 0 | CF₃ | H | 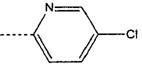 | 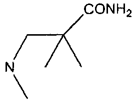 | 458.91 | Yes | 100 |
| **117** | CH(CH₃)₂ | 0 | CF₃ | CH₃ | 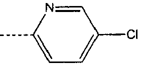 | 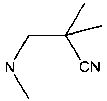 | 440.89 | Yes | 100 |
| **118** | CH(CH₃)₂ | 0 | CF₃ | CH₃ | 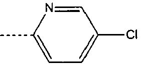 | 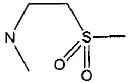 | 465.92 | Yes | 100 |
| **119** | CH(CH₃)₂ | 0 | CF₃ | CH₃ | 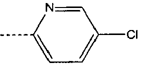 | 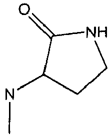 | 442.86 | Yes | 100 |
| **120** | CH(CH₃)₂ | 0 | CF₃ | CH₃ | 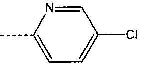 | 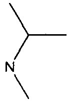 | 401.85 | Yes | 100 |
| **121** | CH(CH₃)₂ | 0 | CF₃ | CH₃ | 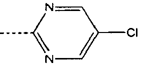 | 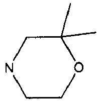 | 444.88 | Yes | 100 |
| **122** | CH(CH₃)₂ | 0 | CF₃ | CH₃ | 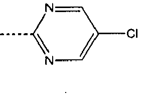 | 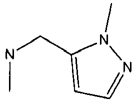 | 454.88 | Yes | 97 |
| **123** | CH(CH₃)₂ | 0 | CF₃ | CH₃ | 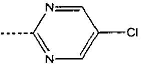 | 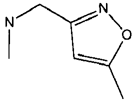 | 455.86 | Yes | 98 |
| **124** | CH(CH₃)₂ | 0 | CF₃ | CH₃ | 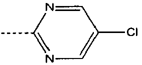 | 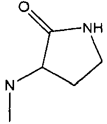 | 443.85 | Yes | 99 |
| **125** | CH(CH₃)₂ | 0 | CF₃ | H | 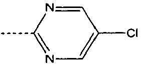 | 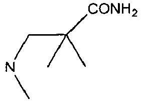 | 459.89 | Yes | 100 |
| **126** | CH(CH₃)₂ | 0 | CF₃ | CH₃ | 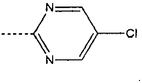 | 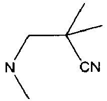 | 441.88 | Yes | 100 |
| **127** | CH(CH₃)₂ | 0 | CF₃ | CH₃ | 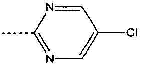 | 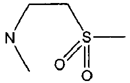 | 466.91 | Yes | 100 |
| **128** | CH(CH₃)₂ | 0 | CF₃ | CH₃ | 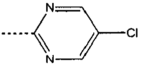 | 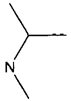 | 402.84 | Yes | 100 |
| **129** | CH(CH₃)₂ | 0 | CF₃ | CH₃ | 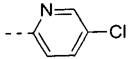 | 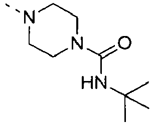 | 513.21 | Yes | >98 |
| **130** | CH(CH₃)₂ | 0 | CF₃ | CH₃ | 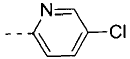 | 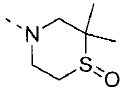 | 475.13 | Yes | >98 |
| **131** | CH(CH₃)₂ | 0 | CF₃ | CH₃ | 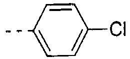 | 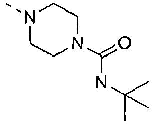 | 512.22 | Yes | >98 |
| **132** | CH(CH₃)₂ | 0 | CF₃ | CH₃ | 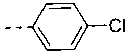 | 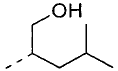 | 458.19 | Yes | 90-95 |
| **133** | CH(CH₃)₂ | 0 | CF₃ | CH₃ | 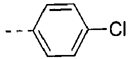 | 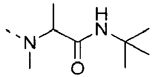 | 485.21 | Yes | >98 |
| **134** | CH(CH₃)₂ | 0 | CF₃ | CH₃ | 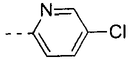 | 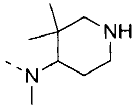 | 470.21 | Yes | >98 |
| **135** | CH(CH₃)₂ | 0 | CF₃ | CH₃ | 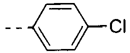 | 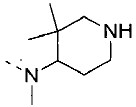 | 469.21 | Yes | ∼90% |

### 2. Assay Descriptions and Biological Data:

### 2.1 Fluorescent assay for CaV2.2 channels using potassium depolarization to induce channel opening

Human CaV2.2 channels were stably expressed in HEK293 cells together with alpha2-delta and beta subunits of voltage gated calcium channels. In addition, an inwardly rectifying potassium channel (Kir2.3) was stably expressed in these cells to augment control of the cell membrane potential by the concentration of extracellular potassium ions. Raise of the extracellular potassium concentration leads to depolarization of the membrane potential and thus regulates the voltage dependent state of CaV2.2 channels. For preparation, cells were seeded in black poly-D-lysine coated 96-well plates (Becton Dickinson, Biocoat 4640) in 100 µL medium [500 mL DMEM/F-12 plus Glutamax (Invitrogen 31331-093) plus 5.5 mL MEM NEAA 100x (Invitrogen 11140-035) plus 50 mL FBS decomplemented (Invitrogen 10270-106) plus 200 µg/mL Geneticin (Invitrogen 10131-027) plus 50 µg/mL Hygromycin B (Invitrogen 10687-010) plus 2 µg/mL Blasticidin (anti-bl5b Invivo-Gen) plus 0.2 µg/mL Puromycin (A 11138-03)] at a cell density of 30.000 cells per well. Plates were incubated at 37°C (5% CO₂) for 20 to 23 h. On the day of experiment medium was discarded and cells were loaded with Fluo 4 by addition of 100 µL of basic assay buffer (10 mM HEPES, 1 mM KCI, 149 mM NaCl, 0.8 mM CaCl₂, 1.7 mM MgCl₂, 10 mM Glucose, 0.1% BSA. pH 7.4) containing 2 µM Fluo 4 (Molecular Probes; F-14201), 0.01% pluronic acid (Molecular Probes; P-6866) and 2.5 mM probenecid (Molecular Probes; P36400). Cells were incubated in the dark at 25°C for 60 min. Then dye containing buffer was discarded and 100 µL basic (1 mM KCl) or alternative (30mM KCI) assay buffer was added. The alternative assay buffer contained altered concentrations of KCI (30 mM) and NaCl (120 mM) and was used in order to promote the inactivated channel state. After that 25 µL of basic or alternative assay buffer with or without test compound were added and cells were incubated again in the dark at 25°C for 15 min. Fluorescence intensity was measured on a FLIPR 3 instrument (Molecular Devices Corp., Sunnyvale, CA) with excitation at 480 nm and emission at 535 nm. After continuously reading fluorescence for 30 sec, 50 µL of basic assay buffer containing 210 mM KCl (NaCl omitted) were added for depolarization. Peak fluorescent signal intensity was determined and the amplitude of the peak signal, normalized to base line, was used to measure channel inhibition by test compounds.

The following table summarizes the inhibitory activity of exemplified compounds according to the present invention.

| **Example No.** | **Activity Category** | **Example No.** | **Activity Category** | **Example No.** | **Activity Category** | **Example No.** | **Activity Category** |
|---|---|---|---|---|---|---|---|
| **001** | B | **036** | B | **069** | B | **104** | A |
| **002** | B | **037** | C | **070** | B | **105** | B |
| **003** | C | **038** | C | **071** | C | **106** | B |
| **004** | B | **039** | B | **073** | B | **107** | A |
| **005** | C | **040** | B | **074** | B | **108** | A |
| **006** | B | **041** | B | **075** | B | **109** | B |
| **007** | C | **042** | B | **076** | B | **110** | B |
| **008** | B | **043** | B | **077** | B | **111** | B |
| **009** | B | **044** | B | **078** | C | **112** | C |
| **010** | B | **045** | B | **079** | C | **113** | B |
| **011** | B | **046** | A | **080** | B | **114** | C |
| **012** | B | **047** | A | **081** | C | **115** | B |
| **013** | B | **048** | A | **082** | C | **116** | C |
| **014** | B | **049** | A | **083** | B | **117** | B |
| **015** | B | **050** | B | **084** | A | **118** | C |
| **016** | A | **051** | A | **085** | B | **119** | C |
| **017** | B | **052** | B | **086** | A | **120** | B |
| **018** | B | **053** | C | **087** | A | **121** | C |
| **019** | C | **054** | B | **088** | B | **122** | C |
| **020** | A | **055** | A | **089** | A | **123** | B |
| **021** | A | **056** | B | **090** | B | **126** | C |
| **022** | A | **057** | A | **091** | A | **127** | D |
| **023** | B | **058** | A | **093** | B | **128** | C |
| **024** | A | **059** | A | **094** | A | **129** | B |
| **025** | B | **060** | A | **095** | B | **130** | C |
| **026** | C | **061** | A | **096** | B | **131** | B |
| **027** | B | **062** | B | **097** | A | **132** | C |
| **028** | B | **063** | C | **098** | A | **133** | C |
| **029** | B | **064** | B | **099** | C | **134** | C |
| **032** | A | **065** | B | **100** | B | **135** | C |
| **033** | B | **066** | B | **101** | B | | |
| **034** | B | **067** | C | **102** | B | | |
| **035** | C | **068** | A | **103** | B | | |

| | | | | | | | |
|---|---|---|---|---|---|---|---|
| *** %-Inhib (CaV2.2) @3µM @30mM KCI:** "A": %-Inhibition > 95 %; "B": %-Inhibition > 75 % up to ≤ 95 %; "C": %-Inhibition > 40 % up to ≤ 75 %, "D": %-Inhibition > 30 % up to ≤ 40 %. | | | | | | | |

### 2.2 Electrophysiological assessment of calcium channel activity

Patch-clamp recordings were performed using HEK293 cells stably expressing human Cav2.2. Cells were plated in T150 flasks and grown a humidified incubator at 37°C and under 5% CO₂ to approximately 50-60% confluency. Cells were maintained at 30°C for 48 hrs prior to recording. On the day of the experiment, cells were harvested with TrypLE cell detachment solution (Invitrogen) diluted to 25% with phosphate buffered saline and maintained in 50% cell culture media, 50% NaCl based external saline (in mM, 140 NaCl, 4 KCI, 1 MgCl₂, 2 CaCl₂, 5 Glucose, 10 HEPES, pH 7.4) up to several hours prior to experiment.

Currents were recorded at room temperature (21-23°C) using the Patchliner planar array technology (Nanion). Patchliner is a multi-well whole-cell automated patch clamp device that operates asynchronously with fully integrated fluidics. Capacitance and series resistance compensation was automated and no correction for liquid junction potential was employed. Leak was subtracted on-line. Whole-cell patch-clamp recordings were obtained using extracellular saline consisting of (mM): 145 TEA-Cl, 10 BaCl₂, 10 HEPES, 10 Glucose. The pH was adjusted to 7.35 with NaOH and the osmolarity was adjusted to 310 mOsm with sucrose. Intracellular solution consisted of (mM): 50 CsCl, 60 CsF, 10 NaCl, 20 EGTA, 5 BAPTA, 10 HEPES. Prior to an experiment, 5 mM MgATP and 0.3 NaGTP were added, the pH was adjusted to 7.2 with CsOH and the osmolarity was adjusted to 290 mOsm with sucrose.

A voltage pulse protocol was utilised to assess compound inhibition. Cells were held at a holding potential of -60 mV and channels were activated using a 10 ms test pulse to +30 mV activated every 10 seconds (0.1 Hz). Increasing concentrations of compound were applied to individual cells with 5 minutes at each test concentration. Compounds were prepared in DMSO as 10 mM stock solutions and subsequent 1:3 serial dilutions performed. Final dilution of 1:1000 in external solution resulted in a final DMSO concentration of 0.1%. For each cell, current responses were normalised to dimethyl sulfoxide vehicle control to generate concentration-response curves. When multiple doses were achieved per cell, IC50 values were calculated from the fits of the Hill equation to the data. The form of the Hill equation used was: Relative current = (100/(1+(IC50/conc)^Slope)). A selection of the foregoing exemplified compounds was tested under these conditions: Several compounds are potent inhibitors (IC50 < 5 µM) or even very potent inhibitors (IC50 < 2 µM).

## Claims

1. A compound of general formula (I), 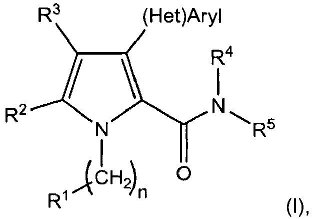 wherein
n represents 0, 1 or 2;
R¹ represents
C₁₋₆-alkyl, C₂₋₆-alkenyl or C₂₋₆-alkynyl;
or
C₃₋₆-cycloalkyl or 3 to 7 membered heterocyclyl;
R² represents CH₂F; CHF₂ or CF₃;
R³ represents H, C₁₋₆-alkyl, C₃₋₆-cycloalkyl, 3 to 7 membered heterocyclyl, OH; O-C₁₋₆-alkyl; NH₂; N(H)(C₁₋₆-alkyl); N(C₁₋₆-alkyl)₂ or SO₂(C₁₋₆-alkyl);
(Het)Aryl represents aryl or heteroaryl, each substituted by zero or one or two or three substituents of the group consisting of R⁶, R⁷ and R⁸,
wherein R⁶, R⁷ and R⁸, are each independently of one another selected from the group consisting of F; Cl; Br; I; NO₂; CN; C₁₋₆-alkyl; CF₃; CF₂H; CFH₂; CF₂Cl; CFCl₂; C(=O)-H; C(=O)-C₁₋₆-alkyl; C(=O)-OH; C(=O)-O-C₁₋₆-alkyl; C(=O)-N(H)(OH); C(=O)-NH₂; C(=O)-N(H)(C₁₋₆-alkyl); C(=O)-N(C₁₋₆-alkyl)₂; C(=N-OH)-H; C(=N-OH)-C₁₋₆-alkyl; C(=N-O-C₁₋₆-alkyl)-H; C(=N-O-C₁₋₆-alkyl)-C₁₋₆-alkyl; OH; OCF₃; OCF₂H; OCFH₂; OCF₂Cl; OCFCl₂; O-C₁₋₆-alkyl; O-C(=O)-C₁₋₆-alkyl; O-C(=O)-O-C₁₋₆-alkyl; O-(C=O)-N(H)(C₁₋₆-alkyl); O-C(=O)-N(C₁₋₆-alkyl)₂; O-S(=O)₂-C₁₋₆-alkyl; O-S(=O)₂-OH; O-S(=O)₂-O-C₁₋₆-alkyl; O-S(=O)₂-NH₂; O-S(=O)₂-N(H)(C₁₋₆-alkyl); O-S(=O)₂-N(C₁₋₆-alkyl)₂; NH₂; N(H)(C₁₋₆-alkyl); N(C₁₋₆-alkyl)₂; N(H)-C(=O)-C₁₋₆-alkyl; N(H)-C(=O)-O-C₁₋₆-alkyl; N(H)-C(=O)-NH₂; N(H)-C(=O)-N(H)(C₁₋₆-alkyl); N(H)-C(=O)-N(C₁₋₆-alkyl)₂; N(C₁₋₆-alkyl)-C(=O)-C₁₋₆-alkyl; N(C₁₋₆-alkyl)-C(=O)-O-C₁₋₆-alkyl; N(C₁₋₆-alkyl)-C(=O)-NH₂; N(C₁₋₆-alkyl)-C(=O)-N(H)(C₁₋₆-alkyl); N(C₁₋₆-alkyl)-C(=O)-N(C₁₋₆-alkyl)₂; N(H)-S(=O)₂OH; N(H)-S(=O)₂-C₁₋₆-alkyl; N(H)-S(=O)₂-O-C₁₋₆-alkyl; N(H)-S(=O)₂-NH₂; N(H)-S(=O)₂-N(H)(C₁₋₆-alkyl); N(H)-S(=O)₂N(C₁₋₆-alkyl)₂; N(C₁₋₆-alkyl)-S(=O)₂-OH; N(C₁₋₆-alkyl)-S(=O)₂-C₁₋₆-alkyl; N(C₁₋₆-alkyl)-S(=O)₂-O-C₁₋₆-alkyl; N(C₁₋₆-alkyl)-S(=O)₂-NH₂; N(C₁₋₆-alkyl)-S(=O)₂-N(H)(C₁₋₆-alkyl); N(C₁₋₆-alkyl)-S(=O)₂-N(C₁₋₆-alkyl)₂; SH; SCF₃; SCF₂H; SCFH₂; SCF₂Cl; SCFCl₂; S-C₁₋₆-alkyl; S(=O)-C₁₋₆-alkyl; S(=O)₂-C₁₋₆-alkyl; S(=O)₂-OH; S(=O)₂-O-C₁₋₆-alkyl; S(=O)₂-NH₂; S(=O)₂-N(H)(C₁₋₆-alkyl); S(=O)₂-N(C₁₋₆-alkyl)₂, C₃₋₆-cycloalkyl or 3 to 7 membered heterocyclyl;
wherein said C₁₋₆-alkyl in each case may be branched or unbranched and unsubstituted or mono- or poly-substituted; and wherein said C₃₋₆-cycloalkyl or 3 to 7 membered heterocyclyl in each case unsubstituted or mono- or polysubstituted;
R⁴ represents H, C₁₋₁₀-alkyl, aryl, or aryl connected via a C₁₋₈-alkylene group;
R⁵ represents H; C₁₋₁₀alkyl, C₃₋₁₀-cycloalkyl, 3 to 10 membered heterocyclyl, aryl or heteroaryl; or C₃₋₁₀-cycloalkyl, 3 to 10 membered heterocyclyl, aryl or heteroaryl, each connected via a C₁₋₈-alkylene group; or
R⁴ and R⁵ together with the nitrogen atom connecting them form a 3 to 10 membered heterocyclyl;
wherein said C₁₋₆-alkyl, said C₁₋₁₀-alkyl, said C₂₋₆-alkenyl, said C₂₋₆-alkynyl and said C₁₋₈-alkylene in each case may be branched or unbranched and unsubstituted or mono- or poly-substituted;
and wherein said C₃₋₆-cycloalkyl, said C₃₋₁₀-cycloalkyl, said 3 to 7 membered heterocyclyl, said 3 to 10 membered heterocyclyl, said aryl and said heteroaryl in each case may be unsubstituted or mono- or polysubstituted;
optionally in the form of an individual stereoisomer or a mixture of stereoisomers,
in the form of the free compound and/or a physiologically acceptable salt and/or a physiologically acceptable solvate thereof.

2. A compound according to claim 1, **characterized in that**
R¹ represents
C₁₋₆-alkyl, C₂₋₆-alkenyl or C₂₋₆-alkynyl,
in each case branched or unbranched, and in each case unsubstituted or mono- or polysubstituted by one or more substituents selected from
F; Cl; CN; CF₃; CF₂H; CFH₂; C(=O)-OH; C(=O)-O-C₁₋₆-alkyl; C(=O)-NH₂; C(=O)-N(H)(C₁₋₆-alkyl); C(=O)-N(C₁₋₆-alkyl)₂; OH; OCF₃; OCF₂H; OCFH₂; O-C₁₋₆-alkyl; O-C(=O)-C₁₋₆-alkyl; O-S(=O)₂-C₁₋₆-alkyl; NH₂; N(H)(C₁₋₆-alkyl); N(C₁₋₆-alkyl)₂; N(H)-C(=O)-C₁₋₆-alkyl; N(H)-C(=O)-N(H)(C₁₋₆-alkyl); N(H)-C(=O)-N(C₁₋₆-alkyl)₂; N(C₁₋₆-alkyl)-C(=O)-C₁₋₆-alkyl; N(H)-S(=O)₂-C₁₋₆-alkyl; N(C₁₋₆-alkyl)-S(=O)₂-C₁₋₆-alkyl; S(=O)-C₁₋₆-alkyl, S(=O)₂-C₁₋₆-alkyl, C₃₋₆-cycloalkyl or 3 to 7 membered heterocyclyl,
or R¹ represents
C₃₋₆-cycloalkyl or 3 to 7 membered heterocyclyl, in each case unsubstituted or mono- or polysubstituted by one or more substituents selected from
F; Cl; CN; C₁₋₆-alkyl; CF₃; CF₂H; CFH₂; C(=O)-OH; C(=O)-O-C₁₋₆-alkyl; C(=O)-NH₂; C(=O)-N(H)(C₁₋₆-alkyl); C(=O)-N(C₁₋₆-alkyl)₂; OH; =O; OCF₃; OCF₂H; OCFH₂; O-C₁₋₆-alkyl; O-C(=O)-C₁₋₆-alkyl; O-S(=O)₂-C₁₋₆-alkyl; NH₂; N(H)(C₁₋₆-alkyl); N(C₁₋₆-alkyl)₂; N(H)-C(=O)-C₁₋₆-alkyl; N(H)-C(=O)-N(H)(C₁₋₆-alkyl); N(H)-C(=O)-N(C₁₋₆-alkyl)₂; N(C₁₋₆-alkyl)-C(=O)-C₁₋₆-alkyl; N(H)-S(=O)₂-C₁₋₆-alkyl; N(C₁₋₆-alkyl)-S(=O)₂-C₁₋₆-alkyl; S(=O)-C₁₋₆-alkyl or S(=O)₂-C₁₋₆-alkyl.

3. A compound according to one or more of the preceding claims, **characterized in that** R² represents CHF₂ or CF₃.

4. A compound according to one or more of the preceding claims, **characterized in that** R³ is selected from the group consisting of H, methyl, ethyl, n-propyl, iso-propyl, n-butyl, sec-butyl, iso-butyl, tert-butyl, cyclopropyl, methoxy, ethoxy, methylsulfonyl, 2-oxetyl, 3-oxetyl, 2-tetrahydrofuranyl and 3-tetrahydrofuranyl.

5. A compound according to one or more of the preceding claims, **characterized in that** Het(aryl) is selected
from phenyl, pyrrol, pyrazolyl, imidazolyl, isoxazolyl, oxazolyl, thiazolyl, pyridinyl, pyrazinyl, pyrimidinyl and pyridazinyl,
preferably Het(aryl) represents phenyl, pyridinyl, pyrazinyl or pyrimidinyl.

6. A compound according to one or more of the preceding claims, **characterized in that**
R⁶, R⁷ and R⁸ are each independently of one another selected from the group consisting of F; Cl; CN; C₁₋₆-alkyl; CF₃; CF₂H; CFH₂; OH; OCF₃; OCF₂H; OCFH₂; O-C₁₋₆-alkyl; O-C(=O)-C₁₋₆-alkyl; NH₂; N(H)(C₁₋₆-alkyl); N(C₁₋₆-alkyl)₂; SCF₃; S(=O)-C₁₋₆-alkyl; S(=O)₂-C₁₋₆-alkyl; S(=O)₂-OH; S(=O)₂-O-C₁₋₆-alkyl; S(=O)₂-NH₂; S(=O)₂-N(H)(C₁₋₆-alkyl); or S(=O)₂-N(C₁₋₆ alkyl)₂; wherein in each case C₁₋₆-alkyl may be branched or unbranched; or a C₃₋₆ cycloaliphatic residue, unsubstituted or mono- or polysubstituted,
preferably
R⁶, R⁷ and R⁸ are each independently of one another selected from the group consisting of F; Cl; CN; C₁₋₆-alkyl; CF₃; CF₂H; CFH₂; OH; OCF₃; OCF₂H; OCFH₂; O-C₁₋₆-alkyl; S(=O)-C₁₋₆-alkyl; S(=O)₂-C₁₋₆-alkyl; cyclopropyl and O-cyclopropyl.

7. A compound according to one or more of the preceding claims, **characterized in that** the compound of general formula (I) is a compound according to general formula (Ia), 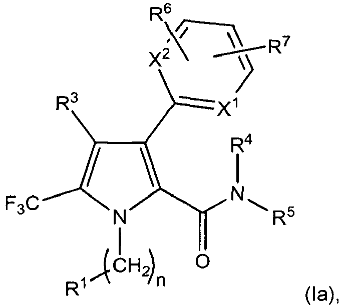 wherein
R³ represents H or CH₃ or cyclopropyl;
X¹ is N or CH; X² is N or CH;
R⁶ and R⁷ are independently absent or are each independently of one another selected from the group consisting of F; Cl; CN; C₁₋₆-alkyl; CF₃; CF₂H; CFH₂; OH; OCF₃; OCF₂H; OCFH₂ or O-C₁₋₆-alkyl;
and n, R¹, R⁴ and R⁵ are defined according to claim 1.

8. A compound according to one or more of the preceding claims, **characterized in that**
R⁴ represents
H or
a C₁₋₆ aliphatic residue, branched or unbranched, unsubstituted or substituted with 1, 2, 3, 4 or 5 substituents independently from one another selected from the group consisting of OH, =O, O-C₁₋₆-alkyl, S(=O)-C₁₋₆-alkyl, S(=O)₂-C₁₋₆-alkyl, N(H)-S(=O)-C₁₋₆-alkyl, N(C₁₋₆-alkyl)-S(=O)-C₁₋₆-alkyl, N(H)-S(=O)₂-C₁₋₆-alkyl, N(C₁₋₆-alkyl)-S(=O)₂-C₁₋₆-alkyl, C(=O)-NH₂, C(=O)-N(H)(C₁₋₆-alkyl), C(=O)-N(C₁₋₆-alkyl)₂, C(=O)-O-C₁₋₆-alkyl; N(H)-C(=O)-C₁₋₆-alkyl, and N(C₁₋₆-alkyl)-C(=O)-C₁₋₆-alkyl
or phenyl, unsubstituted or mono- or polysubstituted with with 1, 2 or 3 substituents independently from one another selected from the group consisting of F; Cl; CN; C₁₋₆-alkyl; CF₃; CF₂H; CFH₂; OH; OCF₃; OCF₂H; OCFH₂ or O-C₁₋₆-alkyl, and optionally connected via a C₁₋₃-alkylene group, branched or unbranched;
and
R⁵ represents
H; or
C₁₋₆-alkyl, branched or unbranched, unsubstituted or substituted with 1, 2, 3, 4 or 5 substituents independently from one another selected from the group consisting of F, Cl, CF₃, CN, OH, =O, OCF₃, O-C₁₋₆-alkyl, O-(C=O)C₁₋₆-alkyl, S(=O)-C₁₋₆-alkyl, S(=O)₂-C₁₋₆-alkyl, S(=O)₂-NH₂, S(=O)₂-N(H)C₁₋₆-alkyl, S(=O)₂-N(C₁₋₆-alkyl)₂, NH₂, NH(C₁₋₆-alkyl), N(C₁₋₆-alkyl)₂, N(H)-S(=O)-C₁₋₆-alkyl, N(C₁₋₆-alkyl)-S(=O)-C₁₋₆-alkyl, N(H)-S(=O)₂-C₁₋₆-alkyl, N(C₁₋₆-alkyl)-S(=O)₂-C₁₋₆-alkyl, N(H)-C(=O)-NH₂, N(H)-C(=O)-N(H)(C₁₋₆-alkyl), N(H)-C(=O)-N(C₁₋₆-alkyl)₂, N(H)-C(=O)-O-C₁₋₆-alkyl; O-C(=O)-NH₂, O-C(=O)-N(H)(C₁₋₆-alkyl), O-C(=O)-N(C₁₋₆-alkyl)₂, C(=O)-NH₂, C(=O)-N(H)(C₁₋₆-alkyl), C(=O)-N(C₁₋₆-alkyl)₂, C(=O)-O-C₁₋₆-alkyl; N(H)-C(=O)-C₁₋₆-alkyl, and N(C₁₋₆-alkyl)-C(=O)-C₁₋₆-alkyl; or
C₃₋₆-cycloalkyl, unsubstituted or substituted with 1, 2, 3, 4 or 5 substituents independently from one another selected from the group consisting of F, Cl, CN, CF₃, =O, OCF₃, C₁₋₆-alkylen-OH, C₁₋₆ alkyl, OH, O-C₁₋₆-alkyl, O-(C=O)C₁₋₆-alkyl, S(=O)-C₁₋₆-alkyl, S(=O)₂-C₁₋₆-alkyl, S(=O)₂-NH₂, S(=O)₂-N(H)C₁₋₆-alkyl, S(=O)₂-N(C₁₋₆-alkyl)₂, NH₂, NH(C₁₋₆-alkyl), N(C₁₋₆-alkyl)₂, N(H)-S(=O)-C₁₋₆-alkyl, N(C₁₋₆-alkyl)-S(=O)-C₁₋₆-alkyl, N(H)-S(=O)₂-C₁₋₆-alkyl, N(C₁₋₆-alkyl)-S(=O)₂-C₁₋₆-alkyl, N(H)-C(=O)-O-C₁₋₆-alkyl; O-C(=O)-NH₂, O-C(=O)-N(H)(C₁₋₆-alkyl), O-C(=O)-N(C₁₋₆-alkyl)₂, N(H)-C(=O)-NH₂, N(H)-C(=O)-N(H)(C₁₋₆-alkyl), N(H)-C(=O)-N(C₁₋₆-alkyl)₂, C(=O)-NH₂, C(=O)-N(H)(C₁₋₆-alkyl), C(=O)-N(C₁₋₆-alkyl)₂, C(=O)-O-C₁₋₆-alkyl; N(H)-C(=O)-C₁₋₆-alkyl, and N(C₁₋₆-alkyl)-C(=O)-C₁₋₆-alkyl; wherein said C₃₋₆-cycloalkyl is optionally connected via C₁₋₆-alkylene, branched or unbranched, which in turn may be unsubstituted or substituted with 1, 2, 3, 4 or 5 substituents independently from one another selected from the group consisting of F, Cl, CF₃, =O, OCF₃, OH, O-C₁₋₆-alkyl and C₁₋₆-alkylen-OH; or
3-7-membered heterocyclyl, which is unsubstituted or substituted with 1, 2, 3, 4 or 5 substituents independently from one another selected from the group consisting of F, Cl, CN, CF₃, =O, OCF₃, C₁₋₆-alkylen-OH, C₁₋₆ alkyl, OH, O-C₁₋₆-alkyl, O-(C=O)C₁₋₆-alkyl, S(=O)-C₁₋₆-alkyl, S(=O)₂-C₁₋₆-alkyl, S(=O)₂-NH₂, S(=O)₂-N(H)C₁₋₆-alkyl, S(=O)₂-N(C₁₋₆-alkyl)₂, NH₂, NH(C₁₋₆-alkyl), N(C₁₋₆-alkyl)₂, N(H)-S(=O)-C₁₋₆-alkyl, N(C₁₋₆-alkyl)-S(=O)-C₁₋₆-alkyl, N(H)-S(=O)₂-C₁₋₆-alkyl, N(C₁₋₆-alkyl)-S(=O)₂-C₁₋₆-alkyl, N(H)-C(=O)-O-C₁₋₆-alkyl; O-C(=O)-NH₂, O-C(=O)-N(H)(C₁₋₆-alkyl), O-C(=O)-N(C₁₋₆-alkyl)₂, N(H)-C(=O)-NH₂, N(H)-C(=O)-N(H)(C₁₋₆-alkyl), N(H)-C(=O)-N(C₁₋₆-alkyl)₂, (C=O)C₁₋₆-alkyl, C(=O)-NH₂, C(=O)-N(H)(C₁₋₆-alkyl), C(=O)-N(C₁₋₆-alkyl)₂, C(=O)-O-C₁₋₆-alkyl; N(H)-C(=O)-C₁₋₆-alkyl, and N(C₁₋₆-alkyl)-C(=O)-C₁₋₆-alkyl, wherein said 3-7-membered heterocyclyl is optionally connected via C₁₋₆-alkylene, branched or unbranched, which in turn may be unsubstituted or substituted with 1, 2, 3, 4 or 5 substituents independently from one another selected from the group consisting of F, Cl, CF₃, =O, OCF₃, OH, O-C₁₋₆-alkyl and C₁₋₆-alkylen-OH;
or aryl or heteroaryl, which in each case is unsubstituted or substituted with 1, 2, 3, 4 or 5 substituents independently from one another selected from the group consisting of F, Cl, CN, CF₃, OCF₃, C₁₋₆-alkylen-OH, C₁₋₆-alkyl, OH, O-C₁₋₆-alkyl, O-(C=O)C₁₋₆-alkyl, S(=O)-C₁₋₆-alkyl, S(=O)₂-C₁₋₆-alkyl, S(=O)₂-NH₂, S(=O)₂-N(H)C₁₋₆-alkyl, S(=O)₂-N(C₁₋₆-alkyl)₂, NH₂, NH(C₁₋₆-alkyl), N(C₁₋₆-alkyl)₂, N(H)-S(=O)-C₁₋₆-alkyl, N(C₁₋₆-alkyl)-S(=O)-C₁₋₆-alkyl, N(H)-S(=O)₂-C₁₋₆-alkyl, N(C₁₋₆-alkyl)-S(=O)₂-C₁₋₆-alkyl, N(H)-C(=O)-O-C₁₋₆-alkyl; O-C(=O)-NH₂, O-C(=O)-N(H)(C₁₋₆-alkyl), O-C(=O)-N(C₁₋₆-alkyl)₂, N(H)-C(=O)-NH₂, N(H)-C(=O)-N(H)(C₁₋₆-alkyl), N(H)-C(=O)-N(C₁₋₆-alkyl)₂, C(=O)-NH₂, C(=O)-N(H)(C₁₋₆-alkyl), C(=O)-N(C₁₋₆-alkyl)₂, C(=O)-O-C₁₋₆-alkyl; N(H)-C(=O)-C₁₋₆-alkyl, and N(C₁₋₆-alkyl)-C(=O)-C₁₋₆-alkyl, wherein said aryl or heteroaryl is optionally connected via C₁₋₆-alkylene, branched or unbranched, which in turn may be unsubstituted or substituted with 1, 2, 3, 4 or 5 substituents independently from one another selected from the group consisting of F, Cl, CF₃, =O, OCF₃, OH, O-C₁₋₆-alkyl and C₁₋₆-alkylen-OH.

9. A compound according to one or more of the preceding claims, **characterized in that** R⁴ and R⁵ together with the nitrogen atom connecting them form a 3-7-membered heterocyclyl, unsubstituted or substituted with 1, 2, 3, 4 or 5 substituents selected from the group consisting of F, Cl, CN, CF₃, =O, OH, C₁₋₆-alkyl, O-C₁₋₆-alkyl, C₁₋₆-alkylen-OH, OCF₃, SO₂(C₁₋₆-alkyl), SO₂NH₂, SO₂N(H)C₁₋₆-alkyl, SO₂N(C₁₋₆-alkyl)₂, C₁₋₆-alkylen-SO₂(C₁₋₆-alkyl), NH₂, NH(C₁₋₆-alkyl), N(C₁₋₆-alkyl)₂, (C=O)C₁₋₆-alkyl, C₃₋₆-cycloalkyl, 3 to 7 membered heterocyclyl, aryl, heteroaryl, O-aryl and O-heteroaryl, in each case unsubstituted or mono- or polysubstituted.

10. A compound according to one or more of the preceding claims, **characterized in that**
R⁴ represents H or C₁₋₆-alkyl or benzyl; and
R⁵ represents
C₃₋₆-cycloalkyl, which is unsubstituted or substituted with 1, 2, 3, 4 or 5 substituents independently from one another selected from the group consisting of F, Cl, CF₃, =O, OCF₃, OH, O-C₁₋₆-alkyl, C₁₋₆-alkylen-OH C₁₋₆-alkyl, C(=O)-NH₂, C(=O)-N(H)(C₁₋₆-alkyl) and C(=O)-N(C₁₋₆-alkyl)₂; or
3-7-membered heterocyclyl, which contains 1 or 2 heteroatoms or heteroatom groups independently from one another selected from the group consisting of O, S, S(=O), S(=O)₂, NH and N-C₁₋₆-alkyl, and which is unsubstituted or substituted with 1, 2, 3, 4 or 5 substituents independently from one another selected from the group consisting of F, Cl, CF₃, OCF₃, CN, =O, C₁₋₆-alkyl, C₁₋₆-alkylen-OH and O-C₁₋₆-alkyl; or
phenyl or heteroaryl, which contains at least one nitrogen atom, in each case unsubstituted or substituted with 1, 2 or 3 substituents independently from one another selected from the group consisting of F, Cl, CN, CF₃, OCF₃, C₁₋₆-alkylen-OH, C₁₋₆₋alkyl, OH, O-C₁₋₆-alkyl, S(=O)₂-C₁₋₆-alkyl, S(=O)₂-NH₂, NH₂, NH(C₁₋₆-alkyl), N(C₁₋₆-alkyl)₂, O-C(=O)-NH₂, C(=O)-NH₂, C(=O)-N(H)(C₁₋₆-alkyl), C(=O)-N(C₁₋₆-alkyl)₂, C(=O)-O-C₁₋₆-alkyl;
or a part structure of general formula SF-III 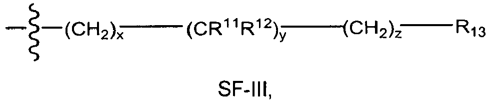 wherein
x represents 0, 1 or 2;
y represents 0, 1 or 2;
z represents 0, 1 or 2;
on the condition that the sum of x, y and z is 1, 2, 3, 4, 5 or 6;
R¹¹ and R¹² are independently from one another selected from H or C₁₋₆-alkyl; or
R¹¹ and R¹² together with the carbon atom connecting them form a C₃₋₆-cycloalkyl or a 3-7-membered heterocyclyl, which contains 1 or 2 heteroatoms or heteroatom groups independently from one another selected from the group consisting of O, S, S(=O), S(=O)₂, NH and N-C₁₋₆-alkyl, wherein said C₃₋₆-cycloalkyl or 3-7-membered heterocyclyl may be unsubstituted or substituted with 1, 2, 3, 4 or 5 substituents independently from one another selected from the group consisting of F, Cl, CF₃, OCF₃, CN, C₁₋₆-alkyl and O-C₁₋₆-alkyl;
R¹³ is selected from the group consisting of
H, F, Cl, CN, OH, O-C₁₋₆-alkyl, O-(C=O)C₁₋₆-alkyl, S(=O)-C₁₋₆-alkyl, S(=O)₂-C₁₋₆-alkyl, S(=O)₂-NH₂, S(=O)₂-N(H)C₁₋₆-alkyl, S(=O)₂-N(C₁₋₆-alkyl)₂, NH₂, NH(C₁₋₆-alkyl), N(C₁₋₆-alkyl)₂, N(H)-C(=O)-C₁₋₆-alkyl, N(C₁₋₆-alkyl)-C(=O)-C₁₋₆-alkyl, N(H)-S(=O)-C₁₋₆-alkyl, N(C₁₋₆-alkyl)-S(=O)-C₁₋₆-alkyl, N(H)-S(=O)₂-C₁₋₆-alkyl, N(C₁₋₆-alkyl)-S(=O)₂-C₁₋₆-alkyl, C(=O)-NH₂, C(=O)-N(H)(C₁₋₆-alkyl), C(=O)-N(C₁₋₆-alkyl)₂, C(=O)-O-C₁₋₆-alkyl, N(H)-C(=O)-NH₂, N(H)-C(=O)-N(H)(C₁₋₆-alkyl), N(H)-C(=O)-N(C₁₋₆-alkyl)₂, N(H)-C(=O)-O-C₁₋₆-alkyl; O-C(=O)-NH₂, O-C(=O)-N(H)(C₁₋₆-alkyl), O-C(=O)-N(C₁₋₆-alkyl)₂; or represents
C₃₋₆-cycloalkyl, which is unsubstituted or substituted with 1, 2, 3, 4, or 5 substituents independently from one another selected from the group consisting of F, Cl, CF₃, =O, OCF₃, OH, O-C₁₋₆-alkyl, C₁₋₆-alkylen-OH and C₁₋₆-alkyl; or
3-7-membered heterocyclyl, which contains 1 or 2 heteroatoms or heteroatom groups independently from one another selected from the group consisting of O, S, S(=O), S(=O)₂, NH and N-C₁₋₆-alkyl, and which is unsubstituted or substituted with 1, 2, 3, 4 or 5 substituents independently from one another selected from the group consisting of F, Cl, CF₃, OCF₃, CN, C₁₋₆-alkyl and O-C₁₋₆-alkyl; or
phenyl or heteroaryl, which contains at least one nitrogen atom, in each unsubstituted or substituted with 1, 2 or 3 substituents independently from one another selected from the group consisting of F, Cl, CN, CF₃, OCF₃, C₁₋₆-alkylen-OH, C₁₋₆₋alkyl, OH, O-C₁₋₆-alkyl, S(=O)₂-C₁₋₆-alkyl, S(=O)₂-NH₂, NH₂, NH(C₁₋₆-alkyl), N(C₁₋₆-alkyl)₂, O-C(=O)-NH₂, C(=O)-NH₂, C(=O)-N(H)(C₁₋₆-alkyl), C(=O)-N(C₁₋₆-alkyl)₂, C(=O)-O-C₁₋₆-alkyl;
or
R⁴ and R⁵ together with the nitrogen atom connecting them form a heterocyclyl, selected from the group consisting of 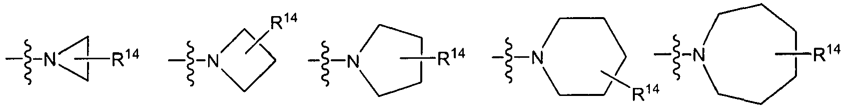 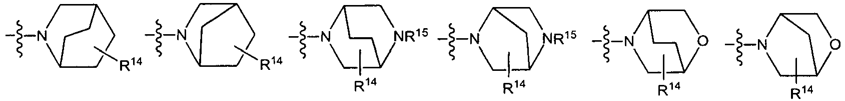 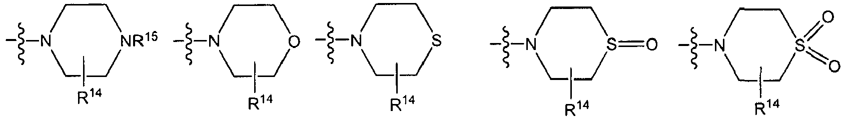 wherein
R¹⁴ denotes 0, 1, 2, 3 or 4 substituents which are in each case independently of each other selected from the group consisting of F, Cl, CF₃, =O, OCF₃, OH, O-C₁₋₆-alkyl, C₁₋₆-alkylen-OH, C₁₋₆-alkylen-SO₂(C₁₋₆-alkyl), SO₂(C₁₋₆-alkyl), C₁₋₆-alkyl, aryl, heteroaryl, O-aryl and O-heteroaryl, wherein said aryl or said heteroaryl is unsubstituted or substituted with 1, 2 or 3 substituents independently from one another selected from the group consisting of F, Cl, CN, CF₃, OCF₃, C₁₋₆-alkylen-OH, C₁₋₆₋alkyl, OH, O-C₁₋₆-alkyl, NH₂, NH(C₁₋₆-alkyl), N(C₁₋₆-alkyl)₂, N(H)-S(=O)₂-C₁₋₆-alkyl, C(=O)-NH₂ or C(=O)-N(H)(C₁₋₆-alkyl), C(=O)-N(C₁₋₆-alkyl)₂;
or
R¹⁴ denotes at least two substituents, wherein two substituents R¹⁴ stand together for a C₁₋₆-alkylen-group, substituted or unsubstituted, wherein optionally one or more C-atoms of the C₁₋₆-alkylen-group is replaced by a heteroatom or heteroatom group, selected of O, N-R¹⁵, S, S(O) and S(O)₂, and wherein these two substituents R¹⁴ are positioned at different carbon atoms of the heterocyclyl, so the C₁₋₆-alkylen-group represents a bridge to form a bicyclic heterocyclyl;
or
R¹⁴ denotes at least two substituents, wherein two substituents R¹⁴ stand together for a C₂₋₆-alkylen-group, substituted or unsubstituted, wherein optionally one or more C-atoms of the C₂₋₆-alkylen-group is replaced by a heteroatom or heteroatom group, selected of O, N-R¹⁵, S, S(O) and S(O)₂, and wherein these two substituents R¹⁴ are positioned at the same carbon atom of the heterocyclyl, so the C₂₋₆-alkylen-group forms a spiro-heterocyclyl; and
R¹⁵ represents H, C₁₋₆-alkyl, (C=O)C₁₋₆-alkyl, (C=O)NH₂, (C=O)NH(C₁₋₆-alkyl) or
(C=O)N(C₁₋₆-alkyl)₂.

11. A compound according to one or more of the preceding claims, **characterized in that** the compound of general formula (I) is a compound according to general formula (Ia), 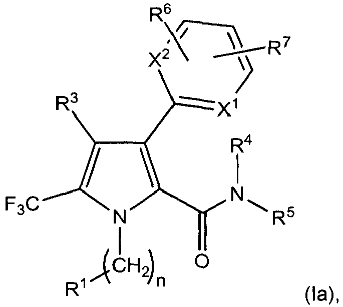 wherein
n is 0 or 1,
R¹ represents C₁₋₆-alkyl, C₂₋₆-alkenyl or C₂₋₆-alkynyl,
in each case branched or unbranched, and in each case unsubstituted or mono- or polysubstituted by one or more substituents selected from F; Cl; CN; CF₃; C(=O)-NH₂; C(=O)-N(H)(C₁₋₆-alkyl); C(=O)-N(C₁-₆-alkyl)₂; OH; O-C₁₋₆-alkyl; NH₂; N(H)(C₁₋₆-alkyl); N(C₁₋₆-alkyl)₂; N(H)-C(=O)-C₁₋₆-alkyl; S(=O)-C₁₋₆-alkyl; S(=O)₂-C₁₋₆-alkyl or cyclopropyl,
or R¹ represents cyclopropyl, cyclobutyl, cyclopentyl, cyclohexyl, oxetanyl, azetidinyl, tetrahydrofuranyl, tetrahydropyranyl, pyrrolidinyl, piperidinyl and piperazinyl,
in each case unsubstituted or mono- or polysubstituted by one or more substituents selected from F; Cl; CN; C₁₋₆-alkyl; CF₃; OH; =O; O-C₁₋₆-alkyl; O-C(=O)-C₁₋₆-alkyl; O-S(=O)₂-C₁₋₆-alkyl; NH₂; N(H)(C₁₋₆-alkyl); N(C₁₋₆-alkyl)₂; N(H)-C(=O)-C₁₋₆-alkyl; N(H)-S(=O)₂-C₁₋₆-alkyl or S(=O)₂-C₁₋₆-alkyl;
R³ represents H or CH₃ or cyclopropyl;
X¹ is N or CH; X² is N or CH;
R⁶ and R⁷ are independently absent or are each independently of one another selected from the group consisting of F; Cl; CN; C₁₋₆-alkyl; CF₃; CF₂H; CFH₂; OH; OCF₃; OCF₂H; OCFH₂ or O-C₁₋₆-alkyl;
R⁴ represents H or C₁₋₆-alkyl or benzyl; and
R⁵ represents
C₃₋₆-cycloalkyl, which is unsubstituted or substituted with 1, 2, 3, 4 or 5 substituents independently from one another selected from the group consisting of F, Cl, CF₃, =O, OCF₃, OH, O-C₁₋₆-alkyl, C₁₋₆-alkylen-OH, C₁₋₆-alkyl, C(=O)-NH₂, C(=O)-N(H)(C₁₋₆-alkyl) and C(=O)-N(C₁₋₆-alkyl)₂; or
5- or 6-membered heterocyclyl, which contains 1 or 2 heteroatoms or heteroatom groups independently from one another selected from the group consisting of O, S, S(=O), S(=O)₂, NH and N-C₁₋₆-alkyl, and which is unsubstituted or substituted with 1, 2, 3, 4 or 5 substituents independently from one another selected from the group consisting of F, Cl, CF₃, OCF₃, CN, =O, C₁₋₆-alkyl, C₁₋₆-alkylen-OH and O-C₁₋₆-alkyl;
or a part structure of general formula SF-III 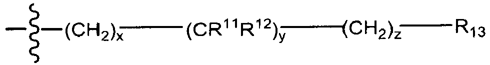 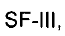 wherein
x represents 1 and y and z each represent 0 or
x and y each represent 1 and z represents 0 or
x and z each represent 1 and y represents 0 or
x, y and z each represent 1;
R¹¹ and R¹² are independently from one another selected from H or CH₃;
R¹³ is selected from the group consisting of
H, F, Cl, CN, OH, O-C₁₋₆-alkyl, O-(C=O)C₁₋₆-alkyl, S(=O)-C₁₋₆-alkyl, S(=O)₂-C₁₋₆-alkyl, NH₂, NH(C₁₋₆-alkyl), N(C₁₋₆-alkyl)₂, N(H)-C(=O)-C₁₋₆-alkyl, N(H)-S(=O)₂-C₁₋₆-alkyl, C(=O)-NH₂, C(=O)-N(H)(C₁₋₆-alkyl), C(=O)-N(C₁₋₆-alkyl)₂, N(H)-C(=O)-NH₂, N(H)-C(=O)-N(H)(C₁₋₆-alkyl), N(H)-C(=O)-N(C₁₋₆-alkyl)₂,
or represents
C₃₋₆-cycloalkyl or
3-7-membered heterocyclyl, which contains 1 or 2 heteroatoms or heteroatom groups independently from one another selected from the group consisting of O, S, S(=O), S(=O)₂, NH and N-C₁₋₆-alkyl, and which is unsubstituted or substituted with 1, 2, 3, 4 or 5 substituents independently from one another selected from the group consisting of F, Cl, CF₃, OCF₃, CN, C₁₋₆-alkyl and O-C₁₋₆-alkyl; or
phenyl or heteroaryl, selected from pyrrolyl, pyrazolyl, imidazolyl, oxazolyl, isoxazolyl, thiazolyl, isothiazolyl, pyridinyl, pyrazinyl, pyrimidinyl and pyridazinyl,
in each unsubstituted or substituted with 1, 2 or 3 substituents independently from one another selected from the group consisting of F, Cl, CN, CF₃, OCF₃, C₁₋₆-alkylen-OH, C₁₋₆alkyl, OH, O-C₁₋₆-alkyl, S(=O)₂-C₁₋₆-alkyl, S(=O)₂-NH₂, NH₂, NH(C₁₋₆-alkyl), N(C₁₋₆-alkyl)₂, O-C(=O)-NH₂, C(=O)-NH₂, C(=O)-N(H)(C₁₋₆-alkyl), C(=O)-N(C₁₋₆-alkyl)₂, C(=O)-O-C₁₋₆-alkyl;
or
R⁴ and R⁵ together with the nitrogen atom connecting them form a heterocyclyl, selected from the group consisting of 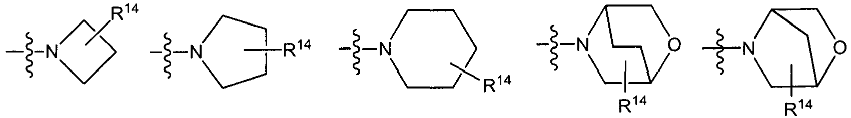 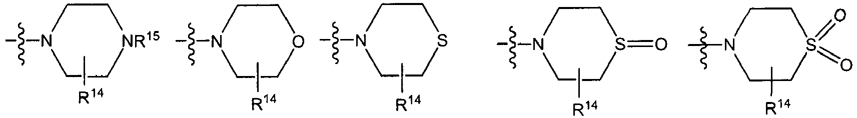 wherein
R¹⁴ denotes 0, 1 or 2 substituents which are in each case independently of each other selected from the group consisting of F, Cl, CF₃, =O, OCF₃, OH, O-C₁₋₆-alkyl, C₁₋₆-alkylen-OH, C₁₋₆-alkylen-SO₂(C₁₋₆-alkyl), SO₂(C₁₋₆-alkyl), C₁₋₆-alkyl, aryl, heteroaryl, O-aryl and O-heteroaryl, wherein said aryl or said heteroaryl is unsubstituted or substituted with 1, 2 or 3 substituents independently from one another selected from the group consisting of F, Cl, CN, CF₃, OCF₃, C₁₋₆-alkylen-OH, C₁₋₆₋alkyl, OH or O-C₁₋₆-alkyl; and
R¹⁵ represents H, C₁₋₆-alkyl, (C=O)C₁₋₆-alkyl, (C=O)NH₂, (C=O)NH(C₁₋₆-alkyl) or
(C=O)N(C₁₋₆-alkyl)₂.

12. A compound according to one or more of the preceding claims selected from the group consisting of
| | |
|---|---|
| **001** | N-Benzyl-3-(4-chlorophenyl)-1,4-dimethyl-N-tetrahydro-pyran-4-yl-5-(trifluoromethyl)-1H-pyrrole-2-carboxylic acid amide |
| **002** | 3-(4-Chlorophenyl)-N-(2,2-dimethyl-propyl)-N,1,4-trimethyl-5-(trifluoromethyl)-1H-pyrrole-2-carboxylic acid amide |
| **003** | N-Benzyl-3-(4-chlorophenyl)-N-(2,2-dimethyl-propyl)-1,4-dimethyl-5-(trifluoromethyl)-1H-pyrrole-2-carboxylic acid amide |
| **004** | [3-(4-Chlorophenyl)-1,4-dimethyl-5-(trifluoromethyl)-1H-pyrrol-2-yl]-morpholin-4-yl-methanone |
| **005** | 3-(4-Chlorophenyl)-N-(2,2-dimethyl-propyl)-1-ethyl-N,4-dimethyl-5-(trifluoromethyl)-1H-pyrrole-2-carboxylic acid amide |
| **006** | [3-(4-Chlorophenyl)-1-isopropyl-4-methyl-5-(trifluoromethyl)-1H-pyrrol-2-yl]-(2,2-dimethyl-morpholin-4-yl)-methanone |
| **007** | N-(1-Carbamoyl-cyclopropyl)-3-(4-chlorophenyl)-1-cyclopropyl-N,4-dimethyl-5-(trifluoromethyl)-1H-pyrrole-2-carboxylic acid amide |
| **008** | 3-(4-Chlorophenyl)-1-cyclopropyl-N-(1,1-dioxo-thian-4-yl)-N,4-dimethyl-5-(trifluoromethyl)-1H-pyrrole-2-carboxylic acid amide |
| **009** | N-(2-Carbamoyl-2-methyl-propyl)-3-(4-chlorophenyl)-1-cyclopropyl-N,4-dimethyl-5-(trifluoromethyl)-1H-pyrrole-2-carboxylic acid amide |
| **010** | 3-(4-Chlorophenyl)-1-cyclopropyl-N-[(4-methoxyphenyl)-methyl]-N,4-dimethyl-5-(trifluoromethyl)-1H-pyrrole-2-carboxylic acid amide |
| **011** | 3-(4-Chlorophenyl)-1-cyclopropyl-N-[(4-methoxyphenyl)-methyl]-N-methyl-5-(trifluoromethyl)-1H-pyrrole-2-carboxylic acid amide |
| **012** | 3-(4-Chlorophenyl)-N,1-dicyclopropyl-N,4-dimethyl-5-(trifluoromethyl)-1H-pyrrole-2-carboxylic acid amide |
| **013** | 3-(4-Chlorophenyl)-N,1-dicyclopropyl-N-methyl-5-(trifluoromethyl)-1H-pyrrole-2-carboxylic acid amide |
| **014** | 3-(4-Chlorophenyl)-1-cyclopropyl-N,4-dimethyl-N-tetrahydro-pyran-4-yl-5-(trifluoromethyl)-1H-pyrrole-2-carboxylic acid amide |
| **015** | 3-(4-Chlorophenyl)-1-cyclopropyl-N-methyl-N-tetrahydro-pyran-4-yl-5-(trifluoromethyl)-1H-pyrrole-2-carboxylic acid amide |
| **016** | 3-(4-Chlorophenyl)-1-cyclopropyl-N-(2,2-dimethyl-3-morpholin-4-yl-propyl)-N,4-dimethyl-5-(trifluoromethyl)-1H-pyrrole-2-carboxylic acid amide |
| **017** | 3-(4-Chlorophenyl)-1-cyclopropyl-N-(2,2-dimethyl-3-morpholin-4-yl-propyl)-N-methyl-5-(trifluoromethyl)-1H-pyrrole-2-carboxylic acid amide |
| **018** | 3-(4-Chlorophenyl)-1-cyclopropyl-N-(2-hydroxy-2-methyl-propyl)-N,4-dimethyl-5-(trifluoromethyl)-1H-pyrrole-2-carboxylic acid amide |
| **019** | 3-(4-Chlorophenyl)-1-cyclopropyl-N-(2-hydroxy-2-methyl-propyl)-N-methyl-5-(trifluoromethyl)-1H-pyrrole-2-carboxylic acid amide |
| **020** | 3-(4-Chloro-2-fluoro-phenyl)-1-cyclopropyl-N,4-dimethyl-N-[(5-methyl-isoxazol-3-yl)-methyl]-5-(trifluoromethyl)-1H-pyrrole-2-carboxylic acid amide |
| **021** | 3-(4-Chloro-2-fluoro-phenyl)-1-cyclopropyl-N,4-dimethyl-N-(pyrimidin-4-yl-methyl)-5-(trifluoromethyl)-1H-pyrrole-2-carboxylic acid amide |
| **022** | 3-(4-Chlorophenyl)-1-cyclopropyl-N,4-dimethyl-N-(tetrahydro-furan-3-yl-methyl)-5-(trifluoromethyl)-1H-pyrrole-2-carboxylic acid amide |
| **023** | 3-(4-Chlorophenyl)-1-cyclopropyl-N-methyl-N-(tetrahydro-furan-3-yl-methyl)-5-(trifluoromethyl)-1H-pyrrole-2-carboxylic acid amide |
| **024** | 3-(4-Chloro-2-fluoro-phenyl)-1-cyclopropyl-N,4-dimethyl-N-[(5-methyl-pyrazin-2-yl)-methyl]-5-(trifluoromethyl)-1H-pyrrole-2-carboxylic acid amide |
| **025** | [3-(4-Chlorophenyl)-1-cyclopropyl-4-methyl-5-(trifluoromethyl)-1H-pyrrol-2-yl]-(3-hydroxy-azetidin-1-yl)-methanone |
| **026** | [3-(4-Chlorophenyl)-1-cyclopropyl-5-(trifluoromethyl)-1H-pyrrol-2-yl]-(3-hydroxy-azetidin-1-yl)-methanone |
| **027** | [3-(4-Chlorophenyl)-1-cyclopropyl-4-methyl-5-(trifluoromethyl)-1H-pyrrol-2-yl]-[4-(3-methyl-[1,2,4]oxadiazol-5-yl)-piperidin-1-yl]-methanone |
| **028** | [3-(4-Chloro-2-fluoro-phenyl)-1-cyclopropyl-4-methyl-5-(trifluoromethyl)-1H-pyrrol-2-yl]-[4-(3-methyl-[1,2,4]oxadiazol-5-yl)-piperidin-1-yl]-methanone |
| **029** | [3-(4-Chlorophenyl)-1-cyclopropyl-5-(trifluoromethyl)-1H-pyrrol-2-yl]-[4-(3-methyl-[1,2,4]oxadiazol-5-yl)-piperidin-1-yl]-methanone |
| **030** | [3-(4-Chlorophenyl)-1-cyclopropyl-4-methyl-5-(trifluoromethyl)-1H-pyrrol-2-yl]-[4-[5-(trifluoromethyl)-pyridin-2-yl]oxy-piperidin-1-yl]-methanone |
| **031** | [3-(4-Chlorophenyl)-1-cyclopropyl-5-(trifluoromethyl)-1H-pyrrol-2-yl]-[4-[5-(trifluoromethyl)-pyridin-2-yl]oxy-piperidin-1-yl]-methanone |
| **032** | [3-(4-Chlorophenyl)-1-cyclopropyl-4-methyl-5-(trifluoromethyl)-1H-pyrrol-2-yl]-(2,2-dimethyl-morpholin-4-yl)-methanone |
| **033** | [3-(4-Chloro-2-fluoro-phenyl)-1-cyclopropyl-4-methyl-5-(trifluoromethyl)-1H-pyrrol-2-yl]-(2,2-dimethyl-morpholin-4-yl)-methanone |
| **034** | [3-(4-Chlorophenyl)-1-cyclopropyl-5-(trifluoromethyl)-1H-pyrrol-2-yl]-(2,2-dimethyl-morpholin-4-yl)-methanone |
| **035** | 3-(4-Chlorophenyl)-1-cyclopropyl-N-(2,2-dimethyl-propyl)-N-methyl-5-(trifluoromethyl)-1H-pyrrole-2-carboxylic acid amide |
| **036** | 3-(4-Chlorophenyl)-1-cyclopropyl-N-(3-hydroxy-2,2-dimethyl-propyl)-N-methyl-5-(trifluoromethyl)-1H-pyrrole-2-carboxylic acid amide |
| **037** | 3-(4-Chlorophenyl)-1-cyclopropyl-N-methyl-N-(2-methylsulfonyl-ethyl)-5-(trifluoromethyl)-1H-pyrrole-2-carboxylic acid amide |
| **038** | [3-(4-Chlorophenyl)-1-cyclopropyl-5-(trifluoromethyl)-1H-pyrrol-2-yl]-morpholin-4-yl-methanone |
| **039** | 3-(4-Chlorophenyl)-1-cyclopropyl-N-(2,2-dimethyl-propyl)-N,4-dimethyl-5-(trifluoromethyl)-1H-pyrrole-2-carboxylic acid amide |
| **040** | 3-(4-Chlorophenyl)-1-cyclopropyl-N-(3-hydroxy-2,2-dimethyl-propyl)-N,4-dimethyl-5-(trifluoromethyl)-1H-pyrrole-2-carboxylic acid amide |
| **041** | 3-(4-Chlorophenyl)-1-cyclopropyl-N,4-dimethyl-N-(2-methylsulfonyl-ethyl)-5-(trifluoromethyl)-1H-pyrrole-2-carboxylic acid amide |
| **042** | [3-(4-Chlorophenyl)-1-cyclobutyl-4-methyl-5-(trifluoromethyl)-1H-pyrrol-2-yl]-(2,2-dimethyl-morpholin-4-yl)-methanone |
| **043** | [3-(4-Chlorophenyl)-1-cyclopropyl-4-methyl-5-(trifluoromethyl)-1H-pyrrol-2-yl]-morpholin-4-yl-methanone |
| **044** | [3-(4-Chlorophenyl)-1-cyclopropyl-4-methyl-5-(trifluoromethyl)-1H-pyrrol-2-yl]-(2,2-dimethyl-1,1-dioxo-[1,4]thiazinan-4-yl)-methanone |
| **045** | (2,2-Dimethyl-morpholin-4-yl)-[3-(4-fluorophenyl)-1-(2-methyl-propyl)-5-(trifluoromethyl)-1H-pyrrol-2-yl]-methanone |
| **046** | 3-(4-Fluorophenyl)-N-methyl-1-(2-methyl-propyl)-N-tetrahydro-pyran-4-yl-5-(trifluoromethyl)-1H-pyrrole-2-carboxylic acid amide |
| **047** | 3-(4-Fluorophenyl)-N-methyl-N-[(5-methyl-isoxazol-3-yl)-methyl]-1-(2-methyl-propyl)-5-(trifluoromethyl)-1H-pyrrole-2-carboxylic acid amide |
| **048** | 3-(4-Chlorophenyl)-N,4-dimethyl-1-(2-methyl-propyl)-N-(pyrimidin-4-yl-methyl)-5-(trifluoromethyl)-1H-pyrrole-2-carboxylic acid amide |
| **049** | 3-(4-Fluorophenyl)-N-methyl-1-(2-methyl-propyl)-N-[(5-methyl-pyrazin-2-yl)-methyl]-5-(trifluoromethyl)-1H-pyrrole-2-carboxylic acid amide |
| **050** | 3-(4-Fluorophenyl)-N-methyl-1-(2-methyl-propyl)-N-(2-methylsulfonyl-ethyl)-5-(trifluoromethyl)-1H-pyrrole-2-carboxylic acid amide |
| **051** | N-(1-Carbamoyl-cyclopropyl)-3-(4-chlorophenyl)-N,4-dimethyl-1-(2-methyl-propyl)-5-(trifluoromethyl)-1H-pyrrole-2-carboxylic acid amide |
| **052** | N-(2-Carbamoyl-2-methyl-propyl)-3-(4-chlorophenyl)-N,4-dimethyl-1-(2-methyl-propyl)-5-(trifluoromethyl)-1H-pyrrole-2-carboxylic acid amide |
| **053** | N-(2,2-Dimethyl-propyl)-3-(4-fluorophenyl)-N-methyl-1-(2-methyl-propyl)-5-(trifluoromethyl)-1H-pyrrole-2-carboxylic acid amide |
| **054** | N-(2,2-Dimethyl-3-morpholin-4-yl-propyl)-3-(4-fluorophenyl)-N-methyl-1-(2-methylpropyl)-5-(trifluoromethyl)-1H-pyrrole-2-carboxylic acid amide |
| **055** | 3-(4-Chlorophenyl)-N-(3-hydroxy-2,2-dimethyl-propyl)-N,4-dimethyl-1-(2-methyl-propyl)-5-(trifluoromethyl)-1H-pyrrole-2-carboxylic acid amide |
| **056** | 3-(4-Fluorophenyl)-N-(3-hydroxy-2,2-dimethyl-propyl)-N-methyl-1-(2-methyl-propyl)-5-(trifluoromethyl)-1H-pyrrole-2-carboxylic acid amide |
| **057** | 3-(4-Chlorophenyl)-N,4-dimethyl-N-[(5-methyl-isoxazol-3-yl)-methyl]-1-(2-methyl-propyl)-5-(trifluoromethyl)-1H-pyrrole-2-carboxylic acid amide |
| **058** | 3-(4-Fluorophenyl)-N-methyl-1-(2-methyl-propyl)-N-(tetrahydro-furan-3-yl-methyl)-5-(trifluoromethyl)-1H-pyrrole-2-carboxylic acid amide |
| **059** | 3-(4-Chlorophenyl)-N,4-dimethyl-1-(2-methyl-propyl)-N-[(5-methyl-pyrazin-2-yl)-methyl]-5-(trifluoromethyl)-1H-pyrrole-2-carboxylic acid amide |
| **060** | N-[(2-Dimethylamino-pyrimidin-5-yl)-methyl]-3-(4-fluorophenyl)-N-methyl-1-(2-methylpropyl)-5-(trifluoromethyl)-1H-pyrrole-2-carboxylic acid amide |
| **061** | 3-(4-Chlorophenyl)-N,4-dimethyl-1-(2-methyl-propyl)-N-(2-methylsulfonyl-ethyl)-5-(trifluoromethyl)-1H-pyrrole-2-carboxylic acid amide |
| **062** | [3-(4-Chlorophenyl)-4-methyl-1-(2-methyl-propyl)-5-(trifluoromethyl)-1H-pyrrol-2-yl]-[4-(3-methyl-[1,2,4]oxadiazol-5-yl)-piperidin-1-yl]-methanone |
| **063** | 4-[3-(4-Fluorophenyl)-1-(2-methyl-propyl)-5-(trifluoromethyl)-1H-pyrrole-2-carbonyl]-piperazin-2-one |
| **064** | [3-(4-Chlorophenyl)-4-methyl-1-(2-methyl-propyl)-5-(trifluoromethyl)-1H-pyrrol-2-yl]-(2,2-dimethyl-morpholin-4-yl)-methanone |
| **065** | [3-(4-Fluorophenyl)-1-(2-methyl-propyl)-5-(trifluoromethyl)-1H-pyrrol-2-yl]-morpholin-4-yl-methanone |
| **066** | N-Cyclopropyl-3-(4-fluorophenyl)-1-(2-methyl-propyl)-5-(trifluoromethyl)-1H-pyrrole-2-carboxylic acid amide |
| **067** | 3-(4-Chlorophenyl)-N-(2-cyano-2-methyl-propyl)-N,4-dimethyl-1-(2-methyl-propyl)-5-(trifluoromethyl)-1H-pyrrole-2-carboxylic acid amide |
| **068** | 3-(4-Chlorophenyl)-N,4-dimethyl-1-[(1-methyl-cyclopropyl)-methyl]-N-(2-methylsulfonylethyl)-5-(trifluoromethyl)-1H-pyrrole-2-carboxylic acid amide |
| **069** | N-[(2-Dimethylamino-pyrimidin-5-yl)-methyl]-3-(4-fluorophenyl)-N-methyl-1-(tetrahydrofuran-2-yl-methyl)-5-(trifluoromethyl)-1H-pyrrole-2-carboxylic acid amide |
| **070** | 3-(4-Fluorophenyl)-N-methyl-1-(tetrahydro-furan-2-yl-methyl)-N-tetrahydro-pyran-4-yl-5-(trifluoromethyl)-1H-pyrrole-2-carboxylic acid amide |
| **071** | N-(1,1-Dioxo-thiolan-3-yl)-3-(4-fluorophenyl)-N-methyl-1-(tetrahydro-furan-2-yl-methyl)-5-(trifluoromethyl)-1H-pyrrole-2-carboxylic acid amide |
| **072** | N-(2-Carbamoyl-2-methyl-propyl)-3-(4-fluorophenyl)-N-methyl-1-(tetrahydro-furan-2-yl-methyl)-5-(trifluoromethyl)-1H-pyrrole-2-carboxylic acid amide |
| **073** | N-(2,2-Dimethyl-propyl)-3-(4-fluorophenyl)-N-methyl-1-(tetrahydro-furan-2-yl-methyl)-5-(trifluoromethyl)-1H-pyrrole-2-carboxylic acid amide |
| **074** | 3-(4-Fluorophenyl)-N-(3-hydroxy-2,2-dimethyl-propyl)-N-methyl-1-(tetrahydro-furan-2-yl-methyl)-5-(trifluoromethyl)-1H-pyrrole-2-carboxylic acid amide |
| **075** | 3-(4-Fluorophenyl)-N-methyl-N-[(5-methyl-isoxazol-3-yl)-methyl]-1-(tetrahydro-furan-2-yl-methyl)-5-(trifluoromethyl)-1H-pyrrole-2-carboxylic acid amide |
| **076** | 3-(4-Fluorophenyl)-N-methyl-N, 1-bis(tetrahydro-furan-2-yl-methyl)-5-(trifluoromethyl)-1H-pyrrole-2-carboxylic acid amide |
| **077** | 3-(4-Fluorophenyl)-N-methyl-N-[(5-methyl-pyrazin-2-yl)-methyl]-1-(tetrahydro-furan-2-yl-methyl)-5-(trifluoromethyl)-1H-pyrrole-2-carboxylic acid amide |
| **078** | 3-(4-Fluorophenyl)-N-methyl-N-(2-methylsulfonyl-ethyl)-1-(tetrahydro-furan-2-yl-methyl)-5-(trifluoromethyl)-1H-pyrrole-2-carboxylic acid amide |
| **079** | 4-[3-(4-Fluorophenyl)-1-(tetrahydro-furan-2-yl-methyl)-5-(trifluoromethyl)-1H-pyrrole-2-carbonyl]-piperazin-2-one |
| **080** | (2,2-Dimethyl-morpholin-4-yl)-[3-(4-fluorophenyl)-1-(tetrahydro-furan-2-yl-methyl)-5-(trifluoromethyl)-1H-pyrrol-2-yl]-methanone |
| **081** | [3-(4-Fluorophenyl)-1-(tetrahydro-furan-2-yl-methyl)-5-(trifluoromethyl)-1H-pyrrol-2-yl]-morpholin-4-yl-methanone |
| **082** | N-Cyclopropyl-3-(4-fluorophenyl)-1-(tetrahydro-furan-2-yl-methyl)-5-(trifluoromethyl)-1H-pyrrole-2-carboxylic acid amide |
| **083** | 3-(4-Chlorophenyl)-N-(2,2-dimethyl-propyl)-N-methyl-1-(tetrahydro-furan-2-yl-methyl)-5-(trifluoromethyl)-1H-pyrrole-2-carboxylic acid amide |
| **084** | [3-(4-Chlorophenyl)-1-(cyclopropyl-methyl)-4-methyl-5-(trifluoromethyl)-1H-pyrrol-2-yl]-(2,2-dimethyl-morpholin-4-yl)-methanone |
| **085** | [3-(4-Chlorophenyl)-1-(cyclobutyl-methyl)-4-methyl-5-(trifluoromethyl)-1H-pyrrol-2-yl]-(2,2-dimethyl-morpholin-4-yl)-methanone |
| **086** | [3-(4-Chlorophenyl)-1-(3-cyclopropyl-prop-2-ynyl)-4-methyl-5-(trifluoromethyl)-1H-pyrrol-2-yl]-morpholin-4-yl-methanone |
| **087** | [3-(4-Chlorophenyl)-4-methyl-1-[(1-methyl-cyclopropyl)-methyl]-5-(trifluoromethyl)-1H-pyrrol-2-yl]-morpholin-4-yl-methanone |
| **088** | 1-[(1-Amino-cyclopropyl)-methyl]-3-(4-chlorophenyl)-N-(2,2-dimethyl-propyl)-N-methyl-5-(trifluoromethyl)-1H-pyrrole-2-carboxylic acid amide |
| **089** | [3-(4-Chlorophenyl)-1-[(1-hydroxy-cyclopropyl)-methyl]-4-methyl-5-(trifluoromethyl)-1H-pyrrol-2-yl]-morpholin-4-yl-methanone |
| **090** | 3-(4-Chlorophenyl)-N-(2,2-dimethyl-propyl)-1-[(1-hydroxy-cyclopentyl)-methyl]-N-methyl-5-(trifluoromethyl)-1H-pyrrole-2-carboxylic acid amide |
| **091** | 3-(4-Chlorophenyl)-N-(2,2-dimethyl-propyl)-N-methyl-1-(pyrrolidin-2-yl-methyl)-5-(trifluoromethyl)-1H-pyrrole-2-carboxylic acid amide |
| **092** | 3-(4-Chlorophenyl)-N-(2,2-dimethyl-propyl)-N-methyl-1-(tetrahydro-pyran-4-yl-methyl)-5-(trifluoromethyl)-1H-pyrrole-2-carboxylic acid amide |
| **093** | 1-[(1-Amino-cyclopropyl)-methyl]-3-(4-chlorophenyl)-N,4-dimethyl-N-(2-methylsulfonylethyl)-5-(trifluoromethyl)-1H-pyrrole-2-carboxylic acid amide |
| **094** | 3-(4-Chlorophenyl)-1-[(1-hydroxy-cyclopentyl)-methyl]-N,4-dimethyl-N-(2-methylsulfonylethyl)-5-(trifluoromethyl)-1H-pyrrole-2-carboxylic acid amide |
| **095** | [1-[(1-Amino-cyclopropyl)-methyl]-3-(4-chlorophenyl)-4-methyl-5-(trifluoromethyl)-1H-pyrrol-2-yl]-morpholin-4-yl-methanone |
| **096** | [3-(4-Chlorophenyl)-1-[(1-hydroxy-cyclopentyl)-methyl]-4-methyl-5-(trifluoromethyl)-1H-pyrrol-2-yl]-morpholin-4-yl-methanone |
| **097** | [3-(4-Chlorophenyl)-4-methyl-1-(tetrahydro-pyran-4-yl-methyl)-5-(trifluoromethyl)-1H-pyrrol-2-yl]-morpholin-4-yl-methanone |
| **098** | [3-(4-Chlorophenyl)-1-[2-(3,3-difluoro-azetidin-1-yl)-ethyl]-4-methyl-5-(trifluoromethyl)-1H-pyrrol-2-yl]-morpholin-4-yl-methanone |
| **099** | [3-(4-Chlorophenyl)-4-methyl-1-(2-methylsulfonyl-ethyl)-5-(trifluoromethyl)-1H-pyrrol-2-yl]-morpholin-4-yl-methanone |
| **100** | 3-(4-Chlorophenyl)-1-[(1-cyano-cyclopropyl)-methyl]-N-(2,2-dimethyl-propyl)-N-methyl-5-(trifluoromethyl)-1H-pyrrole-2-carboxylic acid amide |
| **101** | 3-(4-Chlorophenyl)-N-(2,2-dimethyl-propyl)-1-[(1-hydroxy-cyclobutyl)-methyl]-N-methyl-5-(trifluoromethyl)-1H-pyrrole-2-carboxylic acid amide |
| **102** | [3-(4-Chlorophenyl)-1,4-dimethyl-5-(trifluoromethyl)-1H-pyrrol-2-yl]-(2,2-dimethyl-morpholin-4-yl)-methanone |
| **103** | [3-(4-Chlorophenyl)-1-ethyl-4-methyl-5-(trifluoromethyl)-1H-pyrrol-2-yl]-(2,2-dimethyl-morpholin-4-yl)-methanone |
| **104** | [3-(4-Chlorophenyl)-4-methyl-1-(2-methyl-propyl)-5-(trifluoromethyl)-1H-pyrrol-2-yl]-(2-oxa-5-azabicyclo[2.2.1]heptan-5-yl)-methanone |
| **105** | N-(2-Carbamoyl-2-methyl-propyl)-3-(4-chlorophenyl)-1-isopropyl-N,4-dimethyl-5-(trifluoromethyl)-1H-pyrrole-2-carboxylic acid amide |
| **106** | 3-(4-Chlorophenyl)-N-(2-cyano-2-methyl-propyl)-1-isopropyl-N,4-dimethyl-5-(trifluoromethyl)-1H-pyrrole-2-carboxylic acid amide |
| **107** | 3-(4-Chlorophenyl)-1-isopropyl-N,4-dimethyl-N-[(5-methyl-isoxazol-3-yl)-methyl]-5-(trifluoromethyl)-1H-pyrrole-2-carboxylic acid amide |
| **108** | 3-(4-Chlorophenyl)-N,1-diisopropyl-N,4-dimethyl-5-(trifluoromethyl)-1H-pyrrole-2-carboxylic acid amide |
| **109** | 3-(4-Chlorophenyl)-N-isopropyl-N,4-dimethyl-1-(2-methyl-propyl)-5-(trifluoromethyl)-1H-pyrrole-2-carboxylic acid amide |
| **110** | 3-(4-Chlorophenyl)-1-isopropyl-N,4-dimethyl-N-(2-methylsulfonyl-ethyl)-5-(trifluoromethyl)-1H-pyrrole-2-carboxylic acid amide |
| **111** | 3-(4-Chlorophenyl)-1-isopropyl-N,4-dimethyl-N-[(2-methyl-2H-pyrazol-3-yl)-methyl]-5-(trifluoromethyl)-1H-pyrrole-2-carboxylic acid amide |
| **112** | [3-(5-Chloro-pyridin-2-yl)-1-isopropyl-4-methyl-5-(trifluoromethyl)-1H-pyrrol-2-yl]-(2,2-dimethyl-morpholin-4-yl)-methanone |
| **113** | 3-(5-Chloro-pyridin-2-yl)-1-isopropyl-N,4-dimethyl-N-[(5-methyl-isoxazol-3-yl)-methyl]-5-(trifluoromethyl)-1H-pyrrole-2-carboxylic acid amide |
| **114** | 3-(5-Chloro-pyridin-2-yl)-1-isopropyl-N,4-dimethyl-N-[(2-methyl-2H-pyrazol-3-yl)-methyl]-5-(trifluoromethyl)-1H-pyrrole-2-carboxylic acid amide |
| **115** | 3-(4-Chlorophenyl)-1-isopropyl-N,4-dimethyl-N-(2-oxo-pyrrolidin-3-yl)-5-(trifluoromethyl)-1H-pyrrole-2-carboxylic acid amide |
| **116** | N-(2-Carbamoyl-2-methyl-propyl)-3-(5-chloro-pyridin-2-yl)-1-isopropyl-N,4-dimethyl-5-(trifluoromethyl)-1H-pyrrole-2-carboxylic acid amide |
| **117** | 3-(5-Chloro-pyridin-2-yl)-N-(2-cyano-2-methyl-propyl)-1-isopropyl-N,4-dimethyl-5-(trifluoromethyl)-1H-pyrrole-2-carboxylic acid amide |
| **118** | 3-(5-Chloro-pyridin-2-yl)-1-isopropyl-N,4-dimethyl-N-(2-methylsulfonyl-ethyl)-5-(trifluoromethyl)-1H-pyrrole-2-carboxylic acid amide |
| **119** | 3-(5-Chloro-pyridin-2-yl)-1-isopropyl-N,4-dimethyl-N-(2-oxo-pyrrolidin-3-yl)-5-(trifluoromethyl)-1H-pyrrole-2-carboxylic acid amide |
| **120** | 3-(5-Chloro-pyridin-2-yl)-N,1-diisopropyl-N,4-dimethyl-5-(trifluoromethyl)-1H-pyrrole-2-carboxylic acid amide |
| **121** | [3-(5-Chloro-pyrimidin-2-yl)-1-isopropyl-4-methyl-5-(trifluoromethyl)-1H-pyrrol-2-yl]-(2,2-dimethyl-morpholin-4-yl)-methanone |
| **122** | 3-(5-Chloro-pyrimidin-2-yl)-1-isopropyl-N,4-dimethyl-N-[(2-methyl-2H-pyrazol-3-yl)-methyl]-5-(trifluoromethyl)-1H-pyrrole-2-carboxylic acid amide |
| **123** | 3-(5-Chloro-pyrimidin-2-yl)-1-isopropyl-N,4-dimethyl-N-[(5-methyl-isoxazol-3-yl)-methyl]-5-(trifluoromethyl)-1H-pyrrole-2-carboxylic acid amide |
| **124** | 3-(5-Chloro-pyrimidin-2-yl)-1-isopropyl-N,4-dimethyl-N-(2-oxo-pyrrolidin-3-yl)-5-(trifluoromethyl)-1H-pyrrole-2-carboxylic acid amide |
| **125** | N-(2-Carbamoyl-2-methyl-propyl)-3-(5-chloro-pyrimidin-2-yl)-1-isopropyl-N,4-dimethyl-5-(trifluoromethyl)-1H-pyrrole-2-carboxylic acid amide |
| **126** | 3-(5-Chloro-pyrimidin-2-yl)-N-(2-cyano-2-methyl-propyl)-1-isopropyl-N,4-dimethyl-5-(trifluoromethyl)-1H-pyrrole-2-carboxylic acid amide |
| **127** | 3-(5-Chloro-pyrimidin-2-yl)-1-isopropyl-N,4-dimethyl-N-(2-methylsulfonyl-ethyl)-5-(trifluoromethyl)-1H-pyrrole-2-carboxylic acid amide |
| **128** | 3-(5-Chloro-pyrimidin-2-yl)-N,1-diisopropyl-N,4-dimethyl-5-(trifluoromethyl)-1H-pyrrole-2-carboxylic acid amide |
| **129** | N-tert-Butyl-4-[3-(5-chloro-pyridin-2-yl)-1-isopropyl-4-methyl-5-(trifluoromethyl)-1H-pyrrole-2-carbonyl]-piperazine-1-carboxylic acid amide |
| **130** | [3-(5-Chloro-pyridin-2-yl)-1-isopropyl-4-methyl-5-(trifluoromethyl)-1H-pyrrol-2-yl]-(2,2-dimethyl-1-oxo-[1,4]thiazinan-4-yl)-methanone |
| **131** | N-tert-Butyl-4-[3-(4-chlorophenyl)-1-isopropyl-4-methyl-5-(trifluoromethyl)-1H-pyrrole-2-carbonyl]-piperazine-1-carboxylic acid amide |
| **132** | 3-(4-Chlorophenyl)-N-[1-(hydroxymethyl)-3-methyl-butyl]-1-isopropyl-N,4-dimethyl-5-(trifluoromethyl)-1H-pyrrole-2-carboxylic acid amide |
| **133** | N-[1-(tert-Butyl-carbamoyl)-ethyl]-3-(4-chlorophenyl)-1-isopropyl-N,4-dimethyl-5-(trifluoromethyl)-1H-pyrrole-2-carboxylic acid amide |
| **134** | 3-(5-Chloro-pyridin-2-yl)-N-(3,3-dimethyl-piperidin-4-yl)-1-isopropyl-N,4-dimethyl-5-(trifluoromethyl)-1H-pyrrole-2-carboxylic acid amide |
| **135** | 3-(4-Chlorophenyl)-N-(3,3-dimethyl-piperidin-4-yl)-1-isopropyl-N,4-dimethyl-5-(trifluoromethyl)-1H-pyrrole-2-carboxylic acid amide |
optionally in the form of a single stereoisomer or a mixture of stereoisomers, in the form of the free compound and/or a physiologically acceptable salt or solvate thereof.

13. Pharmaceutical composition comprising at least one compound according to one or more of claims 1 to 12.

14. At least one compound according to one or more of claims 1 to 12 for use in the treatment and/or prophylaxis of one or more disorders selected from the group consisting of pain, preferably pain selected from the group consisting of acute pain, chronic pain, visceral pain, headache pain, inflammatory pain and mixed pain; stroke; mood disorders; epilepsy; schizophrenia, and neurodegenerative disorders.

15. At least one compound according to any one of claims 1 to 12 for use in the treatment and/or prophylaxis of pain, in particular acute pain and/or chronic pain and/or visceral pain and/or headache pain and/or inflammatory pain and/or mixed pain.

## Patentansprüche

1. Verbindung mit der allgemeinen Formel (I), 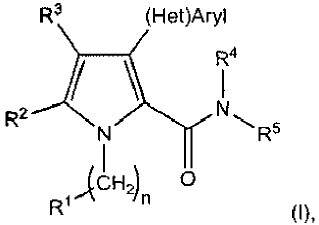 wobei
n 0, 1 oder 2 darstellt;
R¹ darstellt:
C₁₋₆-Alkyl, C₂₋₆-Alkenyl oder C₂₋₆-Alkynyl; oder
C₃₋₆-Cycloalkyl oder 3- bis 7-gliedriges Heterocyclyl;
R² darstellt: CH₂F; CHF₂ oder CF₃;
R³ darstellt: H, C₁₋₆-Alkyl, C₃₋₆-Cycloalkyl, 3- bis 7-gliedriges Heterocyclyl, OH; O-C₁₋₆-Alkyl; NH₂; N(H)(C₁₋₆-Alkyl); N(C₁₋₆-Alkyl)₂ oder SO₂(C₁₋₆-Alkyl);
(Het)Aryl darstellt: Aryl oder Heteroaryl, jeweils substituiert durch null oder einen oder zwei oder drei Substituenten der Gruppe bestehend aus R⁶, R⁷ und R⁸,
wobei R⁶, R⁷ und R⁸ jeweils unabhängig voneinander ausgewählt sind aus der Gruppe bestehend aus F; Cl; Br; I; NO₂; CN; C₁₋₆-Alkyl; CF₃; CF₂H; CFH₂; CF₂Cl; CFCl₂; C(=O)-H; C(=O)-C₁₋₆-Alkyl; C(=O)-OH; C(=O)-O-C₁₋₆-Alkyl; C(=O)-N(H)(OH); C(=O)-NH₂; C(=O)-N(H)(C₁₋₆-Alkyl); C(=O)-N(C₁₋₆-Alkyl)₂; C(=N-OH)-H; C(=N-OH)-C₁₋₆-Alkyl; C(=N-O-C₁₋₆-Alkyl)-H; C(=N-O-C₁₋₆-Alkyl)-C₁₋₆-Alkyl; OH; OCF₃; OCF₂H; OCFH₂; OCF₂Cl; OCFCl₂; O-C₁₋₆-Alkyl; O-C(=O)-C₁₋₆-Alkyl; O-C(=O)-O-C₁₋₆-Alkyl; O-(C=O)-N(H)(C₁₋₆-Alkyl); O-C(=O)-N(C₁₋₆-Alkyl)₂; O-S(=O)₂-C₁₋₆-Alkyl; O-S(=O)₂-OH; O-S(=O)₂-O-C₁₋₆-Alkyl; O-S(=O)₂-NH; O-S(=O)₂-N(H)(C₁₋₆-Alkyl); O-S(=O)₂-N(C₁₋₆-Alkyl)₂; NH₂; N(H)(C₁₋₆-Alkyl); N(C₁₋₆-Alkyl)₂; N(H)-C(=O)-C₁₋₆-Alkyl; N(H)-C(=O)-O-C₁₋₆-Alkyl; N(H)-C(=O)-NH₂; N(H)-C(=O)-N(H)(C₁₋₆-Alkyl); N(H)-C(=O)-N(C₁₋₆-Alkyl)₂; N(C₁₋₆-Alkyl)-C(=O)-C₁₋₆-Alkyl; N(C₁₋₆-Alkyl)-C(=O)-O-C₁₋₆-Alkyl; N(C₁₋₆-Alkyl)-C(=O)-NH₂; N(C₁₋₆-Alkyl)-C(=O)-N(H)(C₁₋₆-Alkyl); N(C₁₋₆-Alkyl)-C(=O)-N(C₁₋₆-Alkyl)₂; N(H)-S(=O)₂OH; N(H)-S(=O)₂-C₁₋₆-Alkyl; N(H)-S(=O)₂-O-C₁₋₆-Alkyl; N(H)-S(=O)₂-NH₂; N(H)-S(=O)₂-N(H)(C₁₋₆-Alkyl); N(H)-S(=O)₂N(C₁₋₆-Alkyl)₂; N(C₁₋₆-Alkyl)-S(=O)₂-OH; N(C₁₋₆-Alkyl)-S(=O)₂-C₁₋₆-Alkyl; N(C₁₋₆-Alkyl)-S(=O)₂-O-C₁₋₆-Alkyl; N(C₁₋₆-Alkyl)-S(=O)₂-NH₂; N(C₁₋₆-Alkyl)-S(=O)₂-N(H)(C₁₋₆-Alkyl); N(C₁₋₆-Alkyl)-S(=O)₂-N(C₁₋₆-Alkyl)₂; SH; SCF₃; SCF₂H; SCFH₂; SCF₂Cl; SCFCl₂; S-C₁₋₆-Alkyl; S(=O)-C₁₋₆-Alkyl; S(=O)₂-C₁₋₆-Alkyl; S(=O)₂-OH; S(=O)₂-O-C₁₋₆-Alkyl; S(=O)₂-NH₂; S(=O)₂-N(H)(C₁₋₆-Alkyl); S(=O)₂-N(C₁₋₆-Alkyl)₂, C₃₋₆-Cycloalkyl oder 3- bis 7-gliedriges Heterocyclyl;
wobei das C₁₋₆-Alkyl in jedem Fall verzweigt oder unverzweigt und unsubstituiert oder mono- oder poly-substituiert sein kann; und wobei das C₃₋₆-Cycloalkyl oder 3- bis 7-gliedrige Heterocyclyl in jedem Fall unsubstituiert oder mono- oder polysubstituiert sein kann;
R⁴ darstellt: H, C₁₋₁₀-Alkyl, Aryl, oder Aryl verbunden über eine C₁₋₈-Alkylen-Gruppe;
R⁵ darstellt: H; C₁₋₁₀-Alkyl, C₃₋₁₀-Cycloalkyl, 3- bis 10-gliedriges Heterocyclyl, Aryl oder Heteroaryl; oder C₃₋₁₀-Cycloalkyl, 3- bis 10-gliedriges Heterocyclyl, Aryl oder Heteroaryl, jeweils verbunden über eine C₁₋₈-Alkylen-Gruppe; oder
R⁴ und R⁵ zusammen mit dem Stickstoffatom, das sie verbindet, ein 3- bis 10-gliedriges Heterocyclyl bilden;
wobei das C₁₋₆-Alkyl, das C₁₋₁₀-Alkyl, das C₂₋₆-Alkenyl, das C₂₋₆-Alkynyl und das C₁₋₈-Alkylen in jedem Fall verzweigt oder unverzweigt und unsubstituiert oder mono- oder poly-substituiert sein können; und wobei das C₃₋₆-Cycloalkyl, das C₃₋₁₀-Cycloalkyl, das 3- bis 7-gliedrige Heterocyclyl, das 3- bis 10-gliedrige Heterocyclyl, das Aryl und das Heteroaryl in jedem Fall unsubstituiert oder mono- oder polysubstituiert sein können;
optional in der Form eines individuellen Stereoisomers oder eines Gemischs aus Stereoisomeren, in der Form der freien Verbindung und/oder eines physiologisch annehmbaren Salzes und/oder eines physiologisch annehmbaren Solvats davon.

2. Verbindung nach Anspruch 1, **dadurch gekennzeichnet, dass**
R¹ darstellt:
C₁₋₆-Alkyl, C₂₋₆-Alkenyl oder C₂₋₆-Alkynyl,
in jedem Fall verzweigt oder unverzweigt, und in jedem Fall unsubstituiert oder mono- oder polysubstituiert durch einen oder mehrere Substituenten, ausgewählt aus
F; Cl; CN; CF₃; CF₂H; CFH₂; C(=O)-OH; C(=O)-O-C₁₋₆-Alkyl; C(=O)-NH₂; C(=O)-N(H)(C₁₋₆-Alkyl); C(=O)-N(C₁₋₆-Alkyl)₂; OH; OCF₃; OCF₂H; OCFH₂; O-C₁₋₆-Alkyl; O-C(=O)-C₁₋₆-Alkyl; O-S(=O)₂-C₁₋₆-Alkyl; NH₂; N(H)(C₁₋₆-Alkyl); N(C₁₋₆-Alkyl)₂; N(H)-C(=O)-C₁₋₆-Alkyl; N(H)-C(=O)-N(H)(C₁₋₆-Alkyl); N(H)-C(=O)-N(C₁₋₆-Alkyl)₂; N(C₁₋₆-Alkyl)-C(=O)-C₁₋₆-Alkyl; N(H)-S(=O)₂-C₁₋₆-Alkyl; N(C₁₋₆-Alkyl)-S(=O)₂-C₁₋₆-Alkyl; S(=O)-C₁₋₆-Alkyl, S(=O)₂-C₁₋₆-Alkyl, C₃₋₆-Cycloalkyl oder 3- bis 7-gliedriges Heterocyclyl, oder R¹ darstellt:
C₃₋₆-Cycloalkyl oder 3- bis 7-gliedriges Heterocyclyl, in jedem Fall unsubstituiert oder mono- oder polysubstituiert durch einen oder mehrere Substituenten, ausgewählt aus
F; Cl; CN; C₁₋₆-Alkyl; CF₃; CF₂H; CFH₂; C(=O)-OH; C(=O)-O-C₁₋₆-Alkyl; C(=O)-NH₂; C(=O)-N(H)(C₁₋₆-Alkyl); C(=O)-N(C₁₋₆-Alkyl)₂; OH; =O; OCF₃; OCF₂H; OCFH₂; O-C₁₋₆-Alkyl; O-C(=O)-C₁₋₆-Alkyl; O-S(=O)₂-C₁₋₆-Alkyl; NH₂; N(H)(C₁₋₆-Alkyl); N(C₁₋₆-Alkyl)₂; N(H)-C(=O)-C₁₋₆-Alkyl; N(H)-C(=O)-N(H)(C₁₋₆-Alkyl); N(H)-C(=O)-N(C₁₋₆-Alkyl)₂; N(C₁₋₆-Alkyl)-C(=O)-C₁₋₆-Alkyl; N(H)-S(=O)₂- C₁₋₆-Alkyl; N(C₁₋₆-Alkyl)-S(=O)₂-C₁₋₆-Alkyl; S(=O)-C₁₋₆-Alkyl oder S(=O)₂-C₁₋₆-Alkyl.

3. Verbindung nach einem oder mehreren der vorherigen Ansprüche, **dadurch gekennzeichnet, dass** R² CHF₂ oder CF₃ darstellt.

4. Verbindung nach einem oder mehreren der vorherigen Ansprüche, **dadurch gekennzeichnet, dass** R³ ist ausgewählt aus der Gruppe bestehend aus H, Methyl, Ethyl, n-Propyl, iso-Propyl, n-Butyl, sec-Butyl, iso-Butyl, tert-Butyl, Cyclopropyl, Methoxy, Ethoxy, Methylsulfonyl, 2-Oxetyl, 3-Oxetyl, 2-Tetrahydrofuranyl und 3-Tetrahydrofuranyl.

5. Verbindung nach einem oder mehreren der vorherigen Ansprüche, **dadurch gekennzeichnet, dass** Het(aryl) ausgewählt ist aus Phenyl, Pyrrol, Pyrazolyl, Imidazolyl, Isoxazolyl, Oxazolyl, Thiazolyl, Pyridinyl, Pyrazinyl, Pyrimidinyl und Pyridazinyl, vorzugsweise, dass Het(aryl) Phenyl, Pyridinyl, Pyrazinyl oder Pyrimidinyl darstellt.

6. Verbindung nach einem oder mehreren der vorherigen Ansprüche, **dadurch gekennzeichnet, dass**
R⁶, R⁷ und R⁸ jeweils unabhängig voneinander ausgewählt sind aus der Gruppe bestehend aus F; Cl; CN; C₁₋₆-Alkyl; CF₃; CF₂H; CFH₂; OH; OCF₃; OCF₂H; OCFH₂; O-C₁₋₆-Alkyl; O-C(=O)-C₁₋₆-Alkyl; NH₂; N(H)(C₁₋₆-Alkyl); N(C₁₋₆-Alkyl)₂; SCF₃; S(=O)-C₁₋₆-Alkyl; S(=O)₂-C₁₋₆-Alkyl; S(=O)₂-OH; S(=O)₂-O-C₁₋₆-Alkyl; S(=O)₂-NH₂; S(=O)₂-N(H)(C₁₋₆-Alkyl); oder S(=O)₂-N(C₁₋₆-Alkyl)₂; wobei in jedem Fall C₁₋₆-Alkyl verzweigt oder unverzweigt sein kann; oder ein C₃₋₆-cycloaliphatischer Rest, unsubstituiert oder mono- oder polysubstituiert, vorzugsweise
R⁶, R⁷ und R⁸ jeweils unabhängig voneinander ausgewählt sind aus der Gruppe bestehend aus F; Cl; CN; C₁₋₆-Alkyl; CF₃; CF₂H; CFH₂; OH; OCF₃; OCF₂H; OCFH₂; O-C₁₋₆-Alkyl; S(=O)-C₁₋₆-Alkyl; S(=O)₂-C₁₋₆-Alkyl; Cyclopropyl und O-Cyclopropyl.

7. Verbindung nach einem oder mehreren der vorherigen Ansprüche, **dadurch gekennzeichnet, dass** die Verbindung mit der allgemeinen Formel (I) eine Verbindung gemäß der allgemeinen Formel (la) ist, 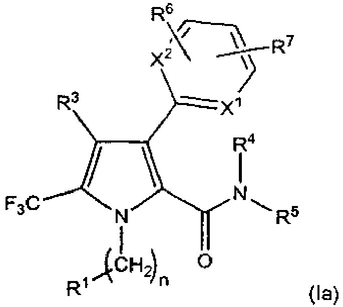 wobei
R³ H oder CH₃ oder Cyclopropyl darstellt;
X¹ N oder CH ist; X² N oder CH ist;
R⁶ und R⁷ unabhängig fehlen oder jeweils unabhängig voneinander ausgewählt sind aus der Gruppe bestehend aus F; Cl; CN; C₁₋₆-Alkyl; CF₃; CF₂H; CFH₂; OH; OCF₃; OCF₂H; OCFH₂ oder O-C₁₋₆-Alkyl;
und n, R¹, R⁴ und R⁵ gemäß Anspruch 1 definiert sind.

8. Verbindung nach einem oder mehreren der vorherigen Ansprüche, **dadurch gekennzeichnet, dass**
R⁴ darstellt:
H oder
einen C₁₋₆ aliphatischen Rest, verzweigt oder unverzweigt, unsubstituiert oder substituiert mit 1, 2, 3, 4 oder 5 Substituenten, unabhängig voneinander ausgewählt aus der Gruppe bestehend aus OH, =O, O- C₁₋₆-Alkyl, S(=O)-C₁₋₆-Alkyl, S(=O)₂-C₁₋₆-Alkyl, N(H)-S(=O)-C₁₋₆-Alkyl, N(C₁₋₆-Alkyl)-S(=O)-C₁₋₆-Alkyl, N(H)-S(=O)₂-C₁₋₆-Alkyl, N(C₁₋₆-Alkyl)-S(=O)₂-C₁₋₆-Alkyl, C(=O)-NH₂, C(=O)-N(H)(C₁₋₆-Alkyl), C(=O)-N(C₁₋₆-Alkyl)₂, C(=O)-O-C₁₋₆-Alkyl; N(H)-C(=O)-C₁₋₆-Alkyl, und N(C₁₋₆-Alkyl)-C(=O)-C₁₋₆-Alkyl
oder Phenyl, unsubstituiert oder mono- oder polysubstituiert mit 1, 2 oder 3 Substituenten, unabhängig voneinander ausgewählt aus der Gruppe bestehend aus F; Cl; CN; C₁₋₆-Alkyl; CF₃; CF₂H; CFH₂; OH; OCF₃; OCF₂H; OCFH₂ oder O-C₁₋₆-Alkyl, und optional verbunden über eine C₁₋₃-Alkylen-Gruppe, verzweigt oder unverzweigt; und
R⁵ darstellt:
H; oder
C₁₋₆-Alkyl, verzweigt oder unverzweigt, unsubstituiert oder substituiert mit 1, 2, 3, 4 oder 5 Substituenten, unabhängig voneinander ausgewählt aus der Gruppe bestehend aus F, Cl, CF₃, CN, OH, =O, OCF₃, O-C₁₋₆-Alkyl, O-(C=O)C₁₋₆-Alkyl, S(=O)-C₁₋₆-Alkyl, S(=O)₂-C₁₋₆-Alkyl, S(=O)₂-NH₂, S(=O)₂-N(H)C₁₋₆-Alkyl, S(=O)₂-N(C₁₋₆-Alkyl)₂, NH₂, NH(C₁₋₆-Alkyl), N(C₁₋₆-Alkyl)₂, N(H)-S(=O)-C₁₋₆-Alkyl, N(C₁-₆-Alkyl)-S(=O)-C₁₋₆-Alkyl, N(H)-S(=O)₂-C₁₋₆-Alkyl, N(C₁₋₆-Alkyl)-S(=O)₂-C₁₋₆-Alkyl, N(H)-C(=O)-NH₂, N(H)-C(=O)-N(H)(C₁₋₆-Alkyl), N(H)-C(=O)-N(C₁₋₆-Alkyl)₂, N(H)-C(=O)-O-C₁₋₆-Alkyl; O-C(=O)-NH₂, O-C(=O)-N(H)(C₁₋₆-Alkyl), O-C(=O)-N(C₁₋₆-Alkyl)₂, C(=O)-NH₂, C(=O)-N(H)(C₁₋₆-Alkyl), C(=O)-N(C₁₋₆-Alkyl)₂, C(=O)-O-C₁₋₆-Alkyl; N(H)-C(=O)-C₁₋₆-Alkyl, und N(C₁₋₆-Alkyl)-C(=O)-C₁₋₆-Alkyl; oder
C₃₋₆-Cycloalkyl, unsubstituiert oder substituiert mit 1, 2, 3, 4 oder 5 Substituenten, unabhängig voneinander ausgewählt aus der Gruppe bestehend aus F, Cl, CN, CF₃, =O, OCF₃, C₁₋₆-Alkylen-OH, C₁₋₆-Alkyl, OH, O-C₁₋₆-Alkyl, O-(C=O)C₁₋₆-Alkyl, S(=O)-C₁₋₆-Alkyl, S(=O)₂-C₁₋₆-Alkyl, S(=O)₂-NH₂, S(=O)₂-N(H)C₁₋₆-Alkyl, S(=O)₂-N(C₁₋₆-Alkyl)₂, NH₂, NH(C₁₋₆-Alkyl), N(C₁₋₆-Alkyl)₂, N(H)-S(=O)-C₁₋₆-Alkyl, N(C₁₋₆-Alkyl)-S(=O)-C₁₋₆-Alkyl, N(H)-S(=O)₂-C₁₋₆-Alkyl, N(C₁₋₆-Alkyl)-S(=O)₂-C₁₋₆-Alkyl, N(H)-C(=O)-O-C₁₋₆-Alkyl; O-C(=O)-NH₂, O-C(=O)-N(H)(C₁₋₆-Alkyl), O-C(=O)-N(C₁₋₆-Alkyl)₂, N(H)-C(=O)-NH₂, N(H)-C(=O)-N(H)(C₁₋₆-Alkyl), N(H)-C(=O)-N(C₁₋₆-Alkyl)₂, C(=O)-NH₂, C(=O)-N(H)(C₁₋₆-Alkyl), C(=O)-N(C₁₋₆-Alkyl)₂, C(=O)-O-C₁₋₆-Alkyl; N(H)-C(=O)-C₁₋₆-Alkyl, und N(C₁₋₆-Alkyl)-C(=O)-C₁₋₆-Alkyl; wobei das C₃₋₆-Cycloalkyl optional über C₁₋₆-Alkylen verbunden ist, verzweigt oder unverzweigt, das wiederum unsubstituiert oder mit 1, 2, 3, 4 oder 5 Substituenten substituiert sein kann, unabhängig voneinander ausgewählt aus der Gruppe bestehend aus F, Cl, CF₃, =O, OCF₃, OH, O-C₁₋₆-Alkyl und C₁₋₆-Alkylen-OH; oder
3-7-gliedriges Heterocyclyl, das unsubstituiert oder mit 1, 2, 3, 4 oder 5 Substituenten substituiert ist, unabhängig voneinander ausgewählt aus der Gruppe bestehend aus F, Cl, CN, CF₃, =O, OCF₃, C₁₋₆-Alkylen-OH, C₁₋₆-Alkyl, OH, O-C₁₋₆-Alkyl, O-(C=O)C₁₋₆-Alkyl, S(=O)-C₁₋₆-Alkyl, S(=O)₂-C₁₋₆-Alkyl, S(=O)₂-NH₂, S(=O)₂-N(H)C₁₋₆-Alkyl, S(=O)₂-N(C₁₋₆-Alkyl)₂, NH₂, NH(C₁₋₆-Alkyl), N(C₁₋₆-Alkyl)₂, N(H)-S(=O)-C₁₋₆-Alkyl, N(C₁₋₆-Alkyl)-S(=O)-C₁₋₆-Alkyl, N(H)-S(=O)₂-C₁₋₆-Alkyl, N(C₁₋₆-Alkyl)-S(=O)₂-C₁₋₆-Alkyl, N(H)-C(=O)-O-C₁₋₆-Alkyl; O-C(=O)-NH₂, O-C(=O)-N(H)(C₁₋₆-Alkyl), O-C(=O)-N(C₁₋₆-Alkyl)₂, N(H)-C(=O)-NH₂, N(H)-C(=O)-N(H)(C₁₋₆-Alkyl), N(H)-C(=O)-N(C₁₋₆-Alkyl)₂, (C=O)C₁₋₆-Alkyl, C(=O)-NH₂, C(=O)-N(H)(C₁₋₆-Alkyl), C(=O)-N(C₁₋₆-Alkyl)₂, C(=O)-O-C₁₋₆-Alkyl; N(H)-C(=O)-C₁₋₆-Alkyl, und N(C₁₋₆-Alkyl)-C(=O)-C₁₋₆-Alkyl, wobei das 3-7-gliedrige Heterocyclyl optional über C₁₋₆-Alkylen verbunden ist, verzweigt oder unverzweigt, das wiederum unsubstituiert oder mit 1, 2, 3, 4 oder 5 Substituenten substituiert sein kann, unabhängig voneinander ausgewählt aus der Gruppe bestehend aus F, Cl, CF₃, =O, OCF₃, OH, O-C₁₋₆-Alkyl und C₁₋₆-Alkylen-OH;
oder Aryl oder Heteroaryl, das in jedem Fall unsubstituiert oder mit 1, 2, 3, 4 oder 5 Substituenten substituiert ist, unabhängig voneinander ausgewählt aus der Gruppe bestehend aus F, Cl, CN, CF₃, OCF₃, C₁₋₆-Alkylen-OH, C₁₋₆-Alkyl, OH, O-C₁₋₆-Alkyl, C-(C=O)C₁₋₆-Alkyl, S(=O)-C-₁₋₆-Alkyl, S(=O)₂-C₁₋₆-Alkyl, S(=O)₂-NH₂, S(=O)₂-N(H)C₁₋₆-Alkyl, S(=O)₂-N(C₁₋₆-Alkyl)₂, NH₂, NH(C₁₋₆-Alkyl), N(C₁₋₆-Alkyl)₂, N(H)-S(=O)-C₁₋₆-Alkyl, N(C₁₋₆-Alkyl)-S(=O)-C₁₋₆-Alkyl, N(H)-S(=O)₂-C₁₋₆-Alkyl, N(C₁₋₆-Alkyl)-S(=O)₂-C₁₋₆-Alkyl, N(H)-C(=O)-O-C₁₋₆-Alkyl; O-C(=O)-NH₂, O-C(=O)-N(H)(C₁₋₆-Alkyl), O-C(=O)-N(C₁₋₆-Alkyl)₂, N(H)-C(=O)-NH₂, N(H)-C(=O)-N(H)(C₁₋₆-Alkyl), N(H)-C(=O)-N(C₁₋₆-Alkyl)₂, C(=O)-NH₂, C(=O)-N(H)(C₁₋₆-Alkyl), C(=O)-N(C₁₋₆-Alkyl)₂, C(=O)-O-C₁₋₆-Alkyl; N(H)-C(=O)-C₁₋₆-Alkyl, und N(C₁₋₆-Alkyl)-C(=O)-C₁₋₆-Alkyl, wobei das Aryl oder Heteroaryl optional über C₁₋₆-Alkylen verbunden ist, verzweigt oder unverzweigt, das wiederum unsubstituiert oder mit 1, 2, 3, 4 oder 5 Substituenten substituiert sein kann, unabhängig voneinander ausgewählt aus der Gruppe bestehend aus F, Cl, CF₃, =O, OCF₃, OH, O-C₁₋₆-Alkyl und C₁₋₆-Alkylen-OH.

9. Verbindung nach einem oder mehreren der vorherigen Ansprüche, **dadurch gekennzeichnet, dass** R⁴ und R⁵ zusammen mit dem Stickstoffatom, das sie verbindet, ein 3-7-gliedriges Heterocyclyl bildet, unsubstituiert oder substituiert mit 1, 2, 3, 4 oder 5 Substituenten, ausgewählt aus der Gruppe bestehend aus F, Cl, CN, CF₃, =O, OH, C₁₋₆-Alkyl, O-C₁₋₆-Alkyl, C₁₋₆-Alkylen-OH, OCF₃, SO₂(C₁₋₆-Alkyl), SO₂NH₂, SO₂N(H)C₁₋₆-Alkyl, SO₂N(C₁₋₆-Alkyl)₂, C₁₋₆-Alkylen-SO₂(C₁₋₆-Alkyl), NH₂, NH(C₁₋₆-Alkyl), N(C₁₋₆-Alkyl)₂, (C=O)C₁₋₆-Alkyl, C₃₋₆-Cycloalkyl, 3- bis 7-gliedriges Heterocyclyl, Aryl, Heteroaryl, O-Aryl und O-Heteroaryl, in jedem Fall unsubstituiert oder mono- oder polysubstituiert.

10. Verbindung nach einem oder mehreren der vorherigen Ansprüche, **dadurch gekennzeichnet, dass**
R⁴ H oder C₁₋₆-Alkyl oder Benzyl darstellt; und
R⁵ darstellt:
C₃₋₆-Cycloalkyl, das unsubstituiert oder mit 1, 2, 3, 4 oder 5 Substituenten substituiert ist, unabhängig voneinander ausgewählt aus der Gruppe bestehend aus F, Cl, CF₃, =O, OCF₃, OH, O-C₁₋₆-Alkyl, C₁₋₆-Alkylen-OH C₁₋₆-Alkyl, C(=O)-NH₂, C(=O)-N(H)(C₁₋₆-Alkyl) und C(=O)-N(C₁₋₆-Alkyl)₂; oder
3-7-gliedriges Heterocyclyl, das 1 oder 2 Heteroatome oder Heteroatomgruppen enthält, unabhängig voneinander ausgewählt aus der Gruppe bestehend aus O, S, S(=O), S(=O)₂, NH und N-C₁₋₆-Alkyl, und das unsubstituiert oder mit 1, 2, 3, 4 oder 5 Substituenten substituiert ist, unabhängig voneinander ausgewählt aus der Gruppe bestehend aus F, Cl, CF₃, OCF₃, CN, =O, C₁₋₆-Alkyl, C₁₋₆-Alkylen-OH und O-C₁₋₆-Alkyl; oder Phenyl oder Heteroaryl, das mindestens ein Stickstoffatom enthält, in jedem Fall unsubstituiert oder mit 1, 2 oder 3 Substituenten substituiert, unabhängig voneinander ausgewählt aus der Gruppe bestehend aus F, Cl, CN, CF₃, OCF₃, C₁₋₆-Alkylen-OH, C₁₋₆-Alkyl, OH, O-C₁₋₆-Alkyl, S(=O)₂-C₁₋₆-Alkyl, S(=O)₂-NH₂, NH₂, NH(C₁₋₆-Alkyl), N(C₁₋₆-Alkyl)₂, O-C(=O)-NH₂, C(=O)-NH₂, C(O)-N(H)(C₁₋₆-Alkyl), C(=O)-N(C₁₋₆-Alkyl)₂, C(=O)-O-C₁₋₆-Alkyl; oder eine Teilstruktur der allgemeinen Formel SF-III 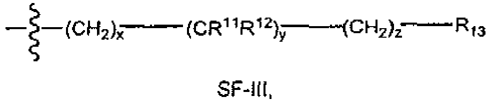 wobei
x 0, 1 oder 2 darstellt;
y 0, 1 oder 2 darstellt;
z 0, 1 oder 2 darstellt;
unter der Bedingung, dass die Summe von x, y und z 1, 2, 3, 4, 5 oder 6 ist;
R¹¹ und R¹² unabhängig voneinander ausgewählt sind aus H oder C₁₋₆-Alkyl; oder
R¹¹ und R¹² zusammen mit dem Kohlenstoffatom, das sie verbindet, ein C₃₋₆-Cycloalkyl oder ein 3-7-gliedriges Heterocyclyl bilden, das 1 oder 2 Heteroatome oder Heteroatomgruppen enthält, unabhängig voneinander ausgewählt aus der Gruppe bestehend aus O, S, S(=O), S(=O)₂, NH und N-C₁₋₆-Alkyl, wobei das C₃₋₆-Cycloalkyl oder 3-7-gliedrige Heterocyclyl unsubstituiert oder mit 1, 2, 3, 4 oder 5 Substituenten substituiert sein kann, unabhängig voneinander ausgewählt aus der Gruppe bestehend aus F, Cl, CF₃, OCF₃, CN, C₁₋₆-Alkyl und O-C₁₋₆-Alkyl;
R¹³ ausgewählt ist aus der Gruppe bestehend aus
H, F, Cl, CN, OH, O-C₁₋₆-Alkyl, O-(C=O)C₁₋₆-Alkyl, S(=O)-C₁₋₆-Alkyl, S(=O)₂-C₁₋₆-Alkyl, S(=O)₂- NH₂, S(=O)₂-N(H)C₁₋₆-Alkyl, S(=O)₂-N(C₁₋₆-Alkyl)₂, NH₂, NH(C₁₋₆-Alkyl), N(C₁₋₆-Alkyl)₂, N(H)-C(=O)-C₁₋₆-Alkyl, N(C₁₋₆-Alkyl)-C(=O)-C₁₋₆-Alkyl, N(H)-S(=O)-C₁₋₆-Alkyl, N(C₁₋₆-Alkyl)-S(=O)-C₁₋₆-Alkyl, N(H)-S(=O)₂-C₁₋₆-Alkyl, N(C₁₋₆-Alkyl)-S(=O)₂-C₁₋₆-Alkyl, C(=O)-NH₂, C(=O)-N(H)(C₁₋₆-Alkyl), C(=O)-N(C₁₋₆-Alkyl)₂, C(=O)-O-C₁₋₆-Alkyl, N(H)-C(=O)-NH₂, N(H)-C(=O)-N(H)(C₁₋₆-Alkyl), N(H)-C(=O)-N(C₁₋₆-Alkyl)₂, N(H)-C(=O)-O-C₁₋₆-Alkyl; O-C(=O)-NH₂, O-C(=O)-N(H)(C₁₋₆-Alkyl), O-C(=O)-N(C₁₋₆-Alkyl)₂; oder darstellt:
C₃₋₆-Cycloalkyl, das unsubstituiert oder mit 1, 2, 3, 4, oder 5 Substituenten substituiert ist, unabhängig voneinander ausgewählt aus der Gruppe bestehend aus F, Cl, CF₃, =O, OCF₃, OH, O-C₁₋₆-Alkyl, C₁₋₆-Alkylen-OH und C₁₋₆-Alkyl; oder
3-7-gliedriges Heterocyclyl, das 1 oder 2 Heteroatome oder Heteroatomgruppen enthält, unabhängig voneinander ausgewählt aus der Gruppe bestehend aus O, S, S(=O), S(=O)₂, NH und N-C₁₋₆-Alkyl, und das unsubstituiert oder mit 1, 2, 3, 4 oder 5 Substituenten substituiert ist, unabhängig voneinander ausgewählt aus der Gruppe bestehend aus F, Cl, CF₃, OCF₃, CN, C₁₋₆-Alkyl und O-C₁₋₆-Alkyl; oder
Phenyl oder Heteroaryl, das mindestens ein Stickstoffatom enthält, in jedem Fall unsubstituiert oder mit 1, 2 oder 3 Substituenten substituiert, unabhängig voneinander ausgewählt aus der Gruppe bestehend aus F, Cl, CN, CF₃, OCF₃, C₁₋₆-Alkylen-OH, C₁₋₆-Alkyl, OH, O-C₁₋₆-Alkyl, S(=O)₂-C₁₋₆-Alkyl, S(=O)₂-NH₂, NH₂, NH(C₁₋₆-Alkyl), N(C₁₋₆-Alkyl)₂, O-C(=O)-NH₂, C(=O)-NH₂, C(=O)-N(H)(C₁₋₆-Alkyl), C(=O)-N(C₁₋₆-Alkyl)₂, C(=O)-O-C₁₋₆-Alkyl; oder
R⁴ und R⁵ zusammen mit dem Stickstoffatom, das sie verbindet, ein Heterocyclyl bilden, ausgewählt aus der Gruppe bestehend aus 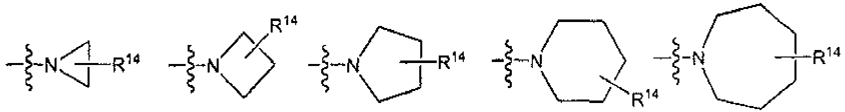 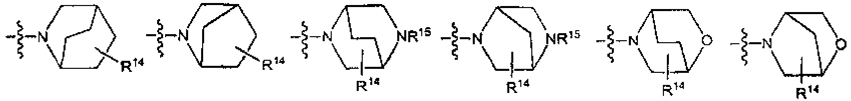 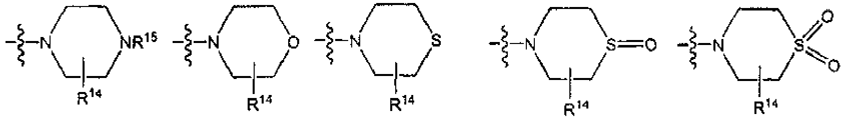 wobei
R¹⁴ 0, 1, 2, 3 oder 4 Substituenten bezeichnet, die in jedem Fall unabhängig voneinander ausgewählt sind aus der Gruppe bestehend aus F, Cl, CF₃, =O, OCF₃, OH, O-C₁₋₆-Alkyl, C₁₋₆-Alkylen-OH, C₁₋₆-Alkylen-SO₂(C₁₋₆-Alkyl), SO₂(C₁₋₆-Alkyl), C₁₋₆-Alkyl, Aryl, Heteroaryl, O-Aryl und O-Heteroaryl, wobei das Aryl oder das Heteroaryl unsubstituiert oder mit 1, 2 oder 3 Substituenten substituiert ist, unabhängig voneinander ausgewählt aus der Gruppe bestehend aus F, Cl, CN, CF₃, OCF₃, C₁₋₆-Alkylen-OH, C₁₋₆-Alkyl, OH, O-C₁₋₆-Alkyl, NH₂, NH(C₁₋₆-Alkyl), N(C₁₋₆-Alkyl)₂, N(H)-S(=O)₂-C₁₋₆-Alkyl, C(=O)-NH₂ oder C(=O)-N(H)(C₁₋₆-Alkyl), C(=O)-N(C₁₋₆-Alkyl)₂; oder
R¹⁴ mindestens zwei Substituenten bezeichnet, wobei zwei Substituenten R¹⁴ zusammen für eine C₁₋₆-Alkylen-Gruppe stehen, substituiert oder unsubstituiert, wobei optional ein oder mehrere C-Atome der C₁₋₆-Alkylen-Gruppe durch ein Heteroatom oder eine Heteroatom-Gruppe ersetzt sind, ausgewählt aus O, N-R¹⁵, S, S(O) und S(O)₂, und wobei diese zwei Substituenten R¹⁴ sind an verschiedenen Kohlenstoffatomen des Heterocyclyls positioniert sind, sodass die C₁₋₆-Alkylen-Gruppe eine Brücke darstellt, um ein bicyclisches Heterocyclyl zu bilden; oder
R¹⁴ mindestens zwei Substituenten bezeichnet, wobei zwei Substituenten R¹⁴ zusammen für eine C₂₋₆-Alkylen-Gruppe stehen, substituiert oder unsubstituiert, wobei optional ein oder mehrere C-Atome der C₂₋₆-Alkylen-Gruppe durch ein Heteroatom oder eine Heteroatom-Gruppe ersetzt sind, ausgewählt aus O, N-R¹⁵, S, S(O) und S(O)₂, und wobei diese zwei Substituenten R¹⁴ an demselben Kohlenstoffatom des Heterocyclyls positioniert sind, sodass die C₂₋₆-Alkylen-Gruppe ein Spiro-Heterocyclyl bildet; und R¹⁴ H, C₁₋₆-Alkyl, (C=O)C₁₋₆-Alkyl, (C=O)NH₂, (C=O)NH(C₁₋₆-Alkyl) oder (C=O)N(C₁₋₆-Alkyl)₂ darstellt.

11. Verbindung nach einem oder mehreren der vorherigen Ansprüche, **dadurch gekennzeichnet, dass** die Verbindung mit der allgemeinen Formel (I) eine Verbindung gemäß der allgemeinen Formel (la) ist, 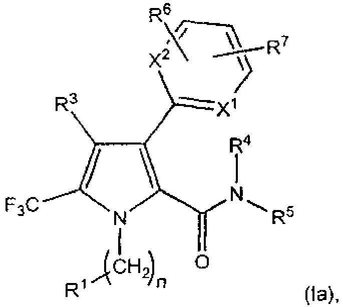 wobei n 0 oder 1 ist,
R¹ C₁₋₆-Alkyl, C₂₋₆-Alkenyl oder C₂₋₆-Alkynyl darstellt,
in jedem Fall verzweigt oder unverzweigt, und in jedem Fall unsubstituiert oder mono- oder polysubstituiert durch einen oder mehrere Substituenten ausgewählt aus F; Cl; CN; CF₃; C(=O)-NH₂; C(=O)-N(H)(C₁₋₆-Alkyl); C(=O)-N(C₁₋₆-Alkyl)₂; OH; O-C₁₋₆-Alkyl; NH₂; N(H)(C₁₋₆-Alkyl); N(C₁₋₆-Alkyl)₂; N(H)-C(=O)-C₁₋₆-Alkyl; S(=O)-C₁₋₆-Alkyl; S(=O)₂-C₁₋₆-Alkyl oder Cyclopropyl,
oder R¹ Cyclopropyl, Cyclobutyl, Cyclopentyl, Cyclohexyl, Oxetanyl, Azetidinyl, Tetrahydrofuranyl, Tetrahydropyranyl, Pyrrolidinyl, Piperidinyl und Piperazinyl darstellt,
in jedem Fall unsubstituiert oder mono- oder polysubstituiert durch einen oder mehrere Substituenten, ausgewählt aus F; Cl; CN; C₁₋₆-Alkyl; CF₃; OH; =O; O-C₁₋₆-Alkyl; O-C(=O)-C₁₋₆-Alkyl; O-S(=O)₂-C₁₋₆-Alkyl; NH₂; N(H)(C₁₋₆-Alkyl); N(C₁₋₆-Alkyl)₂; N(H)-C(=O)-C₁₋₆-Alkyl; N(H)-S(=O)₂-C₁₋₆-Alkyl oder S(=O)₂- C₁₋₆-Alkyl;
R³ H oder CH₃ oder Cyclopropyl darstellt;
X¹ N oder CH ist; X² N oder CH ist;
R⁶ und R⁷ unabhängig fehlen oder jeweils unabhängig voneinander ausgewählt sind aus der Gruppe bestehend aus F; Cl; CN; C₁₋₆-Alkyl; CF₃; CF₂H; CFH₂; OH; OCF₃; OCF₂H; OCFH₂ oder O-C₁₋₆-Alkyl;
R⁴ H oder C₁₋₆-Alkyl oder Benzyl darstellt; und R⁵ darstellt:
C₃₋₆-Cycloalkyl, das unsubstituiert oder mit 1, 2, 3, 4 oder 5 Substituenten substituiert ist, unabhängig voneinander ausgewählt aus der Gruppe bestehend aus F, Cl, CF₃, =O, OCF₃, OH, O-C₁₋₆-Alkyl, C₁₋₆-Alkylen-OH, C₁₋₆-Alkyl, C(=O)-NH₂, C(=O)-N(H)(C₁₋₆-Alkyl) und C(=O)- N(C₁₋₆-Alkyl)₂; oder
5- oder 6-gliedriges Heterocyclyl, das 1 oder 2 Heteroatome oder Heteroatomgruppen enthält, unabhängig voneinander ausgewählt aus der Gruppe bestehend aus O, S, S(=O), S(=O)₂, NH und N-C₁₋₆-Alkyl, und das unsubstituiert oder mit 1, 2, 3, 4 oder 5 Substituenten substituiert ist, unabhängig voneinander ausgewählt aus der Gruppe bestehend aus F, Cl, CF₃, OCF₃, CN, =O, C₁₋₆-Alkyl, C₁₋₆-Alkylen-OH und O-C₁₋₆-Alkyl; oder eine Teilstruktur der allgemeinen Formel SF-III 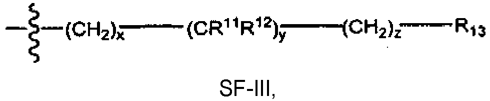 wobei
x 1 darstellt und y und z jeweils 0 darstellen, oder
x und y jeweils 1 darstellen und z 0 darstellt, oder
x und z jeweils 1 darstellen und y 0 darstellt, oder
x, y und z jeweils 1 darstellen;
R¹¹ und R¹⁴ unabhängig voneinander ausgewählt sind aus H oder CH₃;
R¹³ ausgewählt ist aus der Gruppe bestehend aus
H, F, Cl, CN, OH, O-C₁₋₆-Alkyl, O-(C=O)C₁₋₆-Alkyl, S(=O)-C₁₋₆-Alkyl, S(=O)₂-C₁₋₆-Alkyl, NH₂, NH(C₁₋₆-Alkyl), N(C₁₋₆-Alkyl)₂, N(H)-C(=O)-C₁₋₆-Alkyl, N(H)-S(=O)₂-C₁₋₆-Alkyl, C(=O)-NH₂, C(=O)-N(H)(C₁₋₆-Alkyl), C(=O)-N(C₁₋₆-Alkyl)₂, N(H)-C(=O)-NH₂, N(H)-C(=O)-N(H)(C₁₋₆-Alkyl), N(H)-C(=O)-N(C₁₋₆-Alkyl)₂,
oder darstellt:
C₃₋₆-Cycloalkyl oder
3-7-gliedriges Heterocyclyl, das 1 oder 2 Heteroatome oder Heteroatomgruppen enthält, unabhängig voneinander ausgewählt aus der Gruppe bestehend aus O, S, S(=O), S(=O)₂, NH und N-C₁₋₆-Alkyl, und das unsubstituiert oder mit 1, 2, 3, 4 oder 5 Substituenten substituiert ist, unabhängig voneinander ausgewählt aus der Gruppe bestehend aus F, Cl, CF₃, OCF₃, CN, C₁₋₆-Alkyl und O-C₁₋₆-Alkyl; oder
Phenyl oder Heteroaryl, ausgewählt aus Pyrrolyl, Pyrazolyl, Imidazolyl, Oxazolyl, Isoxazolyl, Thiazoiyl, Isothiazolyl, Pyridinyl, Pyrazinyl, Pyrimidinyl und Pyridazinyl,
in jedem Fall unsubstituiert oder mit 1, 2 oder 3 Substituenten substituiert, unabhängig voneinander ausgewählt aus der Gruppe bestehend aus F, Cl, CN, CF₃, OCF₃, C₁₋₆-Alkylen-OH, C₁₋₆-Alkyl, OH, O-C₁₋₆-Alkyl, S(=O)₂-C₁₋₆-Alkyl, S(=O)₂-NH₂, NH₂, NH(C₁₋₆-Alkyl), N(C₁₋₆-Alkyl)₂, O-C(=O)-NH₂, C(=O)-NH₂, C(=O)-N(H)(C₁₋₆-Alkyl), C(=O)-N(C₁₋₆-Alkyl)₂, C(=O)-O-C₁₋₆-Alkyl; oder
R⁴ und R⁵ zusammen mit dem Stickstoffatom, das sie verbindet, ein Heterocyclyl bilden, ausgewählt aus der Gruppe bestehend aus 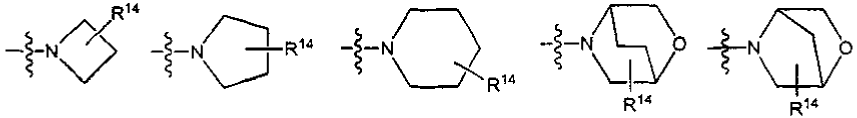 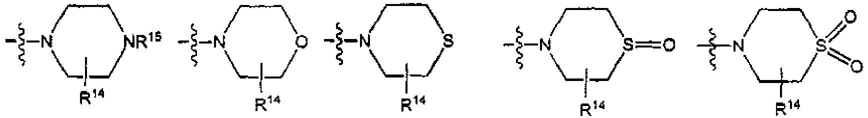 wobei
R¹⁴ 0, 1 oder 2 Substituenten bezeichnet, die in jedem Fall unabhängig voneinander ausgewählt sind aus der Gruppe bestehend aus F, Cl, CF₃, =O, OCF₃, OH, O-C₁₋₆-Alkyl, C₁₋₆-Alkylen-OH, C₁₋₆-Alkylen-SO₂(C₁₋₆-Alkyl), SO₂(C₁₋₆-Alkyl), C₁₋₆-Alkyl, Aryl, Heteroaryl, O-Aryl und O-Heteroaryl, wobei das Aryl oder das Heteroaryl unsubstituiert oder mit 1, 2 oder 3 Substituenten substituiert ist, unabhängig voneinander ausgewählt aus der Gruppe bestehend aus F, Cl, CN, CF₃, OCF₃, C₁₋₆-Alkylen-OH, C₁₋₆-Alkyl, OH oder O-C₁₋₆-Alkyl; und
R¹⁵ H, C₁₋₆-Alkyl, (C=O)C₁₋₆-Alkyl, (C=O)NH₂, (C=O)NH(C₁₋₆-Alkyl) oder (C=O)N(C₁₋₆-Alkyl)₂ darstellt.

12. Verbindung nach einem oder mehreren der vorherigen Ansprüche, ausgewählt aus der Gruppe bestehend aus
| | |
|---|---|
| **001** | N-Benzyl-3-(4-chlorphenyl)-1,4-dimethyl-N-tetrahydro-pyran-4-yl-5-(trifluormethyl)-1H-pyrrol-2-carboxylsäureamid |
| **002** | 3-(4-Chlorphenyl)-N-(2,2-dimethyl-propyl)-N,1,4-trimethyl-5-(trifluormethyl)-1H-pyrrol-2-carboxylsäureamid |
| **003** | N-Benzyl-3-(4-chlorphenyl)-N-(2,2-dimethyl-propyl)-1,4-dimethyl-5-(trifluormethyl)-1H-pyrrol-2-carboxylsäureamid |
| **004** | [3-(4-Chlorphenyl)-1,4-dimethyl-5-(trifluormethyl)-1H-pyrrol-2-yl]-morpholin-4-yl-methanon |
| **005** | 3-(4-Chlorphenyl)-N-(2,2-dimethyl-propyl)-1-ethyl-N,4-dimethyl-5-(trifluormethyl)-1H-pyrrol-2-carboxylsäureamid |
| **006** | [3-(4-Chlorphenyl)-1-isopropyl-4-methyl-5-(trifluormethyl)-1H-pyrrol-2-yl]-(2,2-dimethyl-morpholin-4-yl)-methanon |
| **007** | N-(1-Carbamoyl-cyclopropyl)-3-(4-chlorphenyl)-1-cyclopropyl-N,4-dimethyl-5-(trifluormethyl)-1H-pyrrol-2-carboxylsäureamid |
| **008** | 3-(4-Chlorphenyl)-1-cyclopropyl-N-(1,1-dioxo-thian-4-yl)-N,4-dimethyl-5-(trifluormethyl)-1H-pyrrol-2-carboxylsäureamid |
| **009** | N-(2-Carbamoyl-2-methyl-propyl)-3-(4-chlorphenyl)-1-cyclopropyl-N,4-dimethyl-5-(trifluormethyl)-1H-pyrrol-2-carboxylsäureamid |
| **010** | 3-(4-Chlorphenyl)-1-cyclopropyl-N-[(4-methoxyphenyl)-methyl]-N,4-dimethyl-5-(trifluormethyl)-1H-pyrrol-2-carboxylsäureamid |
| **011** | 3-(4-Chlorphenyl)-1-cyclopropyl-N-[(4-methoxyphenyl)-methyl]-N-methyl-5-(trifluormethyl)-1H-pyrrol-2-carboxylsäureamid |
| **012** | 3-(4-Chlorphenyl)-N,1-dicyclopropyl-N,4-dimethyl-5-(trifluormethyl)-1H-pyrrol-2-carboxylsäureamid |
| **013** | 3-(4-Chlorphenyl)-N,1-dicyclopropyl-N-methyl-5-(trifluormethyl)-1H-pyrrol-2-carboxylsäureamid |
| **014** | 3-(4-Chlorphenyl)-1-cyclopropyl-N,4-dimethyl-N-tetrahydro-pyran-4-yl-5-(trifluormethyl)-1H-pyrrol-2-carboxylsäureamid |
| **015** | 3-(4-Chlorphenyl)-1-cyclopropyl-N-methyl-N-tetrahydro-pyran-4-yl-5-(trifluormethyl)-1H-pyrrol-2-carboxylsäureamid |
| **016** | 3-(4-Chlorphenyl)-1-cyclopropyl-N-(2,2-dimethyl-3-morpholin-4-yl-propyl)-N,4-dimethyl-5-(trifluormethyl)-1H-pyrrol-2-carboxylsäureamid |
| **017** | 3-(4-Chlorphenyl)-1-cyclopropyl-N-(2,2-dimethyl-3-morpholin-4-yl-propyl)-N-methyl-5-(trifluormethyl)-1H-pyrrol-2-carboxylsäureamid |
| **018** | 3-(4-Chlorphenyl)-1-cyclopropyl-N-(2-hydroxy-2-methyl-propyl)-N,4-dimethyl-5-(trifluormethyl)-1H-pyrrol-2-carboxylsäureamid |
| **019** | 3-(4-Chlorphenyl)-1-cyclopropyl-N-(2-hydroxy-2-methyl-propyl)-N-methyl-5-(trifluormethyl)-1H-pyrrol-2-carboxylsäureamid |
| **020** | 3-(4-Chlor-2-fluor-phenyl)-1-cyclopropyl-N,4-dimethyl-N-[(5-methyl-isoxazol-3-yl)-methyl]-5-(trifluormethyl)-1H-pyrrol-2-carboxylsäureamid |
| **021** | 3-(4-Chlor-2-fluor-phenyl)-1-cyclopropyl-N,4-dimethyl-N-(pyrimidin-4-yl-methyl)-5-(trifluormethyl)-1H-pyrrol-2-carboxylsäureamid |
| **022** | 3-(4-Chlorphenyl)-1-cyclopropyl-N,4-dimethyl-N-(tetrahydro-furan-3-yl-methyl)-5-(trifluormethyl)-1H-pyrrol-2-carboxylsäureamid |
| **023** | 3-(4-Chlorphenyl)-1-cyclopropyl-N-methyl-N-(tetrahydro-furan-3-yl-methyl)-5-(trifluormethyl)-1H-pyrrol-2-carboxylsäureamid |
| **024** | 3-(4-Chlor-2-fluor-phenyl)-1-cyclopropyl-N,4-dimethyl-N-[(5-methyl-pyrazin-2-yl)-methyl]-5-(trifluormethyl)-1H-pyrrol-2-carboxylsäureamid |
| **025** | [3-(4-Chlorphenyl)-1-cyclopropyl-4-methyl-5-(trifluormethyl)-1H-pyrrol-2-yl]-(3-hydroxy-azetidin-1-yl)-methanon |
| **026** | [3-(4-Chlorphenyl)-1-cyclopropyl-5-(trifluormethyl)-1H-pyrrol-2-yl]-(3-hydroxy-azetidin-1-yl)-methanon |
| **027** | [3-(4-Chlorphenyl)-1-cyclopropyl-4-methyl-5-(trifluormethyl)-1H-pyrrol-2-yl]-[4-(3-methyl-[1,2,4]oxadiazol-5-yl)-piperidin-1-yl]-methanon |
| **028** | [3-(4-Chlor-2-fluor-phenyl)-1-cyclopropyl-4-methyl-5-(trifluormethyl)-1H-pyrrol-2-yl]-[4-(3-methyl-[1,2,4]oxadiazol-5-yl)-piperidin-1-yl]-methanon |
| **029** | [3-(4-Chlorphenyl)-1-cyclopropyl-5-(trifluormethyl)-1H-pyrrol-2-yl]-[4-(3-methyl-[1,2,4]oxadiazol-5-yl)-piperidin-1-yl]-methanon |
| **030** | [3-(4-Chlorphenyl)-1-cyclopropyl-4-methyl-5-(trifluormethyl)-1H-pyrrol-2-yl]-[4-[5-(trifluormethyl)-pyridin-2-yl]oxy-piperidin-1-yl]-methanon |
| **031** | [3-(4-Chlorphenyl)-1-cyclopropyl-5-(trifluormethyl)-1H-pyrrol-2-yl]-[4-[5-(trifluormethyl)-pyridin-2-yl]oxy-piperidin-1-yl]-methanon |
| **032** | [3-(4-Chlorphenyl)-1-cyclopropyl-4-methyl-5-(trifluormethyl)-1H-pyrrol-2-yl]-(2,2-dimethyl-morpholin-4-yl)-methanon |
| **033** | [3-(4-Chlor-2-fluor-phenyl)-1-cyclopropyl-4-methyl-5-(trifluormethyl)-1H-pyrrol-2-yl]-(2,2-dimethyl-morpholin-4-yl)-methanon |
| **034** | [3-(4-Chlorphenyl)-1-cyclopropyl-5-(trifluormethyl)-1H-pyrrol-2-yl]-(2,2-dimethyl-morpholin-4-yl)-methanon |
| **035** | 3-(4-Chlorphenyl)-1-cyclopropyl-N-(2,2-dimethyl-propyl)-N-methyl-5-(trifluormethyl)-1H-pyrrol-2-carboxylsäureamid |
| **036** | 3-(4-Chlorphenyl)-1-cyclopropyl-N-(3-hydroxy-2,2-dimethyl-propyl)-N-methyl-5-(trifluormethyl)-1H-pyrrol-2-carboxylsäureamid |
| **037** | 3-(4-Chlorphenyl)-1-Cyclopropyl-N-methyl-N-(2-methylsulfonyl-ethyl)-5-(trifluormethyl)-1H-pyrrol-2-carboxylsäureamid |
| **038** | [3-(4-Chlorphenyl)-1-cyclopropyl-5-(trifluormethyl)-1H-pyrrol-2-yl]-morpholin-4-yl-methanon |
| **039** | 3-(4-Chlorphenyl)-1-cyclopropyl-N-(2,2-dimethyl-propyl)-N,4-dimethyl-5-(trifluormethyl)-1H-pyrrol-2-carboxylsäureamid |
| **040** | 3-(4-Chlorphenyl)-1-cyclopropyl-N-(3-hydroxy-2,2-dimethyl-propyl)-N,4-dimethyl-5-(trifluormethyl)-1H-pyrrol-2-carboxylsäureamid |
| **041** | 3-(4-Chlorphenyl)-1-cyclopropyl-N,4-dimethyl-N-(2-methylsulfonyl-ethyl)-5-(trifluormethyl)-1H-pyrrol-2-carboxylsäureamid |
| **042** | [3-(4-Chlorphenyl)-1-cyclobutyl-4-methyl-5-(trifluormethyl)-1H-pyrrol-2-yl]-(2,2-dimethyl-morpholin-4-yl)-methanon |
| **043** | [3-(4-Chlorphenyl)-1-cyclopropyl-4-methyl-5-(trifluormethyl)-1H-pyrrol-2-yl]-morpholin-4-yl-methanon |
| **044** | [3-(4-Chlorphenyl)-1-Cyclopropyl-4-methyl-5-(trifluormethyl)-1H-pyrrol-2-yl]-(2,2-dimethyl-1,1-dioxo-[1,4]thiazinan-4-yl)-methanon |
| **045** | (2,2-Dimethyl-morpholin-4-yl)-[3-(4-fluorphenyl)-1-(2-methyl-propyl)-5-(trifluormethyl)-1H-pyrrol-2-yl]-methanon |
| **046** | 3-(4-Fluorphenyl)-N-methyl-1-(2-methyl-propyl)-N-tetrahydro-pyran-4-yl-5-(trifluormethyl)-1H-pyrrol-2-carboxylsäureamid |
| **047** | 3-(4-Fluorphenyl)-N-methyl-N-[(5-methyl-isoxazol-3-yl)-methyl]-1-(2-methyl-propyl)-5-(trifluormethyl)-1H-pyrrol-2-carboxylsäureamid |
| **048** | 3-(4-Chlorphenyl)-N,4-dimethyl-1-(2-methyl-propyl)-N-(pyrimidin-4-yl-methyl)-5-(trifluormethyl)-1H-pyrrol-2-carboxylsäureamid |
| **049** | 3-(4-Fluorphenyl)-N-methyl-1-(2-methyl-propyl)-N-[(5-methyl-pyrazin-2-yl)-methyl]-5-(trifluormethyl)-1H-pyrrol-2-carboxylsäureamid |
| **050** | 3-(4-Fluorphenyl)-N-methyl-1-(2-methyl-propyl)-N-(2-methylsulfonyl-ethyl)-5-(trifluormethyl)-1H-pyrrol-2-carboxylsäureamid |
| **051** | N-(1-Carbamoyl-cyclopropyl)-3-(4-chlorphenyl)-N,4-dimethyl-1-(2-methyl-propyl)-5-(trifluormethyl)-1H-pyrrol-2-carboxylsäureamid |
| **052** | N-(2-Carbamoyl-2-methyl-propyl)-3-(4-chlorphenyl)-N,4-dimethyl-1-(2-methyl-propyl)-5-(trifluormethyl)-1H-pyrrol-2-carboxylsäureamid |
| **053** | N-(2,2-Dimethyl-propyl)-3-(4-fluorphenyl)-N-methyl-1-(2-methyl-propyl)-5-(trifluormethyl)-1H-pyrrol-2-carboxylsäureamid |
| **054** | N-(2,2-Dimethyl-3-morpholin-4-yl-propyl)-3-(4-fluorphenyl)-N-methyl-1-(2-methyl-propyl)-5-(trifluormethyl)-1H-pyrrol-2-carboxylsäureamid |
| **055** | 3-(4-Chlorphenyl)-N-(3-hydroxy-2,2-dimethyl-propyl)-N,4-dimethyl-1-(2-methyl-propyl)-5-(trifluormethyl)-1H-pyrrol-2-carboxylsäureamid |
| **056** | 3-(4-Fluorphenyl)-N-(3-hydroxy-2,2-dimethyl-propyl)-N-methyl-1-(2-methyl-propyl)-5-(trifluormethyl)-1H-pyrrol-2-carboxylsäureamid |
| **057** | 3-(4-Chlorphenyl)-N,4-dimethyl-N-[(5-methyl-isoxazol-3-yl)-methyl]-1-(2-methyl-propyl)-5-(trifluormethyl)-1H-pyrrol-2-carboxylsäureamid |
| **058** | 3-(4-Fluorphenyl)-N-methyl-1-(2-methyl-propyl)-N-(tetrahydro-furan-3-yl-methyl)-5-(trifluormethyl)-1H-pyrrol-2-carboxylsäureamid |
| **059** | 3-(4-Chlorphenyl)-N,4-dimethyl-1-(2-methyl-propyl)-N-[(5-methyl-pyrazin-2-yl)-methyl]-5-(trifluormethyl)-1H-pyrrol-2-carboxylsäureamid |
| **060** | N-[(2-Dimethylamino-pyrimidin-5-yl)-methyl]-3-(4-fluorphenyl)-N-methyl-1-(2-methyl-propyl)-5-(trifluormethyl)-1H-pyrrol-2-carboxylsäureamid |
| **061** | 3-(4-Chlorphenyl)-N,4-dimethyl-1-(2-methyl-propyl)-N-(2-methylsulfonyl-ethyl)-5-(trifluormethyl)-1H-pyrrol-2-carboxylsäureamid |
| **062** | [3-(4-Chlorphenyl)-4-methyl-1-(2-methyl-propyl)-5-(trifluormethyl)-1H-pyrrol-2-yl]-[4-(3-methyl-[1,2,4]oxadiazol-5-yl)-piperidin-1-yl]-methanon |
| **063** | 4-[3-(4-Fluorphenyl)-1-(2-methyl-propyl)-5-(trifluormethyl)-1H-pyrrol-2-carbonyl]-piperazin-2-on |
| **064** | [3-(4-Chlorphenyl)-4-methyl-1-(2-methyl-propyl)-5-(trifluormethyl)-1H-pyrrol-2-yl]-(2,2-dimethyl-morpholin-4-yl)-methanon |
| **065** | [3-(4-Fluorphenyl)-1-(2-methyl-propyl)-5-(trifluormethyl)-1H-pyrrol-2-yl]-morpholin-4-yl-methanon |
| **066** | N-Cyclopropyl-3-(4-fluorphenyl)-1-(2-methyl-propyl)-5-(trifluormethyl)-1H-pyrrol-2-carboxylsäureamid |
| **067** | 3-(4-Chlorphenyl)-N-(2-cyano-2-methyl-propyl)-N,4-dimethyl-1-(2-methyl-propyl)-5-(trifluormethyl)-1H-pyrrol-2-carboxylsäureamid |
| **068** | 3-(4-Chlorphenyl)-N,4-dimethyl-1-[(1-methyl-Cyclopropyl)-methyl]-N-(2-methylsulfonyl-ethyl)-5-(trifluormethyl)-1H-pyrrol-2-carboxylsäureamid |
| **069** | N-[(2-Dimethylamino-pyrimidin-5-yl)-methyl]-3-(4-fluorphenyl)-N-methyl-1-(tetrahydro-furan-2-yl-methyl)-5-(trifluormethyl)-1H-pyrrol-2-carboxylsäureamid |
| **070** | 3-(4-Fluorphenyl)-N-methyl-1-(tetrahydro-furan-2-yl-methyl)-N-tetrahydro-pyran-4-yl-5-(trifluormethyl)-1H-pyrrol-2-carboxylsäureamid |
| **071** | N-(1,1-Dioxo-thiolan-3-yl)-3-(4-fluorphenyl)-N-methyl-1-(tetrahydro-furan-2-yl-methyl)-5-(trifluormethyl)-1H-pyrrol-2-carboxylsäureamid |
| **072** | N-(2-Carbamoyl-2-methyl-propyl)-3-(4-fluorphenyl)-N-methyl-1-(tetrahydro-furan-2-yl-methyl)-5-(trifluormethyl)-1H-pyrrol-2-carboxylsäureamid |
| **073** | N-(2,2-Dimethyl-propyl)-3-(4-fluorphenyl)-N-methyl-1-(tetrahydro-furan-2-yl-methyl)-5-(trifluormethyl)-1H-pyrrol-2-carboxylsäureamid |
| **074** | 3-(4-Fluorphenyl)-N-(3-hydroxy-2,2-dimethyl-propyl)-N-methyl-1-(tetrahydro-furan-2-yl-methyl)-5-(trifluormethyl)-1H-pyrrol-2-carboxylsäureamid |
| **075** | 3-(4-Fluorphenyl)-N-methyl-N-[(5-methyl-isoxazol-3-yl)-methyl]-1-(tetrahydro-furan-2-yl-methyl)-5-(trifluormethyl)-1H-pyrrol-2-carboxylsäureamid |
| **076** | 3-(4-Fluorphenyl)-N-methyl-N,1-bis(tetrahydro-furan-2-yl-methyl)-5-(trifluormethyl)-1H-pyrrol-2-carboxylsäureamid |
| **077** | 3-(4-Fluorphenyl)-N-methyl-N-[(5-methyl-pyrazin-2-yl)-methyl]-1-(tetrahydro-furan-2-yl-methyl)-5-(trifluormethyl)-1H-pyrrol-2-carboxylsäureamid |
| **078** | 3-(4-Fluorphenyl)-N-methyl-N-(2-methylsulfonyl-ethyl)-1-(tetrahydro-furan-2-yl-methyl)-5-(trifluormethyl)-1H-pyrrol-2-carboxylsäureamid |
| **079** | 4-[3-(4-Fluorphenyl)-1-(tetrahydro-furan-2-yl-methyl)-5-(trifluormethyl)-1H-pyrrol-2-carbonyl]-piperazin-2-on |
| **080** | (2,2-Dimethyl-morpholin-4-yl)-[3-(4-fluorphenyl)-1-(tetrahydro-furan-2-yl-methyl)-5-(trifluormethyl)-1H-pyrrol-2-yl]-methanon |
| **081** | [3-(4-Fluorphenyl)-1-(tetrahydro-furan-2-yl-methyl)-5-(trifluormethyl)-1H-pyrrol-2-yl]-morpholin-4-yl-methanon |
| **082** | N-Cyclopropyl-3-(4-fluorphenyl)-1-(tetrahydro-furan-2-yl-methyl)-5-(trifluormethyl)-1H-pyrrol-2-carboxylsäureamid |
| **083** | 3-(4-Chlorphenyl)-N-(2,2-dimethyl-propyl)-N-methyl-1-(tetrahydro-furan-2-yl-methyl)-5-(trifluormethyl)-1H-pyrrol-2-carboxylsäureamid |
| **084** | [3-(4-Chlorphenyl)-1-(cyclopropyl-methyl)-4-methyl-5-(trifluorrnethyl)-1H-pyrrol-2-yl]-(2,2-dimethyl-morpholin-4-yl)-methanon |
| **085** | [3-(4-Chlorphenyl)-1-(cyclobutyl-methyl)-4-methyl-5-(trifluormethyl)-1H-pyrrol-2-yl]-(2,2-dimethyl-morpholin-4-yl)-methanon |
| **086** | [3-(4-Chlorphenyl)-1-(3-cyclopropyl-prop-2-ynyl)-4-methyl-5-(trifluormethyl)-1H-pyrrol-2-yl]-morpholin-4-yl-methanon |
| **087** | [3-(4-Chlorphenyl)-4-methyl-1-[(1-methyl-cyclopropyl)-methyl]-5-(trifluormethyl)-1H-pyrrol-2-yl]-morpholin-4-yl-methanon |
| **088** | 1-[(1-Amino-cyclopropyl)-methyl]-3-(4-chlorphenyl)-N-(2,2-dimethyl-propyl)-N-methyl-5-(trifluormethyl)-1H-pyrrol-2-carboxylsäureamid |
| **089** | [3-(4-Chlorphenyl)-1-[(1-hydroxy-cyclopropyl)-methyl]-4-methyl-5-(trifluormethyl)-1H-pyrrol-2-yl]-morpholin-4-yl-methanon |
| **090** | 3-(4-Chlorphenyl)-N-(2,2-dimethyl-propyl)-1-[(1-hydroxy-cyclopentyl)-methyl]-N-methyl-5-(trifluormethyl)-1H-pyrrol-2-carboxylsäureamid |
| **091** | 3-(4-Chlorphenyl)-N-(2,2-dimethyl-propyl)-N-methyl-1-(pyrrolidin-2-yl-methyl)-5-(trifiuoromethyl)-1H-pyrrol-2-carboxylsäureamid |
| **092** | 3-(4-Chlorphenyl)-N-(2,2-dimethyl-propyl)-N-methyl-1-(tetrahydro-pyran-4-yl-methyl)-5-(trifluormethyl)-1H-pyrrol-2-carboxylsäureamid |
| **093** | 1-[(1-Amino-cyclopropyl)-methyl]-3-(4-chlorphenyl)-N,4-dimethyl-N-(2-methylsulfonyl-ethyl)-5-(trifluormethyl)-1H-pyrrol-2-carboxylsäureamid |
| **094** | 3-(4-Chlorphenyl)-1-[(1-hydroxy-cyclopentyl)-methyl]-N,4-dimethyl-N-(2-methylsulfonyl-ethyl)-5-(trifluormethyl)-1H-pyrrol-2-carboxylsäureamid |
| **095** | [1-[(1-Amino-cyclopropyl)-methyl]-3-(4-chlorphenyl)-4-methyl-5-(trifluormethyl)-1H-pyrrol-2-yl]-morpholin-4-yl-methanon |
| **096** | [3-(4-Chlorphenyl)-1-[(1-hydroxy-cyclopentyl)-methyl]-4-methyl-5-(trifluormethyl)-1H-pyrrol-2-yl]-morpholin-4-yl-methanon |
| **097** | [3-(4-Chlorphenyl)-4-methyl-1-(tetrahydro-pyran-4-yl-methyl)-5-(trifluormethyl)-1H-pyrrol-2-yl]-morpholin-4-yl-methanon |
| **098** | [3-(4-Chlorphenyl)-1-[2-(3,3-difluor-azetidin-1-yl)-ethyl]-4-methyl-5-(trifluormethyl)-1H-pyrrol-2-yl]-morpholin-4-yl-methanon |
| **099** | [3-(4-Chlorphenyl)-4-methyl-1-(2-methylsulfonyl-ethyl)-5-(trifluormethyl)-1H-pyrrol-2-yl]-morpholin-4-yl-methanon |
| **100** | 3-(4-Chlorphenyl)-1-[(1-cyano-cyclopropyl)-methyl)-N-(2,2-dimethyl-propyl)-N-methyl-5-(trifluormethyl)-1H-pyrrol-2-carboxylsäureamid |
| **101** | 3-(4-Chlorphenyl)-N-(2,2-dimethyl-propyl)-1-[(1-hydroxy-cyclobutyl)-methyl]-N-methyl-5-(trifluormethyl)-1H-pyrrol-2-carboxylsäureamid |
| **102** | [3-(4-Chlorphenyl)-1,4-dimethyl-5-(trifluormethyl)-1H-pyrrol-2-yl]-(2,2-dimethyl-morpholin-4-yl)-methanon |
| **103** | [3-(4-Chlorphenyl)-1-ethyl-4-methyl-5-(trifluormethyl)-1H-pyrrol-2-yl]-(2,2-dimethyl-morpholin-4-yl)-methanon |
| **104** | [3-(4-Chlorphenyl)-4-methyl-1-(2-methyl-propyl)-5-(trifluormethyl)-1H-pyrrol-2-yl]-(2-oxa-5-azabicyclo[2.2.1]heptan-5-yl)-methanon |
| **105** | N-(2-Carbamoyl-2-methyl-propyl)-3-(4-chlorphenyl)-1-isopropyl-N,4-dimethyl-5-(trifluormethyl)-1H-pyrrol-2-carboxylsäureamid |
| **106** | 3-(4-Chlorphenyl)-N-(2-cyano-2-methyl-propyl)-1-isopropyl-N,4-dimethyl-5-(trifluormethyl)-1H-pyrrol-2-carboxylsäureamid |
| **107** | 3-(4-Chlorphenyl)-1-isopropyl-N,4-dimethyl-N-[(5-methyl-isoxazol-3-yl)-methyl]-5-(trifluormethyl)-1H-pyrrol-2-carboxylsäureamid |
| **108** | 3-(4-Chlorphenyl)-N,1-diisopropyl-N,4-dimethyl-5-(trifluormethyl)-1H-pyrrol-2-carboxylsäureamid |
| **109** | 3-(4-Chlorphenyl)-N-isopropyl-N,4-dimethyl-1-(2-methyl-propyl)-5-(trifluormethyl)-1H-pyrrol-2-carboxylsäu reamid |
| **110** | 3-(4-Chlorphenyl)-1-isopropyl-N,4-dimethyl-N-(2-methylsulfonyl-ethyl)-5-(trifluormethyl)-1H-pyrrol-2-carboxylsäureamid |
| **111** | 3-(4-Chlorphenyl)-1-isopropyl-N,4-dimethyl-N-[(2-methyl-2H-pyrazol-3-yl)-methyl]-5-(trifluormethyl)-1H-pyrrol-2-carboxylsäureamid |
| **112** | [3-(5-Chlor-pyridin-2-yl)-1-isopropyl-4-methyl-5-(trifluormethyl)-1H-pyrrol-2-yl]-(2,2-dimethyl-morpholin-4-yl)-methanon |
| **113** | 3-(5-Chlor-pyridin-2-yl)-1-isopropyl-N,4-dimethyl-N-[(5-methyl-isoxazol-3-yl)-methyl]-5-(trifluormethyl)-1H-pyrrol-2-carboxylsäureamid |
| **114** | 3-(5-Chlor-pyridin-2-yl)-1-isopropyl-N,4-dimethyl-N-[(2-methyl-2H-pyrazol-3-yl)-methyl]-5-(trifluormethyl)-1H-pyrrol-2-carboxylsäureamid |
| **115** | 3-(4-Chlorphenyl)-1-isopropyl-N,4-dimethyl-N-(2-oxo-pyrrolidin-3-yl)-5-(trifluormethyl)-1H-pyrrol-2-carboxylsäureamid |
| **116** | N-(2-Carbamoyl-2-methyl-propyl)-3-(5-chlor-pyrid in-2-yl)-1-isopropyl-N,4-d imethyl-5-(trifluormethyl)-1H-pyrrol-2-carboxylsäureamid |
| **117** | 3-(5-Chlor-pyridin-2-yl)-N-(2-cyano-2-methyl-propyl)-1-isopropyl-N,4-dimethyl-5-(trifluormethyl)-1H-pyrrol-2-carboxylsäureamid |
| **118** | 3-(5-Chlor-pyridin-2-yl)-1-isopropyl-N,4-dimethyl-N-(2-methylsulfonyl-ethyl)-5-(trifluormethyl)-1H-pyrrol-2-carboxylsäureamid |
| **119** | 3-(5-Chlor-pyridin-2-yl)-1-isopropyl-N,4-dimethyl-N-(2-oxo-pyrrolidin-3-yl)-5-(trifluormethyl)-1H-pyrrol-2-carboxylsäureamid |
| **120** | 3-(5-Chlor-pyridin-2-yl)-N,1-diisopropyl-N,4-dimethyl-5-(trifluormethyl)-1H-pyrrol-2-carboxylsäureamid |
| **121** | [3-(5-Chlor-pyrimidin-2-yl)-1-isopropyl-4-methyl-5-(trifluormethyl)-1H-pyrrol-2-yl]-(2,2-dimethyl-morpholin-4-yl)-methanon |
| **122** | 3-(5-Chlor-pyrimidin-2-yl)-1-isopropyl-N,4-dimethyl-N-[(2-methyl-2H-pyrazol-3-yl)-methyl]-5-(trifluormethyl)-1H-pyrrol-2-carboxylsäureamid |
| **123** | 3-(5-Chlor-pyrimidin-2-yl)-1-isopropyl-N,4-dimethyl-N-[(5-methyl-isoxazol-3-yl)-methyl]-5-(trifluormethyl)-1H-pyrrol-2-carboxylsäureamid |
| **124** | 3-(5-Chlor-pyrimidin-2-yl)-1-isopropyl-N,4-dimethyl-N-(2-oxo-pyrrolidin-3-yl)-5-(trifluormethyl)-1H-pyrrol-2-carboxylsäureamid |
| **125** | N-(2-Carbamoyl-2-methyl-propyl)-3-(5-chlor-pyrimidin-2-yl)-1-isopropyl-N,4-dimethyl-5-(trifluormethyl)-1H-pyrrol-2-carboxylsäureamid |
| **126** | 3-(5-Chlor-pyrimidin-2-yl)-N-(2-cyano-2-methyl-propyl)-1-isopropyl-N,4-dimethyl-5-(trifluormethyl)-1H-pyrrol-2-carboxylsäureamid |
| **127** | 3-(5-Chlor-pyrimidin-2-yl)-1-isopropyl-N,4-dimethyl-N-(2-methylsulfonyl-ethyl)-5-(trifluormethyl)-1H-pyrrol-2-carboxylsäureamid |
| **128** | 3-(5-Chlor-pyrimidin-2-yl)-N,1-diisopropyl-N,4-dimethyl-5-(trifluormethyl)-1H-pyrrol-2-carboxylsäureamid |
| **129** | N-tert-Butyl-4-[3-(5-chlor-pyridin-2-yl)-1-isopropyl-4-methyl-5-(trifluormethyl)-1H-pyrrol-2-carbonyl]-piperazin-1-carboxylsäureamid |
| **130** | [3-(5-Chlor-pyridin-2-yl)-1-isopropyl-4-methyl-5-(trifluormethyl)-1H-pyrrol-2-yl]-(2,2-dimethyl-1-oxo-[1,4]thiazinan-4-yl)-methanon |
| **131** | N-tert-Butyl-4-[3-(4-chlorphenyl)-1-isopropyl-4-methyl-5-(trifluormethyl)-1H-pyrrol-2-carbonyl]-piperazin-1-carboxylsäureamid |
| **132** | 3-(4-Chlorphenyl)-N-[1-(hydroxymethyl)-3-methyl-butyl]-1-isopropyl-N,4-dimethyl-5-(trifluormethyl)-1H-pyrrol-2-carboxylsäureamid |
| **133** | N-[1-(tert-Butyl-carbamoyl)-ethyl]-3-(4-chlorphenyl)-1-isopropyl-N,4-dimethyl-5-(trifluormethyl)-1H-pyrrol-2-carboxylsäureamid |
| **134** | 3-(5-Chlor-pyridin-2-yl)-N-(3,3-dimethyl-piperidin-4-yl)-1-isopropyl-N,4-dimethyl-5-(trifluormethyl)-1H-pyrrol-2-carboxylsäureamid |
| **135** | 3-(4-Chlorphenyl)-N-(3,3-dimethyl-piperidin-4-yl)-1-isopropyl-N,4-dimethyl-5-(trifluormethyl)-1H-pyrrol-2-carboxylsäureamid |
optional in der Form eines einzelnen Stereoisomers oder eines Gemischs aus Stereoisomeren, in der Form der freien Verbindung und/oder eines physiologisch annehmbaren Salzes oder Solvats davon.

13. Pharmazeutische Zusammensetzung, umfassend mindestens eine Verbindung nach einem oder mehreren der Ansprüche 1 bis 12.

14. Mindestens eine Verbindung nach einem oder mehreren der Ansprüche 1 bis 12 zur Verwendung in der Behandlung und/oder Prophylaxe von einer oder mehreren Störungen, ausgewählt aus der Gruppe bestehend aus Schmerz, vorzugsweise Schmerz ausgewählt aus der Gruppe bestehend aus akutem Schmerz, chronischem Schmerz, viszeralem Schmerz, Kopfschmerz, Entzündungsschmerz und gemischtem Schmerz; Schlaganfall; Gemütszustandsstörungen; Epilepsie; Schizophrenie und neurodegenerativen Störungen.

15. Mindestens eine Verbindung nach einem oder mehreren der Ansprüche 1 bis 12 zur Verwendung in der Behandlung und/oder Prophylaxe von Schmerz, besonders akutem Schmerz und/oder chronischem Schmerz und/oder viszeralem Schmerz und/oder Kopfschmerz und/oder Entzündungsschmerz und/oder gemischtem Schmerz.

## Revendications

1. Composé de formule générale (I), 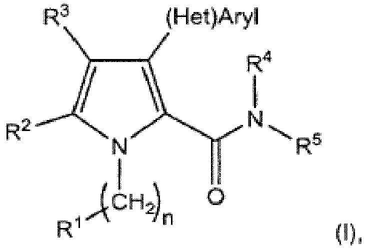 dans lequel :
n représente 0, 1 ou 2 ;
R¹ représente un groupe
alkyle en C₁ à C₆, alcényle en C₂ à C₆ ou alcynyle en C₂ à C₆ ;
ou
cycloalkyle en C₃ à C₆ ou hétérocyclyle de 3 à 7 chaînons ;
R² représente CH₂F ; CHF₂ ou CF₃ ;
R³ représente H, un groupe alkyle en C₁ à C₆, cycloalkyle en C₃ à C₆, hétérocyclyle de 3 à 7 chaînons, OH ; O-alkyle en C₁ à C₆ ; NH₂ ; N (H) (alkyle en C₁ à C₆) ; N(alkyle en C₁ à C₆)₂ ou SO₂(alkyle en C₁ à C₆) ;
(Het)Aryl représente un groupe aryle ou hétéroaryle, chacun substitué par zéro ou un ou deux ou trois substituants du groupe constitué de R⁶, R⁷ et R⁸,
dans lequel R⁶, R⁷ et R⁸ sont choisis chacun indépendamment les uns des autres dans le groupe consistant en F ; Cl ; Br ; I ; NO₂ ; CN ; alkyle en C₁ à C₆ ; CF₃ ; CF₂H ; CFH₂ ; CF₂Cl ; CFCl₂ ; C(=O)-H ; C(=O)-alkyle en C₁ à C₆ ; C(=O)-OH ; C(=O)-O-alkyle en C₁ à C₆ ; C(=O)-N(H)(OH) ; C(=O)-NH₂ ; C(=O)-N(H) (alkyle en C₁ à C₆) ; C(=O)-N(alkyle en C₁ à C₆)₂ ; C(=N-OH)-H ; C(=N-OH)-alkyle en C₁ à C₆ ; C(=N-O-alkyle en C₁ à C₆) -H ; C(=N-O-alkyle en C₁ à C₆) - alkyle en C₁ à C₆ ; OH ; OCF₃ ; OCF₂H ; OCFH₂ ; OCF₂Cl ; OCFCl₂ ; O-alkyle en C₁ à C₆ ; O-C(=O)-alkyle en C₁ à C₆ ; O-C(=O)-O-alkyle en C₁ à C₆ ; O-(C=O)-N(H) (alkyle en C₁ à C₆) ; O-C(=O)-N(alkyle en C₁ à C₆)₂ ; O-S(=O)₂-alkyle en C₁ à C₆ ; O-S(=O)₂-OH ; O-S(=O)₂-O-alkyle en C₁ à C₆ ; O-S(=O)₂-NH₂ ; O-S(=O)₂-N(H) (alkyle en C₁ à C₆) ; O-S(=O)₂-N(alkyle en C₁ à C₆)₂ ; NH₂ ; N(H) (alkyle en C₁ à C₆) ; N(alkyle en C₁ à C₆)₂ ; N(H)-C(=O)-alkyle en C₁ à C₆ ; N(H)-C(=O)-O-alkyle en C₁ à C₆ ; N(H)-C(=O)-NH₂ ; N(H)-C(=O)-N(H) (alkyle en C₁ à C₆) ; N(H)-C(=O)-N(alkyle en C₁ à C₆)₂ ; N(alkyl en C₁ à C₆) - C(=O)-alkyle en C₁ à C₆ ; N(alkyl en C₁ à C₆)-C(=O)-O-alkyle en C₁ à C₆ ; N (alkyl en C₁ à C₆)-C(=O)-NH₂ ; N (alkyl en C₁ à C₆)-C(=O)-N(H) (alkyle en C₁ à C₆) ; N(alkyl en C₁ à C₆) - C(=O)-N(alkyle en C₁ à C₆)₂ ; N(H)-S(=O)₂OH ; N(H)-S(=O)₂-alkyle en C₁ à C₆ ; N(H)-S(=O)₂-O-alkyle en C₁ à C₆ ; N(H)-S(=O)₂-NH₂ ; N(H)-S(=O)₂-N (H) (alkyle en C₁ à C₆) ; N(H)-S(=O)₂N(alkyle en C₁ à C₆)₂ ; N(alkyl en C₁ à C₆) -S(=O)₂-OH ; N(alkyle en C₁ à C₆)-S(=O)₂-alkyle en C₁ à C₆ ; N(alkyle en C₁ à C₆)-S(=O)₂-O-alkyle en C₁ à C₆ ; N(alkyle en C₁ à C₆) - S(=O)₂-NH₂ ; N(alkyl en C₁ à C₆)-S(=O)₂-N(H) (alkyle en C₁ à C₆) ; N(alkyle en C₁ à C₆)-S(=O)₂-N(alkyle en C₁ à C₆)₂ ; SH ; SCF₃ ; SCF₂H ; SCFH₂ ; SCF₂Cl ; SCFCl₂ ; S-alkyle en C₁ à C₆ ; S(=O)-alkyle en C₁ à C₆ ; S(=O)₂-alkyle en C₁ à C₆ ; S(=O)₂-OH ; S(=O)₂-O-alkyle en C₁ à C₆ ; S(=O)₂-NH₂ ; S(=O)₂-N (H) (alkyle en C₁ à C₆) ; S(=O)₂-N(alkyle en C₁ à C₆)₂, cycloalkyle en C₃ à C₆ ou hétérocyclyle de 3 à 7 chaînons ;
dans lequel ledit groupe alkyle en C₁ à C₆ dans chaque cas peut être ramifié ou non ramifié et non substitué ou mono- ou poly-substitué ; et dans lequel ledit groupe cycloalkyle en C₃ à C₆ ou hétérocyclyle de 3 à 7 chaînons dans chaque cas peut être non substitué ou mono- ou poly-substitué ;
R⁴ représente H, un groupe alkyle en C₁ à C₁₀, aryle, ou aryle relié par l'intermédiaire d'un groupe alkylène en C₁ à C₈ ;
R⁵ représente H ; un groupe alkyle en C₁ à C₁₀ ; cycloalkyle en C₃ à C₁₀, hétérocyclyle de 3 à 10 chaînons, aryle ou hétéroaryle ;
ou cycloalkyle en C₃ à C₁₀, hétérocyclyle de 3 à 10 chaînons, aryle ou hétéroaryle, chacun relié par l'intermédiaire d'un groupe alkylène en C₁ à C₈ ; ou
R⁴ et R⁵, conjointement avec l'atome d'azote les reliant, forment un groupe hétérocyclyle de 3 à 10 chaînons ;
dans lequel ledit groupe alkyle en C₁ à C₆, ledit groupe alkyle en C₁ à C₁₀, ledit groupe alcényle en C₂ à C₆, ledit groupe alcynyle en C₂ à C₆ et ledit groupe alkylène en C₁ à C₈ dans chaque cas peut être ramifié ou non ramifié et non substitué ou mono- ou poly-substitué ;
et dans lequel ledit groupe cycloalkyle en C₃ à C₆, ledit groupe cycloalkyle en C₃ à C₁₀, ledit groupe hétérocyclyle de 3 à 7 chaînons, ledit groupe hétérocyclyle de 3 à 10 chaînons, ledit groupe aryle et ledit groupe hétéroaryle dans chaque cas peut être non substitué ou mono- ou poly-substitué ;
éventuellement sous la forme d'un stéréoisomère individuel ou d'un mélange de stéréoisomères,
sous la forme du composé libre et/ou de l'un de ses sels physiologiquement acceptables et/ou de l'un de ses produits de solvatation physiologiquement acceptables.

2. Composé selon la revendication 1, **caractérisé en ce que** :
R¹ représente un groupe
alkyle en C₁ à C₆, alcényle en C₂ à C₆ ou alcynyle en C₂ à C₆,
dans chaque cas ramifié ou non ramifié, et dans chaque cas non substitué ou mono- ou poly-substitué par un ou plusieurs substituants choisis parmi les groupes
F ; Cl ; CN ; CF₃ ; CF₂H ; CFH₂ ; C(=O)-OH ; C(=O)-O-alkyle en C₁ à C₆ ; C(=O)-NH₂; C(=O)-N(H) (alkyle en C₁ à C₆) ; C(=O)-N(alkyle en C₁ à C₆)₂ ; OH ; OCF₃ ; OCF₂H ; OCFH₂ ; O-alkyle en C₁ à C₆ ; O-C(=O)-alkyle en C₁ à C₆ ; 0-S(=O)₂-alkyle en C₁ à C₆ ; NH₂ ; N (H) (alkyle en C₁ à C₆) ; N(alkyle en C₁ à C₆)₂ ; N(H)-C(=O)-alkyle en C₁ à C₆ ; N(H)-C(=O)-N(H) (alkyle en C₁ à C₆) ; N(H)-C(=O)-N(alkyle en C₁ à C₆)₂ ; N(alkyle en C₁ à C₆)-C(=O)-alkyle en C₁ à C₆ ; N(H)-S(=O)₂-alkyle en C₁ à C₆ ; N(alkyle en C₁ à C₆)-S(=O)₂-alkyle en C₁ à C₆ ; S(=O)-alkyle en C₁ à C₆, S(=O)₂-alkyle en C₁ à C₆, cycloalkyle en C₃ à C₆ ou hétérocyclyle de 3 à 7 chaînons,
ou R¹ représente un groupe
cycloalkyle en C₃ à C₆ ou hétérocyclyle de 3 à 7 chaînons, dans chaque cas non substitué ou mono- ou poly-substitué par un ou plusieurs substituants choisis parmi les groupes
F ; Cl ; CN ; alkyle en C₁ à C₆ ; CF₃ ; CF₂H ; CFH₂ ; C(=O)-OH ; C(=O)-O-alkyle en C₁ à C₆ ; C(=O)-NH₂; C(=O)-N(H) (alkyle en C₁ à C₆) ; C(=O)-N(alkyle en C₁ à C₆)₂ ; OH ; =O ; OCF₃ ; OCF₂H ; OCFH₂ ; O-alkyle en C₁ à C₆ ; O-C(=O) - alkyle en C₁ à C₆ ; O-S(=O)₂-alkyle en C₁ à C₆ ; NH₂ ; N(H) (alkyle en C₁ à C₆) ; N(alkyle en C₁ à C₆)₂ ; N(H)-C(=O)-alkyle en C₁ à C₆ ; N(H)-C(=O)-N(H)(alkyle en C₁ à C₆) ; N(H)-C(=O)-N(alkyle en C₁ à C₆)₂ ; N(alkyle en C₁ à C₆)-C(=O)-alkyle en C₁ à C₆ ; N(H)-S(=O)₂-alkyle en C₁ à C₆ ; N(alkyle en C₁ à C₆)-S(=O)₂-alkyle en C₁ à C₆ ; S(=O)-alkyle en C₁ à C₆ ou S(=O)₂-alkyle en C₁ à C₆.

3. Composé selon une ou plusieurs des revendications précédentes, **caractérisé en ce que** R² représente CHF₂ ou CF₃.

4. Composé selon une ou plusieurs des revendications précédentes, **caractérisé en ce que** R³ est choisi dans le groupe constitué de H, des groupes méthyle, éthyle, n-propyle, iso-propyle, n-butyle, sec.-butyle, iso-butyle, tert.-butyle, cyclopropyle, méthoxy, éthoxy, méthyl-sulfonyle, 2-oxétyle, 3-oxétyle, 2-tétrahydrofuranyle et 3-tétrahydrofuranyle.

5. Composé selon une ou plusieurs des revendications précédentes, **caractérisé en ce que** :
Het(aryl) est choisi
parmi les groupes phényle, pyrrole, pyrazolyle, imidazolyle, isoxazolyle, oxazolyle, thiazolyle, pyridinyle, pyrazinyle, pyrimidinyle et pyridazinyle,
de préférence Het(aryl) représente un groupe phényle, pyridinyle, pyrazinyle ou pyrimidinyle.

6. Composé selon une ou plusieurs des revendications précédentes, **caractérisé en ce que** :
R⁶, R⁷ et R⁸ sont choisis chacun indépendamment les uns des autres dans le groupe consistant en F ; Cl ; CN ; alkyle en C₁ à C₆ ; CF₃ ; CF₂H ; CFH₂ ; OH ; OCF₃ ; OCF₂H ; OCFH₂ ; O-alkyle en C₁ à C₆ ; O-C(=O)-alkyle en C₁ à C₆ ; NH₂ ; N(H)(alkyle en C₁ à C₆) ; N(alkyle en C₁ à C₆)₂ ; SCF₃ ; S(=O)-alkyle en C₁ à C₆ ; S(=O)₂-alkyle en C₁ à C₆ ; S(=O)₂-OH ; S(=O)₂-O-alkyle en C₁ à C₆ ; S(=O)₂-NH₂ ; S(=O)₂-N(H) (alkyle en C₁ à C₆) ; ou S(=O)₂-N(alkyle en C₁ à C₆)₂ ; dans lesquels dans chaque cas le groupe alkyle en C₁ à C₆ peut être ramifié ou non ramifié ; ou un résidu cyclo-aliphatique en C₃ à C₆, non substitué ou mono- ou poly-substitué,
de préférence
R⁶, R⁷ et R⁸ sont choisis chacun indépendamment les uns des autres dans le groupe consistant en F ; Cl ; CN ; alkyle en C₁ à C₆ ; CF₃ ; CF₂H ; CFH₂ ; OH ; OCF₃ ; OCF₂H ; OCFH₂ ; O-alkyle en C₁ à C₆ ; S(=O)-alkyle en C₁ à C₆ ; S(=O)₂-alkyle en C₁ à C₆ ; cyclopropyle et O-cyclopropyle.

7. Composé selon une ou plusieurs des revendications précédentes, le composé de formule générale (I) étant **caractérisé en ce qu'**il est un composé selon la formule générale (Ia), 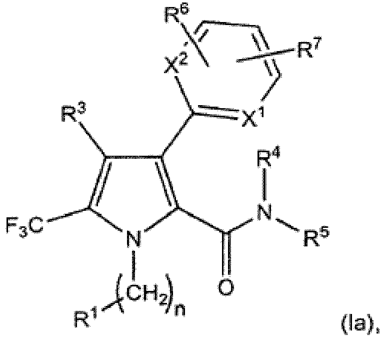 dans laquelle :
R³ représente H ou CH₃ ou un groupe cyclopropyle ;
X¹ représente N ou CH ; X² représente N ou CH ;
R⁶ et R⁷ sont indépendamment absents ou sont choisis chacun indépendamment l'un de l'autre dans le groupe consistant en F ; Cl ; CN ; alkyle en C₁ à C₆ ; CF₃ ; CF₂H ; CFH₂ ; OH ; OCF₃ ; OCF₂H ; OCFH₂ ou O-alkyle en C₁ à C₆ ;
et n, R¹, R⁴ et R⁵ sont définis selon la revendication 1.

8. Composé selon une ou plusieurs des revendications précédentes, **caractérisé en ce que** :
R⁴ représente
H ou
un résidu aliphatique en C₁ à C₆, ramifié ou non ramifié, non substitué ou substitué par 1, 2, 3, 4 ou 5 substituants choisis indépendamment les uns des autres dans le groupe consistant en OH, =0, O-alkyle en C₁ à C₆, S(=O)-alkyle en C₁ à C₆, S(=O)₂-alkyle en C₁ à C₆, N(H)-S(=O)-alkyle en C₁ à C₆, N(alkyl en C₁ à C₆)-S(=O)-alkyle en C₁ à C₆, N(H)-S(=O)₂-alkyle en C₁ à C₆, N(alkyl en C₁ à C₆)- S(=O)₂-alkyle en C₁ à C₆, C(=O)-NH₂, C(=O)-N(H) (alkyle en C₁ à C₆), C(=O)-N(alkyle en C₁ à C₆)₂, C(=O)-O-alkyle en C₁ à C₆ ; N(H)-C(=O)-alkyle en C₁ à C₆, et N(alkyl en C₁ à C₆) - C(=O)-alkyle en C₁ à C₆
ou phényle, non substitué ou mono- ou poly-substitué par 1, 2 ou 3 substituants choisis indépendamment les uns des autres dans le groupe consistant en F ; Cl ; CN ; alkyle en C₁ à C₆ ; CF₃ ; CF₂H ; CFH₂ ; OH ; OCF₃ ; OCF₂H ; OCFH₂ ou O-alkyle en C₁ à C₆, et éventuellement relié par l'intermédiaire d'un groupe alkylène en C₁ à C₃, ramifié ou non ramifié ;
et
R⁵ représente
H ; ou
un groupe alkyle en C₁ à C₆, ramifié ou non ramifié, non substitué ou substitué par 1, 2, 3, 4 ou 5 substituants choisis indépendamment les uns des autres dans le groupe consistant en F, Cl, CF₃, CN, OH, =0, OCF₃, O-alkyle en C₁ à C₆, O-(C=O)alkyle en C₁ à C₆, S(=O)-alkyle en C₁ à C₆, S(=O)₂-alkyle en C₁ à C₆, S(=O)₂-NH₂, S(=O)₂-N(H)alkyle en C₁ à C₆, S(=O)₂-N(alkyle en C₁ à C₆)₂, NH₂, NH(alkyle en C₁ à C₆), N(alkyle en C₁ à C₆)₂, N(H)-S(=O)-alkyle en C₁ à C₆, N(alkyle en C₁ à C₆)-S(=O)-alkyle en C₁ à C₆, N(H)-S(=O)₂-alkyle en C₁ à C₆, N (alkyle en C₁ à C₆)-S(=O)₂-alkyle en C₁ à C₆, N(H)-C(=O)-NH₂, N(H)-C(=O)-N(H) (alkyle en C₁ à C₆), N(H)-C(=O)-N(alkyle en C₁ à C₆)₂, N(H)-C(=O)-O-alkyle en C₁ à C₆ ; O-C(=O)-NH₂, O-C(=O)-N(H) (alkyle en C₁ à C₆), 0-C(=O)-N(alkyle en C₁ à C₆)₂, C(=O)-NH₂, C(=O)-N(H) (alkyle en C₁ à C₆), C(=O)-N(alkyle en C₁ à C₆)₂, C(=O)-O-alkyle en C₁ à C₆ ; N(H)-C(=O)-alkyle en C₁ à C₆, et N(alkyle en C₁ à C₆) - C(=O)-alkyle en C₁ à C₆ ; ou
cycloalkyle en C₃ à C₆, non substitué ou substitué par 1, 2, 3, 4 ou 5 substituants choisis indépendamment les uns des autres dans le groupe consistant en F, Cl, CN, CF₃, =0, OCF₃, alkylène en C₁ à C₆-OH, alkyle en C₁ à C₆, OH, 0-alkyle en C₁ à C₆, O-(C=O)alkyle en C₁ à C₆, S(=O)-alkyle en C₁ à C₆, S(=O)₂-alkyle en C₁ à C₆, S(=O)₂-NH₂, S(=O)₂-N (H) alkyle en C₁ à C₆, S(=O)₂-N(alkyle en C₁ à C₆)₂, NH₂, NH(alkyle en C₁ à C₆), N(alkyle en C₁ à C₆)₂, N(H)-S(=O)-alkyle en C₁ à C₆, N (alkyle en C₁ à C₆)-S(=O)-alkyle en C₁ à C₆, N(H)-S(=O)₂-alkyle en C₁ à C₆, N(alkyle en C₁ à C₆)-S(=O)₂-alkyle en C₁ à C₆, N(H)-C(=O)-O-alkyle en C₁ à C₆ ; O-C(=O)-NH₂, O-C(=O)-N(H) (alkyle en C₁ à C₆), O-C(=O)-N(alkyle en C₁ à C₆)₂, N(H)-C(=O)-NH₂, N(H)-C(=O)-N(H) (alkyle en C₁ à C₆), N(H)-C(=O)-N(alkyle en C₁ à C₆)₂, C(=O)-NH₂, C(=O)-N(H) (alkyle en C₁ à C₆), C(=O)-N(alkyle en C₁ à C₆)₂, C(=O)-O-alkyle en C₁ à C₆ ; N(H)-C(=O)-alkyle en C₁ à C₆, et N (alkyle en C₁ à C₆)-C(=O)-alkyle en C₁ à C₆ ; où ledit groupe cycloalkyle en C₃ à C₆ est éventuellement relié par l'intermédiaire d'un groupe alkylène en C₁ à C₆, ramifié ou non ramifié, qui à son tour peut être non substitué ou substitué par 1, 2, 3, 4 ou 5 substituants choisis indépendamment les uns des autres dans le groupe consistant en F, Cl, CF₃, =0, OCF₃, OH, O-alkyle en C₁ à C₆ et alkylène en C₁ à C₆-OH ; ou
hétérocyclyle de 3 à 7 chaînons, qui est non substitué ou substitué par 1, 2, 3, 4 ou 5 substituants choisis indépendamment les uns des autres dans le groupe consistant en F, Cl, CN, CF₃, =0, OCF₃, alkylène en C₁ à C₆-OH, alkyle en C₁ à C₆, OH, O-alkyle en C₁ à C₆, O-(C=O)alkyle en C₁ à C₆, S(=O)-alkyle en C₁ à C₆, S(=O)₂-alkyle en C₁ à C₆, S(=O)₂-NH₂, S(=O)₂-N(H) alkyle en C₁ à C₆, S(=O)₂-N(alkyle en C₁ à C₆)₂, NH₂, NH(alkyle en C₁ à C₆), N(alkyle en C₁ à C₆)₂, N(H)-S(=O)-alkyle en C₁ à C₆, N(alkyle en C₁ à C₆)-S(=O)-alkyle en C₁ à C₆, N(H)-S(=O)₂-alkyle en C₁ à C₆, N(alkyle en C₁ à C₆)-S(=O)₂-alkyle en C₁ à C₆, N(H)-C(=O)-O-alkyle en C₁ à C₆ ; O-C(=O)-NH₂, O-C(=O)-N(H) (alkyle en C₁ à C₆), O-C(=O)-N(alkyle en C₁ à C₆)₂, N(H)-C(=O)-NH₂, N(H)-C(=O)-N(H) (alkyle en C₁ à C₆), N(H)-C(=O)-N(alkyle en C₁ à C₆)₂, (C=O)alkyle en C₁ à C₆, C(=O)-NH₂, C(=O)-N(H) (alkyle en C₁ à C₆), C(=O)-N(alkyle en C₁ à C₆)₂, C(=O)-O-alkyle en C₁ à C₆ ; N(H)-C(=O)-alkyle en C₁ à C₆, et N(alkyle en C₁ à C₆)-C(=O)-alkyle en C₁ à C₆, où ledit groupe hétérocyclyle de 3 à 7 chaînons est éventuellement relié par l'intermédiaire d'un groupe alkylène en C₁ à C₆, ramifié ou non ramifié, qui à son tour peut être non substitué ou substitué par 1, 2, 3, 4 ou 5 substituants choisis indépendamment les uns des autres dans le groupe consistant en F, Cl, CF₃, =O, OCF₃, OH, O-alkyle en C₁ à C₆ et alkylène en C₁ à C₆-OH ;
ou aryle ou hétéroaryle, lequel dans chaque cas est non substitué ou substitué par 1, 2, 3, 4 ou 5 substituants choisis indépendamment les uns des autres dans le groupe consistant en F, Cl, CN, CF₃, OCF₃, alkylène en C₁ à C₆-OH, alkyle en C₁ à C₆, OH, O-alkyle en C₁ à C₆, O-(C=O)alkyle en C₁ à C₆, S(=O)-alkyle en C₁ à C₆, S(=O)₂-alkyle en C₁ à C₆, S(=O)₂-NH₂, S(=O) ₂-N (H) alkyle en C₁ à C₆, S(=O)₂-N (alkyle en C₁ à C₆)₂, NH₂, NH(alkyle en C₁ à C₆), N(alkyle en C₁ à C₆)₂, N(H)-S(=O)-alkyle en C₁ à C₆, N(alkyle en C₁ à C₆)-S(=O)-alkyle en C₁ à C₆, N(H)-S(=O)₂-alkyle en C₁ à C₆, N(alkyle en C₁ à C₆)-S(=O)₂-alkyle en C₁ à C₆, N(H)-C(=O)-O-alkyle en C₁ à C₆ ; O-C(=O)-NH₂, O-C(=O)-N(H) (alkyle en C₁ à C₆), 0-C(=O)-N(alkyle en C₁ à C₆)₂, N(H)-C(=O)-NH₂ N(H)-C(=O)-N (H) (alkyle en C₁ à C₆), N(H)-C(=O)-N(alkyle en C₁ à C₆)₂, C(=O)-NH₂, C(=O)-N(H) (alkyle en C₁ à C₆), C(=O)-N(alkyle en C₁ à C₆)₂, C(=O)-O-alkyle en C₁ à C₆ ; N(H)-C(=O)-alkyle en C₁ à C₆, et N(alkyle en C₁ à C₆)-C(=O)-alkyle en C₁ à C₆, où ledit groupe aryle ou hétéroaryle est éventuellement relié par l'intermédiaire d'un groupe alkylène en C₁ à C₆, ramifié ou non ramifié, qui à son tour peut être non substitué ou substitué par 1, 2, 3, 4 ou 5 substituants choisis indépendamment les uns des autres dans le groupe consistant en F, Cl, CF₃, =0, OCF₃, OH, O-alkyle en C₁ à C₆ et alkylène en C₁ à C₆-OH.

9. Composé selon une ou plusieurs des revendications précédentes, **caractérisé en ce que** R⁴ et R⁵, conjointement avec l'atome d'azote les reliant, forment un groupe hétérocyclyle de 3 à 7 chaînons, non substitué ou substitué par 1, 2, 3, 4 ou 5 substituants choisis dans le groupe consistant en F, Cl, CN, CF₃, =0, OH, alkyle en C₁ à C₆, 0-alkyle en C₁ à C₆, alkylène en C₁ à C₆-OH, OCF₃, SO₂(alkyle en C₁ à C₆), SO₂NH₂, SO₂N(H)alkyle en C₁ à C₆, SO₂N(alkyle en C₁ à C₆)₂, alkylène en C₁ à C₆-SO₂(alkyle en C₁ à C₆), NH₂, NH(alkyle en C₁ à C₆), N(alkyle en C₁ à C₆)₂, (C=0) alkyle en C₁ à C₆, cycloalkyle en C₃ à C₆, hétérocyclyle de 3 à 7 chaînons, aryle, hétéroaryle, O-aryle et 0-hétéroaryle, dans chaque cas non substitué ou mono- ou poly-substitué.

10. Composé selon une ou plusieurs des revendications précédentes, **caractérisé en ce que** :
R⁴ représente H ou un groupe alkyle en C₁ à C₆ ou benzyle ; et
R⁵ représente
un groupe cycloalkyle en C₃ à C₆, qui est non substitué ou substitué par 1, 2, 3, 4 ou 5 substituants choisis indépendamment les uns des autres dans le groupe consistant en F, Cl, CF₃, =0, OCF₃, OH, O-alkyle en C₁ à C₆, alkylène en C₁ à C₆-OH, alkyle en C₁ à C₆, C(=O)-NH₂, C(=O)-N (H) (alkyle en C₁ à C₆) et C(=O)-N(alkyle en C₁ à C₆)₂ ; ou
hétérocyclyle de 3 à 7 chaînons, qui contient 1 ou 2 hétéroatomes ou groupes d'hétéroatomes choisis indépendamment les uns des autres dans le groupe consistant en 0, S, S(=O), S(=O)₂, NH et N-alkyle en C₁ à C₆, et qui est non substitué ou substitué par 1, 2, 3, 4 ou 5 substituants choisis indépendamment les uns des autres dans le groupe consistant en F, Cl, CF₃, OCF₃, CN, =0, alkyle en C₁ à C₆, alkylène en C₁ à C₆-OH et O-alkyle en C₁ à C₆ ; ou
phényle ou hétéroaryle, qui contient au moins un atome d'azote, dans chaque cas non substitué ou substitué par 1, 2 ou 3 substituants choisis indépendamment les uns des autres dans le groupe consistant en F, Cl, CN, CF₃, OCF₃, alkylène en C₁ à C₆-OH, alkyle en C₁ à C₆, OH, O-alkyle en C₁ à C₆, S(=O)₂-alkyle en C₁ à C₆, S(=O)₂-NH₂, NH₂, NH(alkyle en C₁ à C₆), N(alkyle en C₁ à C₆)₂, O-C(=O)-NH₂, C(=O)-NH₂, C(=O)-N(H)(alkyle en C₁ à C₆), C(=O)-N(alkyle en C₁ à C₆)₂, C(=O)-O-alkyle en C₁ à C₆ ;
ou une structure partielle de formule générale SF-III 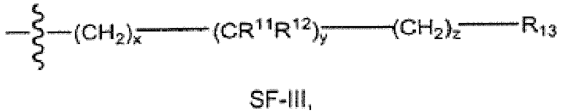 dans laquelle :
x représente 0, 1 ou 2 ;
y représente 0, 1 ou 2 ;
z représente 0, 1 ou 2 ;
à condition que la somme de x, y et z soit de 1, 2, 3, 4, 5 ou 6 ;
R¹¹ et R¹² sont choisis indépendamment les uns des autres parmi H ou un groupe alkyle en C₁ à C₆ ; ou
R¹¹ et R¹², conjointement avec l'atome de carbone les reliant, forment un groupe cycloalkyle en C₃ à C₆ ou hétérocyclyle de 3 à 7 chaînons, qui contient 1 ou 2 hétéroatomes ou groupes d'hétéroatomes choisis indépendamment les uns des autres dans le groupe consistant en 0, S, S(=O), S(=O)₂, NH et N-alkyle en C₁ à C₆, où ledit groupe cycloalkyle en C₃ à C₆ ou hétérocyclyle de 3 à 7 chaînons peut être non substitué ou substitué par 1, 2, 3, 4 ou 5 substituants choisis indépendamment les uns des autres dans le groupe consistant en F, Cl, CF₃, OCF₃, CN, alkyle en C₁ à C₆ et O-alkyle en C₁ à C₆ ;
R¹³ est choisi dans le groupe consistant en
H, F, Cl, CN, OH, O-alkyle en C₁ à C₆, O-(C=O)alkyle en C₁ à C₆, S(=O)-alkyle en C₁ à C₆, S(=O)₂-alkyle en C₁ à C₆, S(=O)₂-NH₂, S(=O)₂-N(H)alkyle en C₁ à C₆, S(=O)₂-N(alkyle en C₁ à C₆)₂, NH₂, NH(alkyle en C₁ à C₆), N(alkyle en C₁ à C₆)₂, N(H)-C(=O)-alkyle en C₁ à C₆, N(alkyle en C₁ à C₆)-C(=O)-alkyle en C₁ à C₆, N(H)-S(=O)-alkyle en C₁ à C₆, N(alkyle en C₁ à C₆)-S(=O)-alkyle en C₁ à C₆, N(H)-S(=O)₂-alkyle en C₁ à C₆, N(alkyle en C₁ à C₆)-S(=O)2-alkyle en C₁ à C₆, C(=O)-NH₂, C(=O)-N(H) (alkyle en C₁ à C₆), C(=O)-N(alkyle en C₁ à C₆)₂, C(=O)-O-alkyle en C₁ à C₆, N(H)-C(=O)-NH₂, N(H)-C(=O)-N(H)(alkyle en C₁ à C₆), N(H)-C(=O)-N(alkyle en C₁ à C₆)₂, N(H)-C(=O)-O-alkyle en C₁ à C₆ ; O-C(=O)-NH₂, O-C(=O)-N(H)(alkyle en C₁ à C₆), O-C(=O)-N(alkyle en C₁ à C₆)₂ ; ou représente un groupe
cycloalkyle en C₃ à C₆, qui est non substitué ou substitué par 1, 2, 3, 4 ou 5 substituants choisis indépendamment les uns des autres dans le groupe consistant en F, Cl, CF₃, =0, OCF₃, OH, O-alkyle en C₁ à C₆, alkylène en C₁ à C₆-OH et alkyle en C₁ à C₆ ; ou
hétérocyclyle de 3 à 7 chaînons, qui contient 1 ou 2 hétéroatomes ou groupes d'hétéroatomes choisis indépendamment les uns des autres dans le groupe consistant en 0, S, S(=O), S(=O)₂, NH et N-alkyle en C₁ à C₆, et qui est non substitué ou substitué par 1, 2, 3, 4 ou 5 substituants choisis indépendamment les uns des autres dans le groupe consistant en F, Cl, CF₃, OCF₃, CN, alkyle en C₁ à C₆ et O-alkyle en C₁ à C₆ ; ou
phényle ou hétéroaryle, qui contient au moins un atome d'azote, dans chaque cas non substitué ou substitué par 1, 2 ou 3 substituants choisis indépendamment les uns des autres dans le groupe consistant en F, Cl, CN, CF₃, OCF₃, alkylène en C₁ à C₆-OH, alkyle en C₁ à C₆, OH, O-alkyle en C₁ à C₆, S(=O)₂-alkyle en C₁ à C₆, S(=O)₂-NH₂, NH₂, NH(alkyle en C₁ à C₆), N(alkyle en C₁ à C₆)₂, O-C(=O)-NH₂, C(=O)-NH₂, C(=O)-N(H)(alkyle en C₁ à C₆), C(=O)-N(alkyle en C₁ à C₆)₂, C(=O)-O-alkyle en C₁ à C₆ ;
ou
R⁴ et R⁵, conjointement avec l'atome d'azote les reliant, forment un groupe hétérocyclyle, choisi dans le groupe consistant en 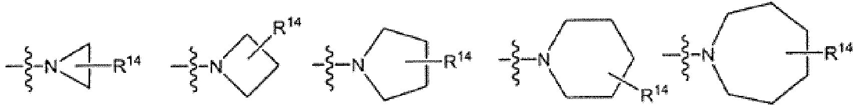 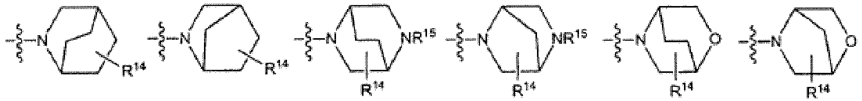 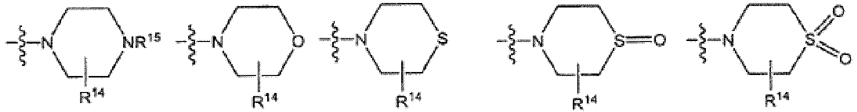
où :
R¹⁴ désigne 0, 1, 2, 3 ou 4 substituants qui sont dans chaque cas choisis indépendamment les uns des autres dans le groupe consistant en F, Cl, CF₃, =0, OCF₃, OH, O-alkyle en C₁ à C₆, alkylène en C₁ à C₆-OH, alkylène en C₁ à C₆-SO₂(alkyle en C₁ à C₆), SO₂(alkyle en C₁ à C₆), alkyle en C₁ à C₆, aryle, hétéroaryle, O-aryle et 0-hétéroaryle, où ledit groupe aryle ou ledit groupe hétéroaryle est non substitué ou substitué par 1, 2 ou 3 substituants choisis indépendamment les uns des autres dans le groupe consistant en F, Cl, CN, CF₃, OCF₃, alkylène en C₁ à C₆-OH, alkyle en C₁ à C₆, OH, O-alkyle en C₁ à C₆, NH₂, NH(alkyle en C₁ à C₆), N(alkyle en C₁ à C₆)₂, N(H)-S(=O)₂-alkyle en C₁ à C₆, C(=O)-NH₂ ou C(=O)-N(H)(alkyle en C₁ à C₆), C(=O)-N(alkyle en C₁ à C₆)₂ ;
ou
R¹⁴ désigne au moins deux substituants, où deux substituants R¹⁴ représentent ensemble un groupe alkylène en C₁ à C₆, substitué ou non substitué, dans lequel éventuellement un ou plusieurs atomes C du groupe alkylène en C₁ à C₆ sont remplacés par un hétéroatome ou un groupe d'hétéroatomes, choisi parmi 0, N-R¹⁵, S, S(O) et S(O)₂, et où ces deux substituants R¹⁴ sont positionnés au niveau d'atomes de carbone différents du groupe hétérocyclyle, de telle façon que le groupe alkylène en C₁ à C₆ représente un pont pour former un groupe hétérocyclyle bicyclique ;
ou
R¹⁴ désigne au moins deux substituants, où deux substituants R¹⁴ représentent ensemble un groupe alkylène en C₂ à C₆, substitué ou non substitué, dans lequel éventuellement un ou plusieurs atomes C du groupe alkylène en C₂ à C₆ sont remplacés par un hétéroatome ou groupe d'hétéroatomes, choisi parmi 0, N-R¹⁵, S, S(O) et S(O)₂, et où ces deux substituants R¹⁴ sont positionnés au niveau du même atome de carbone du groupe hétérocyclyle, de telle façon que le groupe alkylène en C₂ à C₆ forme un groupe spiro-hétérocyclyle ; et
R¹⁵ représente H, un groupe alkyle en C₁ à C₆, (C=O)alkyle en C₁ à C₆, (C=O)NH₂, (C=O)NH(alkyle en C₁ à C₆) ou (C=O)N(alkyle en C₁ à C₆)₂.

11. Composé selon une ou plusieurs des revendications précédentes, le composé de formule générale (I) étant **caractérisé en ce qu'**il est un composé selon la formule générale (Ia), 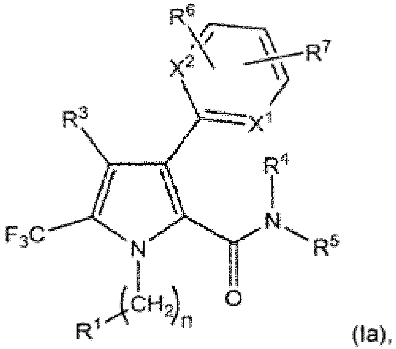 dans laquelle :
n vaut 0 ou 1,
R¹ représente un groupe alkyle en C₁ à C₆, alcényle en C₂ à C₆ ou alcynyle en C₂ à C₆,
dans chaque cas ramifié ou non ramifié, et dans chaque cas non substitué ou mono- ou poly-substitué par un ou plusieurs substituants choisis F ; Cl ; CN ; CF₃ ; C(=O)-NH₂ ; C(=O)-N(H)(alkyle en C₁ à C₆) ; C(=O)-N(alkyle en C₁ à C₆)₂ ; OH ; O-alkyle en C₁ à C₆ ; NH₂ ; N (H) (alkyle en C₁ à C₆) ; N(alkyle en C₁ à C₆)₂ ; N(H)-C(=O)-alkyle en C₁ à C₆ ; S(=O)-alkyle en C₁ à C₆ ; S(=O)₂-alkyle en C₁ à C₆ ou cyclopropyle,
ou R¹ représente un groupe cyclopropyle, cyclobutyle, cyclopentyle, cyclohexyle, oxétanyle, azétidinyle, tétrahydrofuranyle, tétrahydropyranyle, pyrrolidinyle, pipéridinyle et pipérazinyle,
dans chaque cas non substitué ou mono- ou poly-substitué par un ou plusieurs substituants choisis parmi F ; Cl ; CN ; alkyle en C₁ à C₆ ; CF₃ ; OH ; =O ; O-alkyle en C₁ à C₆ ; O-C(=O)-alkyle en C₁ à C₆ ; O-S(=O)₂-alkyle en C₁ à C₆ ; NH₂ ; N(H)(alkyle en C₁ à C₆) ; N(alkyle en C₁ à C₆)₂ ; N(H)-C(=O)-alkyle en C₁ à C₆ ; N(H)-S(=O)₂-alkyle en C₁ à C₆ ou S(=O)₂-alkyle en C₁ à C₆ ;
R³ représente H ou CH₃ ou un groupe cyclopropyle ;
X¹ représente N ou CH ; X² représente N ou CH ;
R⁶ et R⁷ sont indépendamment absents ou sont choisis chacun indépendamment l'un de l'autre dans le groupe consistant en F ; Cl ; CN ; alkyle en C₁ à C₆ ; CF₃ ; CF₂H ; CFH₂ ; OH ; OCF₃ ; OCF₂H ; OCFH₂ ou O-alkyle en C₁ à C₆ ;
R⁴ représente H ou un groupe alkyle en C₁ à C₆ ou benzyle ; et
R⁵ représente un groupe
cycloalkyle en C₃ à C₆, qui est non substitué ou substitué par 1, 2, 3, 4 ou 5 substituants choisis indépendamment les uns des autres dans le groupe consistant en F, Cl, CF₃, =O, OCF₃, OH, O-alkyle en C₁ à C₆, alkylène en C₁ à C₆-OH, alkyle en C₁ à C₆, C(=O)-NH₂, C(=O)-N(H)(alkyle en C₁ à C₆) et C(=O)-N(alkyle en C₁ à C₆)₂ ; ou
hétérocyclyle de 5 ou 6 chaînons, qui contient 1 ou 2 hétéroatomes ou groupes d'hétéroatomes choisis indépendamment les uns des autres dans le groupe consistant en 0, S, S(=O), S(=O)_{2,} NH et N-alkyle en C₁ à C₆, et qui est non substitué ou substitué par 1, 2, 3, 4 ou 5 substituants choisis indépendamment les uns des autres dans le groupe consistant en F, Cl, CF₃, OCF₃, CN, =0, alkyle en C₁ à C₆, alkylène en C₁ à C₆-OH et O-alkyle en C₁ à C₆ ;
ou une structure partielle de formule générale SF-III 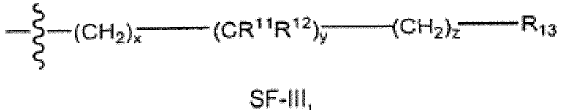 dans laquelle
x représente 1 et y et z représentent chacun 0 ou
x et y représentent chacun 1 et z représente 0 ou
x et z représentent chacun 1 et y représente 0 ou
x, y et z représentent chacun 1 ;
R¹¹ et R¹² sont choisis indépendamment l'un de l'autre parmi H ou CH₃ ;
R¹³ est choisi dans le groupe consistant en
H, F, Cl, CN, OH, O-alkyle en C₁ à C₆, O-(C=O)alkyle en C₁ à C₆, S(=O)-alkyle en C₁ à C₆, S(=)₂-alkyle en C₁ à C₆, NH₂, NH(alkyle en C₁ à C₆), N(alkyle en C₁ à C₆)₂, N(H)-C(=O)-alkyle en C₁ à C₆, N(H)-S(=O)₂-alkyle en C₁ à C₆, C(=O)-NH₂, C(=O)-N(H)(alkyle en C₁ à C₆), C(=O)-N(alkyle en C₁ à C₆)₂, N(H)-C(=O)-NH₂, N(H)-C(=O)-N(H)(alkyle en C₁ à C₆), N(H)-C(=O)-N(alkyle en C₁ à C₆)₂,
ou représente un groupe
cycloalkyle en C₃ à C₆ ou
hétérocyclyle de 3 à 7 chaînons, qui contient 1 ou 2 hétéroatomes ou groupes d'hétéroatomes choisis indépendamment les uns des autres dans le groupe consistant en 0, S, S(=O), S(=O)₂, NH et N-alkyle en C₁ à C₆, et qui est non substitué ou substitué par 1, 2, 3, 4 ou 5 substituants choisis indépendamment les uns des autres dans le groupe consistant en F, Cl, CF₃, OCF₃, CN, alkyle en C₁ à C₆ et O-alkyle en C₁ à C₆ ; ou
phényle ou hétéroaryle, choisi parmi les groupes pyrrolyle, pyrazolyle, imidazolyle, oxazolyle, isoxazolyle, thiazolyle, isothiazolyle, pyridinyle, pyrazinyle, pyrimidinyle et pyridazinyle,
dans chaque cas non substitué ou substitué par 1, 2 ou 3 substituants choisis indépendamment les uns des autres dans le groupe consistant en F, Cl, CN, CF₃, OCF₃, alkylène en C₁ à C₆-OH, alkyle en C₁ à C₆, OH, O-alkyle en C₁ à C₆, S(=O)₂-alkyle en C₁ à C₆, S(=O)₂-NH₂, NH₂, NH(alkyle en C₁ à C₆), N(alkyle en C₁ à C₆)₂, O-C(=O)-NH₂, C(=O)-NH₂, C(=O)-N (H) (alkyle en C₁ à C₆), C(=O)-N(alkyle en C₁ à C₆)₂, C(=O)-0-alkyle en C₁ à C₆ ;
ou
R⁴ et R⁵, conjointement avec l'atome d'azote les reliant, forment un groupe hétérocyclyle, choisi dans le groupe consistant en 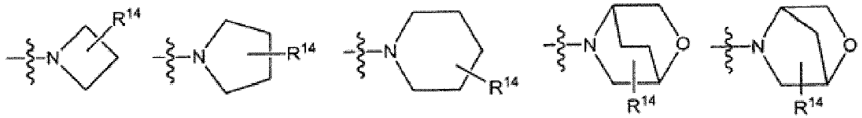 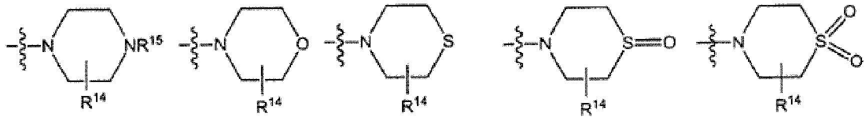 où,
R¹⁴ désigne 0, 1 ou 2 substituants qui sont dans chaque cas choisis indépendamment les uns des autres dans le groupe consistant en F, Cl, CF₃, =0, OCF₃, OH, O-alkyle en C₁ à C₆, alkylène en C₁ à C₆-OH, alkylène en C₁ à C₆-SO₂(alkyle en C₁ à C₆), SO₂(alkyle en C₁ à C₆), alkyle en C₁ à C₆, aryle, hétéroaryle, 0-aryle et 0-hétéroaryle, où ledit groupe aryle ou ledit groupe hétéroaryle est non substitué ou substitué par 1, 2 ou 3 substituants choisis indépendamment les uns des autres dans le groupe consistant en F, Cl, CN, CF₃, OCF₃, alkylène en C₁ à C₆-OH, alkyle en C₁ à C₆, OH, O-alkyle en C₁ à C₆ ; et
R¹⁵ représente H, un groupe alkyle en C₁ à C₆, (C=O)alkyle en C₁ à C₆, (C=O)NH₂, (C=O)NH(alkyle en C₁ à C₆) ou (C=O)N(alkyle en C₁ à C₆)₂.

12. Composé selon une ou plusieurs des revendications précédentes choisi dans le groupe consistant en
| | |
|---|---|
| 001 | Amide d'acide N-benzyl-3-(4-chlorophényl)-1,4-diméthyl-N-tétrahydro-pyrane-4-yl-5-(trifluorométhyl)-1H-pyrrole-2-carboxylique |
| 002 | Amide d'acide 3-(4-chlorophényl)-N-(2,2-diméthyl-propyl)-N,1,4-triméthyl-5-(trifluorométhyl)-1H-pyrrole-2-carboxylique |
| 003 | Amide d'acide N-benzyl-3-(4-chlorophényl)-N-(2,2-diméthyl-propyl)-1,4-diméthyl-5-(trifluorométhyl)-1H-pyrrole-2-carboxylique |
| 004 | [3-(4-chlorophényl)-1,4-diméthyl-5-(trifluorométhyl)-1H-pyrrole-2-yl)-morpholine-4-yl-méthanone |
| 005 | Amide d'acide 3-(4-chlorophényl)-N-(2,2-diméthyl-propyl)-1-éthyl-N,4-diméthyl-5-(trifluorométhyl)-1H-pyrrole-2-carboxylique |
| 006 | [3-(4-chlorophényl)-1-isopropyl-4-méthyl-5-(trifluorométhyl)-1H-pyrrole-2-yl]-(2,2-diméthyl-morpholine-4-yl)-méthanone |
| 007 | Amide d'acide N-(1-carbamoyl-cyclopropyl)-3-(4-chlorophényl)-1-cyclopropyl-N,4-diméthyl-5-(trifluorométhyl)-1H-pyrrole-2-carboxylique |
| 008 | Amide d'acide 3-(4-chlorophényl)-1-cyclopropyl-N-(1,1-dioxo-thiane-4-yl)-N,4-diméthyl-5-(trifluoro-méthyl)-1H-pyrrole-2-carboxylique |
| 009 | Amide d'acide N-(2-carbamoyl-2-méthyl-propyl)-3-(4-chlorophényl)-1-cyclopropyl-N,4-diméthyl-5-(tri-fluorométhyl)-1H-pyrrole-2-carboxylique |
| 010 | Amide d'acide 3-(4-chlorophényl)-1-cyclopropyl-N-[(4-méthoxyphényl)-méthyl]-N,4-diméthyl-5-(trifluoro-méthyl)-1H-pyrrole-2-carboxylique |
| 011 | Amide d'acide 3-(4-chlorophényl)-1-cyclopropyl-N-[(4-méthoxyphényl)-méthyl]-N-méthyl-5-(trifluorométhyl)-1H-pyrrole-2-carboxylique |
| 012 | Amide d'acide 3-(4-chlorophényl)-N,1-dicyclopropyl-N,4-diméthyl-5-(trifluorométhyl)-1H-pyrrole-2-carboxylique |
| 013 | Amide d'acide 3-(4-chlorophényl)-N,1-dicyclopropyl-N-méthyl-5-(trifluorométhyl)-1H-pyrrole-2-carboxylique |
| 014 | Amide d'acide 3-(4-chlorophényl)-1-cyclopropyl-N,4-diméthyl-N-tétrahydro-pyrane-4-yl-5-(trifluoro-méthyl)-1H-pyrrole-2-carboxylique |
| 015 | Amide d'acide 3-(4-chlorophényl)-1-cyclopropyl-N-méthyl-N-tétrahydro-pyrane-4-yl-5-(trifluorométhyl)-1H-pyrrole-2-carboxylique |
| 016 | Amide d'acide 3-(4-chlorophényl)-1-cyclopropyl-N-(2,2-diméthyl-3-morpholine-4-yl-propyl)-N,4-diméthyl-5-(trifluorométhyl)-1H-pyrrole-2-carboxylique |
| 017 | Amide d'acide 3-(4-chlorophényl)-1-cyclopropyl-N-(2,2-diméthyl-3-morpholine-4-yl-propyl)-N-méthyl-5-(trifluorométhyl)-1H-pyrrole-2-carboxylique |
| 018 | Amide d'acide 3-(4-chlorophényl)-1-cyclopropyl-N-(2-hydroxy-2-méthyl-propyl)-N,4-diméthyl-5-(trifluoro-méthyl)-1H-pyrrole-2-carboxylique |
| 019 | Amide d'acide 3-(4-chlorophényl)-1-cyclopropyl-N-(2-hydroxy-2-méthyl-propyl)-N-méthyl-5-(trifluoro-méthyl)-1H-pyrrole-2-carboxylique |
| 020 | Amide d'acide 3-(4-chloro-2-fluoro-phényl)-1-cyclo-propyl-N,4-diméthyl-N-[(5-méthyl-isoxazole-3-yl)-méthyl]-5-(trifluorométhyl)-1H-pyrrole-2-carboxylique |
| 021 | Amide d'acide 3-(4-chloro-2-fluoro-phényl)-1-cyclo-propyl-N,4-diméthyl-N-(pyrimidine-4-yl-méthyl)-5-(trifluorométhyl)-1H-pyrrole-2-carboxylique |
| 022 | Amide d'acide 3-(4-chlorophényl)-1-cyclopropyl-N,4-diméthyl-N-(tétrahydro-furane-3-yl-méthyl)-5-(tri-fluorométhyl)-1H-pyrrole-2-carboxylique |
| 023 | Amide d'acide 3-(4-chlorophényl)-1-cyclopropyl-N-méthyl-N-(tétrahydro-furane-3-yl-méthyl)-5-(tri-fluorométhyl)-1H-pyrrole-2-carboxylique |
| 024 | Amide d'acide 3-(4-chloro-2-fluoro-phényl)-1-cyclo-propyl-N,4-diméthyl-N-[(5-méthyl-pyrazine-2-yl)-méthyl]-5-(trifluorométhyl)-1H-pyrrole-2-carboxylique |
| 025 | [3-(4-chlorophényl)-1-Cyclopropyl-4-méthyl-5-(tri-fluorométhyl)-1H-pyrrole-2-yl]-(3-hydroxy-azétidine-1-yl)-méthanone |
| 026 | [3-(4-chlorophényl)-1-cyclopropyl-5-(trifluoro-méthyl)-1H-pyrrole-2-yl]-(3-hydroxy-azétidine-1-yl)-méthanone |
| 027 | [3-(4-chlorophényl)-1-cyclopropyl-4-méthyl-5-(trifluorométhyl)-1H-pyrrole-2-yl]-[4-(3-méthyl-[1,2,4]oxadiazole-5-yl)-pipéridine-1-yl]-méthanone |
| 028 | [3-(4-chloro-2-fluoro-phényl)-1-cyclopropyl-4-méthyl-5-(trifluorométhyl)-1H-pyrrole-2-yl]-[4-(3-méthyl-[1,2,4]oxadiazole-5-yl)-pipéridine-1-yl]-méthanone |
| 029 | [3-(4-chlorophényl)-1-cyclopropyl-5-(trifluoro-méthyl)-1H-pyrrole-2-yl]-[4-(3-méthyl-[1,2,4]-oxadiazole-5-yl)-pipéridine-1-yl]-méthanone |
| 030 | [3-(4-chlorophényl)-1-cyclopropyl-4-méthyl-5-(tri-fluorométhyl)-1H-pyrrole-2-yl]-[4-[5-(trifluoro-méthyl)-pyridine-2-yl]oxy-pipéridine-1-yl]-méthanone |
| 031 | [3-(4-chlorophényl)-1-cyclopropyl-5-(trifluoro-méthyl)-1H-pyrrole-2-yl]-[4-[5-(trifluorométhyl)-pyridine-2-yl]oxy-pipéridine-1-yl]-méthanone |
| 032 | [3-(4-chlorophényl)-1-cyclopropyl-4-méthyl-5-(tri-fluorométhyl)-1H-pyrrole-2-yl]-(2,2-diméthyl-morpholine-4-yl)-méthanone |
| 033 | [3-(4-chloro-2-fluoro-phényl)-1-cyclopropyl-4-méthyl-5-(trifluorométhyl)-1H-pyrrole-2-yl]-(2,2-diméthyl-morpholine-4-yl)-méthanone |
| 034 | [3-(4-chlorophényl)-1-cyclopropyl-5-(trifluoro-méthyl)-1H-pyrrole-2-yl]-(2,2-diméthyl-morpholine-4-yl)-méthanone |
| 035 | Amide d'acide 3-(4-chlorophényl)-1-cyclopropyl-N-(2,2-diméthyl-propyl)-N-méthyl-5-(trifluorométhyl)-1H-pyrrole-2-carboxylique |
| 036 | Amide d'acide 3-(4-chlorophényl)-1-cyclopropyl-N-(3-hydroxy-2,2-diméthyl-propyl)-N-méthyl-5-(trifluoro-méthyl)-1H-pyrrole-2-carboxylique |
| 037 | Amide d'acide 3-(4-chlorophényl)-1-cyclopropyl-N-méthyl-N-(2-méthylsulfonyl-éthyl)-5-(trifluoro-méthyl)-1H-pyrrole-2-carboxylique |
| 038 | [3-(4-chlorophényl)-1-cyclopropyl-5-(trifluoro-méthyl)-1H-pyrrole-2-yl]-morpholine-4-yl-méthanone |
| 039 | Amide d'acide 3-(4-chlorophényl)-1-cyclopropyl-N-(2,2-diméthyl-propyl)-N,4-diméthyl-5-(trifluoro-méthyl)-1H-pyrrole-2-carboxylique |
| 040 | Amide d'acide 3-(4-chlorophényl)-1-cyclopropyl-N-(3-hydroxy-2,2-diméthyl-propyl)-N,4-diméthyl-5-(tri-fluorométhyl)-1H-pyrrole-2-carboxylique |
| 041 | Amide d'acide 3-(4-chlorophényl)-1-cyclopropyl-N,4-diméthyl-N-(2-méthylsulfonyl-éthyl)-5-(trifluoro-méthyl)-1H-pyrrole-2-carboxylique |
| 042 | [3-(4-chlorophényl)-1-cyclobutyl-4-méthyl-5-(trifluorométhyl)-1H-pyrrole-2-yl]-(2,2-diméthyl-morpholine-4-yl)-méthanone |
| 043 | [3-(4-chlorophényl)-1-cyclopropyl-4-méthyl-5-(trifluorométhyl)-1H-pyrrole-2-yl]-morpholine-4-yl-méthanone |
| 044 | [3-(4-chlorophényl)-1-cyclopropyl-4-méthyl-5-(trifluorométhyl)-1H-pyrrole-2-yl]-(2,2-diméthyl-1,1-dioxo-[1,4]thiazinane-4-yl)-méthanone |
| 045 | (2,2-diméthyl-morpholine-4-yl)-[3-(4-fluorophényl)-1-(2-méthyl-propyl)-5-(trifluorométhyl)-1H-pyrrole-2-yl]-méthanone |
| 046 | Amide d'acide 3-(4-fluorophényl)-N-méthyl-1-(2-méthyl-propyl)-N-tétrahydro-pyrane-4-yl-5-(tri-fluorométhyl)-1H-pyrrole-2-carboxylique |
| 047 | Amide d'acide 3-(4-fluorophényl)-N-méthyl-N-[(5-méthyl-isoxazole-3-yl)-méthyl]-1-(2-méthyl-propyl)-5-(trifluorométhyl)-1H-pyrrole-2-carboxylique |
| 048 | Amide d'acide 3-(4-chlorophényl)-N,4-diméthyl-1-(2-méthyl-propyl)-N-(pyrimidine-4-yl-méthyl)-5-(tri-fluorométhyl)-1H-pyrrole-2-carboxylique |
| 049 | Amide d'acide 3-(4-fluorophényl)-N-méthyl-1-(2-méthyl-propyl)-N-[(5-méthyl-pyrazine-2-yl)-méthyl]-5-(trifluorométhyl)-1H-pyrrole-2-carboxylique |
| 050 | Amide d'acide 3-(4-fluorophényl)-N-méthyl-1-(2-méthyl-propyl)-N-(2-méthylsulfonyl-éthyl)-5-(tri-fluorométhyl)-1H-pyrrole-2-carboxylique |
| 051 | Amide d'acide N-(1-carbamoyl-cyclopropyl)-3-(4-chlorophényl)-N,4-diméthyl-1-(2-méthyl-propyl)-5-(trifluorométhyl)-1H-pyrrole-2-carboxylique |
| 052 | Amide d'acide N-(2-carbamoyl-2-méthyl-propyl)-3-(4-chlorophényl)-N,4-diméthyl-1-(2-méthyl-propyl)-5-(trifluorométhyl)-1H-pyrrole-2-carboxylique |
| 053 | Amide d'acide N-(2,2-diméthyl-propyl)-3-(4-fluoro-phényl)-N-méthyl-1-(2-méthyl-propyl)-5-(trifluoro-méthyl)-1H-pyrrole-2-carboxylique |
| 054 | Amide d'acide N-(2,2-diméthyl-3-morpholine-4-yl-propyl)-3-(4-fluorophényl)-N-méthyl-1-(2-méthyl-propyl)-5-(trifluorométhyl)-1H-pyrrole-2-carboxylique |
| 055 | Amide d'acide 3-(4-chlorophényl)-N-(3-hydroxy-2,2-diméthyl-propyl)-N,4-diméthyl-1-(2-méthyl-propyl)-5-(trifluorométhyl)-1H-pyrrole-2-carboxylique |
| 056 | Amide d'acide 3-(4-fluorophényl)-N-(3-hydroxy-2,2-diméthyl-propyl)-N-méthyl-1-(2-méthyl-propyl)-5-(trifluorométhyl)-1H-pyrrole-2-carboxylique |
| 057 | Amide d'acide 3-(4-chlorophényl)-N,4-diméthyl-N-[(5-méthyl-isoxazole-3-yl)-méthyl]-1-(2-méthyl-propyl)-5-(trifluorométhyl)-1H-pyrrole-2-carboxylique |
| 058 | Amide d'acide 3-(4-fluorophényl)-N-méthyl-1-(2-méthyl-propyl)-N-(tétrahydro-furane-3-yl-méthyl)-5-(trifluorométhyl)-1H-pyrrole-2-carboxylique |
| 059 | Amide d'acide 3-(4-chlorophényl)-N,4-diméthyl-1-(2-méthyl-propyl)-N-[(5-méthyl-pyrazine-2-yl)-méthyl]-5-(trifluorométhyl)-1H-pyrrole-2-carboxylique |
| 060 | Amide d'acide N-[(2-diméthylamino-pyrimidine-5-yl)-méthyl]-3-(4-fluorophényl)-N-méthyl-1-(2-méthyl-propyl)-5-(trifluorométhyl)-1H-pyrrole-2-carboxylique |
| 061 | Amide d'acide 3-(4-chlorophényl)-N,4-diméthyl-1-(2-méthyl-propyl)-N-(2-méthylsulfonyl-éthyl)-5-(tri-fluorométhyl)-1H-pyrrole-2-carboxylique |
| 062 | [3-(4-chlorophényl)-4-méthyl-1-(2-méthyl-propyl)-5-(trifluorométhyl)-1H-pyrrole-2-yl]-[4-(3-méthyl-[1,2,4]oxadiazole-5-yl)-pipéridine-1-yl]-méthanone |
| 063 | 4-[3-(4-fluorophényl)-1-(2-méthyl-propyl)-5-(tri-fluorométhyl)-1H-pyrrole-2-carbonyl]-pipérazine-2-one |
| 064 | [3-(4-chlorophényl)-4-méthyl-1-(2-méthyl-propyl)-5-(trifluorométhyl)-1H-pyrrole-2-yl]-(2,2-diméthyl-morpholine-4-yl)-méthanone |
| 065 | [3-(4-fluorophényl)-1-(2-méthyl-propyl)-5-(trifluoro-méthyl)-1H-pyrrole-2-yl]-morpholine-4-yl-méthanone |
| 066 | Amide d'acide N-cyclopropyl-3-(4-fluorophényl)-1-(2-méthyl-propyl)-5-(trifluorométhyl)-1H-pyrrole-2-carboxylique |
| 067 | Amide d'acide 3-(4-chlorophényl)-N-(2-cyano-2-méthyl-propyl)-N,4-diméthyl-1-(2-méthyl-propyl)-5-(tri-fluorométhyl)-1H-pyrrole-2-carboxylique |
| 068 | Amide d'acide 3-(4-chlorophényl)-N,4-diméthyl-1-[(1-méthyl-cyclopropyl)-méthyl]-N-(2-méthylsulfonyl-éthyl)-5-(trifluorométhyl)-1H-pyrrole-2-carboxylique |
| 069 | Amide d'acide N-[(2-diméthylamino-pyrimidine-5-yl)-méthyl]-3-(4-fluorophényl)-N-méthyl-1-(tétrahydro-furane-2-yl-méthyl)-5-(trifluorométhyl)-1H-pyrrole-2-carboxylique |
| 070 | Amide d'acide 3-(4-fluorophényl)-N-méthyl-1-(tétra-hydro-furane-2-yl-méthyl)-N-tétrahydro-pyrane-4-yl-5-(trifluorométhyl)-1H-pyrrole-2-carboxylique |
| 071 | Amide d'acide N-(1,1-dioxo-thiolane-3-yl)-3-(4-fluorophényl)-N-méthyl-1-(tétrahydro-furane-2-yl-méthyl)-5-(trifluorométhyl)-1H-pyrrole-2-carboxylique |
| 072 | Amide d'acide N-(2-carbamoyl-2-méthyl-propyl)-3-(4-fluorophényl)-N-méthyl-1-(tétrahydro-furane-2-yl-méthyl)-5-(trifluorométhyl)-1H-pyrrole-2-carboxylique |
| 073 | Amide d'acide N-(2,2-diméthyl-propyl)-3-(4-fluoro-phényl)-N-méthyl-1-(tétrahydro-furane-2-yl-méthyl)-5-(trifluorométhyl)-1H-pyrrole-2-carboxylique |
| 074 | Amide d'acide 3-(4-fluorophényl)-N-(3-hydroxy-2,2-diméthyl-propyl)-N-méthyl-1-(tétrahydro-furane-2-yl-méthyl)-5-(trifluorométhyl)-1H-pyrrole-2-carboxylique |
| 075 | Amide d'acide 3-(4-fluorophényl)-N-méthyl-N-[(5-méthyl-isoxazole-3-yl)-méthyl]-1-(tétrahydro-furane-2-yl-méthyl)-5-(trifluorométhyl)-1H-pyrrole-2-carboxylique |
| 076 | Amide d'acide 3-(4-fluorophényl)-N-méthyl-N,1-bis(tétrahydro-furane-2-yl-méthyl)-5-(trifluoro-méthyl)-1H-pyrrole-2-carboxylique |
| 077 | Amide d'acide 3-(4-fluorophényl)-N-méthyl-N-[(5-méthyl-pyrazine-2-yl)-méthyl]-1-(tétrahydro-furane-2-yl-méthyl)-5-(trifluorométhyl)-1H-pyrrole-2-carboxylique |
| 078 | Amide d'acide 3-(4-fluorophényl)-N-méthyl-N-(2-méthylsulfonyl-éthyl)-1-(tétrahydro-furane-2-yl-méthyl)-5-(trifluorométhyl)-1H-pyrrole-2-carboxylique |
| 079 | 4-[3-(4-fluorophényl)-1-(tétrahydro-furane-2-yl-méthyl)-5-(trifluorométhyl)-1H-pyrrole-2-carbonyl]-pipérazine-2-one |
| 080 | (2,2-diméthyl-morpholine-4-yl)-[3-(4-fluorophényl)-1-(tétrahydro-furane-2-yl-méthyl)-5-(trifluorométhyl)-1H-pyrrole-2-yl]-méthanone |
| 081 | [3-(4-fluorophényl)-1-(tétrahydro-furane-2-yl-méthyl)-5-(trifluorométhyl)-1H-pyrrole-2-yl]-morpholine-4-yl-méthanone |
| 082 | Amide d'acide N-cyclopropyl-3-(4-fluorophényl)-1-(tétrahydro-furane-2-yl-méthyl)-5-(trifluorométhyl)-1H-pyrrole-2-carboxylique |
| 083 | Amide d'acide 3-(4-chlorophényl)-N-(2,2-diméthyl-propyl)-N-méthyl-1-(tétrahydro-furane-2-yl-méthyl)-5-(trifluorométhyl)-1H-pyrrole-2-carboxylique |
| 084 | [3-(4-chlorophényl)-1-(cyclopropyl-méthyl)-4-méthyl-5-(trifluorométhyl)-1H-pyrrole-2-yl]-(2,2-diméthyl-morpholine-4-yl)-méthanone |
| 085 | [3-(4-chlorophényl)-1-(cyclobutyl-méthyl)-4-méthyl-5-(trifluorométhyl)-1H-pyrrole-2-yl]-(2,2-diméthyl-morpholine-4-yl)-méthanone |
| 086 | [3-(4-chlorophényl)-1-(3-cyclopropyl-prop-2-ynyl)-4-méthyl-5-(trifluorométhyl)-1H-pyrrole-2-yl]-morpholine-4-yl-méthanone |
| 087 | [3-(4-chlorophényl)-4-méthyl-1-[(1-méthyl-cyclo-propyl)-méthyl]-5-(trifluorométhyl)-1H-pyrrole-2-yl]-morpholine-4-yl-méthanone |
| 088 | Amide d'acide 1-[(1-amino-cyclopropyl)-méthyl]-3-(4-chlorophényl)-N-(2,2-diméthyl-propyl)-N-méthyl-5-(trifluorométhyl)-1H-pyrrole-2-carboxylique |
| 089 | [3-(4-chlorophényl)-1-[(1-hydroxy-cyclopropyl)-méthyl]-4-méthyl-5-(trifluorométhyl)-1H-pyrrole-2-yl]-morpholine-4-yl-méthanone |
| 090 | Amide d'acide 3-(4-chlorophényl)-N-(2,2-diméthyl-propyl)-1-[(1-hydroxy-cÿclopentyl)-méthyl]-N-méthyl-5-(trifluorométhyl)-1H-pyrrole-2-carboxylique |
| 091 | Amide d'acide 3-(4-chlorophényl)-N-(2,2-diméthyl-propyl)-N-méthyl-1-(pyrrolidine-2-yl-méthyl)-5-(trifluorométhyl)-1H-pyrrole-2-carboxylique |
| 092 | Amide d'acide 3-(4-chlorophényl)-N-(2,2-diméthyl-propyl)-N-méthyl-1-(tétrahydro-pyrane-4-yl-méthyl)-5-(trifluorométhyl)-1H-pyrrole-2-carboxylique |
| 093 | Amide d'acide 1-[(1-amino-cyclopropyl)-méthyl]-3-(4-chlorophényl)-N,4-diméthyl-N-(2-méthylsulfonyl-éthyl)-5-(trifluorométhyl)-1H-pyrrole-2-carboxylique |
| 094 | Amide d'acide 3-(4-chlorophényl)-1-[(1-hydroxy-cyclo-pentyl)-méthyl]-N,4-diméthyl-N-(2-méthylsulfonyl-éthyl)-5-(trifluorométhyl)-1H-pyrrole-2-carboxylique |
| 095 | [1-[(1-amino-cÿclopropyl)-méthyl]-3-(4-chlorophényl)-4-méthyl-5-(trifluorométhyl)-1H-pyrrole-2-yl]-morpholine-4-yl-méthanone |
| 096 | [3-(4-chlorophényl)-1-[(1-hydroxy-cyclopentyl)-méthyl]-4-méthyl-5-(trifluorométhyl)-1H-pyrrole-2-yl]-morpholine-4-yl-méthanone |
| 097 | [3-(4-chlorophényl)-4-méthyl-1-(tétrahydro-pyrane-4-yl-méthyl)-5-(trifluorométhyl)-1H-pyrrole-2-yl]-morpholine-4-yl-méthanone |
| 098 | [3-(4-chlorophényl)-1-[2-(3,3-difluoro-azétidine-1-yl)-éthyl]-4-méthyl-5-(trifluorométhyl)-1H-pyrrole-2-yl]-morpholine-4-yl-méthanone |
| 099 | [3-(4-chlorophényl)-4-méthyl-1-(2-méthylsulfonyl-éthyl)-5-(trifluorométhyl)-1H-pyrrole-2-yl]-morpholine-4-yl-méthanone |
| 100 | Amide d'acide 3-(4-chlorophényl)-1-[(1-cyano-cyclo-propyl)-méthyl]-N-(2,2-diméthyl-propyl)-N-méthyl-5-(trifluorométhyl)-1H-pyrrole-2-carboxylique |
| 101 | Amide d'acide 3-(4-chlorophényl)-N-(2,2-diméthyl-propyl)-1-[(1-hydroxy-cyclobutyl)-méthyl]-N-méthyl-5-(trifluorométhyl)-1H-pyrrole-2-carboxylique |
| 102 | [3-(4-chlorophényl)-1,4-diméthyl-5-(trifluorométhyl)-1H-pyrrole-2-yl]-(2,2-diméthyl-morpholine-4-yl)-méthanone |
| 103 | [3-(4-chlorophényl)-1-éthyl-4-méthyl-5-(trifluoro-méthyl)-1H-pyrrole-2-yl]-(2,2-diméthyl-morpholine-4-yl)-méthanone |
| 104 | [3-(4-chlorophényl)-4-méthyl-1-(2-méthyl-propyl)-5-(trifluorométhyl)-1H-pyrrole-2-yl]-(2-oxa-5-azabicyclo[2.2.1]heptane-5-yl)-méthanone |
| 105 | Amide d'acide N-(2-carbamoyl-2-méthyl-propyl)-3-(4-chlorophényl)-1-isopropyl-N,4-diméthyl-5-(trifluoro-méthyl)-1H-pyrrole-2-carboxylique |
| 106 | Amide d'acide 3-(4-chlorophényl)-N-(2-cyano-2-méthyl-propyl)-1-isopropyl-N,4-diméthyl-5-(trifluorométhyl)-1H-pyrrole-2-carboxylique |
| 107 | Amide d'acide 3-(4-chlorophényl)-1-isopropyl-N,4-diméthyl-N-[(5-méthyl-isoxazole-3-yl)-méthyl]-5-(trifluorométhyl)-1H-pyrrole-2-carboxylique |
| 108 | Amide d'acide 3-(4-chlorophényl)-N,1-diisopropyl-N,4-diméthyl-5-(trifluorométhyl)-1H-pyrrole-2-carboxylique |
| 109 | Amide d'acide 3-(4-chlorophényl)-N-isopropyl-N,4-diméthyl-1-(2-méthyl-propyl)-5-(trifluorométhyl)-1H-pyrrole-2-carboxylique |
| 110 | Amide d'acide 3-(4-chlorophényl)-1-isopropyl-N,4-diméthyl-N-(2-méthylsulfonyl-éthyl)-5-(trifluoro-méthyl)-1H-pyrrole-2-carboxylique |
| 111 | Amide d'acide 3-(4-chlorophényl)-1-isopropyl-N,4-diméthyl-N-[(2-méthyl-2H-pyrazole-3-yl)-méthyl]-5-(trifluorométhyl)-1H-pyrrole-2-carboxylique |
| 112 | [3-(5-chloro-pyridine-2-yl)-1-isopropyl-4-méthyl-5-(trifluorométhyl)-1H-pyrrole-2-yl]-(2,2-diméthyl-morpholine-4-yl)-méthanone |
| 113 | Amide d'acide 3-(5-chloro-pyridine-2-yl)-1-isopropyl-N,4-diméthyl-N-[(5-méthyl-isoxazole-3-yl)-méthyl]-5-(trifluorométhyl)-1H-pyrrole-2-carboxylique |
| 114 | Amide d'acide 3-(5-chloro-pyridine-2-yl)-1-isopropyl-N,4-diméthyl-N-[(2-méthyl-2H-pyrazole-3-yl)-méthyl]-5-(trifluorométhyl)-1H-pyrrole-2-carboxylique |
| 115 | Amide d'acide 3-(4-chlorophényl)-1-isopropyl-N,4-diméthyl-N-(2-oxo-pyrrolidine-3-yl)-5-(trifluoro-méthyl)-1H-pyrrole-2-carboxylique |
| 116 | Amide d'acide N-(2-carbamoyl-2-méthyl-propyl)-3-(5-chloro-pyridine-2-yl)-1-isopropyl-N,4-diméthyl-5-(trifluorométhyl)-1H-pyrrole-2-carboxylique |
| 117 | Amide d'acide 3-(5-chloro-pyridine-2-yl)-N-(2-cyano-2-méthyl-propyl)-1-isopropyl-N,4-diméthyl-5-(tri-fluorométhyl)-1H-pyrrole-2-carboxylique |
| 118 | Amide d'acide 3-(5-chloro-pyridine-2-yl)-1-isopropyl-N,4-diméthyl-N-(2-méthylsulfonyl-éthyl)-5-(trifluoro-méthyl)-1H-pyrrole-2-carboxylique |
| 119 | Amide d'acide 3-(5-chloro-pyridine-2-yl)-1-isopropyl-N,4-diméthyl-N-(2-oxo-pyrrolidine-3-yl)-5-(trifluoro-méthyl)-1H-pyrrole-2-carboxylique |
| 120 | Amide d'acide 3-(5-chloro-pyridine-2-yl)-N,1-diiso-propyl-N,4-diméthyl-5-(trifluorométhyl)-1H-pyrrole-2-carboxylique |
| 121 | [3-(5-chloro-pyrimidine-2-yl)-1-isopropyl-4-méthyl-5-(trifluorométhyl)-1H-pyrrole-2-yl]-(2,2-diméthyl-morpholine-4-yl)-méthanone |
| 122 | Amide d'acide 3-(5-chloro-pyrimidine-2-yl)-1-iso-propyl-N,4-diméthyl-N-[(2-méthyl-2H-pyrazole-3-yl)-méthyl]-5-(trifluorométhyl)-1H-pyrrole-2-carboxylique |
| 123 | Amide d'acide 3-(5-chloro-pyrimidine-2-yl)-1-isopropyl-N,4-diméthyl-N-[(5-méthyl-isoxazole-3-yl)-méthyl]-5-(trifluorométhyl)-1H-pyrrole-2-carboxylique |
| 124 | Amide d'acide 3-(5-chloro-pyrimidine-2-yl)-1-isopropyl-N,4-diméthyl-N-(2-oxo-pyrrolidine-3-yl)-5-(trifluorométhyl)-1H-pyrrole-2-carboxylique |
| 125 | Amide d'acide N-(2-carbamoyl-2-méthyl-propyl)-3-(5-chloro-pyrimidine-2-yl)-1-isopropyl-N,4-diméthyl-5-(trifluorométhyl)-1H-pyrrole-2-carboxylique |
| 126 | Amide d'acide 3-(5-chloro-pyrimidine-2-yl)-N-(2-cyano-2-méthyl-propyl)-1-isopropyl-N,4-diméthyl-5-(trifluorométhyl)-1H-pyrrole-2-carboxylique |
| 127 | Amide d'acide 3-(5-chloro-pyrimidine-2-yl)-1-isopropyl-N,4-diméthyl-N-(2-méthylsulfonyl-éthyl)-5-(trifluorométhyl)-1H-pyrrole-2-carboxylique |
| 128 | Amide d'acide 3-(5-chloro-pyrimidine-2-yl)-N,1-diisopropyl-N,4-diméthyl-5-(trifluorométhyl)-1H-pyrrole-2-carboxylique |
| 129 | Amide d'acide N-tert.-butyl-4-[3-(5-chloro-pyridine-2-yl)-1-isopropyl-4-méthyl-5-(trifluorométhyl)-1H-pyrrole-2-carbonyl]-pipérazine-1-carboxylique |
| 130 | [3-(5-chloro-pyridine-2-yl)-1-isopropyl-4-méthyl-5-(trifluorométhyl)-1H-pyrrole-2-yl]-(2,2-diméthyl-1-oxo-[1,4]thiazinane-4-yl)-méthanone |
| 131 | Amide d'acide N-tert.-butyl-4-[3-(4-chlorophényl)-1-isopropyl-4-méthyl-5-(trifluorométhyl)-1H-pyrrole-2-carbonyl]-pipérazine-1-carboxylique |
| 132 | Amide d'acide 3-(4-chlorophényl)-N-[1-(hydroxy-méthyl)-3-méthyl-butyl]-1-isopropyl-N,4-diméthyl-5-(trifluorométhyl)-1H-pyrrole-2-carboxylique |
| 133 | Amide d'acide N-[1-(tert.-butyl-carbamoyl)-éthyl]-3-(4-chlorophényl)-1-isopropyl-N,4-diméthyl-5-(tri-fluorométhyl)-1H-pyrrole-2-carboxylique |
| 134 | Amide d'acide 3-(5-chloro-pyridine-2-yl)-N-(3,3-diméthyl-pipéridine-4-yl)-1-isopropyl-N,4-diméthyl-5-(trifluorométhyl)-1H-pyrrole-2-carboxylique |
| 135 | Amide d'acide 3-(4-chlorophényl)-N-(3,3-diméthyl-pipéridine-4-yl)-1-isopropyl-N,4-diméthyl-5-(tri-fluorométhyl)-1H-pyrrole-2-carboxylique |
éventuellement sous la forme d'un stéréoisomère individuel ou d'un mélange de stéréoisomères, sous la forme du composé libre et/ou de l'un de ses sels ou produits de solvatation physiologiquement acceptables.

13. Composition pharmaceutique comprenant au moins un composé selon une ou plusieurs des revendications 1 à 12.

14. Au moins un composé selon une ou plusieurs des revendications 1 à 12 destiné à être utilisé dans le traitement et/ou la prophylaxie d'un ou plusieurs troubles choisis dans le groupe consistant en la douleur, de préférence la douleur choisie dans le groupe consistant en la douleur aiguë, la douleur chronique, la douleur viscérale, la douleur due à une céphalée, la douleur inflammatoire et la douleur mixte ; l'accident vasculaire cérébral ; les troubles de l'humeur ; l'épilepsie ; la schizophrénie, et les troubles neurodégénératifs.

15. Au moins un composé selon une ou plusieurs des revendications 1 à 12 destiné à être utilisé dans le traitement et/ou la prophylaxie de la douleur, en particulier la douleur aiguë et/ou la douleur chronique et/ou la douleur viscérale et/ou la douleur due à une céphalée et/ou la douleur inflammatoire et/ou la douleur mixte.
